# EUROPÄISCHE PATENTSCHRIFT

(11) **EP 1 874 733 B1**
(45) Veröffentlichungstag und Bekanntmachung des Hinweises auf die Patenterteilung: **20.06.2012**
(21) Anmeldenummer: 06742597.5
(22) Anmeldetag: 18.04.2006
(51) Int. Cl.: C07D 233/36, C07D 233/42, A61K 31/4166, C07D 235/26, C07D 235/28, A61K 31/4184, C07D 239/10, A61K 31/513

(54) **SUBSTITUIERTE ZYKLISCHE HARNSTOFF-DERIVATE UND DEREN VERWENDUNG ALS VANILLOID-REZEPTOR 1 MODULATOREN**
SUBSTITUTED CYCLIC UREA DERIVATIVES AND THE USE THEREOF AS VANILLOID RECEPTOR 1 MODULATORS
DERIVES D'UREE CYCLIQUES SUBSTITUES ET LEUR UTILISATION COMME MODULATEURS DU RECEPTEUR VANILLOIDE 1

(30) Priorität: 19.04.2005 DE 102005018191
(43) Veröffentlichungstag der Anmeldung: 09.01.2008
(73) Patentinhaber: Grünenthal GmbH, 52078 Aachen (DE)
(72) Erfinder: FRANK, Robert, 52070 Aachen (DE); SUNDERMANN, Bernd, 52074 Aachen (DE); SCHICK, Hans, 13086 Berlin (DE); SONNENSCHEIN, Helmut, 10245 Berlin (DE)
(74) Vertreter: Brosch, Oliver
(86) Internationale Anmeldenummer: PCT/EP2006/003519
(87) Internationale Veröffentlichungsnummer: WO 2006/111346

(56) Entgegenhaltungen:
- WO-A-02/16318
- WO-A-2004/103281
- WALPOLE C S J ET AL: "ANALOGUES OF CAPSAICIN WITH AGONIST ACTIVITY AS NOVEL ANALGESIC AGENTS; STRUCTURE-ACTIVITY STUDIES. 2. THE AMIDE BOND B-REGION" JOURNAL OF MEDICINAL CHEMISTRY, AMERICAN CHEMICAL SOCIETY. WASHINGTON, US, Bd. 36, Nr. 16, 1993, Seiten 2373-2380, XP001145824 ISSN: 0022-2623
- LEE ET AL: "Analysis of structure-activity relationships for the 'B-region' of N-(4-t-butylbenzyl)-N'-[4-(methylsulfonyla mino)benzyl]-thiourea analogues as TRPV1 antagonists" BIOORGANIC & MEDICINAL CHEMISTRY LETTERS, OXFORD, GB, Bd. 15, Nr. 18, 15. September 2005 (2005-09-15), Seiten 4143-4150, XP005021114 ISSN: 0960-894X

## Beschreibung

Die vorliegende Erfindung betrifft substituierte zyklische Harnstoff-Derivate, Verfahren zu ihrer Herstellung, Arzneimittel enthaltend diese Verbindungen und die Verwendung dieser Verbindungen zur Herstellung von Arzneimitteln.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Einen geeigneten Ansatzpunkt zur Behandlung von Schmerz; insbesondere von Schmerz ausgewählt aus der Gruppe bestehend aus akutem Schmerz, chronischem Schmerz, neuropathischem Schmerz und visceralem Schmerz, besonders bevorzugt von neurophatischem Schmerz; stellt der Vanilloid-Rezeptor vom Subtyp 1 (VR1/TRPV1) dar, der häufig auch als Capsaicin-Rezeptor bezeichnet wird. Dieser Rezeptor wird u.a. durch Vanilloide wie z.B. Capsaicin, Hitze und Protonen stimuliert und spielt eine zentrale Rolle bei der Schmerzentstehung. Darüber hinaus ist er für eine Vielzahl weiterer physiologischer und pathophysiologischer Prozesse von Bedeutung wie beispielsweise Migräne; Depressionen; neurodegenerativen Erkrankungen; kognitiven Erkrankungen; Angstzuständen; Epilepsie; Husten; Diarrhöe; Pruritus; Störungen des kardiovaskulären Systems; Störungen der Nahrungsaufnahme; Medikamentenabhängigkeit; Medikamentenmißbrauch und insbesondere Harninkontinenz.

WO 02/16318 und WO 2004/103281 lehren 1,3-Dibenzyl (thio)harnstoff-derivate als Modulatoren des Vanilloid-Rezeptors.

Eine Aufgabe der vorliegenden Erfindung bestand daher darin, neue Verbindungen zur Verfügung zu stellen, die sich insbesondere als pharmakologische Wirkstoffe in Arzneimitteln eignen, vorzugsweise in Arzneimitteln zur Behandlung von Störungen oder Krankheiten, die zumindest teilweise durch Vanilloid-Rezeptoren 1 (VR1/TRPV1-Rezeptoren) vermittelt werden.

Überraschenderweise wurde nun gefunden, dass substituierte zyklische Harnstoff-Derivate der nachstehend angegebenen allgemeinen Formel I eine ausgezeichnete Affinität zum Vanilloid-Rezeptor vom Subtyp 1 (VR1/TRPV1-Rezeptor) aufweisen und sich daher insbesondere zur Prophylaxe und/oder Behandlung von Störungen oder Krankheiten eignen, die zumindest teilweise durch Vanilloid-Rezeptoren 1 (VR1/TRPV1) vermittelt werden.

Ein Gegenstand der vorliegenden Erfindung sind daher substituierte zyklische Harnstoff-Derivate der allgemeinen Formel I, 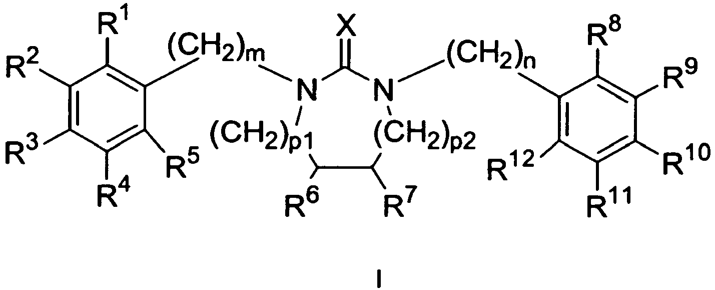 worin
X für O, S oder N-C≡N steht;
m gleich 1 oder 2 ist;
n gleich 1 oder 2 ist;
p1 und p2, unabhängig voneinander, jeweils für 0, 1, 2 oder 3 stehen, wobei die Summe von p1 und p2 0, 1, 2 oder 3 beträgt;
R¹, R², R³, R⁴, R⁵, R⁸, R⁹, R¹⁰, R¹¹ und R¹², unabhängig voneinander, jeweils für
H; F; Cl; Br; I; -SF₅; -NO₂; -NH₂; -OH; -SH; -C(=O)-NH₂; -S(=O)₂-NH₂;
₋NR¹³R¹⁴; -NH-R¹⁵; -OR¹⁶; -SR¹⁷; -O-(CH₂)ₐ-R¹⁸; -O-(CH₂)_{b}-OR¹⁹;
-(CH₂)_{c}-O-(CH₂)_{d}-OR²⁰;
-(CH₂)ₑ-O-C(=O)-R²¹;
-(CH₂)_{f}-O-C(=O)-OR²²;
-NR²³S(=O)₂R²⁴;
-(CH₂)_{g}-C(=O)-NR²⁵R²⁶;
-(CH₂)ₕ-C(=O)-NH-R²⁷;
-S(=O)ᵢR²⁸;
-(CH₂)ⱼ-S(=O)₂-NR²⁹R³⁰;
-(CH₂)ₖ-S(=O)₂-NHR³¹;
-(CH₂)ₗ-NR³²-C(=O)-(CH₂)_{q}-OR³³;
-(CH₂)ᵣ-NH-C(=O)-(CH₂)ₛ-OR³⁴;
-(CH₂)ₜ-NR³⁵-O-C(=O)-OR³⁶;
-(CH₂)ᵤ-NH-O-C(=O)-OR³⁷;
-(CH₂)ᵥ-O-S(=O)₂-R³⁸;
-(CH₂)_{w}-NR³⁹-C(=O)-SR⁴⁰;
-(CH₂)_{y}-C(=O)-NH-OR⁴¹;
-P(=O)-(OR⁴²)2;
-(CH₂)_{z}-C(=S)-NR⁴³R⁴⁴;
-(CH₂)ₐₐ-C(=S)-NH-R⁴⁵;
-(CH₂)_{bb}-NR⁴⁶-C(=O)-R⁴⁷;
-(CH₂)_{cc}-NH-C(=O)-R⁴⁸;
oder -NH-C(=NH)-NH₂ stehen;
wobei
a, b, c, d, q und s, unabhängig voneinander, jeweils gleich 1, 2, 3, 4 oder 5 sind;
e, f, g, h, j, k, l, r, t, u, v, w, x, y, z, aa, bb und cc, unabhängig voneinander, jeweils gleich 0, 1, 2, 3, 4 oder 5 sind, und
i gleich 1 oder 2 ist;
für einen linearen oder verzweigten, gesättigten oder ungesättigten aliphatischen C₁₋₁₀ Rest, der ggf. mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH, -O(C₁₋₅-Alkyl), -S(C₁₋₅-Alkyl), -NH(C₁₋₅-Alkyl), -N(C₁₋₅-Alkyl)(C₁₋₅-Alkyl), -OCF₃ und -SCF₃ substituiert sein kann;
oder für einen ggf. substituierten 6- oder 10-gliedrigen Aryl-Rest, der über eine lineare oder verzweigte, ggf. substituierte C₁₋₅-Alkylen-Gruppe gebunden sein kann, stehen;
oder zwei benachbarte Reste ausgewählt aus der Gruppe bestehend aus R⁸, R⁹, R¹⁰, R¹¹ und R¹² zusammen für eine Methylendioxy(-O-CH₂-O)-Gruppe stehen;
mit der Maßgabe, dass wenigstens einer der Reste R⁸, R⁹, R¹⁰, R¹¹ und R¹² für -NR²³S(=O)₂R²⁴ steht;
R⁶ und R⁷ jeweils für einen Wasserstoff-Rest stehen
oder
R⁶ und R⁷ zusammen mit der sie verbindenden -C-C-Brücke einen unsubstituierten Phenylen-Rest bilden;
R¹³, R¹⁴, R¹⁵, R¹⁶, R¹⁷, R¹⁹, R²⁰, R²¹, R²², R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, R⁴², R⁴³, R⁴⁴, R⁴⁵, R⁴⁶, R⁴⁷ und R⁴⁸, unabhängig voneinander, jeweils
für einen linearen oder verzweigten, gesättigten oder ungesättigten aliphatischen C₁₋₁₀ Rest stehen, der ggf. mit 1, 2, 3, 4, 5, 6, 7, 8 oder 9 Substituenten unabhängig voneinander ausgewählt aus der Gruppe bestehend aus F, Cl, Br, I, -CN, -NO₂. -OH, -NH₂, -SH, -O(C₁₋₅-Alkyl), -S(C₁₋₅-Alkyl), -NH(C₁₋₅-Alkyl), -N(C₁₋₅-Alkyl)(C₁₋₅-Alkyl), -OCF₃ und -SCF₃ substituiert sein kann;
für einen ungesättigten oder gesättigten, ggf. substituierten 3-, 4-, 5-, 6-, 7-, 8- oder 9-gliedrigen cycloaliphatischen Rest stehen
oder für einen ggf. substituierten 5- bis 14-gliedrigen Aryl- oder Heteroaryl-Rest stehen, der mit einem gesättigten oder ungesättigten, ggf. substituierten mono- oder polyzyklischen Ringsystem kondensiert sein kann;
R¹⁸ für einen ungesättigten oder gesättigten, ggf. substituierten 3-, 4-, 5-, 6-, 7-, 8-oder 9-gliedrigen cycloaliphatischen Rest steht
oder für einen ggf. substituierten 5- bis 14-gliedrigen Aryl- oder Heteroaryl-Rest steht, der mit einem gesättigten oder ungesättigten, ggf. substituierten mono- oder polyzyklischen Ringsystem kondensiert sein kann;
R²³ für einen Wasserstoff-Rest steht
oder für einen linearen oder verzweigten, gesättigten oder ungesättigten aliphatischen C₁₋₁₀ Rest steht, der ggf. mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH, -O(C₁₋₅-Alkyl), -S(C₁₋₅-Alkyl), -NH(C₁₋₅-Alkyl), -N(C₁₋₅-Alkyl)(C₁₋₅-Alkyl), -OCF₃ und -SCF₃ substituiert sein kann;
und
R²⁴ für einen linearen oder verzweigten, gesättigten oder ungesättigten aliphatischen C₁₋₁₀ Rest steht, der ggf. mit 1, 2, 3, 4 oder 5 Substituenten unabhängig voneinander ausgewählt aus der Gruppe bestehend aus -CN, - NO₂, -OH, -NH₂, -SH, -O(C₁₋₅-Alkyl), -S(C₁₋₅-Alkyl), -NH(C₁₋₅-Alkyl) und -N(C₁₋₅-Alkyl)(C₁₋₅-Alkyl) substituiert sein kann,
oder für einen ggf. substituierten 5- bis 14-gliedrigen Aryl- oder Heteroaryl-Rest steht, der mit einem gesättigten oder ungesättigten, ggf. substituierten mono- oder polyzyklischen Ringsystem kondensiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Vorzugsweise können die vorstehend genannten cycloaliphatischen Reste ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -NH-C₁₋₅-Alkyl, -N(C₁₋₅-Alkyl)₂, -O-Phenyl, -O-Benzyl, Phenyl und Benzyl substituiert sein, wobei jeweils der zyklische Teil der Reste -O-Phenyl,-O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅,-CN, -NO₂, -C₁₋₅-Alkyl, -O-C₁₋₅-Alkyl, -O-CF₃, -S-CF₃, Phenyl und -O-Benzyl substituiert sein kann, und jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen.

Vorzugsweise kann die vorstehend genannte C₁₋₅-Alkylen-Gruppe ggf. mit 1, 2, 3, 4 oder 5 Substituenten unabhängig voneinander ausgewählt aus der Gruppe bestehend aus F, Cl, Br, -OH, -SH, -NH₂, -CN und NO₂ substituiert sein.

Ebenfalls bevorzugt können die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -NH-C₁₋₅-Alkyl,-N(C₁₋₅-Alkyl)₂, -O-Phenyl, -O-Benzyl, Phenyl und Benzyl substituiert sein, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann.

Vorzugsweise sind die Ringe der vorstehend genannten mono- oder polyzyklischen Ringsysteme jeweils 5-, 6- oder 7-gliedrig und können jeweils ggf. 1, 2, 3, 4 oder 5 Heteroatom(e) als Ringglied(er) aufweisen, die unabhängig voneinander aus der Gruppe bestehend aus Sauerstoff, Stickstoff und Schwefel ausgewählt sind.

Ebenfalls bevorzugt können die vorstehend genannten Aryl- oder Heteroaryl-Reste ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl,-C(=O)-NH₂, -C(=O)-NH-C₁₋₅-Alkyl, -C(=O)-N-(C₁₋₅-Alkyl)₂, -O-Phenyl, -O-Benzyl, Phenyl und Benzyl substituiert sein, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅,-CN, -NO₂, -C₁₋₅-Alkyl, -O-C₁₋₅-Alkyl, -O-CF₃, -S-CF₃, Phenyl und -O-Benzyl substituiert sein kann.

Ebenfalls bevorzugt weisen die vorstehend genannten Heteroaryl-Reste jeweils 1, 2, 3, 4 oder 5 Heteroatom(e) unabhängig voneinander ausgewählt aus der Gruppe bestehend aus Sauerstoff, Stickstoff und Schwefel als Ringglied(er) auf.

Sofern einer oder mehrere der Substituenten R¹ bis R⁵, R⁸ bis R¹⁷, R¹⁹ bis R²³ und R²⁵ bis R⁴⁸ für einen gesättigten oder ungesättigten C₁₋₁₀ aliphatischen Rest, d.h. für einen C₁₋₁₀-Alkyl-, C₂₋₁₀-Alkenyl- oder C₂₋₁₀-Alkinyl-Rest, stehen, kann dieser bevorzugt mit ggf. 1, 2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH,-O(C₁₋₅-Alkyl), -S(C₁₋₅-Alkyl), -NH(C₁₋₅-Alkyl), -N(C₁₋₅-Alkyl)(C₁₋₅-Alkyl), -OCF₃ und-SCF₃ substituiert sein. C₂₋₁₀-Alkenyl-Reste weisen wenigstens eine, vorzugsweise 1, 2, 3 oder 4 C-C-Doppelbindungen und C₂₋₁₀-Alkinyl-Reste wenigstens eine, vorzugsweise 1, 2, 3 oder 4 C-C-Dreifachbindungen auf.

Bevorzugt sind Alkyl-Reste ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, neo-Pentyl und n-Hexyl, die ggf. mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH, -OCH₃, -O-C₂H₅, -SCH₃, -S-C₂H₅, -OCF₃, -SCF₃, -NH-CH₃, -N(CH₃)₂,-N(C₂H₅)₂ und -N(CH₃)(C₂H₅) substituiert sein können.

Ebenfalls bevorzugt sind Alkenyl-Reste ausgewählt aus der Gruppe bestehend aus Vinyl, 1-Propenyl, 2-Propenyl, 1-Butenyl, 2-Butenyl, 3-Butenyl, 2-Methyl-buten-2-yl, 1-Pentenyl, 2-Pentenyl, 3-Pentenyl und 4-Pentenyl, die ggf. mit 1, 2 oder 3 Substituenten unabhängig voneinander ausgewählt aus der Gruppe bestehend aus F, Cl, Br, I, -CN, -NO₂, -OH, -NH₂, -SH, -OCH₃, -O-C₂H₅, -SCH₃, -S-C₂H₅, -OCF₃, - SCF₃, -NH-CH₃, -N(CH₃)₂, -N(C₂H₅)₂ und -N(CH₃)(C₂H₅) substituiert sein können.

Weiterhin bevorzugt sind Alkinyl-Reste ausgewählt aus der Gruppe bestehend aus Ethinyl, 1-Propinyl, 2-Propinyl, 1-Butinyl, 2-Butinyl und 3-Butinyl, die ggf. mit 1, 2 oder 3 Substituenten unabhängig voneinander ausgewählt aus der Gruppe bestehend aus F, Cl, Br, I, -CN, -NO₂, -OH, -NH₂, -SH, -OCH₃, -O-C₂H₅, -SCH₃, -S-C₂H₅, -OCF₃,-SCF₃, -NH-CH₃, -N(CH₃)₂, -N(C₂H₅)₂ und -N(CH₃)(C₂H₅) substituiert sein können.

Besonders bevorzugte ggf. substituierte C₁₋₁₀ aliphatische Rest sind ausgewählt aus der Gruppe bestehend aus Methyl, -CF₃, -CCl₃, -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃, -CH₂-N(CH₃)₂, -CH₂-N(C₂H₅)₂, -CH₂-N(CH₃)(C₂H₅), Ethyl, -CH₂-CH₂-NH₂, -CH₂-CH₂-OH, -CH₂-CH₂-SH,-CH₂-CH₂-NH-CH₃, -CH₂-CH₂-N(CH₃)₂, -CH₂-CH₂-N(C₂H₅)₂, -CH₂-CH₂-N(CH₃)(C₂H₅), -CH₂-CF₃, -C₂F₅, -CH₂-CCl₃, -CH₂-CBr₃, -CH₂-CH₂-CN, n-Propyl, -CH₂-CH₂-CH₂-OH, -CH₂-CH₂-CH₂-SH, -CH₂-CH₂-CH₂-NH₂, -CH₂-CH₂-CH₂-NH-CH₃, -CH₂-CH₂-CH₂-N(CH₃)₂, -CH₂-CH₂-CH₂-N(C₂H₅)₂, -CH₂-CH₂-CH₂-N(CH₃)(C₂H₅), -CH₂-CH₂-O-CH₃,-CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), -CH(CH₃)(S-CH₃), n-Butyl, -CF₂-CF₂-CF₂-CF₃, -CH₂-CH₂-CH₂-CH₂-CN, sec-Butyl, Isobutyl, tert-Butyl, n-Pentyl, sec-Pentyl, neo-Pentyl, n-Hexyl, Vinyl, 1-Propenyl, 2-Propenyl, 1-Butenyl, 2-Butenyl, 3-Butenyl, 2-Methyl-buten-2-yl, (1,1,2)-Trifluor-1-butenyl, 1-Pentenyl, 2-Pentenyl, 3-Pentenyl und 4-Pentenyl.

Sofern der Substituent R²⁴ für einen gesättigten oder ungesättigten C₁₋₁₀ aliphatischen Rest, d.h. für einen C₁₋₁₀-Alkyl-, C₂₋₁₀-Alkenyl- oder C₂₋₁₀-Alkinyl-Rest, steht, kann dieser bevorzugt mit ggf. 1, 2, 3, 4 oder 5 Substituenten unabhängig voneinander ausgewählt aus der Gruppe bestehend aus -CN, -NO₂, -OH, -NH₂, -SH, -O(C₁₋₅-Alkyl), -S(C₁₋₅-Alkyl), -NH(C₁₋₅-Alkyl) und -N(C₁₋₅-Alkyl)(C₁₋₅-Alkyl) substituiert sein. C₂₋₁₀-Alkenyl-Reste weisen wenigstens eine, vorzugsweise 1, 2, 3 oder 4 C-C-Doppelbindungen und C₂₋₁₀-Alkinyl-Reste wenigstens eine, vorzugsweise 1, 2, 3 oder 4 C-C-Dreifachbindungen auf.

Bevorzugt steht R²⁴ für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, neo-Pentyl und n-Hexyl, der ggf. mit 1, 2, 3, 4 oder 5 Substituenten unabhängig voneinander ausgewählt aus der Gruppe bestehend aus -OH, -NH₂,-SH, -NO₂, -O-CH₃, -S-CH₃, -O-C₂H₅, -S-C₂H₅, -NH-CH₃, -N(CH₃)₂ und -CN substituiert sein kann.

Ebenfalls bevorzugt steht R²⁴ für einen Alkenyl-Rest ausgewählt aus der Gruppe bestehend aus Vinyl, 1-Propenyl, 2-Propenyl, 1-Butenyl, 2-Butenyl, 3-Butenyl, 2-Methyl-buten-2-yl, 1-Pentenyl, 2-Pentenyl, 3-Pentenyl und 4-Pentenyl, der ggf. mit 1, 2 oder 3 Substituenten unabhängig voneinander ausgewählt aus der Gruppe bestehend -OH, -NH₂, -SH, -NO₂, -O-CH₃, -S-CH₃, -O-C₂H₅, -S-C₂H₅, -NH-CH₃, - N(CH₃)₂ und -CN substituiert sein kann.

Weiterhin bevorzugt steht R²⁴ für einen Alkinyl-Rest ausgewählt aus der Gruppe bestehend aus Ethinyl, 1-Propinyl, 2-Propinyl, 1-Butinyl, 2-Butinyl und 3-Butinyl, der ggf. mit 1, 2 oder 3 Substituenten unabhängig voneinander ausgewählt aus der Gruppe bestehend aus -OH, -NH₂, -SH, -NO₂, -O-CH₃, -S-CH₃, -O-C₂H₅, -S-C₂H₅,-NH-CH₃, -N(CH₃)₂ und -CN substituiert sein kann.

Besonders bevorzugt steht R²⁴ für einen ggf. substituierten C₁₋₁₀ aliphatischen Rest ausgewählt aus der Gruppe bestehend aus Methyl, -CH₂-CN, -CH₂-O-CH₃, Ethyl,-CH₂-CH₂-CN, n-Propyl, -CH₂-CH₂-O-CH₃, Isopropyl, -CH₂-CH₂-CH₂-CN, -CH₂-O-CH₂-CH₃, -CH(CH₃)(O-CH₃), -CH(CH₃)(S-CH₃), n-Butyl, sec-Butyl, Isobutyl, tert-Butyl, -n-Pentyl, sec-Pentyl, neo-Pentyl, n-Hexyl, Vinyl, 1-Propenyl, 2-Propenyl, 1-Butenyl, 2-Butenyl, 3-Butenyl, 2-Methyl-buten-2-yl, 1-Pentenyl, 2-Pentenyl, 3-Pentenyl und 4-Pentenyl.

Sofern einer oder mehrere der Substituenten R¹³ bis R²² und R²⁵ bis R⁴⁸ für einen (hetero)cycloaliphatischen Rest stehen, kann dieser bevorzugt ausgewählt werden aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl.

Besonders bevorzugt können die (hetero)cycloaliphatischen Reste ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -SCF₃, -SH, -S-CH₃, -S-C₂H₅, -S-C(CH₃)₃, Methyl, Ethyl, n-Propyl, Isopropyl, n-Butyl, sec-Butyl, Isobutyl, tert-Butyl, n-Pentyl, sec-Pentyl, -C(=O)-OH, -C(=O)-O-CH₃, -C(=O)-O-C₂H₅, -N(CH₃)₂, -N(C₂H₅)₂,-N(H)(CH₃), -N(H)(C₂H₅), -O-Phenyl, -O-Benzyl, Phenyl und Benzyl substituiert sein, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, Isobutyl, tert-Butyl,-O-CH₃, -O-C₂H₅, -O-C(CH₃)₃, -O-CF₃, -S-CF₃, Phenyl und -O-Benzyl substituiert sein kann.

Sofern einer oder mehrere der Substituenten R¹ bis R⁵, R⁸ bis R²² und R²⁴ bis R⁴⁸ für einen Aryl-Rest stehen, kann dieser bevorzugt ausgewählt werden aus der Gruppe bestehend aus Phenyl und Naphthyl (1-Naphthyl und 2-Naphthyl).

Sofern einer oder mehrere der Substituenten R¹³ bis R²² und R²⁴ bis R⁴⁸ für einen Heteroaryl-Rest stehen, kann dieser bevorzugt ausgewählt werden aus der Gruppe bestehend aus Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinoxalinyl, Chinolinyl und Isochinolinyl.

Besonders bevorzugt können die Aryl- oder Heteroaryl-Reste ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -O-C(CH₃)₃, -NH₂, -NO₂, -O-CF₃, -S-CF₃, -SH, -S-CH₃, -S-C₂H₅, -S-C(CH₃)₃, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -N(CH₃)₂, -N(C₂H₅)₂, -N(H)(CH₃), -N(H)(C₂H₅), -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-NH₂, -C(=O)-NH-CH₃, -C(=O)-NH-C₂H₅, -C(=O)-N-(CH₃)₂, -C(=O)-N-(C₂H₅)₂, -O-Phenyl, -O-Benzyl, Phenyl und Benzyl substituiert sein, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, Isobutyl, tert-Butyl, -O-CH₃, -O-C₂H₅, -O-C(CH₃)₃, -O-CF₃, -S-CF₃, Phenyl und -O-Benzyl substituiert sein kann.

Unter einem mono- oder polyzyklischen Ringsystem werden im Sinne der vorliegenden Erfindung mono- oder polyzyklische Kohlenwasserstoffreste verstanden, die gesättigt oder ungesättigt sein und ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind. Ein solches mono- bzw. polyzyklisches Ringsystem kann beispielsweise mit einem Aryl-Rest oder einem Heteroaryl-Rest kondensiert (anneliert) sein.

Sofern ein polyzyklisches Ringsystem wie beispielsweise ein bizyklisches Ringsystem vorliegt, können die verschiedenen Ringe, jeweils unabhängig voneinander, einen unterschiedlichen Sättigungsgrad aufweisen, d.h. gesättigt oder ungesättigt sein. Bevorzugt ist ein polyzyklisches Ringsystem ein bizyklisches Ringsystem.

Beispielhaft für Aryl-Reste, die mit einem mono- bzw. polyzyklischen Ringsystem kondensiert sind, seien (1,3)-Benzodioxolyl und (1,4)-Benzodioxanyl genannt. Sofern einer oder mehrere der Substituenten R¹³ bis R²² und R²⁴ bis R⁴⁸ ein mono- oder polyzyklisches Ringsystem aufweisen, kann dieses bevorzugt mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-CH₃, -O-C₂H₅, -O-C(CH₃)₃, -NH₂, -NO₂, -O-CF₃, -SCF₃, -SH, -S-CH₃, -S-C₂H₅, -S-C(CH₃)₃, Methyl, Ethyl, n-Propyl, Isopropyl, n-Butyl, sec-Butyl, Isobutyl, tert-Butyl, n-Pentyl, sec-Pentyl, -C(=O)-OH, -C(=O)-O-CH₃, -C(=O)-O-C₂H₅, -N(CH₃)₂, -N(C₂H₅)₂, -N(H)(CH₃),-N(H)(C₂H₅), -O-Phenyl, -O-Benzyl, Phenyl und Benzyl substituiert sein, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, Isobutyl, tert-Butyl, -O-CH₃, -O-C₂H₅, -O-C(CH₃)₃, -O-CF₃, -S-CF₃, Phenyl und -O-Benzyl substituiert sein kann.

Sofern einer oder mehrere der Substituenten R¹ bis R⁵ und R⁸ bis R¹² eine lineare oder verzweigte C₁₋₅-Alkylen-Gruppe aufweisen, kann diese bevorzugt ausgewählt werden aus der Gruppe bestehend aus -(CH₂)-, -(CH₂)₂-, -C(H)(CH₃)-, -(CH₂)₃-,-(CH₂)₄-, -(CH₂)₅-, -C(H)(C(H)(CH₃)₂)- und -C(C₂H₅)(H)-.

Bevorzugt sind substituierte zyklische Harnstoff-Derivate der vorstehend angegebenen allgemeinen Formel I, worin
R¹, R², R⁴ und R⁵, unabhängig voneinander, jeweils für
H; F; Cl; Br; I; -SF₅; -NO₂; -NH₂; -OH; -SH; -C(=O)-NH₂; -S(=O)₂-NH₂;
-NR¹3R¹⁴; -NH-R¹⁵; -OR¹⁶; -SR¹⁷; -O-(CH₂)ₐ-R¹⁸; -O-(CH₂)_{b}-OR¹⁹;
-(CH₂)_{c}-O-(CH₂)_{d}-OR²⁰_{;}
-(CH₂)ₑ-O-C(=O)-R²¹_{;}
-(CH₂)_{f}-O-C(=O)-OR²²;
-NR²³S(=O)₂R²⁴;
-(CH₂)_{g}-C(=O)-NR²⁵R²⁶;
-(CH₂)ₕ-C(=O)-NH-R²⁷;
-S(=O)ᵢR²⁸_{;}
-(CH₂)ⱼ-S(=O)₂-NR²⁹R³⁰;
-(CH₂)ₖ-S(=O)₂-NHR³¹;
-(CH₂)ₗ-NR³²-C(=O)-(CH₂)_{q}-OR³³;
-(CH₂)ᵣ-NH-C(=O)-(CH₂)ₛ-OR³⁴;
-(CH₂)ₜ-NR³⁵-O-C(=O)-OR³⁶;
-(CH₂)ᵤ-NH-O-C(=O)-OR³⁷;
-(CH₂)ᵥ-O-S(=O)₂-R³⁸;
-(CH₂)_{w}-NR³⁹-C(=O)-SR⁴⁰;
-(CH₂)_{y}-C(=O)-NH-OR⁴¹;
-P(=O)-(OR⁴²)2;
-(CH₂)₂-C(=S)-NR⁴³R⁴⁴;
-(CH₂)ₐₐ-C(=S)-NH-R⁴⁵;
-(CH₂)_{bb}-NR⁴⁶-C(=O)-R⁴⁷;
-(CH₂)_{cc}-NH-C(=O)-R⁴⁸;
oder -NH-C(=NH)-NH₂ stehen;
wobei
a, b, c, d, q und s, unabhängig voneinander, jeweils gleich 1, 2, 3, 4 oder 5 sind,
e, f, g, h, j, k, l, r, t, u, v, w, x, y, z, aa, bb und cc, unabhängig voneinander, jeweils gleich 0, 1, 2, 3, 4 oder 5 sind und
i gleich 1 oder 2 ist;
für einen Rest ausgewählt aus der Gruppe bestehend aus Methyl, -CF₃,-CCl₃, -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃, -CH₂-N(CH₃)₂, -CH₂-N(C₂H₅)₂, -CH₂-N(CH₃)(C₂H₅), Ethyl, -CH₂-CH₂-NH₂, -CH₂-CH₂-OH, -CH₂-CH₂-SH, -CH₂-CH₂-NH-CH₃, -CH₂-CH₂-N(CH₃)₂, -CH₂-CH₂-N(C₂H₅)₂, -CH₂-CH₂-N(CH₃)(C₂H₅), -CH₂-CF₃, -C₂F₅, -CH₂-CCl₃, -CH₂-CBr₃, -CH₂-CH₂-CN, n-Propyl, -CH₂-CH₂-CH₂-OH, -CH₂-CH₂-CH₂-SH, -CH₂-CH₂-CH₂-NH₂, -CH₂-CH₂-CH₂-NH-CH₃, -CH₂-CH₂-CH₂-N(CH₃)₂, -CH₂-CH₂-CH₂-N(C₂H₅)₂, -CH₂-CH₂-CH₂-N(CH₃)(C₂H₅), -CH₂-CH₂-O-CH₃, -CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), -CH(CH₃)(S-CH₃), n-Butyl, -CF₂-CF₂-CF₂-CF₃,-CH₂-CH₂-CH₂-CH₂-CN, sec-Butyl, Isobutyl, tert-Butyl, n-Pentyl, sec-Pentyl, neo-Pentyl, n-Hexyl, Vinyl, 1-Propenyl, 2-Propenyl, 1-Butenyl, 2-Butenyl, 3-Butenyl, 2-Methyl-buten-2-yl, (1,1,2)-Trifluor-1-butenyl, 1-Pentenyl, 2-Pentenyl, 3-Pentenyl und 4-Pentenyl stehen;
oder für einen Anyl-Rest ausgewählt aus der Gruppe bestehend aus Phenyl und Naphthyl stehen, wobei der Aryl-Rest jeweils mit 1, 2, 3, 4 oder 5 Substituenten unabhängig voneinander ausgewählt aus der Gruppe bestehend aus F, Cl, Br, I, -CN, -NO₂, -OH, -SH, Methyl, Ethyl, -CF₃, -O-CF₃, -S-CF₃, -SF₅, -O-CH₃, -O-C₂H₅, -NH₂, -N(CH₃)₂, -N(C₂H₅)₂, -N(H)(CH₃) und-N(H)(C₂H₅) substituiert sein kann;
und
R³ für F; Cl; Br; I; -SF₅; -NO₂; -NH₂; -OH; -SH; -C(=O)-NH₂; -S(=O)₂-NH₂; -NR¹³R¹⁴; -NH-R¹⁵; -OR¹⁶; -SR¹⁷; -O-(CH₂)ₐ-R¹⁸, -O-(CH₂)_{b}-OR¹⁹ oder -NR²³S(=O)₂R²⁴ steht;
wobei
a und b, unabhängig voneinander, jeweils gleich 1, 2, 3, 4 oder 5 sind;
oder für einen Rest ausgewählt aus der Gruppe bestehend aus Methyl, -CF₃, -CCl₃, -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃, -CH₂-N(CH₃)₂, -CH₂-N(C₂H₅)₂, -CH₂-N(CH₃)(C₂H₅), Ethyl, -CH₂-CH₂-NH₂, -CH₂-CH₂-OH, -CH₂-CH₂-SH, -CH₂-CH₂-NH-CH₃, -CH₂-CH₂-N(CH₃)₂, -CH₂-CH₂-N(C₂H₅)₂, -CH₂-CH₂-N(CH₃)(C₂H₅), -CH₂-CF₃, -C₂F₅, -CH₂-CCl₃, -CH₂-CBr₃, -CH₂-CH₂-CN, n-Propyl, -CH₂-CH₂-CH₂-OH, -CH₂-CH₂-CH₂-SH, -CH₂-CH₂-CH₂-NH₂, -CH₂-CH₂-CH₂-NH-CH₃, -CH₂-CH₂-CH₂-N(CH₃)₂, -CH₂-CH₂-CH₂-N(C₂H₅)₂, -CH₂-CH₂-CH₂-N(CH₃)(C₂H₅), -CH₂-CH₂-O-CH₃, -CF₂-CF₂-CF₃, -CF(CF₃)₂,
Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), -CH(CH₃)(S-CH₃), n-Butyl, -CF₂-CF₂-CF₂-CF₃,-CH₂-CH₂-CH₂-CH₂-CN, sec-Butyl, Isobutyl, tert-Butyl, n-Pentyl, sec-Pentyl, neo-Pentyl, n-Hexyl, Vinyl, 1-Propenyl, 2-Propenyl, 1-Butenyl, 2-Butenyl, 3-Butenyl, 2-Methyl-buten-2-yl, (1,1,2)-Trifluor-1-butenyl, 1-Pentenyl, 2-Pentenyl, 3-Pentenyl und 4-Pentenyl stehen;
und jeweils X, m, n, p1, p2 und R⁶ bis R⁴⁸ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte zyklische Harnstoff-Derivate der vorstehend angegebenen allgemeinen Formel I, worin
R¹, R², R⁴ und R⁵, unabhängig voneinander, jeweils für
H; F; Cl; I; Br; -NO₂; -NH₂; -OH; -SH; -NR¹³R¹⁴; -NH-R¹⁵; -OR¹⁶; -SR¹⁷;-NR²³S(=O)₂R²⁴;
oder für einen Rest ausgewählt aus der Gruppe bestehend aus Methyl, -CF₃, Ethyl, -CH₂-CF₃, -C₂F₅, n-Propyl, Isopropyl, n-Butyl, sec-Butyl, Isobutyl, tert-Butyl oder n-Pentyl stehen
und
R³ für F; Cl; Br; I; -OR¹⁶; -NR²³S(=O)₂R²⁴;
oder für einen Rest ausgewählt aus der Gruppe bestehend aus -SF₅, -CF₃, - C₂F₅, -CH₂-CF₃, -CF(CF₃)₂, Isopropyl, -CH(CH₃)(O-CH₃), -CH(CH₃)(S-CH₃), sec-Butyl, Isobutyl und tert-Butyl steht;
und jeweils X, m, n, p1, p2 und R⁶ bis R⁴⁸ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte zyklische Harnstoff-Derivate der vorstehend angegebenen allgemeinen Formel I, worin
R⁸, R⁹, R¹¹ und R¹², unabhängig voneinander, jeweils für
H; F; Cl; Br; I; -SF₅; -NO₂; -NH₂; -OH; -SH; -C(=O)-NH₂; -S(=O)₂-NH₂;
-NR¹³R¹⁴; -NH-R¹⁵; -OR¹⁶; -SR¹⁷; -O-(CH₂)ₐ-R¹⁸; -O-(CH₂)_{b}-OR¹⁹;
-(CH₂)_{c}-O-(CH₂)_{d}-OR²⁰;
-(CH₂)ₑ-O-C(=O)-R²¹;
-(CH₂)_{f}-O-C(=O)-OR²²;
-NR²³S(=O)₂R²⁴;
-(CH₂)ₛ-C(=O)-NR²⁵R²⁶;
-(CH₂)ₕ-C(=O)-NH-R²⁷;
-S(=O)ᵢR²⁸;
-(CH₂)ⱼ-S(=O)₂-NR²⁹R³⁰;
-(CH₂)ₖ-S(=O)₂-NHR³¹;
-(CH₂)ₗ-NR³²-C(=O)-(CH₂)ₐ-OR³³;
-(CH₂)ᵣ-NH-C(=O)-(CH₂)S-OR³⁴;
-(CH₂)ₜ-NR³⁵-O-C(=O)-OR³⁶_{;}
-(CH₂)ᵤNH-O-C(=O)-OR³⁷;
-(CH₂)ᵥ-O-S(=O)₂-R³⁸;
-(CH₂)_{w}-NR³⁹-C(=O)-SR⁴⁰;
-(CH₂)_{y}-C(=O)-NH-OR⁴¹;
-P(=O)-(OR⁴²)₂;
-(CH₂)_{z}-C(=S)-NR⁴³R⁴⁴_{;}
-(CH₂)ₐₐ-C(=S)-NH-R⁴⁵;
-(CH₂)_{bb}-NR⁴⁶-C(=0)-R⁴⁷;
-(CH₂)_{cc}-NH-C(=O)-R⁴⁸;
oder-NH-C(=NH)-NH₂ stehen;
wobei
a, b, c, d, q und s, unabhängig voneinander, jeweils gleich 1. 2, 3, 4 oder 5 sind,
e, f, g, h, j, k, l, r, t, u, v, w, x, y, z, aa, bb und cc, unabhängig voneinander, jeweils gleich 0, 1, 2, 3, 4 oder 5 sind, und
i gleich 1 oder 2 ist;
für einen Rest ausgewählt aus der Gruppe bestehend aus Methyl, -CF₃, - CCl₃, -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃, -CH₂-N(CH₃)₂, -CH₂-N(C₂H₅)₂, -CH₂-N(CH₃)(C₂H₅), Ethyl, -CH₂-CH₂-NH₂, -CH₂-CH₂-OH, -CH₂-CH₂-SH, -CH₂-CH₂-NH-CH₃, -CH₂-CH₂-N(CH₃)₂, -CH₂-CH₂-N(C₂H₅)₂, -CH₂-CH₂-N(CH₃)(C₂H₅), -CH₂-CF₃, -C₂F₅, -CH₂-CCl₃, -CH₂-CBr₃, -CH₂-CH₂-CN, n-Propyl, -CH₂-CH₂-CH₂-OH, -CH₂-CH₂-CH₂-SH, -CH₂-CH₂-CH₂-NH₂, -CH₂-CH₂-CH₂-NH-CH₃, -CH₂-CH₂-CH₂-N(CH₃)₂, -CH₂-CH₂-CH₂-N(C₂H₅)₂, -CH₂-CH₂-CH₂-N(CH₃)(C₂H₅), -CH₂-CH₂-O-CH₃, -CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), -CH(CH₃)(S-CH₃), n-Butyl, -CF₂-CF₂-CF₂-CF₃, - CH₂-CH₂-CH₂-CH₂-CN, sec-Butyl, Isobutyl, tert-Butyl, n-Pentyl, sec-Pentyl, neo-Pentyl, n-Hexyl, Vinyl, 1-Propenyl, 2-Propenyl, 1-Butenyl, 2-Butenyl, 3-Butenyl, 2-Methyl-buten-2-yl, (1,1,2)-Trifluor-1-butenyl, 1-Pentenyl, 2-Pentenyl, 3-Pentenyl und 4-Pentenyl stehen;
oder für einen Aryl-Rest ausgewählt aus der Gruppe bestehend aus Phenyl und Naphthyl stehen, wobei der Aryl-Rest jeweils mit 1, 2, 3, 4 oder 5 Substituenten unabhängig voneinander ausgewählt aus der Gruppe bestehend aus F, Cl, Br, I, -CN, -NO₂, -OH, -SH, Methyl, Ethyl, -CF₃, -O-CF₃, -S-CF₃, -SF₅, -O-CH₃, -O-C₂H₅, -NH₂, -N(CH₃)₂, -N(C₂H₅)₂, -N(H)(CH₃) und - N(H)(C₂H₅) substituiert sein kann;
und
R¹⁰ für -NR²³S(=O)₂R²⁴ steht;
und jeweils X, m, n, p1, p2, R¹ bis R⁷ und R¹³ bis R⁴⁸ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte zyklische Harnstoff-Derivate der vorstehend angegebenen allgemeinen Formel I, worin
R⁸, R⁹, R¹¹ und R¹², unabhängig voneinander, jeweils für
H; F; Cl; Br; I; -NO₂; -NH₂; -OH; -SH; -NR¹³R¹⁴; -NH-R¹⁵; -OR¹⁶; -SR¹⁷;-NR²³S(=O)₂R²⁴;
oder für einen Rest ausgewählt aus der Gruppe bestehend aus Methyl, -CF₃, Ethyl, -CH₂-CF₃, -C₂F₅, n-Propyl, Isopropyl, n-Butyl, sec-Butyl, Isobutyl, tert-Butyl oder n-Pentyl stehen
und
R¹⁰ für -NR²³S(=O)₂R²⁴ steht;
und jeweils X, m, n, p1, p2, R¹ bis R⁷ und R¹³ bis R⁴⁸ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte zyklische Harnstoff-Derivate der vorstehend angegehenen allgemeinen Formel I, worin
der Rest R¹⁰ für -NR²³S(=O)₂R²⁴ steht und die Reste R⁸, R⁹, R¹¹ und R¹² jeweils für H stehen;
und jeweils X, m, n, p1, p2, R¹ bis R⁷ und R¹³ bis R⁴⁸ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte zyklische Harnstoff-Derivate der vorstehend angegebenen allgemeinen Formel I, worin
der Rest R³ für -OR¹⁶; -NR²³S(=O)₂R²⁴; oder für einen Rest ausgewählt aus der Gruppe bestehend aus bestehend aus Isopropyl, sec-Butyl, Isobutyl und tert-Butyl steht; und die Reste R¹, R², R⁴ und R⁵ jeweils für H stehen;
und jeweils X, m, n, p1, p2, R⁶ bis R⁴⁸ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte zyklische Harnstoff-Derivate der vorstehend angegebenen allgemeinen Formel I, worin
R¹³, R¹⁴, R¹⁵, R¹⁶, R¹⁷, R¹⁹, R²⁰, R²¹, R²², R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, R⁴², R⁴³, R⁴⁴, R⁴⁵, R⁴⁶, R⁴⁷ und R⁴⁸, unabhängig voneinander, jeweils
für einen Rest ausgewählt aus der Gruppe bestehend aus Methyl, -CF₃,-CCl₃, -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃, -CH₂-N(CH₃)₂, -CH₂-N(C₂H₅)₂, -CH₂-N(CH₃)(C₂H₅), Ethyl, -CH₂-CH₂-NH₂, -CH₂-CH₂-OH, -CH₂-CH₂-SH, -CH₂-CH₂-NH-CH₃, -CH₂-CH₂-N(CH₃)₂, -CH₂-CH₂-N(C₂H₅)₂, -CH₂-CH₂-N(CH₃)(C₂H₅), -CH₂-CF₃, -C₂F₅, -CH₂-CCl₃, -CH₂-CBr₃, -CH₂-CH₂-CN, n-Propyl, -CH₂-CH₂-CH₂-OH, -CH₂-CH₂-CH₂-SH, -CH₂-CH₂-CH₂-NH₂, -CH₂-CH₂-CH₂-NH-CH₃, -CH₂-CH₂-CH₂-N(CH₃)₂, -CH₂-CH₂-CH₂-N(C₂H₅)₂, -CH₂-CH₂-CH₂-N(CH₃)(C₂H₅), -CH₂-CH₂-O-CH₃, -CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), -CH(CH₃)(S-CH₃), n-Butyl, -CF₂-CF₂-CF₂-CF₃,-CH₂-CH₂-CH₂-CH₂-CN, sec-Butyl, Isobutyl, tert-Butyl, n-Pentyl, sec-Pentyl, neo-Pentyl, n-Hexyl, Vinyl, 1-Propenyl, 2-Propenyl, 1-Butenyl, 2-Butenyl, 3-Butenyl, 2-Methyl-buten-2-yl, (1,1,2)-Trifluor-1-butenyl, 1-Pentenyl, 2-Pentenyl, 3-Pentenyl und 4-Pentenyl stehen;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl stehen, wobei der (hetero)cycloaliphatische Rest jeweils ggf. mit 1, 2, 3, 4 oder 5 Substituenten unabhängig voneinander ausgewählt aus der Gruppe bestehend aus Thioxo (=S), F, Cl, Br, I, -CN,-CF₃, -SF₅, -OH, -NH₂, -O-CF₃, -SH, -O-CH₃, -O-C₂H₅, Methyl, Ethyl, n-Propyl, Isopropyl, n-Butyl, sec-Butyl, Isobutyl, tert-Butyl, -S-CH₃, -S-C₂H₅, -C(=O)-O-CH₃, -C(=O)-O-C₂H₅, Oxo (=O), -N(CH₃)₂, -N(C₂H₅)₂, -N(H)(CH₃),-N(H)(C₂H₅), -NO₂, -SCF₃, -C(=O)-OH, -O-Phenyl, -O-Benzyl, Phenyl und Benzyl substituiert sein kann,
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, indazolyl, Chinoxalinyl, Chinolinyl und Isochinolinyl stehen, wobei der Rest jeweils ggf. mit 1, 2, 3, 4 oder 5 Substituenten unabhängig voneinander ausgewählt aus der Gruppe bestehend aus F, Cl, Br, I, -CN, -NO₂, -OH, -SH, Methyl, Ethyl, n-Propyl, Isopropyl, n-Butyl, sec-Butyl, Isobutyl, tert-Butyl, -CF₃, -O-CF₃, -S-CF₃, -SF₅, -O-CH₃, -O-C₂H₅, -O-Phenyl, -O-Benzyl, -NH₂, -N(CH₃)₂, -N(C₂H₅)₂,-N(H)(CH₃) und -N(H)(C₂H₅) substituiert sein kann;
und jeweils X, m, n, p1, p2, R¹ bis R¹², R¹⁸, R²³ und R²⁴ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte zyklische Harnstoff-Derivate der vorstehend angegebenen allgemeinen Formel I, worin
R¹⁸ für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der (hetero)cycloaliphatische Rest jeweils ggf. mit 1, 2, 3, 4 oder 5 Substituenten unabhängig voneinander ausgewählt aus der Gruppe bestehend aus Thioxo (=S), F, Cl, Br, I, -CN, -CF₃, -SF₅, -OH, -NH₂, -O-CF₃, - SH, -O-CH₃, -O-C₂H₅, Methyl, Ethyl, n-Propyl, Isopropyl, n-Butyl, sec-Butyl, Isobutyl, tert-Butyl, -S-CH₃, -S-C₂H₅, -C(=O)-O-CH₃, -C(=O)-O-C₂H₅, Oxo (=O), -N(CH₃)₂, -N(C₂H₅)₂, -N(H)(CH₃), -N(H)(C₂H₅), -NO₂, -SCF₃, -C(=O)-OH, -O-Phenyl, -O-Benzyl, Phenyl und Benzyl substituiert sein kann,
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinoxalinyl, Chinolinyl und Isochinolinyl steht, wobei der Rest jeweils ggf. mit 1, 2, 3, 4 oder 5 Substituenten unabhängig voneinander ausgewählt aus der Gruppe bestehend aus F, Cl, Br, I, -CN, -NO₂, -OH, -SH, Methyl, Ethyl, n-Propyl, Isopropyl, n-Butyl, sec-Butyl, Isobutyl, tert-Butyl, -CF₃, -O-CF₃, -S-CF₃, -SF₅,-O-CH₃, -O-C₂H₅, -O-Phenyl, -O-Benzyl, -NH₂, -N(CH₃)₂, -N(C₂H₅)₂, -N(H)(CH₃) und -N(H)(C₂H₅) substituiert sein kann;
und jeweils X, m, n, p1, p2, R¹ bis R¹⁷ und R¹⁹ bis R⁴⁸ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte zyklische Harnstoff-Derivate der vorstehend angegebenen allgemeinen Formel I, worin
R²³ für einen Wasserstoff-Rest steht
oder für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, neo-Pentyl und n-Hexyl steht;
und jeweils X, m, n, p1, p2, R¹ bis R²² und R²⁴ bis R⁴⁸ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate. Ebenfalls bevorzugt sind substituierte zyklische Harnstoff-Derivate der vorstehend angegebenen allgemeinen Formel I, worin
R²⁴ für einen Rest ausgewählt aus der Gruppe bestehend aus Methyl, -CH₂-CN, Ethyl, -CH₂-CH₂-CN, n-Propyl, Isopropyl, n-Butyl, sec-Butyl, Isobutyl, tert-Butyl, n-Pentyl, sec-Pentyl, neo-Pentyl und n-Hexyl steht;
und jeweils X, m, n, p1, p2, R¹ bis R²³ und R²⁵ bis R⁴⁸ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte zyklische Harnstoff-Derivate der vorstehend angegebenen allgemeinen Formel I, worin
p1 und p2, unabhängig voneinander, jeweils für 0 oder 1 stehen, wobei die Summe von p1 und p2 0 oder 1 beträgt;
und jeweils X, m, n und R¹ bis R⁴⁸ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Der Fachmann versteht, dass sich für p1 und p2 jeweils gleich 0 die allgemeine Formel Ic ergibt:

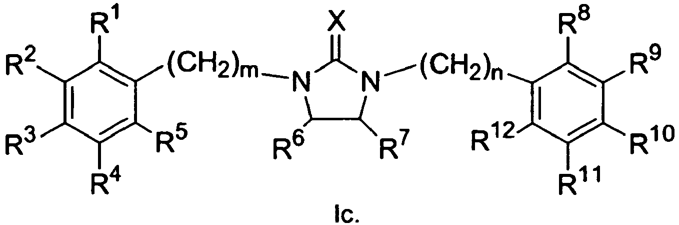

Der Fachmann versteht ebenfalls, dass sich für p1 gleich 0 und p2 gleich 1 die allgemeine Formel Id ergibt:

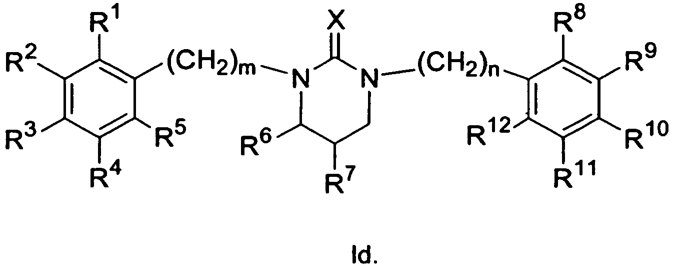

Ferner versteht der Fachmann, dass sich für p1 gleich 1 und p2 gleich 0 die allgemeine Formel Ie ergibt:

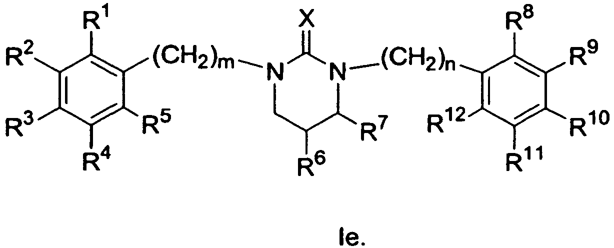

Ebenfalls versteht der Fachmann, dass sich, wenn R⁶ und R⁷ zusammen mit der sie verbindenden -C-C-Brücke einen unsubstituierten Phenylen-Rest bilden, die allgemeine Formel If ergibt:

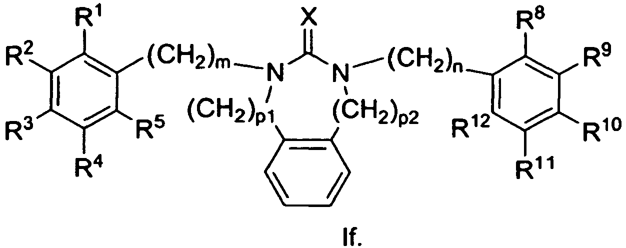

Besonders bevorzugt sind substituierte zyklische Harnstoff-Derivate der vorstehend angegebenen allgemeinen Formel i, worin
X für O, S oder N-C≡N steht;
m gleich 1 ist;
n gleich 1 ist;
p1 und p2, unabhängig voneinander, jeweils für 0 oder 1 stehen, wobei die Summe von p1 und p2 0 oder 1 beträgt;
R¹, R², R⁴, R⁵, R⁸, R⁹, R¹¹ und R¹², unabhängig voneinander, jeweils für
H; F; Cl; I; Br; -NO₂; -NH₂; -OH; -SH; -NR¹³ R¹⁴; -NH-R¹⁵; -OR¹⁶; -SR¹⁷; - NR²³S(=O₎₂R²⁴;
oder für einen Rest ausgewählt aus der Gruppe bestehend aus Methyl, -CF₃, Ethyl, -CH₂-CF₃, -C₂F₅, n-Propyl, Isopropyl, n-Butyl, sec-Butyl, Isobutyl, tert-Butyl oder n-Pentyl stehen;
R³ für F; Cl; Br, I; -OR¹⁶; -NR²³S(=O)₂R²⁴;
oder für einen Rest ausgewählt aus der Gruppe bestehend aus -SF₅, -CF₃, - C₂F₅, -CH₂-CF₃, -CF(CF₃)₂, Isopropyl, -CH(CH₃)(O-CH₃), -CH(CH₃)(S-CH₃), sec-Butyl, Isobutyl und tert-Butyl steht;
R⁶ und R⁷ jeweils für einen Wasserstoff-Rest stehen
oder
R⁶ und R⁷ zusammen mit der sie verbindenden -C-C-Brücke einen unsubstituierten Phenylen-Rest bilden;
R¹⁰ für -NR²³S(=O)₂R²⁴ steht;
R¹³, R¹⁴, R¹⁵, R¹⁶ und R¹⁷, unabhängig voneinander, jeweils
für einen Rest ausgewählt aus der Gruppe bestehend aus -CF₃, -C₂F₅, -CH₂-CF₃, Methyl, Ethyl, n-Propyl, Isopropyl, n-Butyl, sec-Butyl, Isobutyl, tert-Butyl, n-Pentyl, sec-Pentyl, neo-Pentyl und n-Hexyl stehen;
oder für einen Rest ausgewählt aus der Gruppe bestehend aus Phenyl, Thiophenyl, Furanyl und Pyridinyl stehen, wobei der Rest jeweils ggf. mit 1, 2, 3, 4 oder 5 Substituenten unabhängig voneinander ausgewählt aus der Gruppe bestehend aus F, Cl, Br, I, -CN, -NO₂, -OH, -SH, Methyl, Ethyl, n-Propyl, Isopropyl, n-Butyl, sec-Butyl, Isobutyl, tert-Butyl, -CF₃, -O-CF₃, -S-CF₃, -SF₅, -O-CH₃ und -O-C₂H₅ substituiert sein kann;
R²³ für einen Wasserstoff-Rest steht
und
R²⁴ für einen Rest ausgewählt aus der Gruppe bestehend aus Methyl, -CH₂-CN, Ethyl, -CH₂-CH₂-CN, n-Propyl, Isopropyl, n-Butyl, sec-Butyl, Isobutyl, tert-Butyl, n-Pentyl, sec-Pentyl, neo-Pentyl und n-Hexyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls besonders bevorzugt sind substituierte zyklische Harnstoff-Derivate der vorstehend angegebenen allgemeinen Formel I, worin
X für O, S oder N-C≡N steht;
m gleich 1 ist;
n gleich 1 ist;
p1 und p2, unabhängig voneinander, jeweils für 0 oder 1 stehen, wobei die Summe von p1 und p2 0 oder 1 beträgt;
R¹, R², R⁴, R⁸, R¹¹ und R¹² jeweils für H stehen;
R³ für F; Cl; Br, I; -OR¹⁶; -NR²³S(=O)₂R²⁴;
oder für einen Rest ausgewählt aus der Gruppe bestehend aus bestehend aus -CF₃, -C₂F₅, -CH₂-CF₃, Isopropyl, sec-Butyl, Isobutyl und tert-Butyl steht;
R⁵ für H; F; Cl; Br oder I steht;
R⁶ und R⁷ jeweils für einen Wasserstoff-Rest stehen
oder
R⁶ und R⁷ zusammen mit der sie verbindenden -C-C-Brücke einen unsubstituierten Phenylen-Rest bilden;
R⁹ für H; F; Cl; Br; I oder für einen Rest ausgewählt aus der Gruppe bestehend aus Methyl, Ethyl, n-Propyl, Isopropyl, n-Butyl, Isobutyl und tert-Butyl steht;
R¹⁰ für -NR²³S(=O)₂R²⁴ steht;
R¹⁶ für einen Rest ausgewählt aus der Gruppe bestehend aus -CF₃, -C₂F₅ und - CH₂-CF₃ steht;
R²³ für einen Wasserstoff-Rest steht
und
R²⁴ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, neo-Pentyl und n-Hexyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ganz besonders bevorzugt sind substituierte zyklische Harnstoff-Derivate der vorstehend angegebenen allgemeinen Formel I, worin
X für O, S oder N-C≡N steht;
m gleich 1 ist;
n gleich 1 ist;
p1 und p2, unabhängig voneinander, jeweils für 0 oder 1 stehen, wobei die Summe von p1 und p2 0 oder 1 beträgt;
R¹, R², R⁴, R⁵, R⁸, R⁹, R¹¹ und R¹² jeweils für H stehen;
R³ für -OR¹⁶; -NR²³S(=O)₂R²⁴;
oder für einen Rest ausgewählt aus der Gruppe bestehend aus bestehend aus -CF₃, Isopropyl, sec-Butyl, Isobutyl und tert-Butyl steht;
R⁶ und R⁷ jeweils für einen Wasserstoff-Rest stehen
oder
R⁶ und R⁷ zusammen mit der sie verbindenden -C-C-Brücke einen unsubstituierten Phenylen-Rest bilden;
R¹⁰ für -NR²³S(=O)₂R²⁴ steht;
R¹⁶ für einen Rest ausgewählt aus der Gruppe bestehend aus -CF₃, -C₂F₅ und - CH₂-CF₃ steht;
R²³ für einen Wasserstoff-Rest steht
und
R²⁴ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, neo-Pentyl und n-Hexyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Noch weiter bevorzugt sind substituierte zyklische Harnstoff-Derivate der vorstehend angegebenen allgemeinen Formel I ausgewählt aus der Gruppe bestehend aus
[1] N-[4-[3-(4-tert-Butyl-benzyl)-2-thioxo-imidazolidin-ylmethyl]-phenyl]-methansulfonamid,
[2] N-[4-[3-(4-tert-Butyl-benzyl)-2-thioxo-2,3-dihydro-benzoimidazol-1-ylmethyl]-phenyl]-methansulfonamid,
[3] N-[4-[3-(4-tert-Butyl-benzyl)-2-thioxo-tetrahydro-pyrimidin-1-ylmethyl]-phenyl]-methansulfonamid,
[4] N-[4-[3-(4-tert-Butyl-benzyl)-2-oxo-imidazolidin-1-ylmethyl]phenyl]-methansulfonamid,
[5] N-[4-[3-(4-tert-Butyl-benzyl)-2-oxo-tetrahydro-pyrimidin-1-ylmethyl]-phenyl]-methansulfonamid,
[6] N-[4-[3-(4-tert-Butyl-benzyl)-2-oxo-2,3-dihydro-benzoimidazol-1-ylmethyl]-phenyl]-methansulfonamid,
[7] N-[4-[3-(4-Trifluormethoxybenzyl)-2-oxo-2,3-dihydrobenzimidazol-1-ylmethyl]-phenyl]-methansulfonamid,
[8] N-[4-[3-(4-Trifluormethoxybenzyl)-2-thioxo-2,3-dihydrobenzimidazol-1-ylmethyl]-phenyl]-methansulfonamid,
[9] N-[4-[3-(4-Methansulfonylaminobenzyl)-2-oxo-2,3-dihydrobenzimidazol-1-ylmethyl]-phenyl]-methansulfonamid und
[10] N-{4-[2-Oxo-3-(4-trifluormethylbenzyl)-2,3-dihydrobenzimidazol-1-ylmethyl]phenyl}methansulfonamid;
ggf. jeweils in Form entsprechender Salze, oder jeweils in Form entsprechender Solvate.

Ein weiterer Gegenstand der vorliegenden Erfindung ist ein Verfahren zur Herstellung von Verbindungen der vorstehend angegebenen allgemeinen Formel I gemäß dem wenigstens eine Verbindung der allgemeinen Formel II, 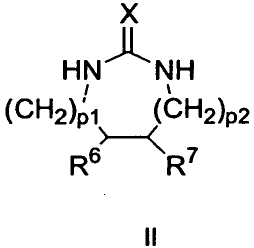 worin R⁶, R⁷, X, p1 und p2 die vorstehend genannte Bedeutung haben, in einem Reaktionsmedium, vorzugsweise in einem Reaktionsmedium ausgewählt aus der Gruppe bestehend aus Diethylether, Tetrahydrofuran, Dichlormethan, Dimethylformamid, Acetonitril, Pyridin, Dimethylsulfoxid, Toluol und entsprechenden Gemischen, ggf. in Gegenwart wenigstens einer Base, vorzugsweise in Gegenwart wenigstens eines Metallhydridsalzes, besonders bevorzugt in Gegenwart von Kalium- und/oder Natriumhydrid,
oder in Gegenwart wenigstens eines Alkalimetallcarbonatsalzes, vorzugsweise in Gegenwart von Kalium- und/oder Natriumcarbonat, und wenigstens eines Alkalimetalliodides, vorzugsweise Kalium- und/oder Natriumiodid, mit wenigstens einer Verbindung der allgemeinen Formel III, 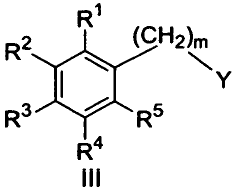 worin R¹ bis R⁵ und m die vorstehend genannte Bedeutung haben und Y für eine Abgangsgruppe, vorzugsweise für einen Halogen-Rest, besonders bevorzugt ein Brom- oder Chloratom steht, zu wenigstens einer Verbindung der allgemeinen Formel IV, 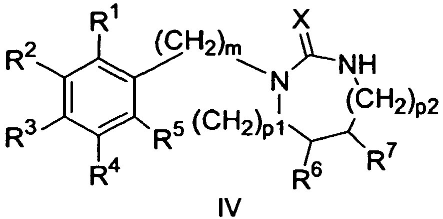 worin R¹ bis R⁷, X, p1, p2 und m die vorstehend genannte Bedeutung haben, umgesetzt wird und diese ggf. gereinigt. und/oder isoliert wird,
oder
wenigstens eine Verbindung der allgemeinen Formel V, 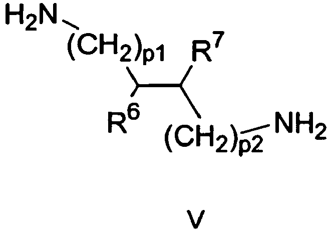 worin R⁶, R⁷, p1 und p2 die vorstehend genannte Bedeutung haben, in einem Reaktionsmedium, vorzugsweise in einem Reaktionsmedium ausgewählt aus der Gruppe bestehend aus Diethylether, Tetrahydrofuran, Acetonitril, Methanol, Ethanol, Dimethylformamid, Dichlormethan und entsprechenden Gemischen, ggf. in Gegenwart wenigstens einer Base, vorzugsweise in Gegenwart wenigstens einer Base ausgewählt aus der Gruppe bestehend aus Pyridin, Triethylamin und Diisopropylethylamin, mit wenigstens einer Verbindung der allgemeinen Formel III zu wenigstens einer Verbindung der allgemeinen Formel VI, 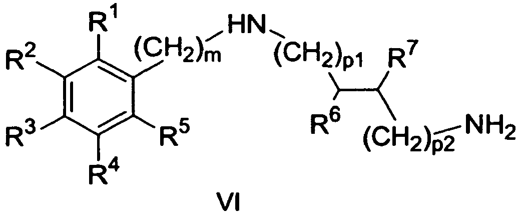 worin R¹ bis R⁷, m, p1 und p2 die vorstehend genannte Bedeutung haben, umgesetzt wird und diese ggf. gereinigt und/oder isoliert wird und wenigstens eine Verbindung der allgemeinen Formel VI in einem Reaktionsmedium, vorzugsweise in einem Reaktionsmedium ausgewählt aus der Gruppe bestehend aus Diethylether, Tetrahydrofuran, Acetonitril, Methanol, Ethanol, Dimethylformamid, Dichlormethan und entsprechenden Gemischen, ggf. in Gegenwart wenigstens einer Base, vorzugsweise in Gegenwart wenigstens einer Base ausgewählt aus der Gruppe bestehend aus Pyridin, Triethylamin und Diisopropylethylamin, mit wenigstens einer Verbindung der allgemeinen Formel Z-C(=X)-Z, worin X für ein Sauerstoff- oder Schwefelatom steht und Z jeweils für eine Abgangsgruppe, vorzugsweise jeweils für einen Halogen-Rest, besonders bevorzugt jeweils für ein Chloratom, steht, zu wenigstens einer Verbindung der allgemeinen Formel IV umgesetzt wird und diese ggf. gereinigt und/oder isoliert wird
oder
wenigstens eine Verbindung der allgemeinen Formel VII, 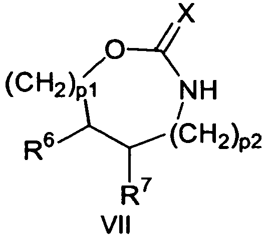 worin R⁶, R⁷, X, p1 und p2 die vorstehend genannte Bedeutung haben, in einem Reaktionsmedium, vorzugsweise in einem Reaktionsmedium ausgewählt aus der Gruppe bestehend aus Diethylether, Tetrahydrofuran, Acetonitril, Methanol, Ethanol, Dimethylformamid, Dichlormethan und entsprechenden Gemischen, mit wenigstens einer Verbindung der allgemeinen Formel VIII, 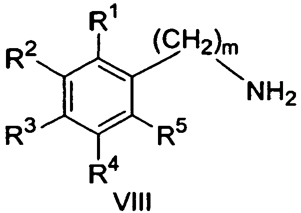 worin R¹ bis R⁵ und m die vorstehend genannte Bedeutung haben, zu wenigstens einer Verbindung der allgemeinen Formel IV umgesetzt wird, und diese ggf. gereinigt und/oder isoliert wird,
und wenigstens eine Verbindung der allgemeinen Formel IV in einem Reaktionsmedium, vorzugsweise in einem Reaktionsmedium ausgewählt aus der Gruppe bestehend aus Diethylether, Tetrahydrofuran, Dichlormethan, Dimethylformamid, Acetonitril, Pyridin, Dimethylsulfoxid, Toluol und entsprechenden Gemischen, in Gegenwart wenigstens einer Base, vorzugsweise in Gegenwart wenigstens eines Metallhydridsalzes, besonders bevorzugt in Gegenwart von Kalium- und/oder Natriumhydrid, oder in Gegenwart wenigstens eines Alkalimetallcarbonatsalzes, vorzugsweise in Gegenwart von Kalium- und/oder Natriumcarbonat, und wenigstens eines Alkalimetalliodides, vorzugsweise Kalium- und/oder Natriumiodid, mit wenigstens einer Verbindung der allgemeinen Formel IX, 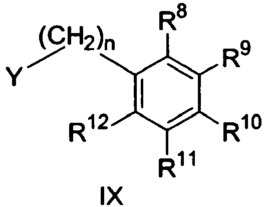 worin R⁸ bis R¹² und n die vorstehend genannte Bedeutung haben mit der Maßgabe, dass wenigstens einer der Substituenten R⁸ bis R¹² für eine -N(PG)₂-Gruppe steht, wobei PG jeweils für eine Schutzgruppe, vorzugsweise jeweils für eine tert.-Butoxycarbonyl-Gruppe oder Benzyloxycarbonyl-Gruppe, steht, oder (PG)₂ zusammen mit dem verbindenden Stickstoffatom eine zyklische Schutzgruppe, bevorzugt eine Phthalimid-Gruppe, bilden, und Y für eine Abgangsgruppe, vorzugsweise für ein Halogenatom, besonders bevorzugt für ein Chlor- oder Bromatom steht, zu wenigstens einer Verbindung der allgemeinen Formel XI, 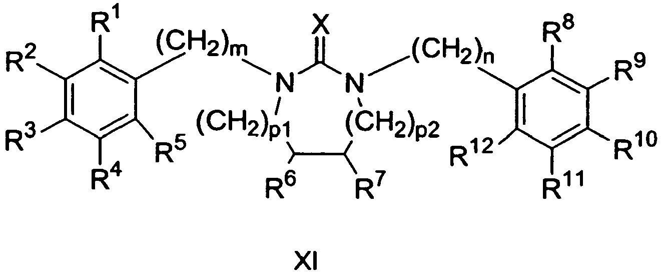 worin R¹ bis R¹², X, m, n, p1 und p2 die vorstehend genannte Bedeutung haben mit der Maßgabe, dass wenigstens einer der Substituenten R⁸ bis R¹² für eine -N(PG)₂-Gruppe steht, wobei PG die vorstehend genannte Bedeutung hat, umgesetzt wird und diese ggf. gereinigt und/oder isoliert wird und wenigstens eine Verbindung der allgemeinen Formel XI in einem Reaktionsmedium, vorzugsweise in einem Reaktionsmedium ausgewählt aus der Gruppe bestehend aus Methanol, Ethanol, Isopropanol, Aceton, Diethylether, Tetrahydrofuran, Dichlormethan, Dimethylformamid, Acetonitril, Pyridin, Dimethylsulfoxid, Toluol und entsprechenden Gemischen, in Gegenwart wenigstens einer Base, vorzugsweise in Gegenwart wenigstens einer Base ausgewählt aus der Gruppe bestehend aus Dimethylamin, Triethylamin, Düsopropylethylamin, Natriumhydroxid, Lithiumhydroxid, Kaliumhydroxid, Kaliumcarbonat und Cäsiumcarbonat, besonders bevorzugt in Gegenwart von Dimethylamin, oder in Gegenwart wenigstens einer Säure, vorzugsweise in Gegenwart wenigstens einer Säure ausgewählt aus der Gruppe bestehend aus Salzsäure, Essigsäure, Trifluoressigsäure, Zitronensäure und Schwefelsäure, oder in Gegenwart von Hydrazin und/oder Phenylhydrazin oder in Gegenwart wenigstens eines Alkalimetallborhydrids, vorzugsweise in Gegenwart von Natriumborhydrid, zu wenigstens einer Verbindung der allgemeinen Formel XII, 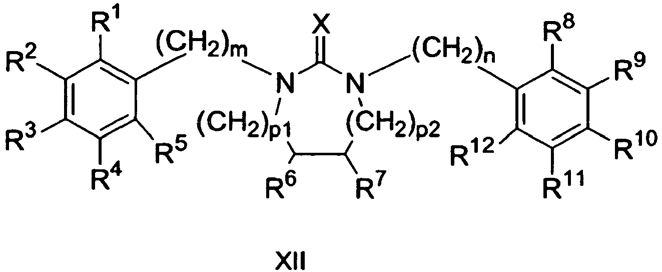 worin R¹ bis R¹², X, m, n, p1 und p2 die vorstehend genannte Bedeutung haben mit der Maßgabe, dass wenigstens einer der Substituenten R⁸ bis R¹² für eine -NH₂-Gruppe steht, umgesetzt wird und diese ggf. gereinigt und/oder isoliert wird,
oder
wenigstens eine Verbindung der allgemeinen Formel IV in einem Reaktionsmedium, vorzugsweise in einem Reaktionsmedium ausgewählt aus der Gruppe bestehend aus Diethylether, Tetrahydrofuran, Dichlomethan, Dimethylformamid, Acetonitril, Pyridin, Dimethylsulfoxid, Toluol und entsprechenden Gemischen, in Gegenwart wenigstens einer Base, vorzugsweise in Gegenwart wenigstens eines Metallhydridsalzes, besonders bevorzugt in Gegenwart von Kalium- und/oder Natriumhydrid, oder in Gegenwart wenigstens eines Alkalimetallcarbonatsalzes, vorzugsweise in Gegenwart von Kalium- und/oder Natriumcarbonat, und wenigstens eines Alkalimetalliodides, vorzugsweise Kalium- und/oder Natriumiodid, mit wenigstens einer Verbindung der allgemeinen Formel XIII, 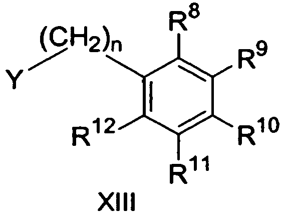 worin R⁸ bis R¹² und n die vorstehend genannte Bedeutung haben mit der Maßgabe, dass wenigstens einer der Substituenten R⁸ bis R¹² für eine -NO₂-Gruppe steht, und Y für eine Abgangsgruppe, vorzugsweise für einen Halogen-Rest, besonders bevorzugt für ein Chlor- oder Bromatom, steht, zu wenigstens einer Verbindung der allgemeinen Formel XIV, 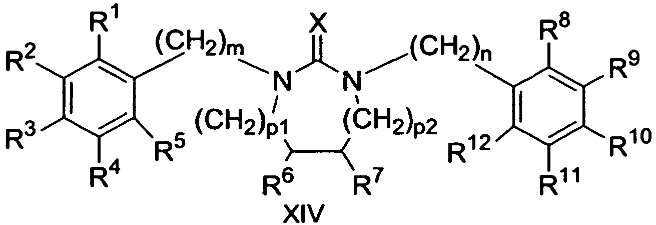 worin R¹ bis R¹², X, m, n, p1 und p2 die vorstehend genannte Bedeutung haben mit der Maßgabe, dass wenigstens einer der Substituenten R⁸ bis R¹² für eine -NO₂-Gruppe steht, umgesetzt wird und diese ggf. gereinigt und/oder isoliert wird, und wenigstens eine Verbindung der allgemeinen Formel XIV in einem Reaktionsmedium, vorzugsweise in einem Reaktionsmedium ausgewählt aus der Gruppe bestehend aus Methanol, Ethanol, Isopropanol, Aceton, Diethylether, Tetrahydrofuran, Dichlormethan, Dimethylformamid, Acetonitril, Pyridin, Dimethylsulfoxid, Toluol und entsprechenden Gemischen, in Gegenwart von Wasserstoff und in Gegenwart eines Katalysators, vorzugsweise in Gegenwart eines Katalysators basierend auf Palladium und/oder Platin, besonders bevorzugt in Gegenwart von Palladium, zu wenigstens einer Verbindung der allgemeinen Formel XII, worin R¹ bis R¹², X, m, n, p1 und p2 die vorstehend genannte Bedeutung haben mit der Maßgabe, dass wenigstens einer der Substituenten R⁸ bis R¹² für eine -NH₂-Gruppe steht, umgesetzt wird und diese ggf. gereinigt und/oder isoliert wird,
und wenigstens eine Verbindung der allgemeinen Formel XII in einem Reaktionsmedium, vorzugsweise in einem Reaktionsmedium ausgewählt aus der Gruppe bestehend aus Aceton, Diethylether, Tetrahydrofuran, Dichlormethan, Dimethylformamid, Acetonitril, Pyridin, Dimethylsulfoxid, Toluol und entsprechenden Gemischen, ggf. in Gegenwart wenigstens einer Base, vorzugsweise in Gegenwart wenigstens einer Base ausgewählt aus der Gruppe bestehend aus Pyridin, Triethylamin und Diisopropylethylamin, mit wenigstens einer Verbindung der allgemeinen Formel R²⁴-S(=O)₂-Z, worin R²⁴ die vorstehend genannte Bedeutung hat und Z für eine Abgangsgruppe, bevorzugt für ein Halogenatom, besonders bevorzugt für ein Chloratom, steht, zu wenigstens einer Verbindung der allgemeinen Formel Ia, 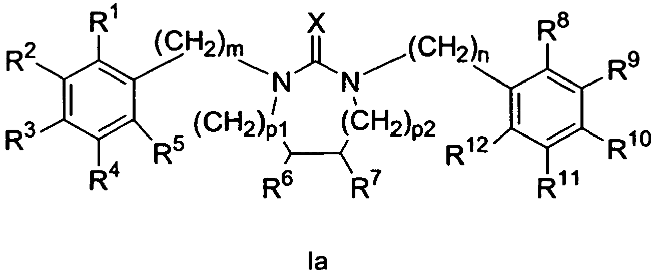 worin R¹ bis R¹², X, m, n, p1 und p2 die vorstehend genannte Bedeutung haben mit der Maßgabe, dass wenigstens einer der Substituenten R⁸ bis R¹² für eine -NH-S(=O)₂-R²⁴-Gruppe steht, wobei R²⁴ die vorstehend genannte Bedeutung hat, umgesetzt wird, und diese ggf. gereinigt und/oder isoliert wird oder wenigstens eine Verbindung der allgemeinen Formel IV in einem Reaktionsmedium, vorzugsweise in einem Reaktionsmedium ausgewählt aus der Gruppe bestehend aus Diethylether, Tetrahydrofuran, Dichlormethan, Dimethylformamid, Acetonitril, Pyridin, Dimethylsulfoxid, Toluol und entsprechenden Gemischen, in Gegenwart wenigstens einer Base, vorzugsweise in Gegenwart wenigstens eines Metallhydridsalzes, besonders bevorzugt in Gegenwart von Kalium- und/oder Natriumhydrid, oder in Gegenwart wenigstens eines Alkalimetallcarbonatsalzes, vorzugsweise in Gegenwart von Kalium- und/oder Natriumcarbonat, und wenigstens eines Alkalimetalliodides, vorzugsweise Kalium- und/oder Natriumiodid, mit wenigstens einer Verbindung der allgemeinen Formel XV, 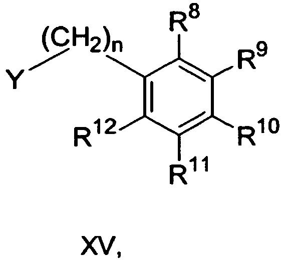 worin R⁸ bis R¹² und n die vorstehend genannte Bedeutung haben mit der Maßgabe, dass wenigstens einer der Substituenten R⁸ bis R¹² für eine -NH-S(=O)₂-R²⁴-Gruppe, wobei R²⁴ die vorstehend genannte Bedeutung hat, steht, zu wenigstens einer Verbindung der allgemeinen Formel la umgesetzt wird, und diese ggf. gereinigt und/oder isoliert wird
und ggf. wenigstens eine Verbindung der allgemeinen Formel Ia, in einem Reaktionsmedium, vorzugsweise in einem Reaktionsmedium ausgewählt aus der Gruppe bestehend aus Diethylether, Tetrahydrofuran, Dichlormethan, Dimethylformamid, Acetonitril, Pyridin, Dimethylsulfoxid, Toluol und entsprechenden Gemischen, ggf. in Gegenwart wenigstens einer Base, vorzugsweise in Gegenwart wenigstens eines Metallhydridsalzes, besonders bevorzugt in Gegenwart von Kalium- und/oder Natriumhydrid, oder in Gegenwart wenigstens eines Alkalimetallcarbonatsalzes, vorzugsweise in Gegenwart von Kalium- und/oder Natriumcarbonat, und wenigstens eines Alkalimetalliodides, vorzugsweise Kalium- und/oder Natriumiodid, mit wenigstens einer Verbindung der allgemeinen Formel R²³-Z, worin R²³ die vorstehend genannte Bedeutung mit Ausnahme von Wasserstoff hat, und Z für eine Abgangsgruppe, bevorzugt für ein Halogenatom, besonders bevorzugt für ein Chloratom, steht, zu wenigstens einer Verbindung der allgemeinen Formel Ib, 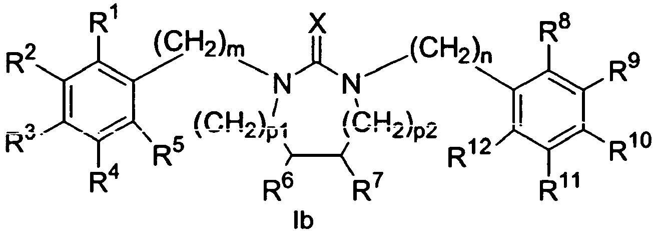 worin R¹ bis R¹², X, m, n, p1 und p2 die vorstehend genannte Bedeutung haben mit der Maßgabe, dass wenigstens einer der Substituenten R⁸ bis R¹² für eine -NR²³-S(=O)₂-R²⁴-Gruppe steht, wobei R²³ und R²⁴ die vorstehend genannte Bedeutung hat, umgesetzt wird, und diese ggf. gereinigt und/oder isoliert wird,
und ggf. wenigstens eine Verbindung der allgemeinen Formel Ib, worin R¹ bis R¹², m, n, p1 und p2 die vorstehend genannte Bedeutung haben und X für ein Sauerstoffatom steht mit der Maßgabe, dass wenigstens einer der Substituenten R⁸ bis R¹² für eine -NR²³-S(=O)₂-R²⁴-Gruppe steht, wobei R²³ und R²⁴ die vorstehend genannte Bedeutung hat, in einem Reaktionsmedium, vorzugsweise in einem Reaktionsmedium ausgewählt aus der Gruppe bestehend aus Toluol, para-Xylol, ortho-Xylol, meta-Xylol, Dichlormethan, Dimethylformamid, Acetonitril und entsprechenden Gemischen, mit wenigstens einer Verbindung der allgemeinen Formel XVI, 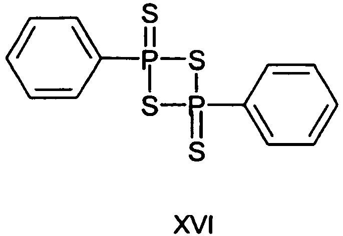 worin die Phenyl-Reste jeweils mit 1 oder 2 Substituenten unabhängig voneinander ausgewählt aus der Gruppe bestehend aus Methoxy, Phenoxy, Cl, Methyl und Br, bevorzugt jeweils mit einem Phenoxy-Rest oder Methoxy-Rest, besonders bevorzugt jeweils mit einem Methoxy-Rest in para-Position, substituiert sind, oder mit Phosphorpentasulfid, zu wenigstens einer Verbindung der allgemeinen Formel Ib, worin R¹ bis R¹², m, n und p die vorstehend genannte Bedeutung haben und X für ein Schwefelatom steht mit der Maßgabe, dass wenigstens einer der Substituenten R⁸ bis R¹² für eine -NR²³-S(=O)₂-R²⁴-Gruppe steht, wobei R²³ und R²⁴ die vorstehend genannte Bedeutung haben, umgesetzt wird, und diese ggf. gereinigt und/oder isoliert wird.

Ein weiterer Gegenstand der vorliegende Erfindung ist ein Verfahren zur Herstellung von Verbindungen der vorstehend angegebenen allgemeinen Formel I gemäß dem wenigstens eine Verbindung der allgemeinen Formel II, 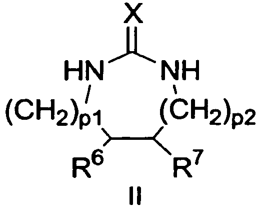 worin R⁶, R⁷, X, p1 und p2 die vorstehend genannte Bedeutung haben, in einem Reaktionsmedium, vorzugsweise in einem Reaktionsmedium ausgewählt aus der Gruppe bestehend aus Diethylether, Tetrahydrofuran, Dichlormethan, Dimethylformamid, Acetonitril, Pyridin, Dimethylsulfoxid, Toluol und entsprechenden Gemischen, in Gegenwart wenigstens einer Base, vorzugsweise in Gegenwart wenigstens eines Metallhydridsalzes, besonders bevorzugt in Gegenwart von Kalium- und/oder Natriumhydrid,
oder in Gegenwart wenigstens eines Alkalimetallcarbonatsalzes, vorzugsweise in Gegenwart von Kalium- und/oder Natriumcarbonat, und wenigstens eines Alkalimetalliodides, vorzugsweise Kalium- und/oder Natriumiodid, mit wenigstens einer Verbindung der allgemeinen Formel XVII, 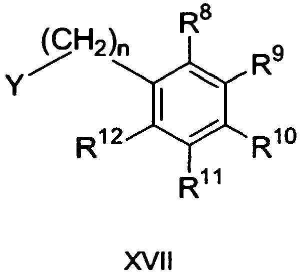 worin R⁸ bis R¹² und n die vorstehend genannte Bedeutung haben mit der Maßgabe, dass wenigstens einer der Substituenten R⁸ bis R¹² für eine -N(PG)₂-Gruppe steht, wobei PG jeweils für eine Schutzgruppe, vorzugsweise jeweils für eine tert.-Butoxycarbonyl-Gruppe oder Benzyloxycarbonyl-Gruppe, steht oder zwei PG-Gruppen zusammen mit dem sie verbindenden Stickstoffatom eine zyklische Schutzgruppe, bevorzugt zusammen mit dem sie verbindenden Stickstoffatom eine Phthalimid-Gruppe, bilden, oder wenigstens einer der Substituenten R⁸ bis R¹² für eine -NO₂-Gruppe steht, und Y für eine Abgangsgruppe, vorzugsweise für ein Halogenatom, besonders bevorzugt für ein Chlor- oder Bromatom steht, zu wenigstens einer Verbindung der allgemeinen Formel XVIII, 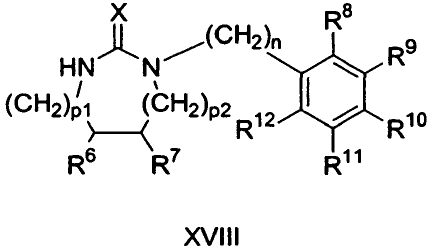 worin R⁶ bis R¹², p1, p2 und n die vorstehend genannte Bedeutung haben mit der Maßgabe, dass wenigstens einer der Substituenten R⁸ bis R¹² für eine -N(PG)₂-Gruppe oder eine -NO₂-Gruppe steht, umgesetzt wird und diese ggf. gereinigt und/oder isoliert wird
oder
wenigstens eine Verbindung der allgemeinen Formel XIX, 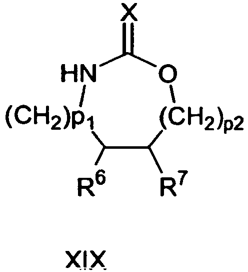 worin R⁶, R⁷, X, p1 und p2 die vorstehend genannte Bedeutung haben, in einem Reaktionsmedium, vorzugsweise in einem Reaktionsmedium ausgewählt aus der Gruppe bestehend aus Diethylether, Tetrahydrofuran, Acetonitril, Methanol, Ethanol, Dimethylformamid, Dichlormethan und entsprechenden Gemischen, mit wenigstens einer Verbindung der allgemeinen Form XX, 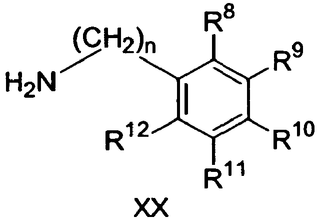 worin R⁸ bis R¹² und n die vorstehend genannte Bedeutung haben mit der Maßgabe, dass wenigstens einer der Substituenten R⁸ bis R¹² für eine -N(PG)₂-Gruppe steht, wobei PG jeweils für eine Schutzgruppe, vorzugsweise jeweils für eine tert.-Butoxycarbonyl-Gruppe oder Benzyloxycarbonyl-Gruppe, steht oder (PG)₂ zusammen mit dem sie verbindenden Stickstoffatom eine zyklische Schutzgruppe, bevorzugt eine Phthalimid-Gruppe, bilden, oder wenigstens einer der Substituenten R⁸ bis R¹² für eine -NO₂-Gruppe steht, zu wenigstens einer Verbindung der allgemeinen Formel XVIII umgesetzt wird und diese ggf. gereinigt und/oder isoliert wird,

oder
wenigstens eine Verbindung der allgemeinen Formel V, 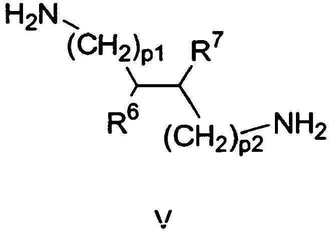 worin R⁶, R⁷, p1 und p2 die vorstehend genannte Bedeutung haben, in einem Reaktionsmedium, vorzugsweise in einem Reaktionsmedium ausgewählt aus der Gruppe bestehend aus Diethylether, Tetrahydrofuran, Acetonitril, Methanol, Ethanol, Dimethylformamid, Dichlormethan und entsprechenden Gemischen, ggf. in Gegenwart wenigstens Base, vorzugsweise in Gegenwart wenigstens einer Base ausgewählt aus der Gruppe bestehend aus Pyridin, Triethylamin und Diisopropylethylamin, mit wenigstens einer Verbindung der allgemeinen Formel XVII zu wenigstens einer Verbindung der allgemeinen Formel XXI, 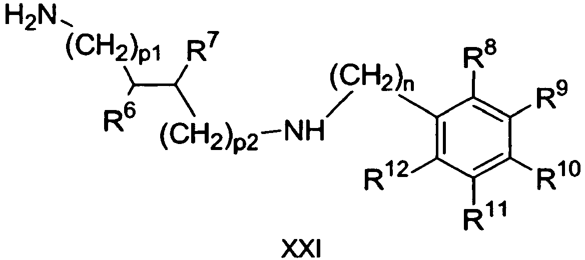 worin R⁶ bis R¹², p1, p2 und n die vorstehend genannte Bedeutung haben mit der Maßgabe, dass wenigstens einer der Substituenten R⁸ bis R¹² für eine -N(PG)₂-Gruppe oder eine -NO₂-Gruppe steht, umgesetzt wird und diese ggf. gereinigt und/oder isoliert wird und wenigstens eine Verbindung der allgemeinen Formel XXI in einem Reaktionsmedium, vorzugsweise in einem Reaktionsmedium ausgewählt aus der Gruppe bestehend aus Diethylether, Tetrahydrofuran, Acetonitril, Methanol, Ethanol, Dimethylformamid, Dichlormethan und entsprechenden Gemischen, ggf. in Gegenwart wenigstens einer Base, vorzugsweise in Gegenwart wenigstens einer Base ausgewählt aus der Gruppe bestehend aus Pyridin, Triethylamin und Diisopropylethylamin, mit wenigstens einer Verbindung der allgemeinen Formel Z-C(=X)-Z, worin X für ein Sauerstoff- oder Schwefelatom und Z jeweils für eine Abgangsgruppe, vorzugsweise jeweils für einen Halogen-Rest, besonders bevorzugt jeweils für ein Chloratom steht, zu wenigstens einer Verbindung der allgemeinen Formel XVIII umgesetzt wird und diese ggf. gereinigt und/oder isoliert wird
und wenigstens eine Verbindung der allgemeinen Formel XVIII in einem Reaktionsmedium, vorzugsweise in einem Reaktionsmedium ausgewählt aus der Gruppe bestehend aus Diethylether, Tetrahydrofuran, Dichlormethan, Dimethylformamid, Acetonitril, Pyridin, Dimethylsulfoxid, Toluol und entsprechenden Gemischen, in Gegenwart wenigstens einer Blase, vorzugsweise in Gegenwart wenigstens eines Metallhydridsalzes, besonders bevorzugt in Gegenwart von Kalium- und/oder Natriumhydrid, oder in Gegenwart wenigstens eines Alkalimetallcarbonatsalzes, vorzugsweise in Gegenwart von Kalium- und/oder Natriumcarbonat, und wenigstens eines Alkalimetalliodides, vorzugsweise Kalium- und/oder Natriumiodid, mit wenigstens einer Verbindung der allgemeinen Formel III, 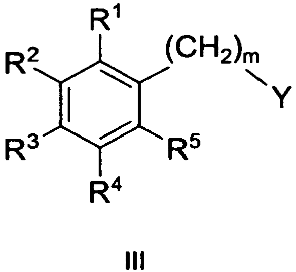 worin R¹ bis R⁵ und m die vorstehend genannte Bedeutung haben und Y für eine Abgangsgruppe, vorzugsweise für einen Halogen-Rest, besonders bevorzugt ein Chlor- oder Bromatom steht, zu wenigstens einer Verbindung der allgemeinen Formel XXII, 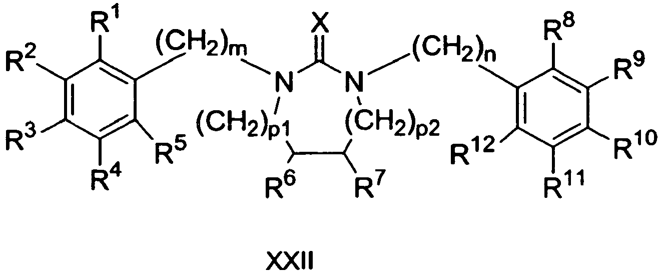 worin R¹ bis R¹², X, m, n, p1 und p2 die vorstehend genannte Bedeutung haben mit der Maßgabe, dass wenigstens einer der Substituenten R⁸ bis R¹² für -N(PG)₂-Gruppe oder -NO₂-Gruppe steht, umgesetzt wird und diese ggf. gereinigt und/oder isoliert wird
und wenigstens eine Verbindung der allgemeinen Formel XXII in einem Reaktionsmedium, vorzugsweise in einem Reaktionsmedium ausgewählt aus der Gruppe bestehend aus Methanol, Ethanol, Isopropanol, Aceton, Diethylether, Tetrahydrofuran, Dichlormethan, Dimethylformamid, Acetonitril, Pyridin, Dimethylsulfoxid, Toluol und entsprechenden Gemischen, in Gegenwart wenigstens einer Blase, vorzugsweise in Gegenwart wenigstens einer Base ausgewählt aus der Gruppe bestehend aus Dimethylamin, Triethylamin, Diisopropylethylamin, Natriumhydroxid, Lithiumhydroxid, Kaliumhydroxid, Kaliumcarbonat und Cäsiumcarbonat, besonders bevorzugt in Gegenwart von Dimethylamin, oder in Gegenwart wenigstens einer Säure, vorzugsweise in Gegenwart wenigstens einer Säure ausgewählt aus der Gruppe bestehend aus Salzsäure, Essigsäure, Trifluoressigsäure, Zitronensäure und Schwefelsäure, oder in Gegenwart von Hydrazin und/oder Phenylhydrazin oder in Gegenwart wenigstens eines Alkalimetallbofiydrids, vorzugsweise in Gegenwart von Natriumborhydrid, oder in Gegenwart von Wasserstoff und eines Katalysators, bevorzugt eines Katalysators basierend auf Palladium und/oder Platin, besonders bevorzugt Palladium, zu wenigstens einer Verbindung der allgemeinen Formel XII, 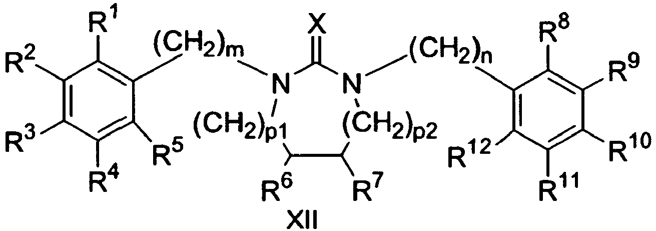 worin R¹ bis R¹², X, m, n, p1 und p2 die vorstehend genannte Bedeutung haben mit der Maßgabe, dass wenigstens einer der Substituenten R⁸ bis R¹² für eine -NH₂-Gruppe steht, umgesetzt wird und diese ggf. gereinigt und/oder isoliert wird,
und wenigstens eine Verbindung der allgemeinen Formel XII in einem Reaktionsmedium, vorzugsweise in einem Reaktionsmedium ausgewählt aus der Gruppe bestehend aus Aceton, Diethylether, Tetrahydrofuran, Dichlormethan, Dimethylformamid, Acetonitril, Pyridin, Dimethylsulfoxid, Toluol und entsprechenden Gemischen, ggf. in Gegenwart wenigstens einer Base, vorzugsweise in Gegenwart wenigstens einer Base ausgewählt aus der Gruppe bestehend aus Pyridin, Triethylamin und Diisopropylethylamin, mit wenigstens einer Verbindung der allgemeinen Formel R²⁴-S(=O)₂-Z, worin R²⁴ die vorstehend genannte Bedeutung hat und Z für eine Abgangsgruppe, bevorzugt für ein Halogenatom, besonders bevorzugt für ein Chloratom, steht, zu wenigstens einer Verbindung der allgemeinen Formel Ia, 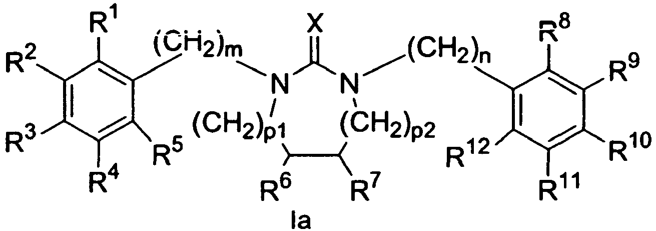 worin R¹ bis R¹², X, m, n, p1 und p2 die vorstehend genannte Bedeutung haben mit der Maßgabe, dass wenigstens einer der Substituenten R⁸ bis R¹² für eine -NH-S(=O)₂-R²⁴-Gruppe steht, wobei R²⁴ die vorstehend genannte Bedeutung hat, umgesetzt wird, und diese ggf. gereinigt und/oder isoliert wird
und ggf. wenigstens eine Verbindung der allgemeinen Formel Ia in einem Reaktionsmedium, vorzugsweise in einem Reaktionsmedium ausgewählt aus der Gruppe bestehend aus Diethylether, Tetrahydrofuran, Dichlormethan, Dimethylformamid, Acetonitril, Pyridin, Dimethylsulfoxid, Toluol und entsprechenden Gemischen, ggf. in Gegenwart wenigstens einer Base, vorzugsweise in Gegenwart wenigstens eines Metallhydridsalzes, besonders bevorzugt in Gegenwart von Kalium- und/oder Natriumhydrid,
oder in Gegenwart wenigstens eines Alkalimetallcarbonatsalzes, vorzugsweise in Gegenwart von Kalium- und/oder Natriumcarbonat, und wenigstens eines Alkalimetalliodides, vorzugsweise Kalium- und/oder Natriumiodid, mit wenigstens einer Verbindung der allgemeinen Formel R²³-Z, worin R²³ die vorstehend genannte Bedeutung mit Ausnahme von Wasserstoff hat und Z für eine Abgangsgruppe, bevorzugt für ein Halogenatom, besonders bevorzugt für ein Chloratom, steht, zu wenigstens einer Verbindung der allgemeinen Formel Ib, 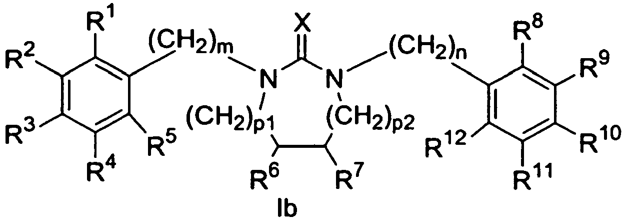 worin R¹ bis R¹², X, m, n, p1 und p2 die vorstehend genannte Bedeutung haben mit der Maßgabe, das wenigstens einer der Substituenten R⁸ bis R¹² für eine -NR²³-S(=O)₂-R²⁴-Gruppe steht, wobei R²³ und R²⁴ die vorstehend genannte Bedeutung haben, umgesetzt wird, und diese ggf. gereinigt und/oder isoliert wird,
und ggf. wenigstens eine Verbindung der allgemeinen Formel Ib, worin R¹ bis R¹², m, n, p1 und p2 die vorstehend genannte Bedeutung haben und X für ein Sauerstoffatom steht mit der Maßgabe, dass wenigstens einer der Substituenten R⁸ bis R¹² für eine -NR²³-S(=O)₂-R²⁴-Gruppe steht, wobei R²³ und R²⁴ die vorstehend genannte Bedeutung haben, in einem Reaktionsmedium, vorzugsweise in einem Reaktionsmedium ausgewählt aus der Gruppe bestehend aus Toluol, para-Xylol, meta-Xylol, ortho-Xylol, Dichlormethan, Dimethylformamid, Acetonitril und entsprechenden Gemischen, mit wenigstens einer Verbindung der allgemeinen Formel XVI, 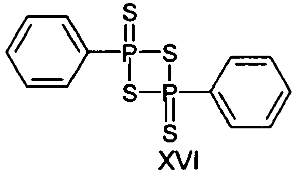 worin die Phenyl-Reste jeweils mit 1 oder 2 Substituenten unabhängig voneinander ausgewählt aus der Gruppe bestehend aus Methoxy, Phenoxy, CI, Methyl und Br, bevorzugt jeweils mit einem Phenoxy-Rest oder Methoxy-Rest, besonders bevorzugt jeweils mit einem Methoxy-Rest in para-Position, substituiert sind, oder mit Phosphorpentasulfid, zu wenigstens einer Verbindung der allgemeinen Formel Ib, worin R¹ bis R¹², m, n und p die vorstehend genannte Bedeutung haben und X für ein Schwefelatom steht mit der Maßgabe, dass wenigstens einer der Substituenten R⁸ bis R¹² für eine -NR²³-S(=O)₂-R²⁴-Gruppe steht, wobei R²³ und R²⁴ die vorstehend genannte Bedeutung haben, umgesetzt wird und diese ggf. gereinigt und/oder isoliert wird.

Ebenfalls ein Gegenstand der vorliegenden Erfindung ist ein Verfahren zur Herstellung von Verbindungen der vorstehend angegebenen allgemeinen Formel I gemäß dem wenigstens eine Verbindung der allgemeinen Formel II, 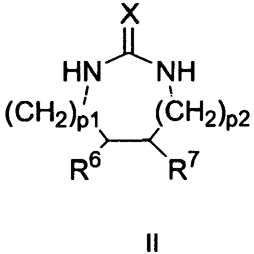 worin R⁶, R⁷, X, p1 und p2 die vorstehend genannte Bedeutung haben, in einem Reaktionsmedium, vorzugsweise in einem Reaktionsmedium ausgewählt aus der Gruppe bestehend aus Diethylether, Tetrahydrofuran, Dichlormethan, Dimethylformamid, Acetonitril, Pyridin, Dimethylsulfoxid, Toluol und entsprechenden Gemischen, in Gegenwart wenigstens einer Base, vorzugsweise in Gegenwart wenigstens eines Metallhydridsalzes, besonders bevorzugt in Gegenwart von Kalium- und/oder Natriumhydrid, oder in Gegenwart wenigstens eines Alkalimetallcarbonatsalzes, vorzugsweise in Gegenwart von Kalium- und/oder Natriumcarbonat, und wenigstens eines Alkalimetalliodides, vorzugsweise Kalium- und/oder Natriumiodid, mit wenigstens einer Verbindung der allgemeinen Formel XXIII, 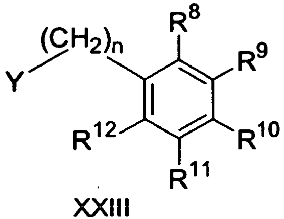 worin R⁸ bis R¹² und n die vorstehend genannte Bedeutung haben mit der Maßgabe, dass wenigstens einer der Substituenten R⁸ bis R¹² für eine -NR²³-S(=O)₂-R²⁴-Gruppe steht und Y für eine Abgangsgruppe, vorzugsweise für ein Halogenatom, besonders bevorzugt für ein Chlor oder Bromatom steht, zu wenigstens einer Verbindung der allgemeinen Formel XXIV, 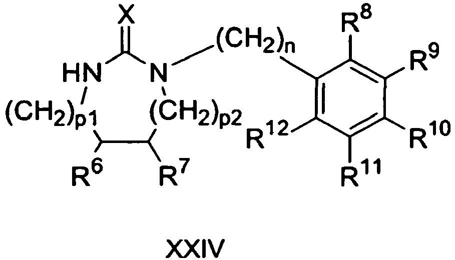 worin R⁶ bis R¹², p1, p2 und n die vorstehend genannte Bedeutung haben mit der Maßgabe, dass wenigstens einer der Substituenten R⁸ bis R¹² für eine -NR²³-S(=O)₂-R²⁴-Gruppe steht, umgesetzt wird und diese ggf. gereinigt und/oder isoliert wird,
und wenigstens eine Verbindung der allgemeinen Formel XXIV in einem Reaktionsmedium, vorzugsweise in einem Reaktionsmedium ausgewählt aus der Gruppe bestehend aus Diethylether, Tetrahydrofuran, Dichlormethan, Dimethylformamid, Acetonitril, Pyridin, Dimethylsulfoxid, Toluol und entsprechenden Gemischen, in Gegenwart wenigstens einer Base, vorzugsweise in Gegenwart wenigstens eines Metallhydridsalzes, besonders bevorzugt in Gegenwart von Kalium- und/oder Natriumhydrid, oder in Gegenwart wenigstens eines Alkalimetallcarbonatsalzes, vorzugsweise in Gegenwart von Kalium- und/oder Natriumcarbonat, und wenigstens eines Alkalimetalliodides, vorzugsweise Kalium- und/oder Natriumiodid, mit wenigstens einer Verbindung der allgemeinen Formel III, 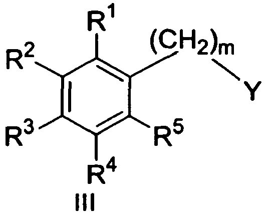 worin R¹ bis R⁵ und m die vorstehend genannte Bedeutung haben und Y für eine Abgangsgruppe, vorzugsweise für einen Halogen-Rest, besonders bevorzugt für ein Chlor- oder Bromatom steht, zu wenigstens einer Verbindung der allgemeinen Formel Ib, 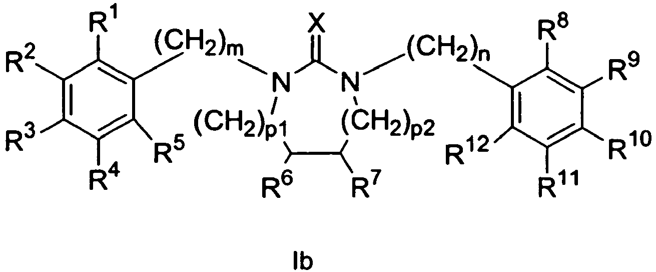 worin R¹ bis R¹², X, m, n, p1 und p2 die vorstehend genannte Bedeutung haben mit der Maßgabe, dass wenigstens einer der Substituenten R⁸ bis R¹² für eine -NR²³-S(=O)₂-R²⁴-Gruppe steht, wobei R²³ und R²⁴ die vorstehend genannte Bedeutung haben, umgesetzt wird, und diese ggf. gereinigt und/oder isoliert wird,
und ggf. wenigstens eine Verbindung der allgemeinen Formel Ib, worin R¹ bis R¹², m, n, p1 und p2 die vorstehend genannte Bedeutung haben und X für ein Sauerstoffatom steht mit der Maßgabe, dass wenigstens einer der Substituenten R⁸ bis R¹² für eine -NR²³-S(=O)₂-R²⁴-Gruppe steht, wobei R²³ und R²⁴ die vorstehend genannte Bedeutung haben, in einem Reaktionsmedium, vorzugsweise in einem Reaktionsmedium ausgewählt aus der Gruppe bestehend aus Toluol, para-Xylol, ortho-Xylol, meta-Xylol, Dichlormethan, Dimethylformamid, Acetonitril und entsprechenden Gemischen, mit wenigstens einer Verbindung der allgemeinen Formel XVI, 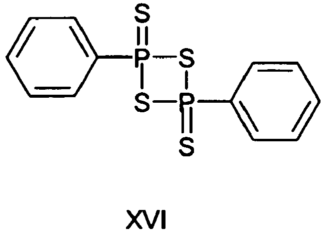 worin die Phenyl-Reste jeweils mit 1 oder 2 Substituenten unabhängig voneinander ausgewählt aus der Gruppe bestehend aus Methoxy, Phenoxy, Cl, Methyl und Br, bevorzugt jeweils mit einem Phenoxy-Rest oder Methoxy-Rest, besonders bevorzugt jeweils mit einem Methoxy-Rest in para-Position, substituiert sind, oder mit Phosphorpentasulfid, zu wenigstens einer Verbindung der allgemeinen Formel Ib, worin R¹ bis R¹², m, n und p die vorstehend genannte Bedeutung haben und X für ein Schwefelatom steht mit der Maßgabe, dass wenigstens einer der Substituenten R⁸ bis R¹² für eine -NR²³-S(=O)₂-R²⁴-Gruppe steht, wobei R²³ und R²⁴ die vorstehend genannte Bedeutung haben, umgesetzt wird und diese ggf. gereinigt und/oder isoliert wird.

Die Verbindungen der vorstehend angegebenen Formeln II, III, V, VII, IX, XIII, XV, XVI, XVII, XIX, XX und XXIII, sowie der allgemeinen Formeln R²³-Z, R²⁴-S(=O)₂-Z und Z-C(=X)-Z sind jeweils am Markt käuflich erhältlich und/oder können nach den üblichen, dem Fachmann bekannten Verfahren hergestellt werden.

Die Synthese von Verbindungen der allgemeinen Formel II, worin X für N-C≡N steht, ist auch im nachfolgenden Schema verdeutlicht.

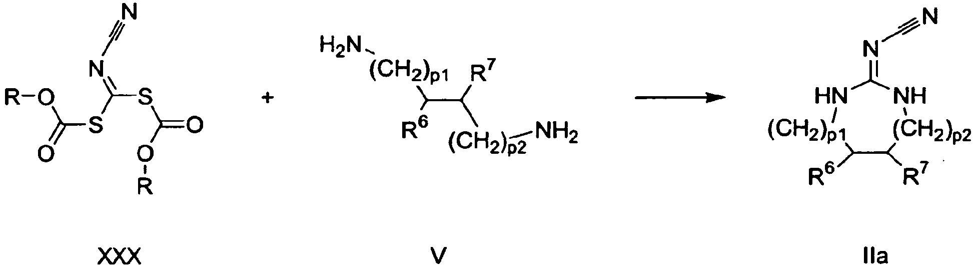

Verbindungen der allgemeinen Formel XXX, worin R für einen C₁₋₁₀-Alkyl-Rest, vorzugsweise für Methyl, Ethyl oder tert-Butyl, steht, werden mit Verbindungen der allgemeinen Formel V, worin R⁶, R⁷, p1 und p2 die vorstehend genannte Bedeutung haben, in einem Reaktionsmedium, vorzugsweise in Chloroform bei einer Temperatur von 20 bis 50 °C oder in n-Propanol bei einer Temperatur von 20 bis 100 °C, zu Verbindungen der allgemeinen Formel IIa, worin R⁶, R⁷, p1 und p2 die vorstehend genannte Bedeutung haben, umgesetzt.

Die vorstehend beschriebenen Umsetzungen können jeweils unter üblichen, dem Fachmann geläufigen Bedingungen, beispielsweise in Hinblick auf Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden.

Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie.

Sämtliche der vorstehend beschriebenen Verfahrensschritte sowie jeweils auch die Reinigung und/oder Isolierung von Zwischen- oder Endprodukten können teilweise oder vollständig unter einer Inertgasatmosphäre, vorzugsweise unter Stickstoffatmosphäre oder Argonatmosphäre, durchgeführt werden.

Die erfindungsgemäßen substituierten zyklischen Harnstoff-Derivate der vorstehend genannten allgemeinen Formeln I sowie entsprechende Stereoisomere können sowohl in Form ihrer freien Basen, ihrer freien Säuren wie auch in Form entsprechender Salze, insbesondere physiologisch verträglicher Salze, isoliert werden.

Die freien Basen der jeweiligen erfindungsgemäßen zyklischen Harnstoff-Derivate der vorstehend genannten allgemeinen Formeln I sowie entsprechender Stereoisomere können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Zitronensäure, Glutaminsäure oder Asparaginsäure, in die entsprechenden Salze, vorzugsweise physiologisch verträglichen Salze, überführt werden.

Die freien Basen der jeweiligen substituierten zyklischen Harnstoff-Derivate der vorstehend genannten allgemeinen Formeln I und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der substituierten zyklischen Harnstoff-Derivate der vorstehend genannten allgemeinen Formeln I und entsprechender Stereoisomere durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze [NHₓR₄₋ₓ]⁺, worin x = 0, 1, 2, 3 oder 4 ist und R für einen linearen oder verzweigten C₁₋₄-Alkyl-Rest steht, genannt.

Die erfindungsgemäßen substituierten zyklischen Harnstoff-Derivate der vorstehend genannten allgemeinen Formeln I und entsprechende Stereoisomere können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erhalten werden.

Sofern die erfindungsgemäßen substituierten zyklischen Harnstoff-Derivate der vorstehend genannten allgemeinen Formeln I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten zyklischen Harnstoff-Derivate der vorstehend genannten allgemeinen Formel I bezeichnet, und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens ein erfindungsgemäßes zyklisches Harnstoff-Derivat der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Diese erfindungsgemäßen Arzneimittel eignen sich insbesondere zur Vanilloid-Rezeptor 1-(VR1/TRPV1)-Regulation, vorzugsweise zur Vanilloid-Rezeptor 1-(VR1/TRPV1)-Hemmung und/oder zur Vanilloid-Rezeptor 1 (VR1/TRPV1)-Stimulation.

Ebenfalls bevorzugt eignen sich die erfindungsgemäßen Arzneimittel zur Prophylaxe und/oder Behandlung von Störungen oder Krankheiten, die zumindest teilweise durch Vanilloid-Rezeptoren 1 vermittelt werden.

Bevorzugt eignet sich das erfindungsgemäße Arzneimittel zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Besonders bevorzugt eignet sich das erfindungsgemäße Arzneimittel zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Harninkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Ganz besonders bevorzugt eignet sich das erfindungsgemäße Arzneimittel zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz, und/oder Harninkontinenz.

Ein weiterer Gegenstand der vorliegenden Erfindung ist die Verwendung wenigstens einer erfindungsgemäßen substituierten zyklischen Harnstoff-Derivats der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Vanilloid-Rezeptor 1-(VR1 /TRPV1)-Regulation, vorzugsweise zur Vanilloid-Rezeptor 1-(VR1/TRPV1)-Hemmung und/oder zur Vanilloid-Rezeptor 1 (VR1/TRPV1)-Stimulation.

Bevorzugt ist die Verwendung wenigstens eines substituierten zyklischen Harnstoff-Derivats der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Störungen oder Krankheiten, die zumindest teilweise durch Vanilloid-Rezeptoren 1 vermittelt werden.

Besonders bevorzugt ist die Verwendung wenigstens eines substituierten zyklischen Harnstoff-Derivats der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Ganz besonders bevorzugt ist die Verwendung wenigstens eines substituierten zyklischen Harnstoff-Derivats der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Harninkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Noch weiter bevorzugt ist die Verwendung wenigstens eines substituierten zyklischen Harnstoff-Derivats der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von Schmerz, vorzugsweise ausgewählt aus der Gruppe bestehend aus akutem Schmerz, chronischem Schmerz, neuropathischem Schmerz und visceralem Schmerz, und/oder Harninkontinenz.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kasein, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden. Neben wenigstens einem substituierten zyklischen Harnstoff-Derivat der vorstehend angegebenen allgemeinen Formel I, ggf. in Form eines seiner reinen Stereoisomeren, insbesondere Enantiomeren oder Diastereomeren, seines Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden erfindungsgemäßen substituierten zyklischen Harnstoff-Derivate der vorstehend angegebenen allgemeinen Formel I können in einem Depot, in gelöster Form oder in einem Pflaster, gegebenenfalls unter Zusatz von die Hautpenetration fördernden Mitteln, als geeignete perkutane Applikationszubereitungen vorliegen. Oral oder perkutan anwendbare Zubereitungsformen können die jeweilige erfindungsgemäßen substituierten zyklischen Harnstoff-Derivate der vorstehend angegebenen allgemeinen Formel I auch verzögert freisetzen. Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remingtons Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung.

Die an den Patienten zu verabreichende Menge der jeweiligen erfindungsgemäßen substituierten zyklischen Harnstoff-Derivate der vorstehend angegebenen allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0,005 bis 100 mg/kg, vorzugsweise 0,05 bis 75 mg/kg Körpergewicht des Patienten wenigstens einer solchen erfindungsgemäßen Verbindung appliziert.

### Pharmakologische Methoden:

### I. Funktionelle Untersuchung am Vanilloid-Rezeptor 1 (VRI/TRPV1-Rezeptor)

Die agonistische bzw. antagonistische Wirkung der zu untersuchenden Substanzen am Vanilloid-Rezeptor 1 (VR1/TRPV1) der Spezies Ratte kann mit folgendem Assay bestimmt werden. Gemäß diesem Assay wird der Ca²⁺-Einstrom durch den Rezeptorkanal mit Hilfe eines Ca²⁺-sensitiven Farbstoffs (Typ Fluo-4, Molecular Probes Europa BV, Leiden Niederlande) im Fiuorescent imaging Plate Reader (FLIPR, Molecular Devices, Sunnyvale, USA) quantifiziert.

### Methode:

Komplett-Medium: 50 mL HAMS F12 Nutrient Mixture (Gibco Invitrogen GmbH, Karlsruhe, Deutschland) mit
10 Vol-% FCS (fetal calf serum, Gibco Invitrogen GmbH, Karlsruhe, Deutschland, hitzeinaktiviert);
2mM L-Glutamin (Sigma, München, Deutschland);
1 Gew-% AA-Lösung (Antibiotika/Antimykotika-Lösung, PAA, Pasching, Österreich) und 25 ng/ml Medium NGF (2.5 S, Gibco Invitrogen GmbH, Karlsruhe, Deutschland)

Zellkultur-Platte: Poly-D-Lysin-beschichtete, schwarze 96-Loch-Platten mit klarem Boden (96 well black/clear plate, BD Biosciences, Heidelberg, Deutschland) werden zusätzlich mit Laminin (Gibco Invitrogen GmbH, Karlsruhe, Deutschland) beschichtet, indem Laminin auf eine Konzentration 100 µg/mL mit PBS (Ca-Mg-free PBS, Gibco Invitrogen GmbH, Karlsruhe, Deutschland) verdünnt wird. Es werden Aliquots mit einer Konzentration von 100 µg/mL an Laminin entnommen und bei -20 °C gelagert. Die Aliquots werden mit PBS im Verhältnis 1:10 auf 10 µg/mL Laminin verdünnt und jeweils 50 µL der Lösung in eine Vertiefung der Zellkultur-Platte pipettiert. Die Zellkultur-Platten werden mindestens zwei Stunden bei 37 °C inkubiert, die überstehende Lösung abgesaugt und die Vertiefungen werden jeweils zweimal mit PBS gewaschen. Die beschichteten Zellkultur-Platten werden mit überstehendem PBS aufbewahrt und dieses erst direkt vor der Aufgabe der Zellen entfernt.

### Präparation der Zellen:

Enthaupteten Ratten wird die Wirbelsäule entnommen und diese direkt in einen kalten, d. h. in einem Eisbad befindlichen, HBSS-Puffer (Hank's buffered saline solution, Gibco Invitrogen GmbH, Karlsruhe, Deutschland) versetzt mit 1 Vol-% (Volumenprozent) einer AA-Lösung (Antibiotika/Antimykotika-Lösung, PAA, Pasching, Österreich) gelegt. Die Wirbelsäule wird längs durchtrennt und zusammen mit Fascien dem Wirbelkanal entnommen. Anschließend werden die Dorsalwurzelgänglien (DRGs; dorsai root ganglia) entnommen und wiederum in kaltem HBSS-Puffer versetzt mit 1 Vol-% einer AA-Lösung aufbewahrt. Die vollständig von Blutresten und Spinalnerven befreiten DRGs werden jeweils in 500 µL kalte Collagenase Typ 2 (PAA, Pasching, Österreich) überführt und 35 Minuten bei 37 °C inkubiert. Nach Zugabe von 2.5 Vol% Trypsin (PAA, Pasching, Österreich) wird weitere 10 Minuten bei 37 °C inkubiert. Nach der vollständigen Inkubation wird die Enzymlösung vorsichtig abpipettiert und die DRGs werden jeweils mit 500 µL Komplett-Medium versetzt.
Die DRGs werden jeweils mehrfach suspendiert, mittels einer Spritze durch Kanülen Nr. 1, Nr. 12 und Nr. 16 gezogen und in 50 mL Falcon-Röhrchen überführt und dieses mit Komplett-Medium auf 15 mL aufgefüllt. Der Inhalt jedes Falcon-Röhrchen wird jeweils durch einen 70 µm Falcon-Filtereinsatz filtriert und 10 Minuten bei 1200 Umdrehungen und Raumtemperatur zentrifugiert. Das resultierende Pellet wird jeweils in 250 µL Komplett-Medium aufgenommen und die Zellzahl ermittelt.

Die Anzahl der Zellen in der Suspension wird auf 3 mal 10⁵ pro mL eingestellt und jeweils 150 µL dieser Suspension in eine Vertiefung der wie vorstehend beschrieben beschichteten Zellkultur-Platten gegeben. Im Brutschrank werden die Platten bei 37 °C, 5 Vol-% CO₂ und 95 % relativer Luftfeuchtigkeit zwei bis drei Tage stehen gelassen.

Anschließend werden die Zellen mit 2 µM Fluo-4 und 0,01 Vol-% Pluronic F127 (Molecular Probes Europe BV, Leiden Niederlande) in HBSS-Puffer (Hank's buffered saline solution, Gibco Invitrogen GmbH, Karlsruhe, Deutschland) für 30 min bei 37 °C beladen, 3 x mit HBSS-Puffer gewaschen und nach einer weiteren Inkubation von 15 Minuten bei Raumtemperatur zur Ca²⁺-Messung im FLIPR-Assay eingesetzt. Die Ca²⁺-abhängige Fluoreszenz wird dabei vor und nach Zugabe von Substanzen gemessen (λₑₓ = 488 nm, λₑₘ = 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Verbindungen (10 µM) auf die Zellen pipettiert und der Ca²⁺-Einstrom mit der Kontrolle (Capsaicin 10 µM) verglichen. Daraus ergibt sich die Angabe in % Aktivierung bezogen auf das Ca²⁺-Signal nach Zugabe von 10 µM Capsaicin (CP). Nach 5 Minuten Inkubation werden 100 nM Capsaicin appliziert und ebenfalls der Einstrom von Ca²⁺ ermittelt.

Desensitisierende Agonisten und Antagonisten führen zu einer Unterdrückung des Ca2⁺-Einstroms. Es werden % Inhibierung im Vergleich zu der maximal erreichbaren Inhibierung mit 10 µM Capsaicin berechnet.

Es werden Dreifach-Bestimmungen (n=3) durchgeführt und diese in mindestens 3 unabhängigen Experimenten wiederholt (N=4).

Ausgehend von der prozentualen Verdrängung durch unterschiedliche Konzentrationen der zu prüfenden Verbindungen der allgemeinen Formel I werden IC₅₀ Hemmkonzentrationen berechnet, die eine 50-prozentige Verdrängung des Capsaicin bewirken.

### II. Formalin-Test an der Maus

Die Untersuchung zur Bestimmung der antinociceptiven Wirkung der erfindungsgemäßen substituierten zyklischen Harnstoff-Derivate wird im Formalin-Test an männlichen Mäusen (NMRI, 20 bis 30 g Körpergewicht, Iffa, Credo, Belgien) durchgeführt.

Im Formalin-Test werden gemäß D. Dubuisson et al., Pain 1977, 4, 161-174 die erste (frühe) Phase (0 bis 15 Minuten nach der Formalin-injektion) und die zweite (späte) Phase (15 bis 60 Minuten nach der Formalin-Injektion) unterschieden. Die frühe Phase stellt als direkte Reaktion auf die Formalin-Injektion ein Modell für Akutschmerz dar, während die späte Phase als Modell für persistierenden (chronischen) Schmerz angesehen wird (T. J. Coderre et al., Pain 1993, 52, 259-285). Die entsprechenden Literaturbeschreibungen werden hiermit als Referenz eingeführt und gelten als Teil der Offenbarung.

Die erfindungsgemäßen substituierten zyklischen Harnstoff-Derivate werden in der zweiten Phase des Formalin-Tests untersucht, um Aussagen über Substanzwirkungen auf chronisch/entzündlichen Schmerz zu erhalten.

In Abhängigkeit von der Applikationsart der erfindungsgemäßen Verbindungen wird der Applikationszeitpunkt der erfindungsgemäßen substituierten zyklischen Harnstoff-Derivate vor der Formalin-Injektion gewählt. Die intravenöse Applikation von 10 mg/kg Körpergewicht der Testsubstanzen erfolgt 5 Minuten vor der Formalin-Injektion. Diese erfolgt durch eine einmalige subkutane Formalin-Injektion (20 µL, 1 %-ige wäßrige Lösung) in die dorsale Seite der rechten Hinterpfote, so dass bei freibeweglichen Versuchstieren eine nociceptive Reaktion induziert wird, die sich in deutlichem Lecken und Beißen der betreffenden Pfote äußert.

Anschließend wird für einen Untersuchungszeitraum von drei Minuten in der zweiten (späten) Phase des Formalin-Tests (21 bis 24 Minuten nach der Formalin-Injektion) das nociceptive Verhalten durch Beobachtung der Tiere kontinuierlich erfasst. Die Quantifizierung des Schmerzverhaltens erfolgt durch Summation der Sekunden, in denen die Tiere in dem Untersuchungszeitraum ein Lecken und Beißen der betroffenen Pfote zeigen.

Der Vergleich erfolgt jeweils mit Kontrolltieren, die anstelle der erfindungsgemäßen Verbindungen Vehikel (0.9%-ige wäßrige Natriumchlorid-Lösung) vor Formalinapplikation erhalten.
Basierend auf der Quantifizierung des Schmerzverhaltens wird die Substanzwirkung im Formalin-Test als Änderung gegen die entsprechende Kontrolle in Prozent ermittelt.

Nach Injektion von Substanzen, die im Formalin-Test antinociceptiv wirksam sind, werden die beschriebenen Verhaltensweisen der Tiere, d. h. Lecken und Beißen, reduziert bzw. aufgehoben.

Im Folgenden wird die Erfindung mit Hilfe einiger Beispiele erläutert. Diese Erläuterungen sind lediglich beispielhaft und schränken den allgemeinen Erfindungsgedanken nicht ein.

### Beispiele:

Die Ausbeuten der hergestellten Verbindungen sind nicht optimiert.

Alle Temperaturen sind unkorrigiert.

### Abkürzungen

| | |
|---|---|
| abs. | absolutiert |
| DCM | Dichlormethan |
| DMF | Dimethylformamid |
| DMSO | Dimethylsulfoxid |
| EtOH | Ethanol |
| MeOH | Methanol |
| THF | Tetrahydrofuran |
| h | Stunden |
| min | Minuten |
| NMR | Kernresonanzspektroskopie |
| RT | Raumtemperatur |
| Smp. | Schmelzpunkt |

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, TCI, etc.) bezogen oder nach den für den Fachmann bekannten Methoden synthetisiert.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt. Die Mischungsverhältnisse von Lösungsmitteln, Laufmitteln oder für chromatographische Untersuchungen sind stets in Volumen/Volumen angegeben.

Die Analytik erfolgte durch Massenspektroskopie und NMR.

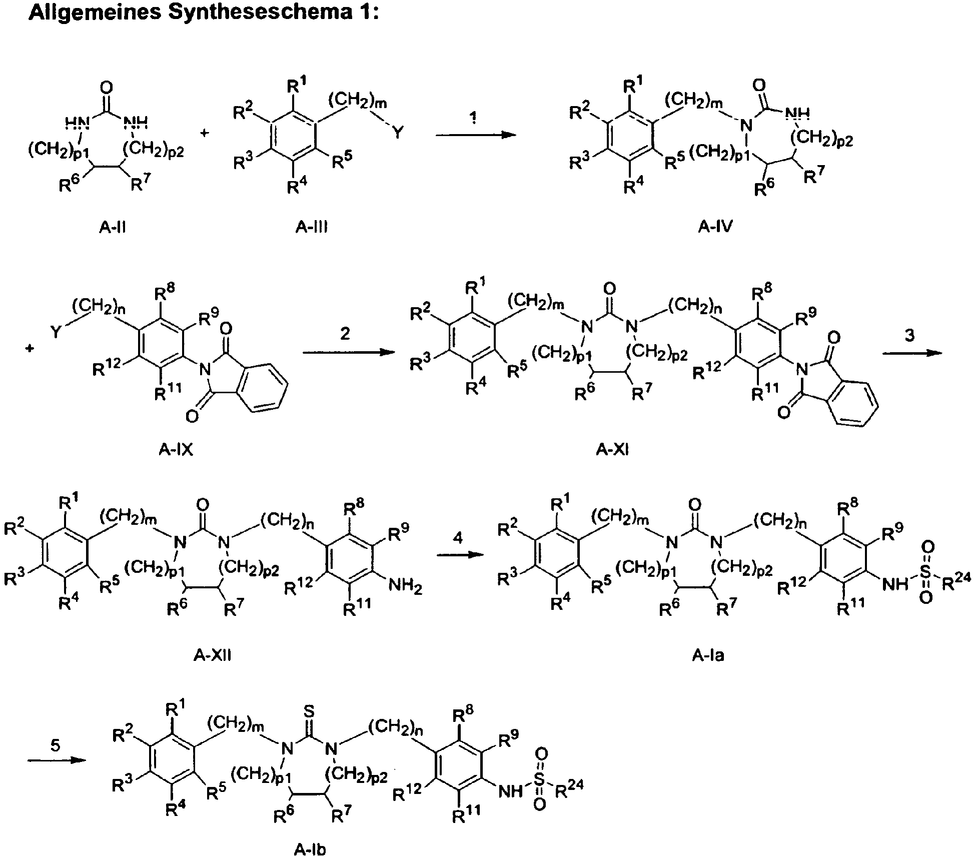

In Stufe 1 wurde die Umsetzung von Verbindungen der allgemeinen Formel A-II mit Verbindungen der allgemeinen Formel A-III unter einer Argonatmosphäre in organischen Lösungsmitteln oder Lösungsmittelgemischen, beispielsweise von Diethylether, THF, DCM, DMF, Acetonitril, Pyridin, DMSO und Toluol, unter Zusatz eines Metallhydridsalzes, beispielsweise unter Zusatz von Natriumhydrid oder Kaliumhydrid, bei Temperaturen von 20 bis 30 °C zu Verbindungen der allgemeinen Formel A-IV durchgeführt. Sofern das Monoalkylierungsprodukt der allgemeinen Formel A-IV als Gemisch mit dem entsprechenden Dialkylierungsprodukt erhalten wurde, wurde das Monoalkylierungsprodukt durch Umkristallisieren des Gemisches aus einem Lösungsmittelgemisch aus H₂O und EtOH erhalten.

In Stufe 2 wurde die Umsetzung von Verbindungen der allgemeinen Formel A-IV mit Verbindungen der allgemeinen Formel A-IX unter einer Argonatmosphäre in organischen Lösungsmitteln oder Lösungsmittelgemischen, beispielsweise von Diethylether, THF, DCM, DMF, Acetonitril, Pyridin, DMSO und Toluol, unter Zusatz eines Metallhydridsalzes, beispielsweise unter Zusatz von Natriumhydrid oder Kaliumhydrid, bei Temperaturen von 20 bis 30 °C, und anschließend bei Temperaturen von 80 bis 120 °C, zu Verbindungen der allgemeinen Formel A-XI durchgeführt.

In Stufe 3 wurde die Umsetzung von Verbindungen der allgemeinen Formel A-XI in organischen Lösungsmitteln oder Lösungsmittelgemischen, beispielsweise von MeOH, EtOH, Isopropanol und Wasser, unter Zusatz von Hydrazinhydrat, Phenylhydrazin oder Dimethylamin, bei Temperaturen von 20 bis 30 °C zu Verbindungen der allgemeinen Formel A-XII durchgeführt. Alternativ wurden Verbindungen der allgemeinen Formel A-XI in organischen Lösungsmitteln oder Lösungsmittelgemischen, beispielsweise von MeOH, EtOH und Isopropanol, unter Zusatz von Natriumborhydrid, bei Temperaturen von 20 bis 30 °C zu Verbindungen der allgemeinen Formel A-XII umgesetzt.

In Stufe 4 wurde die Umsetzung von Verbindungen der allgemeinen Formel A-XII mit Verbindungen der allgemeinen Formel R²⁴-S(=O)₂-Z in organischen Lösungsmitteln oder Lösungsmittelgemischen, beispielsweise von Aceton, Diethylether, THF, DCM, DMF, Acetonitril, Pyridin, DMSO und Toluol, ggf. unter Zusatz einer Base, beispielsweise von Pyridin, Triethylamin und Diisopropylethylamin, bei Temperaturen von 20 bis 30 °C zu Verbindungen der allgemeinen Formel A-Ia durchgeführt.

In Stufe 5 wurde die Umsetzung von Verbindungen der allgemeinen Formel A-Ia in organischen Lösungsmitteln oder Lösungsmittelgemischen, beispielsweise von Toluol, para-Xylol, ortho-Xylol, meta-Xylol, Acetonitril, DCM und DMF, unter Zusatz eines Dithiaphosphetans, beispielsweise von 2,4-Bis(4-methoxyphenyl)-1,3,2,4-dithiadiphosphetan-2,4-disulfide (Lawesson-Reagenz), oder unter Zusatz von Phosphorpentasulfid, bei Temperaturen von 50 bis 150 °C zu Verbindungen der allgemeinen Formel A-Ib durchgeführt.

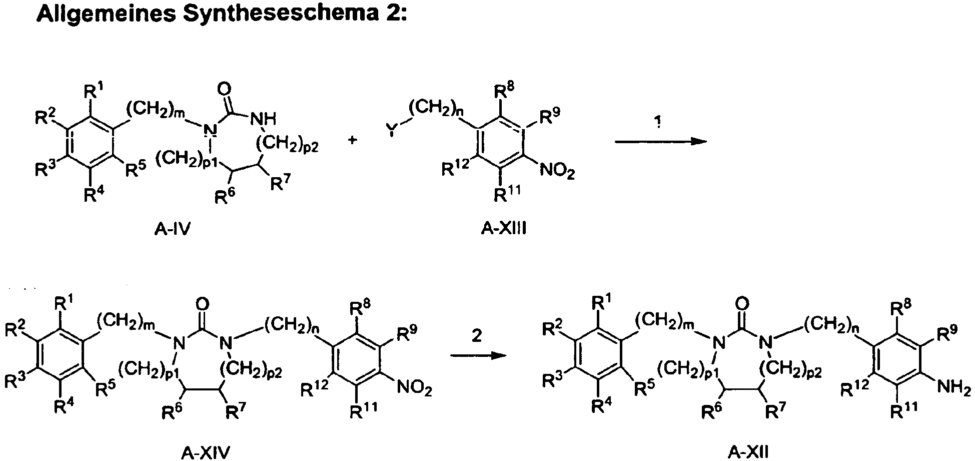

Die Verbindungen der allgemeinen Formel A-IV wurden wie im Allgemeinen Syntheseschema 1, Stufe 1, beschrieben erhalten.

In Stufe 1 wurde die Umsetzung von Verbindungen der allgemeinen Formel A-IV mit Verbindungen der allgemeinen Formel A-XII unter einer Argonatmosphäre in organischen Lösungsmitteln oder Lösungsmittelgemischen, beispielsweise von Diethylether, THF, DCM, DMF, Acetonitril, Pyridin, DMSO und Toluol, unter Zusatz eines Metallhydridsalzes, beispielsweise unter Zusatz von Natriumhydrid oder Kaliumhydrid, bei Temperaturen von 20 bis 30 °C, und anschließend bei Temperaturen von 80 bis 120 °C, zu Verbindungen der allgemeinen Formel A-XIV durchgeführt.

In Stufe 2 wurde die Umsetzung von Verbindungen der allgemeinen Formel A-XIV in organischen Lösungsmitteln oder Lösungsmittelgemischen, beispielsweise von MeOH, EtOH, Isopropanol, Aceton, Diethylether, THF, DCM, DMF, Acetonitril, Pyridin, DMSO und Toluol, in einer Wasserstoffatmosphäre, beispielsweise einer Wasserstoffatmosphäre mit einem Druck von 2 bar, bei Temperaturen von 20 bis 30 °C zu Verbindungen der allgemeinen Formel A-XII durchgeführt.

Die Verbindungen der allgemeinen Formel A-XII wurden, wie im Allgemeinen Syntheseschema 1, Stufe 4 und 5, beschrieben, weiter zu Verbindungen der allgemeinen Formel A-Ia bzw. A-Ib umgesetzt.

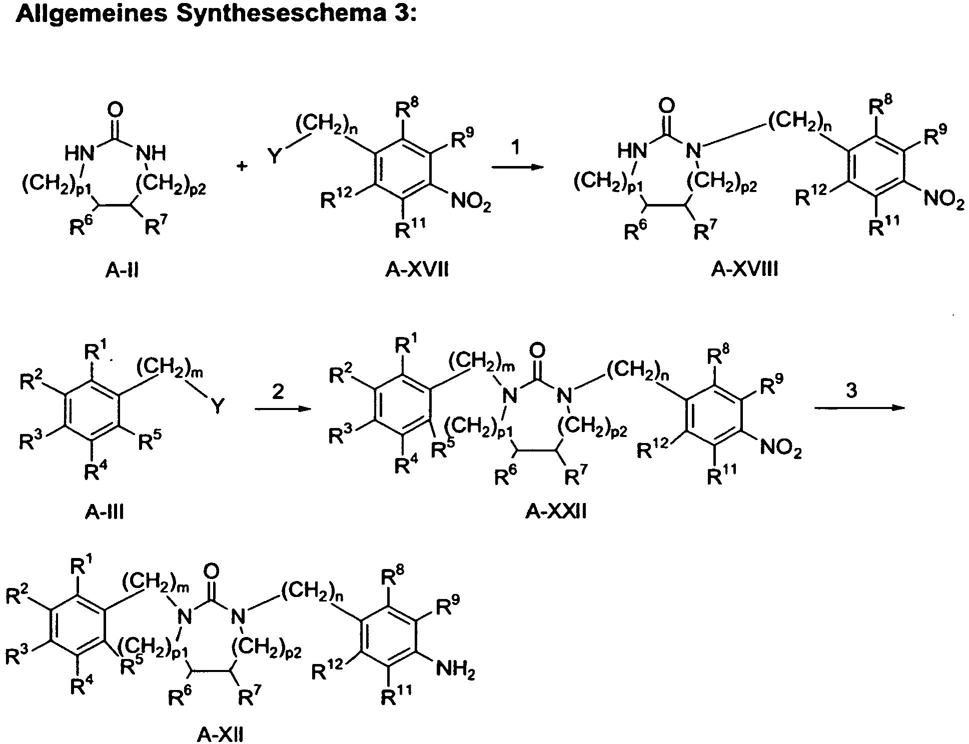

In Stufe 1 wurde die Umsetzung von Verbindungen der allgemeinen Formel A-II mit Verbindungen der allgemeinen Formel A-XVII in organischen Lösungsmitteln oder Lösungsmittelgemischen, beispielsweise von Diethylether, THF, DCM, DMF, Acetonitril, Pyridin, DMSO und Toluol, unter Zusatz eines Alkalimetallcarbonatsalzes, beispielsweise unter Zusatz von Kaliumcarbonat oder Natriumcarbonat, und unter Zusatz eines Alkalimetalliodides, beispielsweise unter Zusatz von Kaliumiodid oder Natriumiodid, bei Temperaturen von 70 bis 120 °C zu Verbindungen der allgemeinen Formel A-XVIII durchgeführt. Sofern das Monoalkylierungsprodukt der allgemeinen Formel A-XVIII als Gemisch mit dem entsprechenden Dialkylierungsprodukt erhalten wurde, wurde das Monoalkylierungsprodukt durch Säulenchromatographie an SiO₂ mit Gemischen aus Cyclohexan und EtOAc als Laufmittel als die weniger polare Verbindung erhalten.

In Stufe 2 wurde die Umsetzung von Verbindungen der allgemeinen Formel A-XVIII mit Verbindungen der allgemeinen Formel A-III in organischen Lösungsmitteln oder Lösungsmittelgemischen, beispielsweise von Diethylether, THF, DCM, DMF, Acetonitril, Pyridin, DMSO und Toluol, unter Zusatz eines Alkalimetallcarbonatsalzes, beispielsweise unter Zusatz von Kaliumcarbonat oder Natriumcarbonat, und unter Zusatz eines Alkalimetalliodides, beispielsweise unter Zusatz von Kaliumiodid oder Natriumiodid, bei Temperaturen von 70 bis 120 °C zu Verbindungen der allgemeinen Formel A-XXII durchgeführt.

In Stufe 3 wurde die Umsetzung von Verbindungen der allgemeinen Formel A-XXII in organischen Lösungsmitteln oder Lösungsmittelgemischen, beispielsweise von MeOH, EtOH, Isopropanol, Aceton, Diethylether, THF, DCM, DMF, Acetonitril, Pyridin, DMSO und Toluol, in einer Wasserstoffatmosphäre, beispielsweise einer Wasserstoffatmosphäre mit einem Druck von 2 bar, bei Temperaturen von 20 bis 30 °C zu Verbindungen der allgemeinen Formel A-XII durchgeführt.

Die Verbindungen der allgemeinen Formel A-XII wurden, wie im Allgemeinen Syntheseschema 1, Stufe 4 und 5, beschrieben, weiter zu Verbindungen der allgemeinen Formel A-Ia bzw. A-Ib umgesetzt.

### I. Synthese von Verbindungen der allgemeinen Formel II mit X = N-C≡N

### a. Synthese von 2-Cyanoimino-imidazolidin

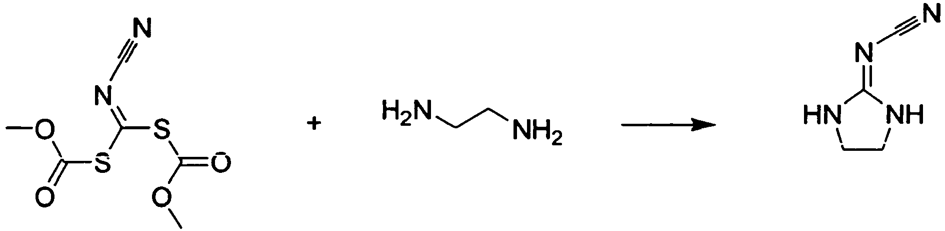

Ethylendiamin (200 mL) wurde in Chloroform (150 mL) gegeben und unter Rühren so mit einer Lösung von N-Cyanimino-dithiokohlensäuredimethylester (50 g, 0.34 mol) in Chloroform versetzt, dass die Temperatur unterhalb von 45 °C gehalten wurde. Die Reaktionsmischung wurde anschließend 30 Minuten bei RT gerührt, die flüchtigen Bestandteile im Vakuum entfernt und der Rückstand aus EtOH umkristallisiert. Es wurden 28.1 g (256 mmol, 75% der Theorie) der gewünschten Verbindung 2-Cyanoimino-imidazolidin erhalten.

### b. Synthese von 2-Cyanimino-hexahydropyrimidin

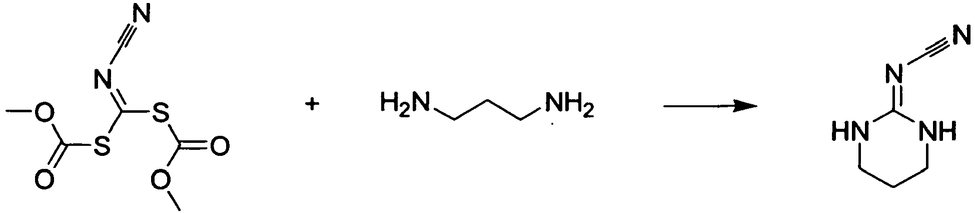

1,3-Diamino-propan (250 mL) wurde in n-Propanol (150 mL) gegeben und unter Rühren so mit einer Lösung von N-Cyanimino-dithiokohlensäuredimethylester (50 g, 0.34 mol) in n-Propanol versetzt, dass die Temperatur unterhalb von 45 °C gehalten wurde. Die Reaktionsmischung wurde anschließend 2.5 h zum Rückfluß erhitzt, die flüchtigen Bestandteile im Vakuum entfernt und der Rückstand aus EtOH umkristallisiert. Es wurden 11.0 g (88.5 mmol, 26% der Theorie) der gewünschten Verbindung 2-Cyanimino-hexahydropyrimidin erhalten.

### II. Synthese der Beispielverbindungen 1 und 4:

### N-[4-[3-(4-tert-Butyl-benzyl)-2-thioxo-imidazolidin-ylmethyl]-phenyl]-methansulfonamid (1) und N-{4-[3-(4-tert-Butylbenzyl)-2-oxoimidazolidin-1-ylmethyl]phenyl}-methansulfonamid (4)

### a. Synthese von 2-p-Tolylisoindol-1,3-dion (A)

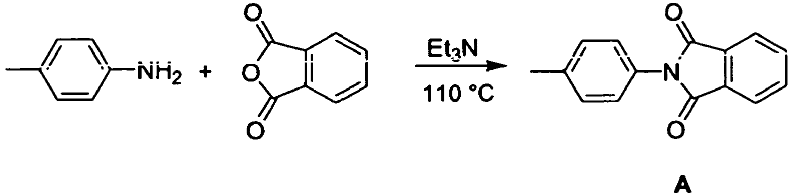

Eine Mischung von Phthalsäureanhydrid (7.4 g, 50.0 mmol) und *p*-Toluidin (53.5 g, 50.0 mmol) wurde mit Toluol (200 mL) und Triethylamin (1 mL, 7.2 mmol) versetzt und 3 h am Wasserabscheider zum Sieden erhitzt. Die entstandene Lösung wurde in der Hitze über eine Fritte mit Kieselgur (0,5 - 1 cm Schichtdicke) filtriert. Der Rückstand wurde mit heißem Toluol gewaschen (2 x 50 mL). Die erhaltene Lösung wurde auf ca. 100 mL eingeengt, wobei es zur Kristallbildung kam. Der ausgefallene Feststoff (7.8 g, 66 % der Theorie) stellt das gewünschte Produkt 2-p-Tolylisoindol-1,3-dion (**A**) dar und weist einen der Literatur [Kaupp, G. et al. Tetrahedron 2000, 56, 6899-6912] entsprechenden Schmelzpunkt (201-203 °C) auf.

### b. Synthese von 2-(4-Brommethylphenyl)isoindol-1,3-dion (B)

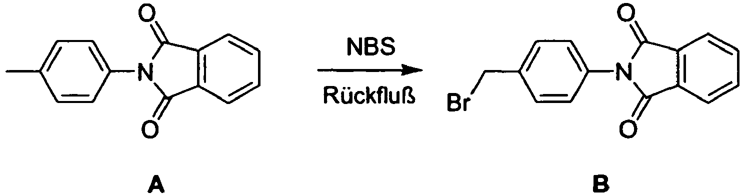

Eine Mischung aus 2-p-Tolylisoindol-1,3-dion (**A**) (4.0 g, 17 mmol) und N-Bromsuccinimid (3.0 g, 17 mmol) wurde mit Tetrachlorkohlenstoff (70 mL) und Benzoylperoxid (8 mL) versetzt und 2 h im Halogenstrahlerlicht zum Sieden erhitzt. Die erhaltene Reaktionslösung wurde eingeengt. Der Rückstand wurde mit Chloroform (300 mL) 15 min in der Hitze ausgerührt und anschließend das Gemisch zur Abtrennung des entstandenen Feststoffs heiß filtriert. Das Filtrat wurde im Vakuum eingeengt und der Rückstand wurde mittels Säulenchromatographie (SiO₂ (150 g) mit Cyclohexan/EtOAc 4:1 (2000 mL) als Eluent) gereinigt. Das Bromid 2-(4-Brommethytphenyl)isoindol-1,3-dion (**B**) (4.1 g, 77 %) wurde als weißer Feststoff mit einem Schmelzpunkt von 191-199 °C isoliert.

### c. Synthese von 1-(4-tert-Butylbenzyl)imidazolidin-2-on (C)

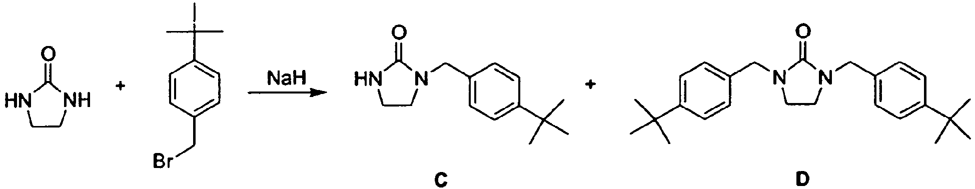

Zu einer Lösung von Imidazolidin-2-on (2.58 g, 30 mmol) in abs. DMF (40 mL) wurde unter Argon portionsweise Natriumhydrid (ca. 60-%ige Suspension in Öl, 1.2 g, ca. 30 mmol) innerhalb von 10 min gegeben. Die Reaktionsmischung wurde 90 min bei RT gerührt und anschließend mit einer Lösung von 4-tert-Butylbenzylbromid (3.7 mL, 20.1 mmol) in abs. DMF (3 mL) versetzt. Die Reaktionsmischung wurde 3 h bei RT gerührt und danach auf eine Mischung aus H₂O (100 mL) und 2N Salzsäure-Lösung in H₂O (10 mL) gegeben. Das entstandene Gemisch wurde 30 min gerührt, die entstandenen Kristalle wurden mit Hilfe einer Fritte abgetrennt und mit H₂O (3 x 30 mL) gewaschen. Der isolierte Feststoff wurde in einem Gemisch aus H₂O (100 mL) und EtOH (120 mL) umkristallisiert. Bei der dabei anfallenden Substanz (1.4 g, Smp.: 122-127 °C) handelt es sich um die Verbindung **D**.
Die verbliebene Mutterlauge wurde im Vakuum eingeengt. Die dabei ausfallenden Kristalle (1.87 g, 40 % der Theorie, Smp.: 161-165 °C) erwiesen sich als die gewünschte Verbindung 1-(4-tert-Butylbenzyl)imidazolidin-2-on (**C**).

### d. Synthese von 2-{4-[3-(4-tert-Butytbenzyl)-2-oxoimidazolidin-1-ylmethyljphenyl}isoindol-1,3-dion (E)

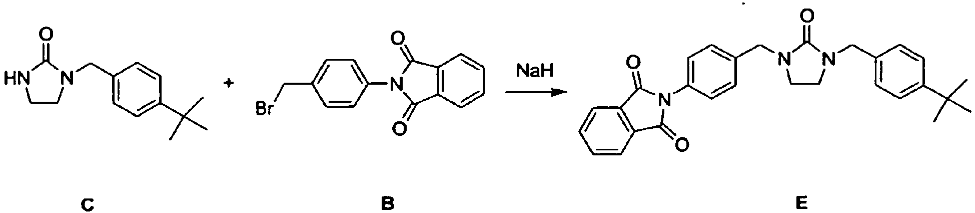

Zu einer Lösung von 1-(4-tert-Butylbenzyl)imidazolidin-2-on (C) (730 mg, 3.15 mmol) in abs. DMF (15 mL) wurde unter Argon innerhalb von 10 min portionsweise Natriumhydrid (ca. 60-%ige Suspension in Öl, 140 mg, ca. 3.5 mmol) gegeben. Die Reaktionsmischung wurde 60 min bei RT gerührt und anschließend innerhalb von 60 min mit einer Lösung von 2-(4-Brommethylphenyl)isoindol-1,3-dion (**B**) (1 g, 3.16 mmol) in abs. DMF (20 mL) versetzt. Die Reaktionsmischung wurde 3 h bei RT gerührt, anschließend 1 h auf 100 °C erwärmt, abgekühlt und auf eine Mischung aus Eiswasser (300 mL) und 2N Salzsäure-Lösung in H₂O (20 mL) gegeben. Die entstandene Mischung wurde 30 min gerührt und anschließend mit DCM (4 x 20 mL) extrahiert. Die vereinigten organischen Phasen wurden über Na₂SO₄ getrocknet und das Lösungsmittel im Vakuum entfernt. Nach säulenchromatographischer Reinigung des Rückstands (SiO₂, Cyclohexan/EtOAc 1:1) wurden das gewünschte Produkt 2-{4-[3-(4-tert-Butylbenzyl)-2-oxoimidazolidin-1-ylmethyl]phenyl}isoindol-1,3-dion (**E**) als glasiger Feststoff (680 mg, 46 % der Theorie) erhalten.

### e. Synthese von 1-(4-Aminobenzyl)-3-(4-tert-butylbenzyl)imidazolidin-2-on (F)

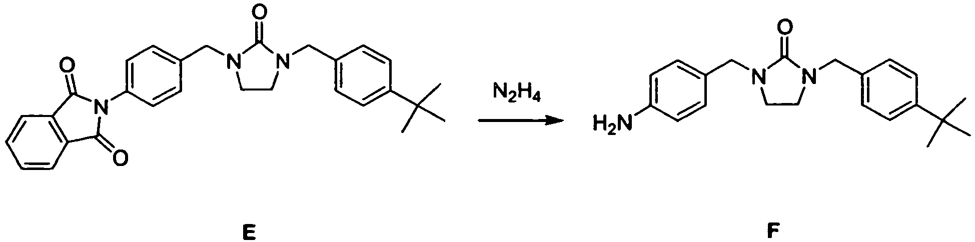

Zu einer Lösung von 2-{4-[3-(4-*tert*-Butylbenzyl)-2-oxoimidazolidin-1-ylmethyl]phenyl}-isoindol-1,3-dion (**E**) (654 mg, 1.4 mmol) in MeOH (30 mL) wurde unter Rühren bei RT Hydrazinhydrat als 0.8 M Lösung in MeOH (10 mL, 8 mmol) gegeben. Die Reaktionsmischung wurde 90 min bei RT gerührt und dann mit H₂O (50 mL) versetzt. Das Lösungsmittel wurde im Vakuum entfernt und der Rückstand wurde mit EtOAc (3 x 20 mL) extrahiert. Die vereinigten organischen Phasen wurden über Na₂SO₄ getrocknet und anschließend wiederum das Lösungsmittel im Vakuum entfernt. Es wurden 430 g (91 % der Theorie) des gewünschten Produkts 1-(4-Aminobenzyl)-3-(4-*tert*-butylbenzyl)imidazolidin-2-on (F) erhalten.

### f. Synthese von N-{4-[3-(4-tert-Butylbenzyl)-2-oxoimidazolidin-1-ylmethyl]phenyl}methansulfonamid (4)

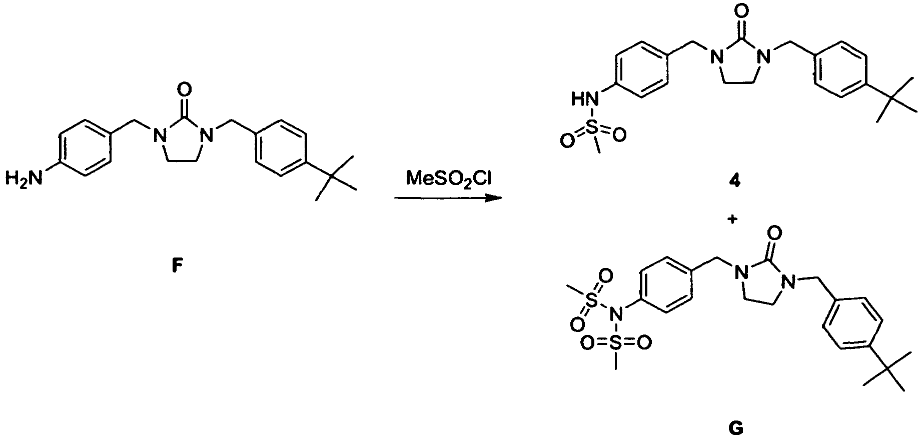

Zu einer Lösung von 1-(4-Aminobenzyl)-3-(4-tert-butylbenzyl)imidazolidin-2-on (F) (410 mg, 1.22 mmol) und Triethylamin (0.2 mL, 1.44 mmol) in abs. THF wurde innerhalb von 20 min eine Lösung von Methansulfonylchlorid (0.12 mL, 1.56 mmol) in 0.8 mL abs. THF gegeben. Die Reaktionsmischung wurde 15 h bei RT gerührt, mit ges. NaHCO₃-Lösung (10 mL) versetzt und 30 min gerührt. Die Mischung wurde mit H₂O (30 mL) versetzt und mit EtOAc (3 x 20 mL) extrahiert. Die vereinigten organischen Phasen wurden mit ges. NaCl-Lösung gewaschen, über Na₂SO₄ getrocknet und das Lösungsmittel im Vakuum entfernt. Nach säulenchromatographischer Reinigung des Rückstands (SiO₂, EtOAc/Cyclohexan 2 :1) wurden das gewünschte Produkt *N*-{4-[3-(4-*tert*-Butylbenzyl)-2-oxoimidazolidin-l-ytmethyl]phenyl}methansulfonamid **(4)** (70 mg, 14 % der Theorie, Smp.: 166-168 °C) und *N*-{4-[3-(4-*tert*-Butylbenzyl)-2-oxoimidazolidin-1-ylmethyl]phenyl}-*N,N*-bis-(methansulfonyl)imid **(G)** (160 mg, Smp.: 184-187 °C) erhalten.

### g. Synthese von N-[4-[3-(4-tert-Butyl-benzyl)-2-thioxo-imidazolidin-ylmethyl]-phenyl]-methansulfonamid (1)

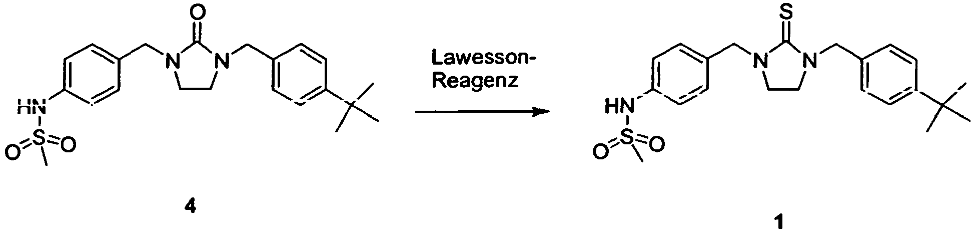

Zu einer Lösung von *N*-{4-[3-(4-tert-Butylbenzyl)-2-oxoimidazolidin-1-ylmethyl]phenyl}methansultonamid **(4)** (1008 mg, 2.63 mmol) in abs. Toluol (30 mL) wurde Lawesson-Reagenz (2,4-Bis(4-methoxyphenyl)-1,3,2,4-dithiadiphosphetan-2,4-disulfide) (640 mg, 1.58 mmol) gegeben. Das Reaktionsgemisch wurde 5 h zum Sieden erhitzt. Anschließend wurde die Reaktionsmischung über Nacht gerührt. Die Reaktionsmischung wurde mit Wasser (40 mL) versetzt und mit Chloroform (3 x 30 mL) extrahiert. Die vereinigten organischen Phasen wurden mit H₂O (2 x 20 mL) gewaschen, über Na₂SO₄ getrocknet und das Lösungsmittel wurde im Vakuum entfernt. Nach Reinigung des Rückstands mittels Säulenchromatographie (SiO₂ (150 g); 1000 mL Cyclohexan/EtOAc 4:1) wurden das gewünschte Produkt N-[4-[3-(4-tert-Butyl-benzyl)-2-thioxo-imidazolidin-ylmethyl]-phenyl]-methansulfonamid (1) (545 mg, 52 % der Theorie) erhalten.

### III. Synthese der Beispielverbindung 3 und 5:

### N-[4-[3-(4-tert-Butyl-benzyl)-2-thioxo-tetrahydro-pyrimidin-1-ylmethyl]-phenyl]-methansulfonamid (3) und N-[4-[3-(4-tert-Butyl-benzyl)-2-oxo-tetrahydro-pyrimidin-1-ylmethyl]-phenyl]-methansulfonamid (5)

### a. Synthese von 1-(4-Nitrobenzyl)tetrahydropyrimidin-2-on (H)

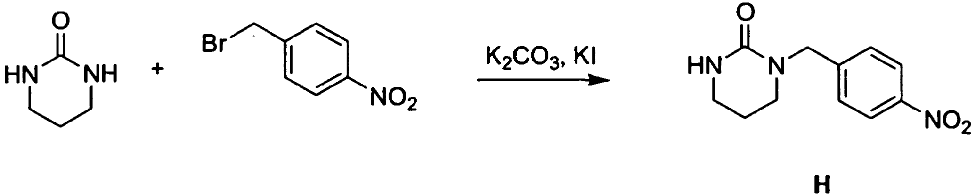

Zu einer Lösung 3,4,5,6-Tetrahydro-2(1H)-pyrimidinon (4 g, 0.04 mol) in abs. DMF (50 mL) wurden bei RT Kaliumcarbonat (5.5 g, ca. 0.04 mol), Kaliumiodid (3.3 g, 0.02 mol) und 4-Nitrobenzylbromid (8.6 g, 0.04 mol) gegeben. Die Reaktionsmischung wurde 2 h bei 100 °C gerührt. Nach Abkühlen der Reaktionsmischung wurde diese auf Eiswasser (500 mL) gegeben. Der ausgefallene Feststoff wurde abgesaugt und mit wenig Aceton (4 x 10 mL) gewaschen. Das gewünschte Produkt 1-(4-Nitrobenzyl)tetrahydropyrimidin-2-on **(H)** wurde als gelber Feststoff (3.5 g, 40 % der Theorie) mit einem Schmelzpunkt von 150-162 °C erhalten.

### b. Synthese von 1-(4-tert-Butylbenzyl)-3-(4-nitrobenzyl)tetrahydropyrimidin-2-on (K)

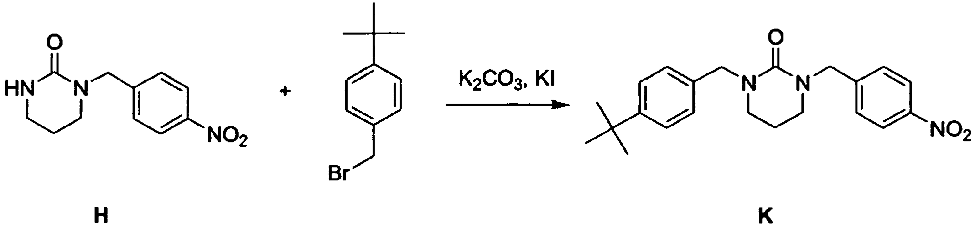

Zu einer Lösung von 1-(4-Nitrobenzyl)tetrahydropyrimidin-2-on **(H)** (1.94 g, 8.15 mmol) in abs. DMF (30 mL) wurden bei RT Kaliumcarbonat (1.12 g, ca. 8.15 mmol), Kaliumiodid (0.7 g, 4 mmol) und 4-*tert*-Butylbenzylbromid (1.85 g, 8.15 mmol) gegeben. Die Reaktionsmischung wurde 3 h bei 100 °C gerührt. Nach Abkühlen der Reaktionsmischung wurde diese in Eiswasser (350 mL) gegossen. Das Gemisch wurde mit Dichlormethan (3 x 40 mL) extrahiert. Die organische Phase wurde mit H₂O (2 x 30 mL) gewaschen, über Na₂SO₄ getrocknet und das Lösungsmittel im Vakuum entfernt. Nach säulenchromatographischer Reinigung des Rückstands (SiO₂ (150 g), Cyclohexan/EtOAc 1:1 (2000 mL) als Eluent) wurde die gewünschte Verbindung 1-(4-*tert*-Butylbenzyl)-3-(4-nitrobenzyl)tetrahydropyrimidin-2-on **(K)** (786 mg, 25 % der Theorie) als gelbes Öl erhalten.

### c. Synthese von 1-(4-Aminobenzyl)-3-(4-tert-butylbenzyl)tetrahydropyrimidin-2-on (L)

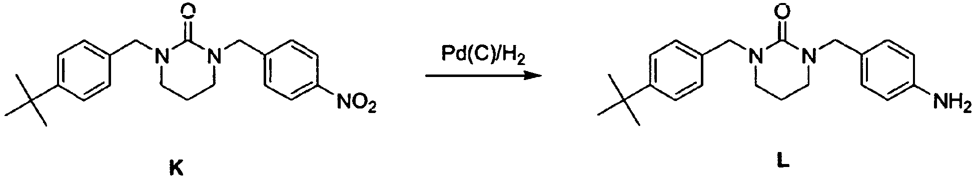

Zu einer Lösung von 1-(4-*tert*-Butylbenzyl)-3-(4-nitrobenzyl)tetrahydropyrimidin-2-on **(K)** (500 mg, 1.31 mmol) in abs. MeOH (50 mL) wurde Palladium auf Kohle (5 %, 600 mg) gegeben. Die Reaktionsmischung wurde 20 min bei RT einem Wasserstoffdruck von 2 bar ausgesetzt. Der Katalysator wurde durch Filtration über Celite abgetrennt und das Filtrat wurde im Vakuum eingeengt. Das gewünschte Produkt 1-(4-Aminobenzyl)-3-(4-*tert*-butylbenzyl)tetrahydropyrimidin-2-on (**L**) wurde als farbloses Öl (364 mg, 79 % der Theorie) erhalten.

### d. Synthese von N-[4-[3-(4-tert-Butyl-benzyl)-2-oxo-tetrahydro-pyrimidin-1-ylmethyl]-phenyl]-methansulfonamid (5)

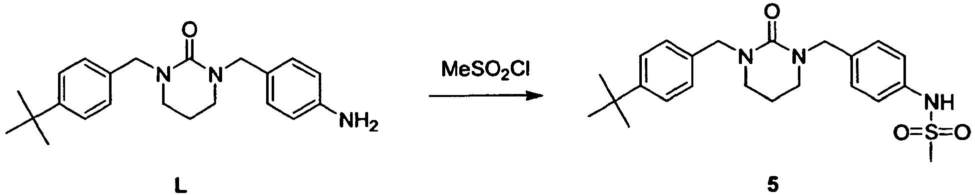

Zu einer Lösung von 1-(4-Aminobenzyl)-3-(4-*tert*-butylbenzyl)tetrahydropyrimidin-2-on (**L**) (438 mg, 1.24 mmol) und Triethylamin (0.22 mL, 1.6 mmol) in abs. THF (50 mL) wurde innerhalb von 15 min eine Lösung von Methansulfonylchlorid (0.11 mL, 1.48 mmol) in 5 mL abs. THF gegeben. Die Reaktionsmischung wurde 48 h bei RT gerührt, mit ges. NaHCO₃-Lösung (20 mL) versetzt und 30 min gerührt. Die Reaktionsmischung wurde mit H₂O (30 mL) versetzt und mit EtOAc (3 x 30 mL) extrahiert. Die vereinigten organischen Phasen wurden mit ges. NaCI-Lösung (30 mL) gewaschen, über Na₂SO₄ getrocknet und das Lösungsmittel wurde im Vakuum entfernt. Der Rückstand wurde mittels Säulenchromatographie (SiO₂ (50 g) mit Cyclohexan/EtOAc 1:1 (500 mL) als Eluent) gereinigt. Das gewünschte Produkt N-[4-[3-(4-tert-Buty)-benzyl)-2-oxo-tetrahydro-pyrimidin-1-ylmethyl]-phenyl-methansulfonamid **(5)** wurde als weißer Feststoff (398 mg, 75 % der Theorie) mit einem Schmelzpunkt von 132-135 °C erhalten.

Die Verbindung N-[4-[3-(4-tert-Butyl-benzyl)-2-thioxo-tetrahydro-pyrimidin-1-ylmethyl]-phenyl]-methansulfonamid **(3)** wurde analog zu der unter II g. beschriebenen Reaktion aus N-[4-[3-(4-tert-Butyl-benzyl)-2-oxo-tetrahydro-pyrimidin-1-ylmethyl]-phenyl-methansulfonamid **(5)** erhalten.

### IV. Synthese der Beispielverbindungen 2 und 6:

### N-[4-[3-(4-tert-Butyl-benzyl)-2-thioxo-2,3-dihydro-benzoimidazol-1-ylmethyl]-phenyl]-methansulfonamid (2) und N-[4-[3-(4-tert-Butyl-benzyl)-2-oxo-2,3-dihydro-benzoimidazol-1-ylmethylj-phenyl]-methansulfonamid (6)

### a. Synthese von 1-(4-tert-Butylbenzyl)-1,3-dihydrobenzimidazol-2-on (N)

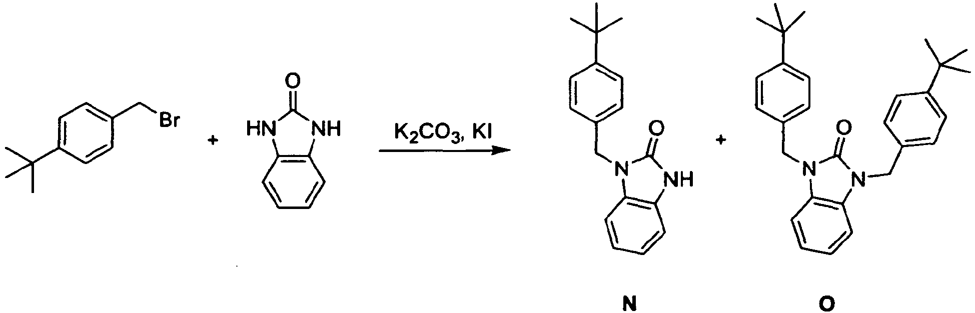

Zu einer Lösung von Benzimidazolinon (1.5 g, 11.2 mmol) in abs. DMF (25 mL) wurde unter Argon 4-tert-Butyl-benzylbromid (1.8 mL, 9.8 mmol), Kaliumcarbonat (2.76 g, 20 mmol) und eine katalytische Menge Kaliumiodid gegeben. Die Reaktionsmischung wurde 2 h auf 75 °C erhitzt. Nach Abkühlen wurde die Reaktionsmischung in eine Lösung von H₂O (200 mL) und 2N Salzsäure-Lösung in H₂O (10 mL) gegossen. Das entstandene Gemisch wurde mit EtOAc (5 x 40 mL) extrahiert, die vereinigten organischen Phasen wurden mit ges. NaCl-Lösung (2 x 30 mL) gewaschen, über Na₂SO₄ getrocknet und das Lösungsmittel wurde im Vakuum entfernt. Der Rückstand wurde in Chlorofom (50 mL) aufgenommen und der erhaltene Feststoff wurde abfiltriert. Das Filtrat wurde im Vakuum eingeengt. Der Rückstand wurde mittels Säulenchromatographie (SiO₂, Cyclohexan/EtOAc 2:1) gereinigt und es wurden 1,3-Bis-(4-tert-butylbenzyl)-1,3-dihydrobenzimidazol-2-on (**O**) (0.9 g. Smp.: 164-168 °C) und das gewünschte Produkt 1-(4-tert-Butylbenzyl)-1,3-dihydrobenzimidazol-2-on (**N**) (615 mg, 22 % der Theorie, Smp.: 179-180 °C) als die stärker polare Substanz erhalten.

### b. Synthese von 1-(4-tert-Butylbenzyl)-3-(4-nitrobenzyl)-1,3-dihydrobenzimidazol-2-on (P)

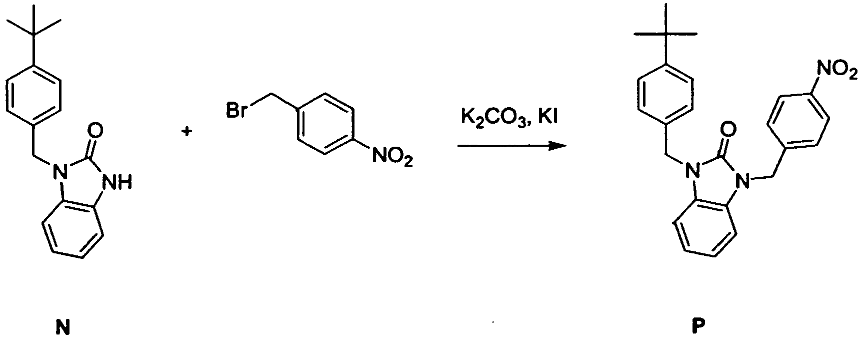

Zu einer Lösung von Verbindung **N** (1.12 g, 4.0 mmol) in abs. DMF (30 mL) wurde unter Argon 4-Nitro-benzylbromid (864 mg, 4.0 mmol), Kaliumcarbonat (1.14 g, 8.30 mmol) und eine katalytische Menge Kaliumiodid gegeben. Die Reaktionsmischung wurde 4 h auf 75 °C erhitzt. Nach Abkühlen wurde die Reaktionsmischung in eine Mischung aus Eis (300 g) und 2N Salzsäure-Lösung in H₂O (20 mL) gegossen und die Reaktionsmischung eine Stunde gerührt. Der entstandene Feststoff wurde über eine Fritte abgetrennt und im Vakuum über Calciumchlorid getrocknet. Das gewünschte Produkt 1-(4-tert-Butylbenzyl)-3-(4-nitrobenzyl)-1,3-dihydrobenzimidazol-2-on **(P)** wurde in einer für synthetische Zwecke ausreichenden reinen Form mit einer Ausbeute von 1.48 g (89 % der Theorie) erhalten.

### c. Synthese von 1-(4-Aminobenzyl)-3-(4-tert-butylbenzyl)-1,3-dihydrobenzimidazol-2-on (Q)

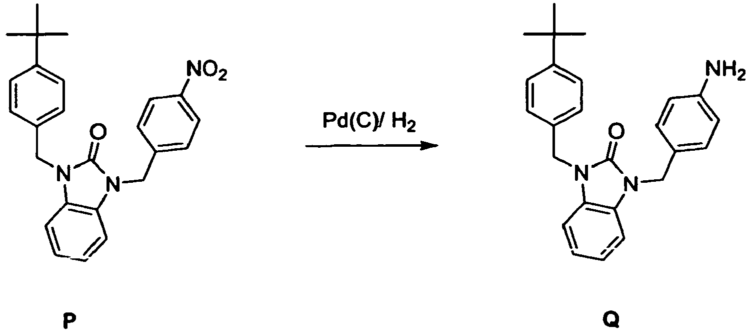

Zu einer Lösung von 1-(4-tert-Butylbenzyl)-3-(4-nitrobenzyl)-1,3-dihydrobenzimidazol-2-on (**P**) (1.13 g, 2.73 mmol) in abs. MeOH (60 mL) wurde Palladium auf Kohle (5 %, 650 mg) gegeben. Die Reaktionsmischung wurde 20 min bei RT einem Wasserstoffdruck von 3 bar ausgesetzt. Der Katalysator wurde durch Filtration über einen Filter abgetrennt und das Filtrat wurde im Vakuum eingeengt. Der Rückstand wurde in EtOAc (10 mL) aufgenommen und über eine Fritte mit Kieselgur (Schichtdicke 1 cm) filtriert. Die Fritte wurde mit Ethylacetat (4 x 20 mL) gewaschen. Das gewünschte Produkt 1-(4-Aminobenzyl)-3-(4-tert-butylbenzyl)-1,3-dihydrobenzimidazol-2-on (**Q**) wurde in einer Ausbeute von 960 mg (91 % der Theorie) erhalten. Das entsprechende Hydrochlorid wurde durch Umsetzung von 1-(4-Aminobenzyl)-3-(4-tert-butylbenzyl)-1,3-dihydrobenzimidazol-2-on (Q) mit Chlortrimethylsilan (1 mL, 7.9 mmol) in DCM (50 mL) erhalten. Das Reaktionsgemisch wurde 17 h ohne Feuchtigkeitsausschluß gerührt, der entstandene Feststoff mit Hilfe einer Fritte abgetrennt und anschließend getrocknet. Das Hydrochlorid wurde als Feststoff (750 mg, 68 % der Theorie, Smp.: 198-219 °C) erhalten.

### d. Synthese von N-[4-[3-(4-tert-Butyl-benzyl)-2-oxo-2,3-dihydro-benzoimidazol-1-ylmethyl]-phenyl]-methansulfonamid (6)

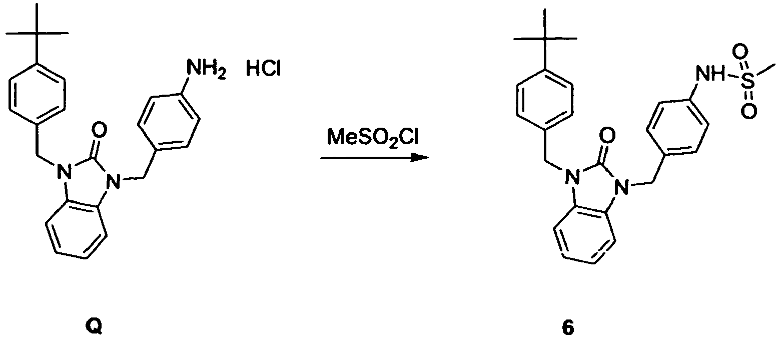

Zu einer Suspension des Hydrochlorids von 1-(4-Aminobenzyl)-3-(4-tert-butylbenzyl)-1,3-dihydrobenzimidazol-2-on (**Q**) (717 mg, 1.7 mmol) in abs. THF (20 mL) wurde Pyridin (0.3 mL, 3.72 mmol) gegeben. Die Reaktionsmischung wurde 10 min bei RT gerührt, Methansulfonylchlorid (0.13 mL, 1.7 mmol, gelöst in abs. THF (1 mL)) innerhalb von 5 min zugegeben und das Reaktionsgemisch wiederum 17 h bei RT gerührt. Anschließend wurde die Reaktionsmischung mit H₂O (10 mL) und nach 10 min noch einmal mit H₂O (100 mL) versetzt. Nach einer Stunde wurde der ausgefallene Feststoff über eine Fritte abgetrennt und anschließend im Vakuum getrocknet. Das gewünschte Produkt N-[4-[3-(4-tert-Butyl-benzyl)-2-oxo-2,3-dihydro-benzoimidazol-1-ylmethyl]-phenyl]-methansulfonamid (**6**) wurde in einer Ausbeute von 680 mg (86 % der Theorie, Smp.: 228-230 °C) erhalten.

### e. Synthese von N-[4-[3-(4-tert-Butyl-benzyl)-2-thioxo-2,3-dihydro-benzoimidazol-1-ylmethyl]-phenyl]-methansulfonamid (2)

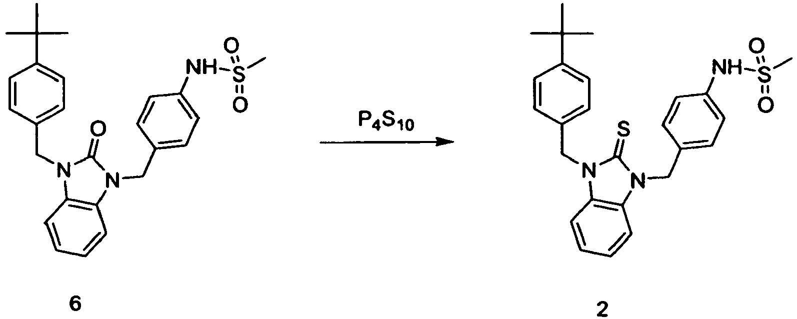

Eine Suspension von N-[4-[3-(4-tert-Butyl-benzyl)-2-oxo-2,3-dihydro-benzoimidazol-1-ylmethyl]-phenyl]-methansulfonamid (**6**) (235 mg, 0.51 mmol) in para-Xylol (20 mL) wurde unter Argon mit Phosphorpentasulfid (300 mg, 0.67 mmol) versetzt. Das Reaktionsgemisch wurde 5 h zum Sieden erhitzt. Nach dem Abkühlen wurde die Reaktionsmischung mit Wasser (1 mL) und anschließend mit 2N NaOH-Lösung in Wasser (10 mL) versetzt und 30 min bei RT gerührt. Die Reaktionsmischung wurde mit wäßriger NaCl-Lösung versetzt und das entstandene Gemisch wurde mit Ethylacetat (4 x 20 mL) extrahiert. Die vereinigten organischen Phasen wurden über Na₂SO₄ getrocknet und das Lösungsmittel im Vakuum entfernt. Nach Reinigung des Rückstands mittels Säulenchromatographie (SiO₂; Cyclohexan/EtOAc 7:3) wurde das gewünschte Produkt N-[4-[3-(4-tert-Butyl-benzyl)-2-thioxo-2,3-dihydro-benzoimidazol-1-ylmethyl]-phenyl]-methansulfonamid (2) (99 mg, 40 % der Theorie, Smp.: 176-178 °C) erhalten.

### V. Synthese der Beispielverbindungen 7 und 8:

### N-[4-[3-(4-Trifluormethoxybenzyl)-2-oxo-2,3-dihydrobenzimidazol-1-ylmethyl]-phenyl]-methansulfonamid (7) und N-[4-[3-(4-Trifluormethoxybenzyl)-2-thioxo-2,3-dihydrobenzimidazol-1-ylmethyl]-phenyl]-methansulfonamid (8)

### a. Synthese von 1-(4-Trifluormethoxybenzyl)-1,3-dihydrobenzimidazol-2-on (R)

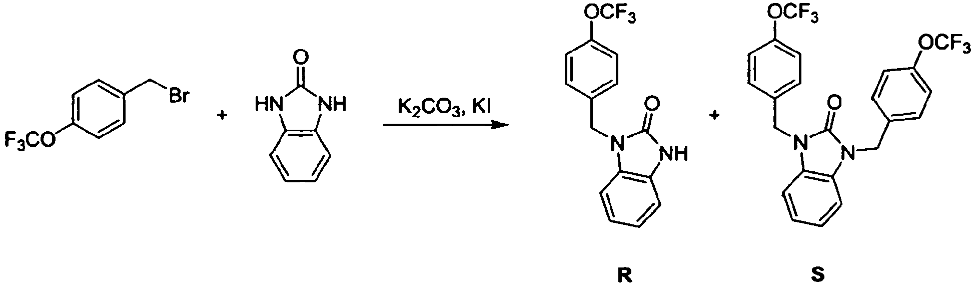

Zu einer Lösung von Benzimidazolinon (4.02 g, 30 mmol) in abs. DMF (20 mL) wurde unter Argon 4-Trifluormethoxy-benzylbromid (5 g, 19.6 mmol), Kaliumcarbonat (7.0 g, 50.7 mmol) und eine katalytische Menge Kaliumiodid gegeben. Die Reaktionsmischung wurde 2 h auf 100 °C erhitzt. Nach Abkühlen wurde die Reaktionsmischung in eine Mischung aus Eis (200 g) und 2N Salzsäure-Lösung in H₂O (100 mL) gegossen. Der entstandene Niederschlag wurde mit Hilfe einer Fritte abgetrennt und anschließend getrocknet. Der Rückstand wurde in EtOAc (150 mL) aufgenommen und die unlöslichen Bestandteile abgetrennt. Das Lösungsmittel wurde im Vakuum entfernt und der Rückstand wurde aus wenig EtOAc (20 mL) umkristallisiert, wobei das dibenzylierte Produkt (**S**) ausfiel. Die Mutterlauge wurde wiederum im Vakuum eingeengt, der Rückstand aus Ethanol umkristallisiert und der ausgefallene Feststoff (**S**) durch Filtration abgetrennt. Das Filtrat wurde mittels Säulenchromatographie (SiO₂, Cyclohexan/EtOAc 1:4) gereinigt und es wurde 1-(4-Trifluormethoxybenzyl)-1,3-dihydrobenzimidazol-2-on (**R**) (1.60 mg, 26 % der Theorie, Smp.: 145-148 °C) erhalten.

### b. Synthese von 1-(4-Nitrobenzyl)-3-(4-trifluormethoxybenzyl)-1,3-dihydro-benzimidazol-2-on (T)

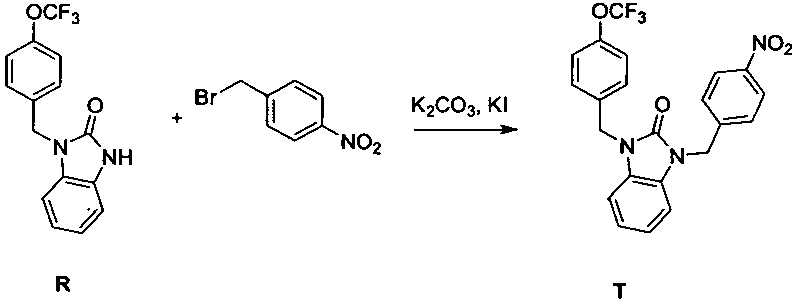

Zu einer Lösung von Verbindung R (770 mg, 2.5 mmol) in abs. DMF (20 mL) wurde unter Argon 4-Nitro-benzylbromid (540 mg, 2.5 mmol), Kaliumcarbonat (828 mg, 6 mmol) und eine katalytische Menge Kaliumiodid gegeben. Die Reaktionsmischung wurde 5 h auf 80 bis 85 °C erhitzt. Nach Abkühlen wurde die Reaktionsmischung in eine Mischung aus Eis (200 g) und 2N Salzsäure-Lösung in H₂O (10 mL) gegossen und die Reaktionsmischung eine Stunde gerührt. Der entstandene Feststoff wurde über eine Fritte abgetrennt und aus Ethanol (20 mL) umkristallisiert. Das gewünschte Produkt 1-(4-Nitrobenzyl)-3-(4-trifluormethoxybenzyl)-1,3-dihydro-benzimidazol-2-on (T) wurde mit einer Ausbeute von 830 g (74 % der Theorie) erhalten.

### c. Synthese von 1-(4-Aminobenzyl)-3-(4-trifluormethoxybenzyl)-1,3-dihydrobenzimidazol-2-on (U)

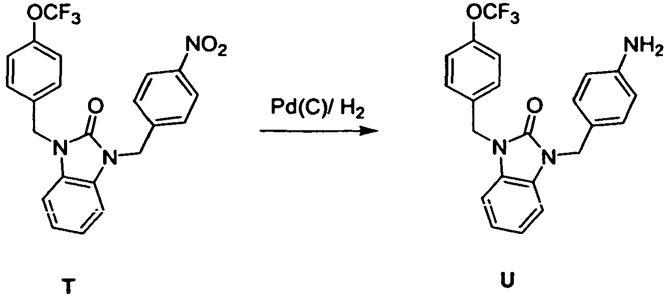

Zu einer Lösung von 1-(4-Nitrobenzyl)-3-(4-trifluormethoxybenzyl)-1,3-dihydro-benzimidazol-2-on (**T**) (1.00 g, 2.26 mmol) in abs. MeOH (200 mL) wurde Palladium auf Kohle (5 %, 830 mg) gegeben. Die Reaktionsmischung wurde 30 min bei RT einem Wasserstoffdruck von 3 bar ausgesetzt. Der Katalysator wurde durch Filtration über einen Filter abgetrennt und das Filtrat wurde im Vakuum eingeengt. Der Rückstand wurde in DCM (20 mL) aufgenommen und über eine Fritte mit Kieselgur (Schichtdicke 1 cm) filtriert. Die Fritte wurde mit DCM (3 x 20 mL) gewaschen.
Die so erhaltene Lösung wurde mit Chlortrimethylsilan (1 mL, 7.9 mmol) versetzt. Das Reaktionsgemisch wurde 17 h ohne Feuchtigkeitsausschluß gerührt, der ausgefallene Feststoff mit Hilfe einer Fritte abgetrennt und anschließend getrocknet. Das Hydrochlorid von 1-(4-Aminobenzyl)-3-(4-trifluormethoxybenzyl)-1,3-dihydrobenzimidazol-2-on (**U**) wurde als Feststoff (791 mg, 76 % der Theorie, Smp.: 222-232 °C) erhalten.

### d. Synthese von N-[4-[3-(4-Trifluormethoxybenzyl)-2-oxo-2,3-dihydrobenzimidazol-1-ylmethyl]-phenyl]-methansulfonamid (7)

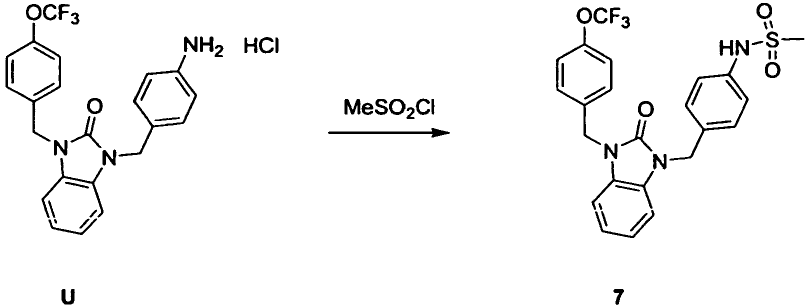

Zu einer Suspension des Hydrochlorids von 1-(4-Aminobenzyl)-3-(4-trifluormethoxybenzyl)-1,3-dihydrobenzimidazol-2-on (**U**) (806 mg, 1.79 mmol) in abs. THF (40 mL) wurde Pyridin (0.4 mL, 5.0 mmol) gegeben. Die Reaktionsmischung wurde 30 min bei RT gerührt, Methansulfonylchlorid (0.16 mL, 2.1 mmol, gelöst in abs. THF (1 mL)) innerhalb von 20 min zugegeben und das Reaktionsgemisch wiederum 18 h bei RT gerührt. Anschließend wurde die Reaktionsmischung mit H₂O (150 mL) und wäßriger 2N Salzsäure-Lösung (5 mL) versetzt. Nach zwei Stunden wurde der ausgefallene Feststoff über eine Fritte abgetrennt, anschließend im Vakuum getrocknet und aus Ethanol (20 mL) umkristallisiert. Das gewünschte Produkt N-[4-[3-(4-Trifluormethoxybenzyl)-2-oxo-2,3-dihydrobenzimidazol-1-ylmethyl]-phenyl]-methansulfonamid (**7**) wurde in einer Ausbeute von 505 mg (57 % der Theorie, Smp.: 182-187 °C) erhalten.

### e. Synthese von N-[4-[3-(4-Trifluormethoxybenzyl)-2-thioxo-2,3-dihydrobenzimidazol-1-ylmethyl]-phenyl]-methansulfonamid (8)

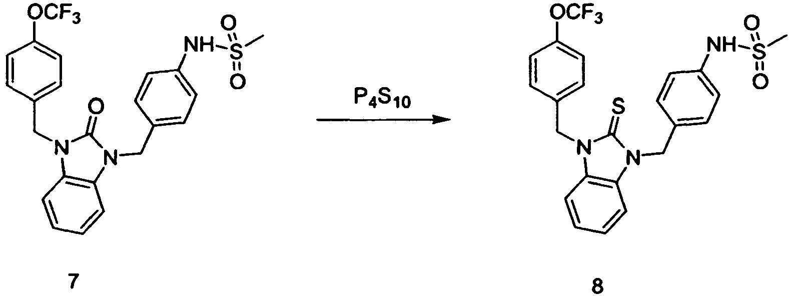

Eine Suspension von N-[4-[3-(4-Trifluormethoxybenzyl)-2-oxo-2,3-dihydrobenzimidazol-1-ylmethyl]-phenyl]-methansulfonamid (**7**) (300 mg, 0.61 mmol) in para-Xylol (20 mL) wurde unter Argon mit Phosphorpentasulfid (444 mg, 1.0 mmol) versetzt. Das Reaktionsgemisch wurde 10 h zum Sieden erhitzt. Nach dem Abkühlen wurde die Reaktionsmischung mit Wasser (1 mL) und anschließend mit 2N NaOH-Lösung in Wasser (10 mL) versetzt und 30 min bei RT gerührt. Die Reaktionsmischung wurde mit Ethylacetat (4 x 20 mL) extrahiert. Die vereinigten organischen Phasen wurden über Na₂SO₄ getrocknet und das Lösungsmittel im Vakuum entfernt. Nach Reinigung des Rückstands mittels Säulenchromatographie (SiO₂; Cyclohexan/EtOAc 2:1) wurden das gewünschte Produkt N-[4-[3-(4-Trifluormethoxybenzyl)-2-thioxo-2,3-dihydrobenzimidazol-1-ylmethyl]-phenyl]-methansulfonamid (**8**) (162 mg, 52 % der Theorie, Smp.: 167-170 °C) erhalten. Nach Umkristallisieren aus Methanol wurden Kristalle erhalten, die einen Schmelzpunkt von 175-179 °C aufwiesen.

### VI. Synthese von Beispielverbindung 9:

### N-[4-[3-(4-Methansulfonylaminobenzyl)-2-oxo-2,3-dihydrobenzimidazol-1-ylmethyl]-phenyl]-methansulfonamid

### a. Synthese von 1,3-Bis-(4-nitrobenzyl)-1,3-dihydrobenzimidazol-2-on (v)

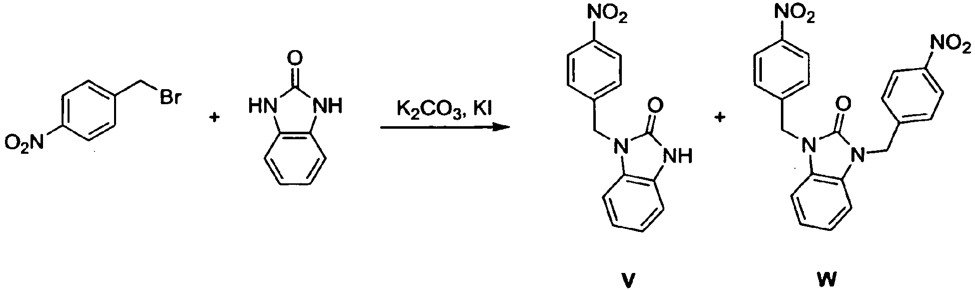

Zu einer Lösung von Benzimidazolinon (1.5 g, 11.2 mmol) in abs. DMF (25 mL) wurde unter Argon 4-Nitro-benzylbromid (2.16 g, 10 mmol), Kaliumcarbonat (2,76 g, 20 mmol) und eine katalytische Menge Kaliumiodid gegeben. Die Reaktionsmischung wurde 2 h auf 100 °C erhitzt. Nach Abkühlen wurde die Reaktionsmischung in eine Lösung von H₂O (200 mL) und 2N Salzsäure-Lösung in H₂O (10 mL) gegossen. Das entstandene Gemisch wurde mit EtOAc (5 x 40 mL) extrahiert, die vereinigten organischen Phasen wurden mit ges. NaCl-Lösung (2 x 30 mL) gewaschen, über Na₂SO₄ getrocknet und das Lösungsmittel wurde im Vakuum entfernt. Der Rückstand wurde in heißem Toluol (100 mL) aufgenommen und der erhaltene Feststoff wurde abfiltriert. Das Filtrat wurde im Vakuum eingeengt. Der Rückstand wurde mittels Säulenchromatographie (SiO₂, Cyclohexan/EtOAc 1:1) gereinigt und es wurden 1,3-Bis-(4-nitrobenzyl)-1,3-dihydrobenzimidazol-2-on (**W**) (1.65 g, Smp.: 200-201 °C) und 1-(4-Nitrobenzyl)-1,3-dihydrobenzimidazol-2-on (**V**) (600 mg, 22 % der Theorie) erhalten.

### b. Synthese von 1,3-Bis-4-(4-aminobenzyl)-1,3-dihydrobenzimidazol-2-on (X)

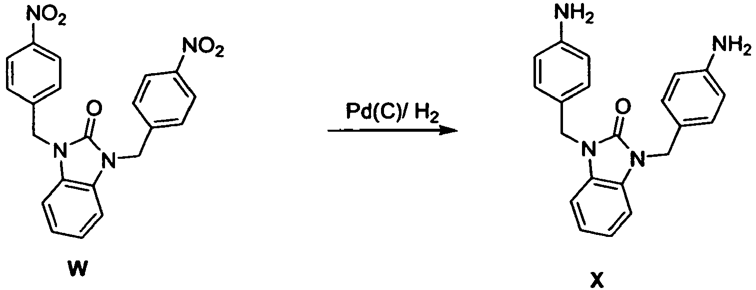

Zu einer Lösung von Bis-1,3-(4-nitrobenzyl)-1,3-dihydro-benzimidazol-2-on (**W**) (1.00 g, 2.4 mmol) in abs. THF (50 mL) wurde Palladium auf Kohle (5 %, 1.2 g) gegeben. Die Reaktionsmischung wurde 30 min bei RT einem Wasserstoffdruck von 2 bar ausgesetzt. Der Katalysator wurde durch Filtration über Celite abgetrennt und das Filtrat wurde im Vakuum eingeengt. Das gewünschte Produkt von 1,3-Bis-4-(4-aminobenzyl)-1,3-dihydrobenzimidazol-2-on (**X**) wurde als farbloses Öl in einer Ausbeute von 840 mg (99 % der Theorie) erhalten.
Zur Herstellung des entsprechenden Dihydrochlorids wurde die Verbindung (**X**) (200 mg, 0.58 mmol) in DCM (10 mL) mit Chlortrimethylsilan (184 µL, 1.5 mmol) versetzt. Das Reaktionsgemisch wurde 17 h ohne Feuchtigkeitsausschluß gerührt, der ausgefallene Feststoff mit Hilfe einer Fritte abgetrennt und anschließend getrocknet. Das Dihydrochlorid von 1,3-Bis-4-(4-aminobenzyl)-1,3-dihydrobenzimidazol-2-on (**X**) wurde als Feststoff (188 mg, 77 % der Theorie, Smp.: 212-218 °C) erhalten.

### c. Synthese von N-[4-[3-(4-Methansulfonylaminobenzyl)-2-oxo-2,3-dihydrobenzimidazol-1-ylmethyl]-phenyl]-methansulfonamid (9)

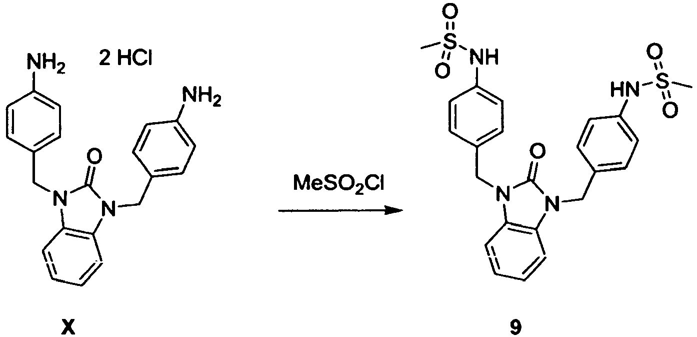

Zu einer Suspension des Dihydrochlorids von 1,3-Bis-4-(4-aminobenzyl)-1,3-dihydrobenzimidazol-2-on (**X**) (600 mg, 1.75 mmol) in abs. THF (50 mL) wurde Triethylamin (0.63 mL, 4.55 mmol) gegeben. Die Reaktionsmischung wurde 30 min bei RT gerührt, Methansulfonylchlorid (0.32 mL, 4.2 mmol, gelöst in abs. THF (10 mL)) innerhalb von 15 min zugegeben und das Reaktionsgemisch wiederum 48 h bei RT gerührt. Anschließend wurde die Reaktionsmischung mit H₂O (150 mL) und ges. Natriumhydrogencarbonat-Lösung (30 mL) versetzt, 30 min bei RT gerührt und mit EtOAc (3 x 20 mL) extrahiert. Die vereinigten organischen Phasen wurden mit ges. NaCl-Lösung (30 mL) gewaschen, über Na₂SO₄ getrocknet und das Lösungsmittel wurde im Vakuum entfernt. Nach Reinigung des Rückstands mittels Säulenchromatographie (SiO₂ (70 g), 700 mL Cyclohexan/EtOAc 1:4) wurde das gewünschte Produkt N-[4-[3-(4-Methansulfonylaminobenzyl)-2-oxo-2,3-dihydrobenzimidazol-1-ylmethyl]-phenyl]-methansulfonamid (**9**) mit einer Ausbeute von 110 mg (12 % der Theorie, Smp.: 204-214 °C) erhalten.

### VII. Synthese von Beispielverbindung 10:

### N-{4-[2-Oxo-3-(4-trifluormethylbenzyl)-2,3-dihydrobenzimidazol-1-ylmethyl]phenyl}methansulfonamid

### a. Synthese von 1-(4-Trifluormethylbenzyl)-1,3-dihydrobenzimidazol-2-on Y

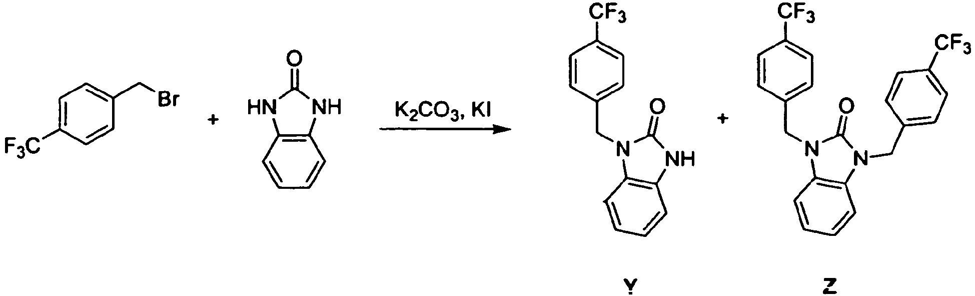

Eine Lösung von 2-Hydroxybenzimidazol (4 g, 29 mmol) in abs. DMF (40 ml) wurde unter Argon mit (4 g, 16,7 mmol), Kaliumcarbonat (7 g, 50,7 mmol) und einer Spatelspitze Kaliumiodid versetzt. Die Reaktionsmischung wurde 5 h unter Rühren auf 100 °C (Badtemperatur) erhitzt. Nach dem Abkühlen wurde der Ansatz auf eine Mischung aus Eis (200 g) und 2N HCl (50 ml) gegeben. Die entstandene wäßrige Suspension wurde 30 min gerührt, der entstandene Feststoff wurde mittels einer Fritte abgetrennt, getrocknet und aus Ethanol (100 ml) umkristallisiert. Dabei fiel das zweifach substituierte Benzimidazol Z aus, das gewünschte Produkt blieb überwiegend in Lösung. Zur Isolierung von Y wurde die ethanolische Lösung eingeengt. Zur Entfernung von nicht umgesetzten 2-Hydroxybenzimidazol wurde der verbliebene Rückstand in Chloroform (ca. 50 ml) aufgenommen, die unlöslichen Bestandteile wurden abgetrennt. Der nach Abdampfen des Lösungsmittels erhaltene Rückstand (1,7 g, 34 %) bestand zu über 90 % aus dem gewünschten Produkt **4C** und konnte ohne weitere Reinigung bei der folgenden Umsetzung verwendet werden.

### b. Synthese von 1-(4-Nitrobenzyl)-3-(4-trifluormethylbenzyl)-1,3-dihydrobenzimidazol-2-on

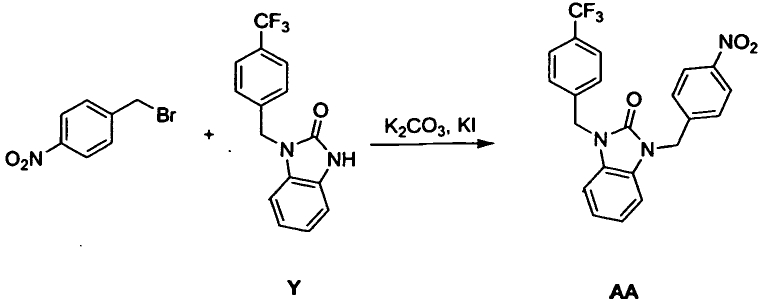

Zu einer Lösung der Verbindung Y (876 mg, 3 mmol) in abs. DMF (30 mL) wurde unter Argon 4-Nitrobenzylbromid (648 mg, 3 mmol), Kaliumcarbonat (1.1 g, 8 mmol) und einer Spatelspitze Kaliumiodid gegeben. Die Reaktionsmischung wurde 5 h unter Rühren auf 85 °C (Badtemperatur) erhitzt. Nach Abkühlen wurde der Ansatz zu einer Mischung aus Eis (150 g) und aq. 2N HCl (20 mL) gegeben. Die Lösung wurde 1 h bei RT gerührt, der entstandene Feststoff wurde mittels einer Fritte abgetrennt und im Vakuum über CaCl₂ getrocknet. Das gewünschte Produkt wurde aus dem Rohprodukt durch Umkristallisation in einer Ausbeute von 700 mg (54 %) mit einem Schmelzpunkt von 134-137 °C aus Ethanol (20 ml) erhalten.

### c. Synthese von 4-[2-Oxo-3-(4-trifluormethylbenzyl)-2,3-dihydrobenzimidazol-1-ylmethyl]phenylammoniumchlorid

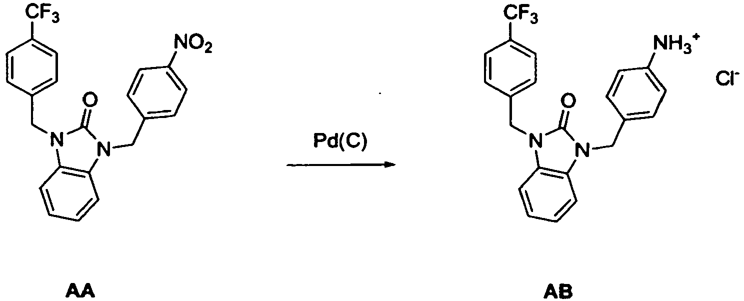

Eine Lösung von Verbindung AA (590 mg, 1.38 mmol) in Methanol (100 mL) wurde mit Palladiumkatalysator (5 % Pd auf Aktivkohle, 420 mg) versetzt und 30 min bei einem Druck von 2 bar bei RT hydriert. Der Katalysator wurde über ein Filter abgetrennt, die erhaltene methanolische Lösung wurde bis zur Trockene eingeengt. Nach Entfernen des Lösungsmittels wurde das Rohprodukt erhalten, das in Dichlormethan (30 mL) gelöst wurde. Die Lösung wurde filtriert, der Filterrückstand wurde 2 x mit CH₂Cl₂ (je 10 mL) gewaschen. Die erhaltene Lösung wurde mit Chlortrimethylsilan (0.5 ml, 4 mmol) versetzt. Die Lösung wurde 5 h ohne Feuchtigkeitsausschluß gerührt, der ausgefallene Feststoff wurde mittels einer Fritte abgetrennt und anschließend getrocknet. Das gewünschte Produkt wurde so in einer Ausbeute von 572 mg (95 %) mit einem Schmelzpunkt von 221 - 225 °C erhalten.

### d. Synthese von N-{4-[2-Oxo-3-(4-trifluormethylbenzyl)-2,3-dihydrobenzimidazol-1-ylmethyl]phenyl}methansulfonamid

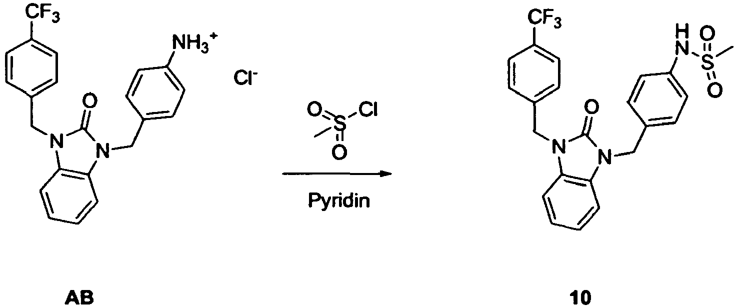

Zu einer Suspension von Verbindung AB (700 mg, 1.6 mmol) in abs. Dioxan (30 mL) wurde Pyridin (0.5 mL, 6.4 mmol) gegeben. Der Ansatz wurde zum Sieden erhitzt, bis das Hydrochlorid vollständig als freie Base gelöst war. Der Ansatz wurde auf ca. 40 °C gekühlt, dann wurde die klare Lösung innerhalb von 5 min mit Methansulfonylchlorid (0.2 ml, 2.6 mmol, gelöst in trockenem Dioxan (1 mL)) versetzt. Das Reaktionsgemisch wurde 2 d bei RT gerührt, anschließend wurde das Dioxan im Vakuum entfernt. Der Rückstand wurde mit Wasser versetzt (20 mL) und mit Ethylacetat (3 x 20 mL) extrahiert. Die organischen Phasen wurden mit Na₂SO₄ getrocknet und das Lösungsmittel im Vakuum entfernt. Der Rückstand wurde säulenchromatographisch (Laufmittel: Cyclohexan/Ethylacetat =1:1) gereinigt. Das Lösungsmittel wurde im Vakuum entfernt und der Rückstand aus Ethanol (7 mL) umkristallisiert, um das gewünschte Produkt in einer Ausbeute von 150 mg (19 %) mit einem Schmelzpunkt von 193-197 °C zu erhalten.

### Pharmakologische Daten:

### I.

Die untersuchten erfindungsgemäßen substituierten zyklischen Harnstoff-Derivate zeigen eine ausgezeichnete Affinität zu dem Vanilloid-Rezeptor 1 (VR1/TRPV1-Rezeptor).

| Verbindung gemäß Beispiel | IC₅₀-Wert [µM] |
|---|---|
| 4 | 9.4 |
| 5 | 2.9 |

## Patentansprüche

1. Substituierte zyklische Harnstoff-Derivate der allgemeinen Formel I, 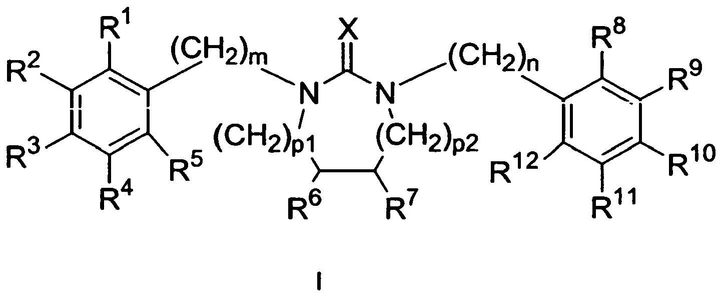 worin
X für O, S oder N-C≡N steht;
m gleich 1 oder 2 ist;
n gleich 1 oder 2 ist;
p1 und p2, unabhängig voneinander, jeweils für 0, 1, 2 oder 3 stehen, wobei die Summe von p1 und p2 0, 1, 2 oder 3 beträgt;
R¹, R², R³, R⁴, R⁵, R⁸, R⁹, R¹⁰, R¹¹ und R¹², unabhängig voneinander, jeweils für
H; F; Cl; Br; I; -SF₅; -NO₂; -NH₂; -OH; -SH; -C(=O)-NH₂; -S(=O)₂-NH₂;
-NR¹³R¹⁴; -NH-R¹⁵; -OR¹⁶; -SR¹⁷; -O-(CH₂)ₐ-R¹⁸; -O-(CH₂)_{b}-OR¹⁹;
-(CH₂)_{c}-O-(CH₂)_{d}-OR²⁰;
-(CH₂)ₑ-O-C(=O)-R²¹;
-(CH₂)_{f}-O-C(=O)-OR²²;
-NR²³S(=O)₂R²⁴;
-(CH₂)_{g}-C(=O)-NR²⁵R²⁶;
-(CH₂)ₕ-C(=O)-NH-R²⁷;
-S(=O)ᵢR²⁸;
-(CH₂)ⱼ-S(=O)₂-NR²⁹R³⁰;
-(CH₂)ₖ-S(=O)₂-NHR³¹;
-(CH₂)ₗ-NR³²-C(=O)-(CH₂)_{q}-OR³³;
-(CH₂)ᵣ-NH-C(=O)-(CH₂)ₛ-OR³⁴;
-(CH₂)ₜNR³⁵-O-C(=O)-OR³⁶;
-(CH₂)ᵤ-NH-O-C(=O)-OR³⁷;
-(CH₂)ᵥ-O-S(=O)₂-R³⁸;
-(CH₂)_{w}-NR³⁹-C(=O)-SR⁴⁰;
-(CH₂)_{y}-C(=O)-NH-OR⁴¹;
-P(=O)-(OR⁴²)₂;
-(CH₂)_{z}-C(=S)-NR⁴³R⁴⁴;
-(CH₂)ₐₐ-C%(=S)-NH-R⁴⁵;
-(CH₂)_{bb}-NR⁴⁶-C(=O)-R⁴⁷;
-(CH₂)_{cc}-NH-C(=O)-R⁴⁸;
oder -NH-C(=NH)-NH₂;
wobei
a, b, c, d, q und s, unabhängig voneinander, jeweils gleich 1, 2, 3, 4 oder 5 sind;
e, f, g, h, j, k, l, r, t, u, v, w, x, y, z, aa, bb und cc, unabhängig voneinander, jeweils gleich 0, 1, 2, 3, 4 oder 5 sind, und
i gleich 1 oder 2 ist, stehen;
für einen linearen oder verzweigten, gesättigten oder ungesättigten aliphatischen C₁₋₁₀ Rest, der ggf. mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH, -O(C₁₋₅-Alkyl),-S(C₁₋₅-Alkyl), -NH(C₁₋₅-Alkyl), -N(C₁₋₅-Alkyl)(C₁₋₅-Alkyl), -OCF₃ und-SCF₃ substituiert sein kann;
oder für einen ggf. substituierten 6- oder 10-gliedrigen Aryl-Rest, der über eine lineare oder verzweigte, ggf. substituierte C₁₋₅-Alkylen-Gruppe gebunden sein kann, stehen;
oder zwei benachbarte Reste ausgewählt aus der Gruppe bestehend aus R⁸, R⁹, R¹⁰, R¹¹ und R¹² zusammen für eine Methylendioxy(-O-CH₂-O)-Gruppe stehen;
mit der Maßgabe, dass wenigstens einer der Reste R⁸, R⁹, R¹⁰, R¹¹ und R¹² für -NR²³S(=O)₂R²⁴ steht;
R⁶ und R⁷ jeweils für einen Wasserstoff-Rest stehen
oder
R⁶ und R⁷ zusammen mit der sie verbindenden -C-C-Brücke einen unsubstituierten Phenylen-Rest bilden;
R¹³, R¹⁴, R¹⁵, R¹⁶, R¹⁷, R¹⁹, R²⁰, R²¹, R²², R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, R⁴², R⁴³, R⁴⁴, R⁴⁵, R⁴⁶, R⁴⁷ und R⁴⁸, unabhängig voneinander, jeweils
für einen linearen oder verzweigten, gesättigten oder ungesättigten aliphatischen C₁₋₁₀ Rest stehen, der ggf. mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH, -O(C₁₋₅-Alkyl),-S(C₁₋₅-Alkyl), -NH(C₁₋₅-Alkyl), -N(C₁₋₅-Alkyl)(C₁₋₅-Alkyl), -OCF₃ und-SCF₃ substituiert sein kann;
für einen ungesättigten oder gesättigten, ggf. substituierten 3-, 4-, 5-, 6-, 7-, 8- oder 9-gliedrigen cycloaliphatischen Rest stehen oder für einen ggf. substituierten 5- bis 14-gliedrigen Aryl- oder Heteroaryl-Rest stehen, der mit einem gesättigten oder ungesättigten, ggf. substituierten mono- oder polyzyklischen Ringsystem kondensiert sein kann;
R¹⁸ für einen ungesättigten oder gesättigten, ggf. substituierten 3-, 4-, 5-, 6-, 7-, 8- oder 9-gliedrigen cycloaliphatischen Rest steht
oder für einen ggf. substituierten 5- bis 14-gliedrigen Aryl- oder Heteroaryl-Rest steht, der mit einem gesättigten oder ungesättigten, ggf. substituierten mono- oder polyzyklischen Ringsystem kondensiert sein kann;
R²³ für einen Wasserstoff-Rest steht
oder für einen linearen oder verzweigten, gesättigten oder ungesättigten aliphatischen C₁₋₁₀ Rest steht, der ggf. mit 1, 2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH, -O(C₁₋₅-Alkyl),-S(C₁₋₅-Alkyl), -NH(C₁₋₅-Alkyl), -N(C₁₋₅-Alkyl)(C₁₋₅-Alkyl), -OCF₃ und-SCF₃ substituiert sein kann;
und
R²⁴ für einen linearen oder verzweigten, gesättigten oder ungesättigten aliphatischen C₁₋₁₀ Rest steht, der ggf. mit 1, 2, 3, 4 oder 5 Substituenten unabhängig voneinander ausgewählt aus der Gruppe bestehend aus -CN, -NO₂, -OH, -NH₂, -SH, -O(C₁₋₅-Alkyl), -S(C₁₋₅-Alkyl), -NH(C₁₋₅-Alkyl) und -N(C₁₋₅-Alkyl)(C₁₋₅-Alkyl) substituiert sein kann,
oder für einen ggf. substituierten 5- bis 14-gliedrigen Aryl- oder Heteroaryl-Rest steht, der mit einem gesättigten oder ungesättigten, ggf. substituierten mono- oder polyzyklischen Ringsystem kondensiert sein kann;
wobei
die vorstehend genannten cycloaliphatischen Reste ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -NH-C₁₋₅-Alkyl, -N(C₁₋₅-Alkyl)₂, -O-Phenyl, -O-Benzyl, Phenyl und Benzyl substituiert sein können, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
und die vorstehend genannten cycloaliphatischen Reste jeweils ggf. 1, 2, 3, 4 oder 5 Heteroatom(e) unabhängig voneinander ausgewählt aus der Gruppe bestehend aus Sauerstoff, Stickstoff und Schwefel aufweisen können;
die vorstehend genannte C₁₋₅-Alkylen-Gruppe ggf. mit 1, 2, 3, 4 oder 5 Substituenten unabhängig voneinander ausgewählt aus der Gruppe bestehend aus F, Cl, Br, -OH, -SH, -NH₂, -CN und NO₂ substituiert sein kann;
die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -NH-C₁₋₅-Alkyl, -N(C₁₋₅-Alkyl)₂, -O-Phenyl, -O-Benzyl, Phenyl und Benzyl substituiert sein können, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, - NO₂, -C₁₋₅-Alkyl, -O-C₁₋₅-Alkyl, -O-CF₃, -S-CF₃, Phenyl und -O-Benzyl substituiert sein kann,
und die Ringe der vorstehend genannten mono- oder polyzyklischen Ringsysteme jeweils 5-, 6- oder 7-gliedrig sind und jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
und die vorstehend genannten Aryl- oder Heteroaryl-Reste ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -NH₂,-NO₂, -O-CF₃, -S-CF₃, -SH, -S-C₁₋₅-Alkyl, -C₁₋₅-Alkyl, -C(=O)-OH, -C(=O)-O-C₁-₅-Alkyl, -NH-C₁₋₅-Alkyl, -N(C₁₋₅-Alkyl)₂, -NH-C(=O)-O-C₁₋₅-Alkyl, -C(=O)-H,-C(=O)-C₁₋₅-Alkyl, -C(=O)-NH₂, -C(=O)-NH-C₁₋₅-Alkyl, -C(=O)-N-(C₁₋₅-Alkyl)₂,-O-Phenyl, -O-Benzyl, Phenyl und Benzyl substituiert sein können, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl,-O-C₁₋₅-Alkyl, -O-CF₃, -S-CF₃, Phenyl und -O-Benzyl substituiert sein kann,
und
die vorstehend genannten Heteroaryl-Reste jeweils 1, 2, 3, 4 oder 5 Heteroatom(e) unabhängig voneinander ausgewählt aus der Gruppe bestehend aus Sauerstoff, Stickstoff und Schwefel als Ringglied(er) aufweisen;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

2. Verbindungen gemäß Anspruch 1, **dadurch gekennzeichnet, dass**
R¹, R², R⁴ und R⁵, unabhängig voneinander, jeweils für
H; F; Cl; Br; I; -SF₅; -NO₂; -NH₂; -OH; -SH; -C(=O)-NH₂; -S(=O)₂-NH₂;
-NR¹³R¹⁴; -NH-R¹⁵; -OR¹⁶; -SR¹⁷; -O-(CH₂)ₐ-R¹⁸; -O-(CH₂)_{b}-OR¹⁹;
-(CH₂)_{c}-O-(CH₂)_{d}-OR²⁰;
-(CH₂)ₑ-O-C(=O)-R²¹;
-(CH₂)_{f}-O-C(=O)-OR²²;
-NR²³S(=O)₂R²⁴;
-(CH₂)_{g}-C(=O)-NR²⁵R²⁶;
-(CH₂)ₕ-C(=O)-NH-R²⁷_{;}
-S(=O)ᵢR²⁸;
-(CH₂)ⱼ-S(=O)₂-NR²⁹R³⁰;
-(CH₂)ₖ-S(=O)₂-NHR³¹;
-(CH₂)ᵢ-NR³²-C(=O)-(CH₂)_{q}-OR³³;
-(CH₂)ᵣ-NH-C(=O)-(CH₂)ₛ-OR³⁴;
-(CH₂)ₜ-NR³⁵-O-C(=O)-OR³⁶;
-(CH₂)ᵤ-NH-O-C(=O)OR³⁷;
-(CH₂)ᵥ-O-S(=O)₂-R³¹;
-(CH₂)_{w}-NR³⁹-C(=O)-SR⁴⁰;
-(CH₂)_{y}-C(=O)-NH-OR⁴¹;
-P(=O)-(OR⁴²)₂;
-(CH₂)_{z}-C(=S)-NR⁴³R⁴⁴;
-(CH₂)ₐₐ-C(=S)-NH-R⁴⁵;
-(CH₂)_{bb}-NR⁴⁶-C(=O)-R⁴⁷;
-(CH₂)_{cc}-NH-C(=O)-R⁴⁸;
oder -NH-C(=NH)-NH₂ stehen;
wobei
a, b, c, d, q und s, unabhängig voneinander, jeweils gleich 1, 2, 3, 4 oder 5 sind,
e, f, g, h, j, k, I, r, t, u, v, w, x, y, z, aa, bb und cc, unabhängig voneinander, jeweils gleich 0, 1, 2, 3, 4 oder 5 sind und
i gleich 1 oder 2 ist;
für einen Rest ausgewählt aus der Gruppe bestehend aus Methyl, -CF₃, -CCl₃, -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃, -CH₂-N(CH₃)₂, -CH₂-N(C₂Hₛ)₂, -CH₂-N(CH₃)(C₂H₅), Ethyl, -CH₂-CH₂-NH₂, -CH₂-CH₂-OH, -CH₂-CH₂-SH,-CH₂-CH₂-NH-CH₃, -CH₂-CH₂-N(CH₃)₂, -CH₂-CH₂-N(C₂Hs)₂, -CH₂-CH₂-N(CH₃)(C₂H₅), -CH₂-CF₃, -C₂F₅, -CH₂-CCl₃, -CH₂-CBr₃, -CH₂-CH₂-CN, n-Propyl, -CH₂-CH₂-CH₂-OH, -CH₂-CH₂-CH₂-SH, -CH₂-CH₂-CH₂-NH₂,-CH₂-CH₂-CH₂-NH-CH₃, -CH₂-CH₂-CH₂-N(CH₃)₂, -CH₂-CH₂-CH₂-N(C₂H₅)₂, -CH₂-CH₂-CH₂-N(CH₃)(C₂H₅), -CH₂-CH₂-O-CH₃, -CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), -CH(CH₃)(S-CH₃), n-Butyl, -CF₂-CF₂-CF₂-CF₃, -CH₂-CH₂-CH₂-CH₂-CN, sec-Butyl, Isobutyl, tert-Butyl, n-Pentyl, sec-Pentyl, neo-Pentyl, n-Hexyl, Vinyl, 1-Propenyl, 2-Propenyl, 1-Butenyl, 2-Butenyl, 3-Butenyl, 2-Methyl-buten-2-yl, (1,1,2)-Trifluor-1-butenyl, 1-Pentenyl, 2-Pentenyl, 3-Pentenyl und 4-Pentenyl stehen;
oder für einen Aryl-Rest ausgewählt aus der Gruppe bestehend aus Phenyl und Naphthyl stehen, wobei der Aryl-Rest jeweils mit 1, 2, 3, 4 oder 5 Substituenten unabhängig voneinander ausgewählt aus der Gruppe bestehend aus F, Cl, Br, I, -CN, -NO₂, -OH, -SH, Methyl, Ethyl,-CF₃, -O-CF₃, -S-CF₃, -SF₅, -O-CH₃, -O-C₂H₅, -NH₂, -N(CH₃)₂, -N(C₂H₅)₂, -N(H)(CH₃) und -N(H)(C₂H₅) substituiert sein kann;
und
R³ für F; Cl; Br; I; -SF₅; -NO₂; -NH₂; -OH; -SH; -C(=O)-NH₂; -S(=O)₂-NH₂; -NR¹³R¹⁴; -NH-R¹⁵; -OR¹⁶; -SR¹⁷; -O-(CH₂)ₐ-R¹⁸, -O-(CH₂)_{b}-OR¹⁹ oder -NR²³S(=O)₂R²⁴ steht;
wobei
a und b, unabhängig voneinander, jeweils gleich 1, 2, 3, 4 oder 5 sind;
oder für einen Rest ausgewählt aus der Gruppe bestehend aus Methyl, -CF₃, -CCl₃, -CBr₃, -CH₂-CN, -CH₂-O-CH₃, -CH₂-O-CF₃, -CH₂-SF₃, - CH₂-NH₂, -CH₂-OH, -CH₂-SH, -CH₂-NH-CH₃, -CH₂-N(CH₃)₂, -CH₂-N(C₂H₅)₂, -CH₂-N(CH₃)(C₂H₅), Ethyl, -CH₂-CH₂-NH₂, -CH₂-CH₂-OH,-CH₂-CH₂-SH, -CH₂-CH₂-NH-CH₃, -CH₂-CH₂-N(CH₃)₂, -CH₂-CH₂-N(C₂H₅)₂, -CH₂-CH₂-N(CH₃)(C₂H₅), -CH₂-CF₃, -C₂F₅, -CH₂-CCl₃, -CH₂-CBr₃, -CH₂-CH₂-CN, n-Propyl, -CH₂-CH₂-CH₂-OH, -CH₂-CH₂-CH₂-SH, - CH₂-CH₂-CH₂-NH₂, -CH₂-CH₂-CH₂-NH-CH₃, -CH₂-CH₂-CH₂-N(CH₃)₂,-CH₂-CH₂-CH₂-N(C₂H₅)₂, -CH₂-CH₂-CH₂-N(CH₃)(C₂H₅), -CH₂-CH₂-O-CH₃, -CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), - CH(CH₃)(S-CH₃), n-Butyl, -CF₂-CF₂-CF₂-CF₃, -CH₂-CH₂-CH₂-CH₂-CN, sec-Butyl, Isobutyl, tert-Butyl, n-Pentyl, sec-Pentyl, neo-Pentyl, n-Hexyl, Vinyl, 1-Propenyl, 2-Propenyl, 1-Butenyl, 2-Butenyl, 3-Butenyl, 2-Methyl-buten-2-yl, (1,1,2)-Trifluor-1-butenyl, 1-Pentenyl, 2-Pentenyl, 3-Pentenyl und 4-Pentenyl stehen.

3. Verbindungen gemäß Anspruch 1 oder 2, **dadurch gekennzeichnet, dass**
R¹, R², R⁴ und R⁵, unabhängig voneinander, jeweils für
H; F; Cl; I; Br, -NO₂; -NH₂; -OH; -SH; -NR¹³R¹⁴; -NH-R¹⁵; -OR¹⁶; -SR¹⁷;-NR²³S(=O)₂R²⁴;
oder für einen Rest ausgewählt aus der Gruppe bestehend aus Methyl, -CF₃, Ethyl, -CH₂-CF₃, -C₂F₅, n-Propyl, Isopropyl, n-Butyl, sec-Butyl, Isobutyl, tert-Butyl oder n-Pentyl stehen
und
R³ für F; Cl; Br; I; -OR¹⁶; -NR²³S(=O)₂R²⁴;
oder für einen Rest ausgewählt aus der Gruppe bestehend aus -SF₅,-CF₃, -C₂F₅, -CH₂-CF₃, -CF(CF₃)₂, Isopropyl, -CH(CH₃)(O-CH₃),-CH(CH₃)(S-CH₃), sec-Butyl, Isobutyl und tert-Butyl steht.

4. Verbindungen gemäß einem oder mehreren der Ansprüche 1 bis 3, **dadurch gekennzeichnet, dass**
R⁸, R⁹, R¹¹ und R¹², unabhängig voneinander, jeweils für
H; F; Cl; Br; I; -SF₅; -NO₂; -NH₂; -OH; -SH; -C(=O)-NH₂; -S(=O)₂-NH₂;
-NR¹³R¹⁴ ; -NH-R¹⁵; -OR¹⁶; -SR¹⁷; -O-(CH₂)ₐ-R¹⁸; -O-(CH₂)_{b}-OR¹⁹;
-(CH₂)_{c}-O-(CH₂)_{d}-OR²⁰;
-(CH₂)ₑ-O-C(=O)-R²¹;
-(CH₂)_{f}-O-C(=O)-OR²²;
-NR²³S(=O)₂R²⁴;
-(CH₂)_{g}-C(=O)-NR²⁵R²⁶;
-(CH₂)ₕ-C(=O)-NH-R²⁷;
-S(=O)ᵢR²⁸;
-(CH₂)ⱼ-S(=O)₂-NR²⁹R³⁰;
-(CH₂)ₖ-S(=O)₂-NHR³¹;
-(CH₂)ₗ-NR³²-C(=O)-(CH₂)_{q}-OR³³;
-(CH₂)ᵣ-NH-C(=O)-(CH₂)ₛ-OR³⁴;
-(CH₂)ₜ-NR³⁵-O-C(=O)-OR³⁵;
-(CH₂)ᵤ-NH-O-C(=O)-OR³⁷;
-(CH₂)ᵥ-O-S(=O)₂-R³⁸;
-(CH₂)_{w}-NR³⁹-C(=O)-SR⁴⁰;
-(CH₂)_{y}-C(=O)-NH-OR⁴¹;
-P(=O)-(OR⁴²)₂;
-(CH₂)_{z}-C(=S)-NR⁴³R⁴⁴;
-(CH₂)ₐₐ-C(=S)-NH-R⁴⁵;
-(CH₂)_{bb}-NR⁴⁶-C(=O)-R⁴⁷;
-(CH₂)_{cc}-NH-C(=O)-R⁴⁸;
oder -NH-C(=NH)-NH₂ stehen;
wobei
a, b, c, d. q und s, unabhängig voneinander, jeweils gleich 1, 2, 3, 4 oder 5 sind,
e, f, g, h, j, k, l, r, t, u, v, w, x, y, z, aa, bb und cc, unabhängig voneinander, jeweils gleich 0, 1, 2, 3, 4 oder 5 sind, und
i gleich 1 oder 2 ist;
für einen Rest ausgewählt aus der Gruppe bestehend aus Methyl, -CF₃, -CCl₃, -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃, -CH₂-N(CH₃)₂, -CH₂-N(C₂H₅)₂, -CH₂-N(CH₃)(C₂Hₛ), Ethyl, -CH₂-CH₂-NH₂, -CH₂-CH₂-OH, -CH₂-CH₂-SH, - CH₂-CH₂-NH-CH₃, -CH₂-CH₂-N(CH₃)₂, -CH₂-CH₂-N(C₂H₅)₂, -CH₂-CH₂-N(CH₃)(C₂H₅), -CH₂-CF₃, -C₂F₅, -CH₂-CCl₃, -CH₂-CBr₃, -CH₂-CH₂-CN, n-Propyl, -CH₂-CH₂-CH₂-OH, -CH₂-CH₂-CH₂-SH, -CH₂-CH₂-CH₂-NH₂,-CH₂-CH₂-CH₂-NH-CH₃, -CH₂-CH₂-CH₂-N(CH₃)₂, -CH₂-CH₂-CH₂-N(C₂H₅)₂, -CH₂-CH₂-CH₂-N(CH₃)(C₂H₅), -CH₂-CH₂-O-CH₃, -CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), -CH(CH₃)(S-CH₃), n-Butyl, -CF₂-CF₂-CF₂-CF₃, -CH₂-CH₂-CH₂-CH₂-CN, sec-Butyl, Isobutyl, tert-Butyl, n-Pentyl, sec-Pentyl, neo-Pentyl, n-Hexyl, Vinyl, 1-Propenyl, 2-Propenyl, 1-Butenyl, 2-Butenyl, 3-Butenyl, 2-Methyl-buten-2-yl, (1,1,2)-Trifluor-1-butenyl, 1-Pentenyl, 2-Pentenyl, 3-Pentenyl und 4-Pentenyl stehen;
oder für einen Aryl-Rest ausgewählt aus der Gruppe bestehend aus Phenyl und Naphthyl stehen, wobei der Aryl-Rest jeweils mit 1, 2, 3, 4 oder 5 Substituenten unabhängig voneinander ausgewählt aus der Gruppe bestehend aus F, Cl, Br, I, -CN, -NO₂, -OH, -SH, Methyl, Ethyl,-CF₃, -O-CF₃, -S-CF₃, -SF₅, -O-CH₃, -O-C₂H₅, -NH₂, -N(CH₃)₂, -N(C₂H₅)₂, -N(H)(CH₃) und -N(H)(C₂H₅) substituiert sein kann;
und
R¹⁰ für -NR²³S(=O)₂R²⁴ steht.

5. Verbindungen gemäß einem oder mehreren der Ansprüche 1 bis 4, **dadurch gekennzeichnet, dass**
R⁸, R⁹, R¹¹ und R¹², unabhängig voneinander, jeweils für
H; F; Cl; Br; I; -NO₂; -NH₂; -OH; -SH; -NR¹³R¹⁴; -NH-R¹⁵; -OR¹⁶; -SR¹⁷;-NR²³S(=O)₂R²⁴;
oder für einen Rest ausgewählt aus der Gruppe bestehend aus Methyl, -CF₃, Ethyl, -CH₂-CF₃, -C₂F₅, n-Propyl, Isopropyl, n-Butyl, sec-Butyl, Isobutyl, tert-Butyl oder n-Pentyl stehen
und
R¹⁰ für -NR²³S(=O)₂R²⁴ steht.

6. Verbindungen gemäß einem oder mehreren der Ansprüche 1 bis 5, **dadurch gekennzeichnet, dass**
der Rest R¹⁰ für -NR²³S(=O)₂R²⁴ steht und die Reste R⁸, R⁹, R¹¹ und R¹² jeweils für H stehen.

7. Verbindungen gemäß einem oder mehreren der Ansprüche 1 bis 6, **dadurch gekennzeichnet, dass**
R¹³, R¹⁴, R¹⁵, R¹⁶, R¹⁷, R¹⁹, R²⁰, R²¹, R²², R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, R⁴², R⁴³, R⁴⁴, R⁴⁵, R⁴⁶, R⁴⁷ und R⁴⁸, unabhängig voneinander, jeweils
für einen Rest ausgewählt aus der Gruppe bestehend aus Methyl, -CF₃, -CCl₃. -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃, -CH₂-N(CH₃)₂, -CH₂-N(C₂H₅)₂, -CH₂-N(CH₃)(C₂H₅), Ethyl, -CH₂-CH₂-NH₂, -CH₂-CH₂-OH, -CH₂-CH₂-SH,-CH₂-CH₂-NH-CH₃, -CH₂-CH₂-N(CH₃)₂, -CH₂-CH₂-N(C₂Hs)2, -CH₂-CH₂-N(CH₃)(C₂H₅), -CH₂-CF₃, -C₂F₅, -CH₂-CCl₃, -CH₂-CBr₃, -CH₂-CH₂-CN, n-Propyl, -CH₂-CH₂-CH₂-OH, -CH₂-CH₂-CH₂-SH, -CH₂-CH₂-CH₂-NH₂,-CH₂-CH₂-CH₂-NH-CH₃, -CH₂-CH₂-CH₂-N(CH₃)₂, -CH₂-CH₂-CH₂-N(C₂H₅)₂, -CH₂-CH₂-CH₂-N(CH₃)(C₂H₅), -CH₂-CH₂-O-CH₃, -CF₂-CF₂-CF₃, -CF(CF₃)₂, Isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), -CH(CH₃)(S-CH₃), n-Butyl, -CF₂-CF₂-CF₂-CF₃, -CH₂-CH₂-CH₂-CH₂-CN, sec-Butyl, Isobutyl, tert-Butyl, n-Pentyl, sec-Pentyl, neo-Pentyl, n-Hexyl, Vinyl, 1-Propenyl, 2-Propenyl, 1-Butenyl, 2-Butenyl, 3-Butenyl, 2-Methyl-buten-2-yl, (1,1,2)-Trifluor-1-butenyl, 1-Pentenyl, 2-Pentenyl, 3-Pentenyl und 4-Pentenyl stehen;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl stehen, wobei der (hetero)cycloaliphatische Rest jeweils ggf. mit 1, 2, 3, 4 oder 5 Substituenten unabhängig voneinander ausgewählt aus der Gruppe bestehend aus Thioxo (=S), F, Cl, Br, I, -CN, -CF₃, -SF₅, -OH, -NH₂, -O-CF₃, -SH, -O-CH₃, -O-C₂H₅, Methyl, Ethyl, n-Propyl, Isopropyl, n-Butyl, sec-Butyl, Isobutyl, tert-Butyl, -S-CH₃, -S-C₂H₅, -C(=O)-O-CH₃, -C(=O)-O-C₂H₅, Oxo (=O), -N(CH₃)₂, -N(C₂H₅)₂, -N(H)(CH₃), -N(H)(C₂H₅), -NO₂, -SCF₃, -C(=O)-OH, -O-Phenyl, -O-Benzyl, Phenyl und Benzyl substituiert sein kann,
oder für einen Rest ausgewählt aus der Gruppe bestehend aus Phenyl, Naphthyl, (1,3)-Benzodioxolyl, (1,4)-Benzodioxany), Thiophenyl, Furanyl, Pyrrolyl, Pyrazolyl, Pyrazinyl, Pyranyl, Triazolyl, Pyridinyl, imidazolyl, indoiyi, isoindoiyi, Benzo[b]furanyi, Benzo[b]thiophenyl, Thiazolyl, Oxazolyl, Isoxazolyl, Pyridazinyl, Pyrazinyl, Pyrimidinyl, Indazolyl, Chinoxalinyl, Chinolinyl und Isochinolinyl stehen, wobei der Rest jeweils ggf. mit 1, 2, 3, 4 oder 5 Substituenten unabhängig voneinander ausgewählt aus der Gruppe bestehend aus F, Cl, Br, I,-CN, -NO₂, -OH, -SH, Methyl, Ethyl, n-Propyl, Isopropyl, n-Butyl, sec-Butyl, Isobutyl, tert-Butyl, -CF₃, -O-CF₃, -S-CF₃, -SF₅, -O-CH₃, -O-C₂H₅, -O-Phenyl, -O-Benzyl, -NH₂, -N(CH₃)₂, -N(C₂H₅)₂, -N(H)(CH₃) und-N(H)(C₂H₅) substituiert sein kann.

8. Verbindungen gemäß einem oder mehreren der Ansprüche 1 bis 7, **dadurch gekennzeichnet, dass**
R¹⁸ für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der (hetero)cycloaliphatische Rest jeweils ggf. mit 1, 2, 3, 4 oder 5 Substituenten unabhängig voneinander ausgewählt aus der Gruppe bestehend aus Thioxo (=S), F, Cl, Br, I, -CN, -CF₃, -SF₅, -OH, -NH₂, -O-CF₃, -SH, -O-CH₃, -O-C₂H₅, Methyl, Ethyl, n-Propyl, Isopropyl, n-Butyl, sec-Butyl, Isobutyl, tert-Butyl, -S-CH₃, -S-C₂H₅, -C(=O)-O-CH₃, -C(=O)-O-C₂H₅, Oxo (=O), -N(CH₃)₂, -N(C₂H₅)₂, -N(H)(CH₃), -N(H)(C₂H₅), -NO₂, -SCF₃, -C(=O)-OH, -O-Phenyl, -O-Benzyl, Phenyl und Benzyl substituiert sein kann,
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinoxalinyl, Chinolinyl und Isochinolinyl steht, wobei der Rest jeweils 99f. mit 1, 2, 3, 4 oder 5 Subsiiiuenten unabhängig voneinander ausgewählt aus der Gruppe bestehend aus F, Cl, Br, I,-CN, -NO₂, -OH, -SH, Methyl, Ethyl, n-Propyl, Isopropyl, n-Butyl, sec-Butyl, Isobutyl, tert-Butyl, -CF₃, -O-CF₃, -S-CF₃, -SF5_{,} -O-CH₃, -O-C₂H₅, -O-Phenyl, -O-Benzyl, -NH₂, -N(CH₃)₂, -N(C₂H₅)₂, -N(H)(CH₃),-N(H)(C₂H₅) und -NH-C(=O)-O-CH₃ substituiert sein kann.

9. Verbindungen gemäß einem oder mehreren der Ansprüche 1 bis 8, **dadurch gekennzeichnet, dass**
R²³ für einen Wasserstoff-Rest steht
oder für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, neo-Pentyl und n-Hexyl steht.

10. Verbindungen gemäß einem oder mehreren der Ansprüche 1 bis 9, **dadurch gekennzeichnet, dass**
R²⁴ für einen Rest ausgewählt aus der Gruppe bestehend aus Methyl, -CH₂-CN, Ethyl, -CH₂-CH₂-CN, n-Propyl, Isopropyl, n-Butyl, sec-Butyl, Isobutyl, tert-Butyl, n-Pentyl, sec-Pentyl, neo-Pentyl und n-Hexyl steht.

11. Verbindungen gemäß einem oder mehreren der Ansprüche 1 bis 10, **dadurch gekennzeichnet, dass**
p1 und p2, unabhängig voneinander, jeweils für 0 oder 1 stehen, wobei die Summe von p1 und p2 0 oder 1 beträgt.

12. Verbindungen gemäß einem oder mehreren der Ansprüche 1 bis 11, **dadurch gekennzeichnet, dass**
X für O, S oder N-C≡N steht;
m gleich 1 ist;
n gleich 1 ist;
p1 und p2, unabhängig voneinander, jeweils für 0 oder 1 stehen, wobei die Summe von p1 und p2 0 oder 1 beträgt;
R¹, R², R⁴, R⁵, R⁸, R⁹, R¹¹ und R¹², unabhängig voneinander, jeweils für
H; F; Cl; I; Br; -NO₂; -NH₂; -OH; -SH; -NR¹³R¹⁴; -NH-R¹⁵; -OR¹⁶; -SR¹⁷; NR²³S(=O)2R²⁴_{;}
oder für einen Rest ausgewählt aus der Gruppe bestehend aus Methyl, -CF₃, Ethyl, -CH₂-CF₃, -C₂F₅, n-Propyl, Isopropyl, n-Butyl, sec-Butyl, Isobutyl, tert-Butyl oder n-Pentyl stehen;
R³ für F; Cl; Br; I; -OR¹⁶; -NR²³S(=O)₂R²⁴;
oder für einen Rest ausgewählt aus der Gruppe bestehend aus -SF₅,-CF₃, -C₂F₅, -CH₂-CF₃, -CF(CF₃)₂, Isopropyl, -CH(CH₃)(O-CH₃),-CH(CH₃)(S-CH₃), sec-Butyl, Isobutyl und tert-Butyl steht.
R⁶ und R⁷ jeweils für einen Wasserstoff-Rest stehen
oder
R⁶ und R⁷ zusammen mit der sie verbindenden -C-C-Brücke einen unsubstituierten Phenylen-Rest bilden;
R¹⁰ für -NR ²3S(=O)₂R²⁴ steht;
R¹³, R¹⁴, R¹⁵, R¹⁶ und R¹⁴, unabhängig voneinander, jeweils
für einen Rest ausgewählt aus der Gruppe bestehend aus -CF₃, -C₂F₅, -CH₂CF₃, Methyl, Ethyl, n-Propyl, Isopropyl, n-Butyl, sec-Butyl, Isobutyl, tert-Butyl, n-Pentyl, sec-Pentyl, neo-Pentyl und n-Hexyl stehen;
oder für einen Rest ausgewählt aus der Gruppe bestehend aus Phenyl, Thiophenyl, Furanyl und Pyridinyl stehen, wobei der Rest jeweils ggf. mit 1, 2, 3, 4 oder 5 Substituenten unabhängig voneinander ausgewählt aus der Gruppe bestehend aus F, Cl, Br, 1, -CN, -NO₂, -OH, -SH, Methyl, Ethyl, n-Propyl, Isopropyl, n-Butyl, sec-Butyl, Isobutyl, tert-Butyl, -CF₃, -O-CF₃, -S-CF₃, -SF₅, -O-CH₃ und -O-C₂H₅ substituiert sein kann;
R²³ für einen Wasserstoff-Rest steht
und R²⁴ für einen Rest ausgewählt aus der Gruppe bestehend aus Methyl, -CH₂-CN, Ethyl, -CH₂-CH₂-CN, n-Propyl, Isopropyl, n-Butyl, sec-Butyl, Isobutyl, tert-Butyl, n-Pentyl, sec-Pentyl, neo-Pentyl und n-Hexyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

13. Verbindungen gemäß einem oder mehreren der Ansprüche 1 bis 12, **dadurch gekennzeichnet, dass**
X für O, S oder N-C≡N steht;
m gleich 1 ist;
n gleich 1 ist;
p1 und p2, unabhängig voneinander, jeweils für 0 oder 1 stehen, wobei die Summe von p1 und p2 0 oder 1 beträgt;
R¹, R², R⁴, R⁵, R⁸, R⁹, R¹¹ und R¹² jeweils für H stehen;
R³ für -OR¹⁶; -NR²³S(=O)₂R²⁴;
oder für einen Rest ausgewählt aus der Gruppe bestehend aus bestehend aus -CF₃, Isopropyl, sec-Butyl, Isobutyl und tert-Butyl steht;
R⁶ und R⁷ jeweils für einen Wasserstoff-Rest stehen
oder
R⁶ und R⁷ zusammen mit der sie verbindenden -C-C-Brücke einen unsubstituierten Phenylen-Rest bilden;
R¹⁰ für -NR²³S(=O)₂R²⁴ steht;
R¹⁶ für einen Rest ausgewählt aus der Gruppe bestehend aus -CF₃, -C₂F₅ und -CH₂CF₃ steht;
R²³ für einen Wasserstoff-Rest steht
und
R²⁴ für einen Rest ausgewählt aus der Gruppe bestehend aus Methyl, Ethyl, n-Propyl, isopropyl, n-Butyl, sec-Butyl, isobutyl, tert-Butyl, n-Pentyl, sec-Pentyl, neo-Pentyl und n-Hexyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

14. Verbindungen gemäß einem oder mehreren der Ansprüche 1 bis 13 ausgewählt aus der Gruppe bestehend aus
[1] N-[4-[3-(4-tert-Butyl-benzyl)-2-thioxo-imidazolidin-ylmethyl]-phenyl]-methansulfonamid,
[2] N-[4-[3-(4-tert-Butyl-benzyl)-2-thioxo-2,3-dihydro-benzoimidazol-1-ylmethyl]-phenyl]-methansulfonamid,
[3] N-[4-[3-(4-tert-Butyl-benzyl)-2-thioxo-tetrahydro-pyrimidin-1-ylmethyl]-phenyl]-methansulfonamid,
[4] N-[4-[3-(4-tert-Butyl-benzyl)-2-oxo-imidazolidin-1-ylmethyl]phenyl]-methansulfonamid,
[5] N-[4-[3-(4-tert-Butyl-benzyl)-2-oxo-tetrahydro-pyrimidin-1-ylmethyl]-phenyl]-methansulfonamid
[6] N-[4-[3-(4-tert-Butyl-benzyl)-2-oxo-2,3-dihydro-benzoimidazol-1-ylmethyl]-phenyl]-methansulfonamid,
[7] N-[4-[3-(4-Triftuormethoxybenzy)-2-oxo-2,3-dihydrobenzimidazol-1-ylmethyl]-phenyl]-methansulfonamid,
[8] N-[4-[3-(4-Trifluormethoxybenzyl)-2-thioxo-2,3-dihydrobenzimidazol-1-ylmethyl]-phenyl]-methansulfonamid,
[9] N-[4-[3-(4-Methansulfonylaminobenzyl)-2-oxo-2,3-dihydrobenzimidazol-1-ylmethyl]-phenyl]-methansulfonamid und
[10] N-{4-[2-Oxo-3-(4-trifluormethylbenzyl)-2,3-dihydrobenzimidazol-1-ylmethyl]phenyl}methansulfonamid;
ggf. jeweils in Form entsprechender Salze, oder jeweils in Form entsprechender Solvate.

15. Verfahren zur Herstellung substituierter zyklischer Harnstoff-Derivate gemäß einem oder mehreren der Ansprüche 1 bis 14, **dadurch gekennzeichnet, dass** wenigstens eine Verbindung der allgemeinen Formel II, 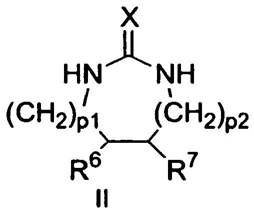 worin R⁶, R⁷, X, p1 und p2 die Bedeutung gemäß einem oder mehreren der Ansprüche 1 bis 14 haben, in einem Reaktionsmedium, ggf. in Gegenwart wenigstens einer Base, vorzugsweise in Gegenwart wenigstens eines Metallhydridsalzes, besonders bevorzugt in Gegenwart von Kalium- und/oder Natriumhydrid,
oder in Gegenwart wenigstens eines Alkalimetallcarbonatsalzes, vorzugsweise in Gegenwart von Kalium- und/oder Natriumcarbonat, und wenigstens eines Alkalimetalliodides, vorzugsweise Kalium- und/oder Natriumiodid, mit wenigstens einer Verbindung der allgemeinen Formel III, 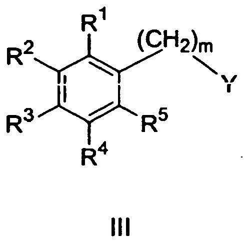 worin R¹ bis R⁵ und m die Bedeutung gemäß einem oder mehreren der Ansprüche 1 bis 14 haben und Y für eine Abgangsgruppe, vorzugsweise für einen Halogen-Rest, besonders bevorzugt ein Brom- oder Chloratom steht, zu wenigstens einer Verbindung der allgemeinen Formel IV, 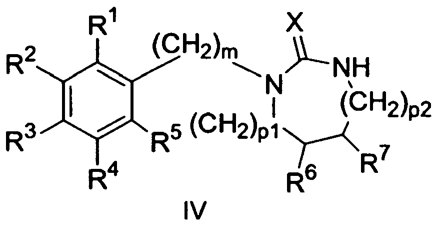 worin R¹ bis R⁷, X, p1, p2 und m die vorstehend genannte Bedeutung haben, umgesetzt wird und diese ggf. gereinigt und/oder isoliert wird,
oder wenigstens eine Verbindung der allgemeinen Formel V, 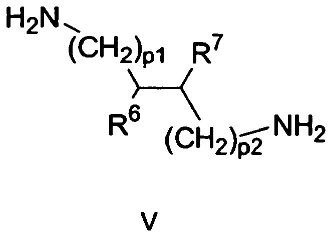 worin R⁶, R⁷, p1 und p2 die Bedeutung gemäß einem oder mehreren der Ansprüche 1 bis 14 haben, in einem Reaktionsmedium, ggf. in Gegenwart wenigstens einer Base, vorzugsweise wenigstens einer Base ausgewählt aus der Gruppe bestehend aus Pyridin, Triethylamin und Düsopropylethylamin, mit wenigstens einer Verbindung der allgemeinen Formel III zu wenigstens einer Verbindung der allgemeinen Formel VI, 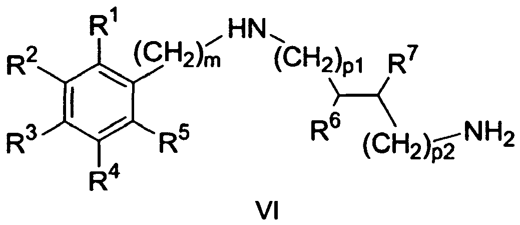 worin R¹ bis R⁷, m, p1 und p2 die vorstehend genannte Bedeutung haben, umgesetzt wird und diese ggf. gereinigt und/oder isoliert wird und wenigstens eine Verbindung der allgemeinen Formel VI in einem Reaktionsmedium, ggf. in Gegenwart wenigstens einer Base, vorzugsweise wenigstens einer Base ausgewählt aus der Gruppe bestehend aus Pyridin, Triethylamin und Diisopropylethylamin, mit wenigstens einer Verbindung der allgemeinen Formel Z-C(=X)-Z, worin X für ein Sauerstoff- oder Schwefelatom steht und Z jeweils für eine Abgangsgruppe, vorzugsweise jeweils für einen Halogen-Rest, besonders bevorzugt jeweils für ein Chloratom, steht, zu wenigstens einer Verbindung der allgemeinen Formel IV umgesetzt wird und diese ggf. gereinigt und/oder isoliert wird
oder
wenigstens eine Verbindung der allgemeinen Formel VII, 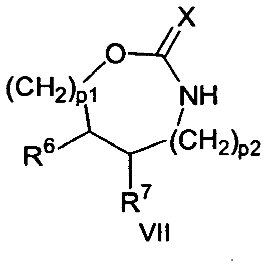 worin R⁶, R⁷, X, p1 und p2 die vorstehend genannte Bedeutung haben, in einem Reaktionsmedium mit wenigstens einer Verbindung der allgemeinen Formel VIII, 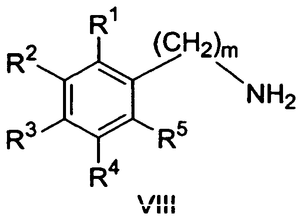 worin R¹ bis R⁵ und m die vorstehend genannte Bedeutung haben, zu wenigstens einer Verbindung der allgemeinen Formel IV umgesetzt wird, und diese ggf. gereinigt und/oder isoliert wird,
und wenigstens eine Verbindung der allgemeinen Formel IV in einem Reaktionsmedium, in Gegenwart wenigstens einer Base, vorzugsweise in Gegenwart wenigstens eines Metallhydridsalzes, besonders bevorzugt in Gegenwart von Kalium- und/oder Natriumhydrid,
oder in Gegenwart wenigstens eines Alkalimetallcarbonatsalzes, vorzugsweise in Gegenwart von Kalium- und/oder Natriumcarbonat, und wenigstens eines Alkalimetalliodides, vorzugsweise Kalium- und/oder Natriumiodid, mit wenigstens einer Verbindung der allgemeinen Formel IX, 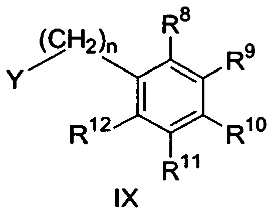 worin R⁸ bis R¹² und n die Bedeutung gemäß einem oder mehreren der Ansprüche 1 bis 14 haben, mit der Maßgabe, dass wenigstens einer der Substituenten R⁸ bis R¹² für eine -N-(PG)₂-Gruppe steht, wobei PG jeweils für eine Schutzgruppe steht oder zwei PG-Gruppen zusammen mit dem sie verbindenden Stickstoffatom eine zyklische Schutzgruppe, bevorzugt zusammen mit dem sie verbindenden Stickstoffatom eine Phthalimid-Gruppe, bilden, und Y für eine Abgangsgruppe, vorzugsweise für ein Halogenatom, besonders bevorzugt für ein Chlor- oder Bromatom steht, zu wenigstens einer Verbindung der allgemeinen Formel XI, 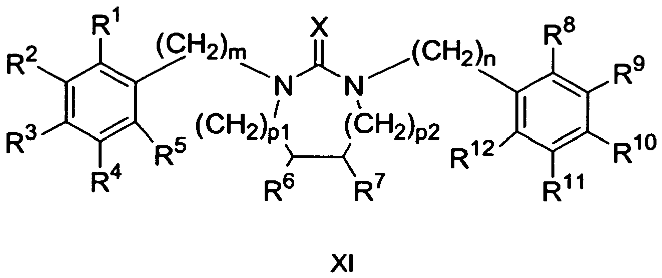 worin R¹ bis R¹², X, m, n, p1 und p2 die vorstehend genannte Bedeutung haben, mit der Maßgabe, dass wenigstens einer der Substituenten R⁸ bis R¹² für eine -N-(PG)₂-Gruppe steht, wobei PG die vorstehend genannte Bedeutung hat, umgesetzt wird und diese ggf. gereinigt und/oder isoliert wird und wenigstens eine Verbindung der allgemeinen Formel XI in einem Reaktionsmedium in Gegenwart wenigstens einer Base, vorzugsweise in Gegenwart von Dimethylamin, oder in Gegenwart wenigstens einer Säure oder in Gegenwart von Hydrazin und/oder Phenylhydrazin oder in Gegenwart wenigstens eines Alkalimetallborhydrids, vorzugsweise in Gegenwart von Natriumborhydrid, zu wenigstens einer Verbindung der allgemeinen Formel XII, 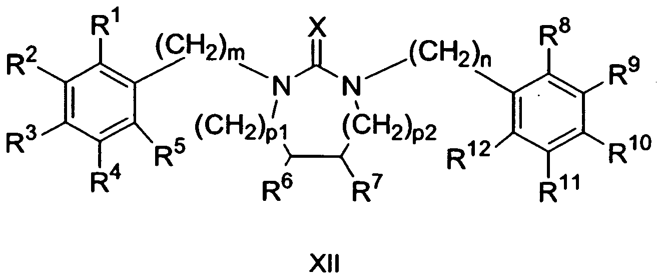 worin R¹ bis R¹², X, m, n, p1 und p2 die vorstehend genannte Bedeutung haben, mit der Maßgabe, dass wenigstens einer der Substituenten R⁸ bis R¹² für eine -NH₂-Gruppe steht, umgesetzt wird und diese ggf. gereinigt und/oder isoliert wird,
oder
wenigstens eine Verbindung der allgemeinen Formel IV in einem Reaktionsmedium, in Gegenwart wenigstens einer Base, vorzugsweise in Gegenwart wenigstens eines Metallhydridsalzes, besonders bevorzugt in Gegenwart von Kalium- und/oder Natriumhydrid,
oder in Gegenwart wenigstens eines Alkalimetallcarbonatsalzes, vorzugsweise in Gegenwart von Kalium- und/oder Natriumcarbonat, und wenigstens eines Alkalimetalliodides, vorzugsweise Kalium- und/oder Natriumiodid, mit wenigstens einer Verbindung der allgemeinen Formel XIII, 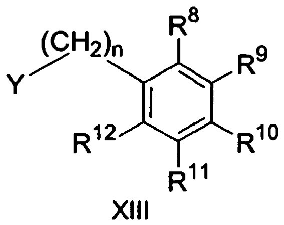 worin R⁸ bis R¹² und n die Bedeutung gemäß einem oder mehreren der Ansprüche 1 bis 14 haben, mit der Maßgabe, dass wenigstens einer der Substituenten R^{a} bis R¹² für eine -NO₂-Gruppe steht, und Y für eine Abgangsgruppe, vorzugsweise für einen Halogen-Rest, besonders bevorzugt für ein Chlor- oder Bromatom, steht, zu wenigstens einer Verbindung der allgemeinen Formel XIV, 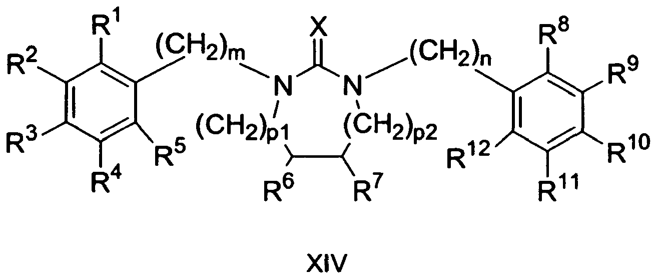 worin R¹ bis R¹², X, m, n, p1 und p2 die vorstehend genannte Bedeutung haben, mit der Maßgabe, dass wenigstens einer der Substituenten R⁸ bis R¹² für eine -NO₂-Gruppe steht, umgesetzt wird und diese ggf. gereinigt und/oder isoliert wird, und wenigstens eine Verbindung der allgemeinen Formel XIV in einem Reaktionsmedium in Gegenwart von Wasserstoff und in Gegenwart eines Katalysator zu wenigstens einer Verbindung der allgemeinen Formel XII, worin R¹ bis R¹², X, m, n, p1 und p2 die vorstehend genannte Bedeutung haben, mit der Maßgabe, dass wenigstens einer der Substituenten R⁸ bis R¹² für eine -NH₂-Gruppe steht, umgesetzt wird und diese ggf. gereinigt und/oder isoliert wird,
und wenigstens eine Verbindung der allgemeinen Formel XII in einem Reaktionsmedium ggf. in Gegenwart wenigstens einer Base mit wenigstens einer Verbindung der allgemeinen Formel R²⁴-S(=O)₂-Z, worin R²⁴ die Bedeutung gemäß einem oder mehreren der Ansprüche 1 bis 14 hat und Z für eine Abgangsgruppe, bevorzugt für ein Halogenatom, besonders bevorzugt für ein Chloratom, steht, zu wenigstens einer Verbindung der allgemeinen Formel Ia, 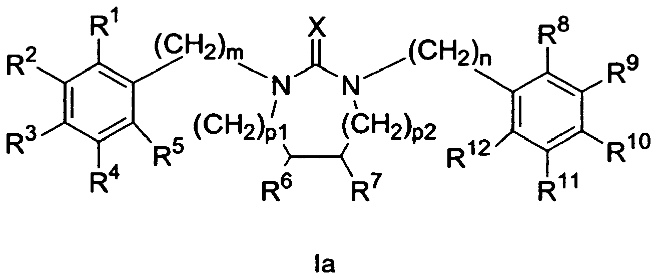 worin R¹ bis R¹², X, m, n, p1 und p2 die vorstehend genannte Bedeutung haben, mit der Maßgabe, dass wenigstens einer der Substituenten R⁸ bis R¹² für eine -NH-S(=O)₂-R²⁴-Gruppe steht, wobei R²⁴ die vorstehend genannte Bedeutung hat, umgesetzt wird, und diese ggf. gereinigt und/oder isoliert wird oder wenigstens eine Verbindung der allgemeinen Formel IV in einem Reaktionsmedium, in Gegenwart wenigstens einer Base, vorzugsweise in Gegenwart wenigstens eines Metallhydridsalzes, besonders bevorzugt in Gegenwart von Kalium- und/oder Natriumhydrid,
oder in Gegenwart wenigstens eines Alkalimetallcarbonatsalzes, vorzugsweise in Gegenwart von Kalium- und/oder Natriumcarbonat, und wenigstens eines Alkalimetalliodides, vorzugsweise Kalium- und/oder Natriumiodid, mit wenigstens einer Verbindung der allgemeinen Formel XV, 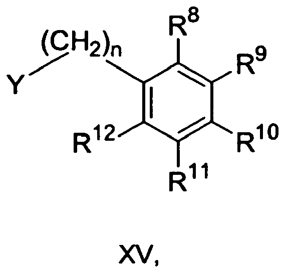 worin R² bis R¹² und n die Bedeutung gemäß einem oder mehreren der Ansprüche 1 bis 14 haben, mit der Maßgabe, dass wenigstens einer der Substituenten R⁸ bis R¹² für eine -NH-S(=O)₂-R²⁴-Gruppe, wobei R²⁴ die vorstehend genannte Bedeutung hat, steht, zu wenigstens einer Verbindung der allgemeinen Formel Ia umgesetzt wird, und diese ggf. gereinigt und/oder isoliert wird
und ggf. wenigstens eine Verbindung der allgemeinen Formel Ia, in einem Reaktionsmedium, ggf. in Gegenwart wenigstens einer Base, mit wenigstens einer Verbindung der allgemeinen Formel R²³-Z, worin R²³ die Bedeutung gemäß einem oder mehreren der Ansprüche 1 bis 14 mit Ausnahme von Wasserstoff hat und Z für eine Abgangsgruppe, bevorzugt für ein Halogenatom, besonders bevorzugt für ein Chloratom, steht, zu wenigstens einer Verbindung der allgemeinen Formel Ib, 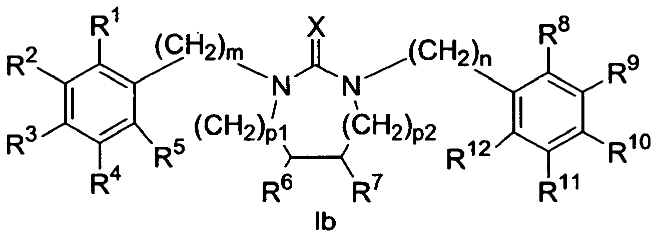 worin R¹ bis R¹², X, m, n, p1 und p2 die vorstehend genannte Bedeutung haben, mit der Maßgabe, dass wenigstens einer der Substituenten R⁸ bis R¹² für eine -NR²³-S(=O)₂-R²⁴-Gruppe steht, wobei R²³ und R²⁴ die vorstehend genannte Bedeutung haben, umgesetzt wird, und diese ggf. gereinigt und/oder und ggf. wenigstens eine Verbindung der allgemeinen Formel Ib, worin R¹ bis R¹², m, n, p1 und p2 die vorstehend genannte Bedeutung haben und X für ein Sauerstoffatom steht, mit der Maßgabe, dass wenigstens einer der Substituenten R⁸ bis R¹² für eine -NR²³-S(=O)₂-R²⁴-Gruppe steht, wobei R²³ und R²⁴ die vorstehend genannte Bedeutung haben, in einem Reaktionsmedium mit wenigstens einer Verbindung der allgemeinen Formel XVI, 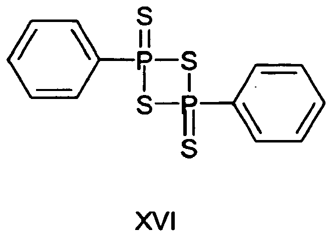 worin die Phenyl-Reste jeweils mit 1 oder 2 Substituenten unabhängig voneinander ausgewählt aus der Gruppe bestehend aus Methoxy, Phenoxy, Cl, Methyl und Br, bevorzugt jeweils mit einem Phenoxy-Rest oder Methoxy-Rest, besonders bevorzugt jeweils mit einem Methoxy-Rest in para-Position, substituiert sind, oder mit Phosphorpentasulfid, zu wenigstens einer Verbindung der allgemeinen Formel Ib, worin R¹ bis R¹², m, n und p die vorstehend genannte Bedeutung haben und X für ein Schwefelatom steht, mit der Maßgabe, dass wenigstens einer der Substituenten R⁸ bis R¹² für eine - NR²³-S(=O)₂-R²⁴- Gruppe steht, wobei R²³ und R²⁴ die vorstehend genannte Bedeutung haben, umgesetzt wird, und diese ggf. gereinigt und/oder isoliert wird.

16. Verfahren zur Herstellung substituierter zyklischer Harnstoff-Derivate gemäß einem oder mehreren der Ansprüche 1 bis 14, **dadurch gekennzeichnet, dass** wenigstens eine Verbindung der allgemeinen Formel II, 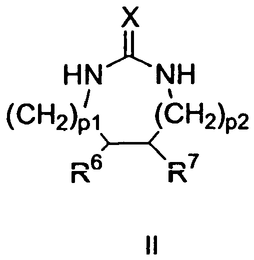 worin R⁶, R⁷, X, p1 und p2 die Bedeutung gemäß einem oder mehreren der Ansprüche 1 bis 14 haben, in einem Reaktionsmedium, in Gegenwart wenigstens einer Base, vorzugsweise in Gegenwart wenigstens eines Metallhydridsalzes, besonders bevorzugt in Gegenwart von Kalium- und/oder Natriumhydrid,
oder in Gegenwart wenigstens eines Alkalimetallcarbonatsalzes, vorzugsweise in Gegenwart von Kalium- und/oder Natriumcarbonat, und wenigstens eines Alkalimetalliodides, vorzugsweise Kalium- und/oder Natriumiodid, mit wenigstens einer Verbindung der allgemeinen Formel XVII, 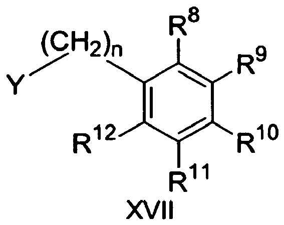 worin R⁸ bis R¹² und n die Bedeutung gemäß einem oder mehreren der Ansprüche 1 bis 14 haben, mit der Maßgabe, dass wenigstens einer der Substituenten R⁸ bis R¹² für eine -N(PG)₂-Gruppe steht, wobei PG jeweils für eine Schutzgruppe steht oder zwei PG-Gruppen zusammen mit dem sie verbindenden Stickstoffatom eine zyklische Schutzgruppe, bevorzugt zusammen mit dem sie verbindenden Stickstoffatom eine Phthalimid-Gruppe, bilden, oder wenigstens einer der Substituenten R⁸ bis R¹² für eine -NO₂-Gruppe steht, und Y für eine Abgangsgruppe, vorzugsweise für ein Halogenatom, besonders bevorzugt für ein Chlor- oder Bromatom steht, zu wenigstens einer Verbindung der allgemeinen Formel XVIII, 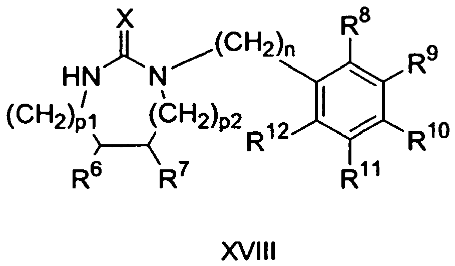 worin R⁶ bis R¹², p1, p2 und n die vorstehend genannte Bedeutung haben, mit der Maßgabe, dass wenigstens einer der Substituenten R⁸ bis R¹² für eine-N(PG)₂-Gruppe oder eine -NO₂-Gruppe steht, umgesetzt wird und diese ggf. gereinigt und/oder isoliert wird
oder
wenigstens eine Verbindung der allgemeinen Formel XIX, 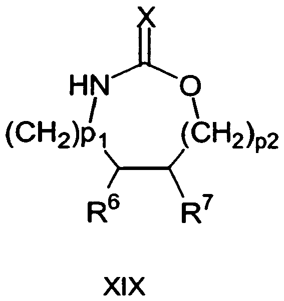 worin R⁶, R⁷, X, p1 und p2 die Bedeutung gemäß einem oder mehreren der Ansprüche 1 bis 14 haben, in einem Reaktionsmedium mit wenigstens einer Verbindung der allgemeinen Form XX, 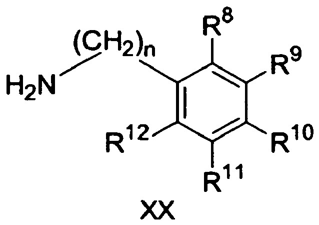 worin R⁸ bis R¹² und n die Bedeutung gemäß einem oder mehreren der Ansprüche 1 bis 14 haben, mit der Maßgabe, dass wenigstens einer der substituenten R⁸ bis R¹² für eine -N(PG)₂-Gruppe steht, wobei PG jeweils für eine Schutzgruppe steht oder zwei PG-Gruppen zusammen mit dem sie verbindenden Stickstoffatom eine zyklische Schutzgruppe, bevorzugt zusammen mit dem sie verbindenden Stickstoffatom eine Phthalimid-Gruppe, bilden, oder wenigstens einer der Substituenten R⁸ bis R¹² für eine -NO₂-Gruppe steht, zu wenigstens einer Verbindung der allgemeinen Formel XVIII umgesetzt wird und diese ggf. gereinigt und/oder isoliert wird,
oder wenigstens eine Verbindung der allgemeinen Formel V, 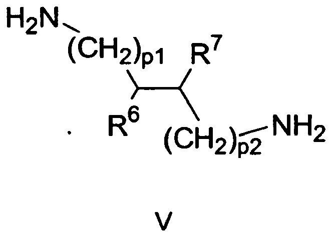 worin R⁶, R⁷, p1 und p2 die Bedeutung gemäß einem oder mehreren der Ansprüche 1 bis 14 haben, in einem Reaktionsmedium, ggf. in Gegenwart wenigstens Base mit wenigstens einer Verbindung der allgemeinen Formel XVII zu wenigstens einer Verbindung der allgemeinen Formel XXI, 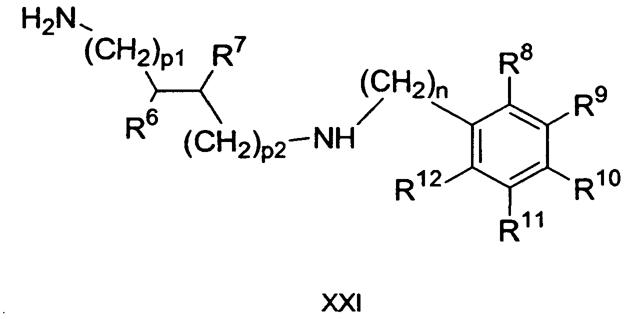 worin R⁶ bis R¹², p1, p2 und n die Bedeutung gemäß einem oder mehreren der Ansprüche 1 bis 14 haben, mit der Maßgabe, dass wenigstens einer der Substituenten R⁸ bis R¹² für eine -N(PG)₂-Gruppe oder eine -NO₂-Gruppe steht, umgesetzt wird und diese ggf. gereinigt und/oder isoliert wird und wenigstens eine Verbindung der allgemeinen Formel XXI in einem Reaktionsmedium, ggf. in Gegenwart wenigstens einer Base mit wenigstens einer Verbindung der allgemeinen Formel Z-C(=X)-Z, worin X für ein Sauerstoff- oder Schwefelatom und Z jeweils für eine Abgangsgruppe, vorzugsweise jeweils für einen Halogen-Rest, besonders bevorzugt jeweils für ein Chloratom steht, zu wenigstens einer Verbindung der allgemeinen Formel XVIII umgesetzt wird und diese ggf. gereinigt und/oder isoliert wird
und wenigstens eine Verbindung der allgemeinen Formel XVIII in einem Reaktionsmedium, in Gegenwart wenigstens einer Base, vorzugsweise in Gegenwart wenigstens eines Metaiihydridsaizes, besonders bevorzugt in Gegenwart von Kalium- und/oder Natriumhydrid,
oder in Gegenwart wenigstens eines Alkalimetallcarbonatsalzes, vorzugsweise in Gegenwart von Kalium- und/oder Natriumcarbonat, und wenigstens eines Alkalimetalliodides, vorzugsweise Kalium- und/oder Natriumiodid, mit wenigstens einer Verbindung der allgemeinen Formel III, 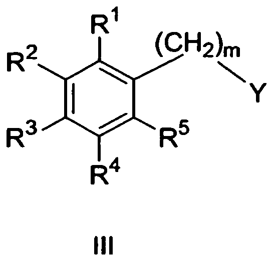 worin R¹ bis R⁵ und m die Bedeutung gemäß einem oder mehreren der Ansprüche 1 bis 14 haben und Y für eine Abgangsgruppe, vorzugsweise für einen Halogen-Rest, besonders bevorzugt ein Chlor- oder Bromatom steht, zu wenigstens einer Verbindung der allgemeinen Formel XXII, 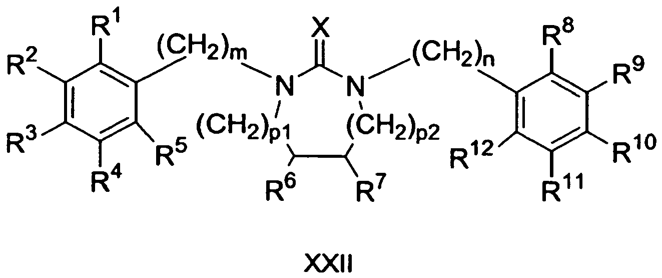 worin R¹ bis R¹², X, m, n, p1 und p2 die Bedeutung gemäß einem oder mehreren der Ansprüche 1 bis 14 haben, mit der Maßgabe, dass wenigstens einer der Substituenten R⁸ bis R¹² für -N(PG)₂-Gruppe oder **-**NO₂-Gruppe steht, umgesetzt wird und diese ggf. gereinigt und/oder isoliert wird
und wenigstens eine Verbindung der allgemeinen Formel XXII in einem Reaktionsmedium in Gegenwart wenigstens einer Base, vorzugsweise in Gegenwart von Dimethylamin, oder in Gegenwart wenigstens einer Säure oder in Gegenwart von Hydrazin und/oder Phenythydrazin oder in Gegenwart wenigstens eines Alkalimetallborhydrids, vorzugsweise in Gegenwart von Natriumborhydrid, oder in Gegenwart von Wasserstoff und eines Katalysators, zu wenigstens einer Verbindung der allgemeinen Formel XII, 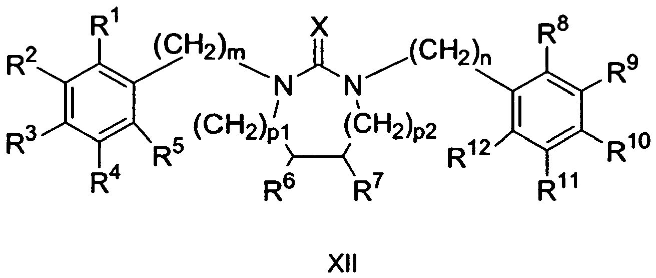 worin R¹ bis R¹², X, m, n, p1 und p2 die vorstehend genannte Bedeutung haben, mit der Maßgabe, dass wenigstens einer der Substituenten R⁸ bis R¹² für eine -NH₂-Gruppe steht, umgesetzt wird und diese ggf. gereinigt und/oder isoliert wird,
und wenigstens eine Verbindung der allgemeinen Formel XII in einem Reaktionsmedium ggf. in Gegenwart wenigstens einer Base mit wenigstens einer Verbindung der allgemeinen Formel R²⁴-S(=O)₂-Z, worin R²⁴ die Bedeutung gemäß einem oder mehreren der Ansprüche 1 bis 14 hat und Z für eine Abgangsgruppe, bevorzugt für ein Halogenatom, besonders bevorzugt für ein Chloratom, steht, zu wenigstens einer Verbindung der allgemeinen Formel Ia, 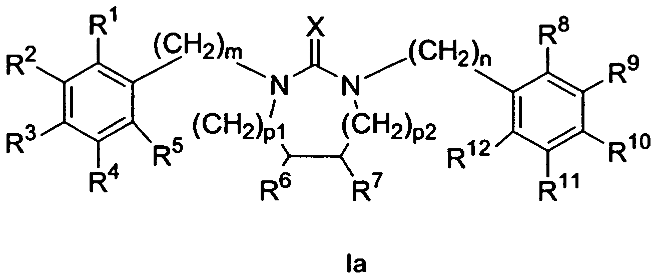 worin R¹ bis R¹², X, m, n, p1 und p2 die vorstehend genannte Bedeutung haben, mit der Maßgabe, dass wenigstens einer der Substituenten R⁸ bis R¹² für eine -NH-S(=O)₂-R²⁴-Gruppe steht, wobei R²⁴ die vorstehend genannte Bedeutung hat, umgesetzt wird, und diese ggf. gereinigt und/oder isoliert wird und ggf. wenigstens eine Verbindung der allgemeinen Formel la in einem Reaktionsmedium, ggf. in Gegenwart wenigstens einer Base, mit wenigstens einer Verbindung der allgemeinen Formel R²³-Z, worin R²³ die Bedeutung gemäß einem oder mehreren der Ansprüche 1 bis 14 mit Ausnahme von Wasserstoff hat und Z für eine Abgangsgruppe, bevorzugt für ein Halogenatom, besonders bevorzugt für ein Chloratom, steht, zu wenigstens einer Verbindung der allgemeinen Formel Ib, 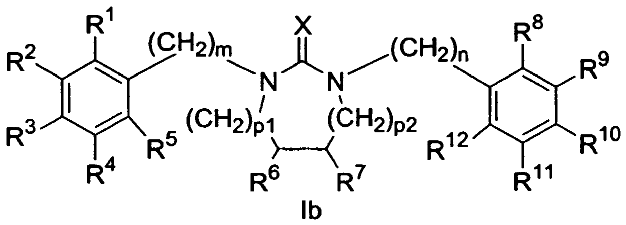 worin R¹ bis R¹², X, m, n, p1 und p2 die vorstehend genannte Bedeutung haben, mit der Maßgabe, dass wenigstens einer der Substituenten R⁸ bis R¹² für eine -NR²³-S(=O)₂-R²⁴-Gruppe steht, wobei R²³ und R²⁴ die vorstehend genannte Bedeutung haben, umgesetzt wird, und diese ggf. gereinigt und/oder isoliert wird,
und ggf. wenigstens eine Verbindung der allgemeinen Formel Ib, worin R¹ bis R¹², m, n, p1 und p2 die vorstehend genannte Bedeutung haben und X für ein Sauerstoffatom steht, mit der Maßgabe, dass wenigstens einer der Substituenten R⁸ bis R¹² für eine -NR²³-S(=O)₂-R²⁴-Gruppe steht, wobei R²³ und R²⁴ die vorstehend genannte Bedeutung haben, in einem Reaktionsmedium mit wenigstens einer Verbindung der allgemeinen Formel XVI, 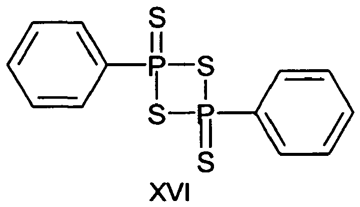 worin die Phenyl-Reste jeweils mit 1 oder 2 Substituenten unabhängig voneinander ausgewählt aus der Gruppe bestehend aus Methoxy, Phenoxy, Cl, Methyl und Br, bevorzugt jeweils mit einem Phenoxy-Rest oder Methoxy-Rest, besonders bevorzugt jeweils mit einem Methoxy-Rest in para-Position, substituiert sind, oder mit Phosphorpentasulfid, zu wenigstens einer Verbindung der allgemeinen Formel Ib, worin R¹ bis R¹², m, n und p die vorstehend genannte Bedeutung haben und X für ein Schwefelatom steht, mit der Maßgabe, dass wenigstens einer der Substituenten R⁸ bis R¹² für eine-NR²³-S(=O)₂-R²⁴-Gruppe steht, wobei R²³ und R²⁴ die vorstehend genannte Bedeutung haben, umgesetzt wird und diese ggf. gereinigt und/oder isoliert wird.

17. Verfahren zur Herstellung substituierter zyklischer Harnstoff-Derivate gemäß einem oder mehreren der Ansprüche 1 bis 14, **dadurch gekennzeichnet, dass** wenigstens eine Verbindung der allgemeinen Formel II, 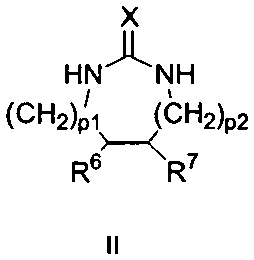 worin R⁶, R⁷, X, p1 und p2 die Bedeutung gemäß einem oder mehreren der Ansprüche 1 bis 14 haben, in einem Reaktionsmedium, in Gegenwart wenigstens einer Base, vorzugsweise in Gegenwart wenigstens eines Metallhydridsalzes, besonders bevorzugt in Gegenwart von Kalium- und/oder Natriumhydrid,
oder in Gegenwart wenigstens eines Alkalimetallcarbonatsalzes, vorzugsweise in Gegenwart von Kalium- und/oder Natriumcarbonat, und wenigstens eines Alkalimetalliodides, vorzugsweise Kalium- und/oder Natriumiodid, mit wenigstens einer Verbindung der allgemeinen Formel XXIII, 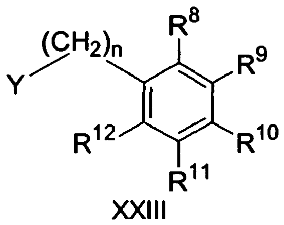 worin R⁸ bis R¹² und n die Bedeutung gemäß einem oder mehreren der Ansprüche 1 bis 14 haben, mit der Maßgabe, dass wenigstens einer der Substituenten R⁸ bis R¹² für eine -NR²³-S(=O)₂-R²⁴-Gruppe steht und Y für eine Abgangsgruppe, vorzugsweise für ein Halogenatom, besonders bevorzugt für ein Chlor- oder Bromatom steht, zu wenigstens einer Verbindung der allgemeinen Formel XXIV, 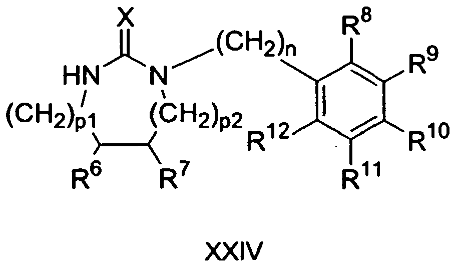 worin R⁶ bis R¹², p1, p2 und n die vorstehend genannte Bedeutung haben, mit der Maßgabe, dass wenigstens einer der Substituenten R⁸ bis R¹² für eine-NR²³-S(=O)₂-R²⁴-Gruppe steht, umgesetzt wird und diese ggf. gereinigt und/oder isoliert wird,
und wenigstens eine Verbindung der allgemeinen Formel XXIV in einem Reaktionsmedium in Gegenwart wenigstens einer Base, vorzugsweise in Gegenwart wenigstens eines Metallhydridsalzes, besonders bevorzugt in Gegenwart von Kalium- und/oder Natriumhydrid,
oder in Gegenwart wenigstens eines Alkalimetallcarbonatsalzes, vorzugsweise in Gegenwart von Kalium- und/oder Natriumcarbonat, und wenigstens eines Alkalimetalliodides, vorzugsweise Kalium- und/oder Natriumiodid, mit wenigstens einer Verbindung der allgemeinen Formel III, 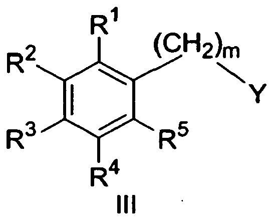 worin R¹ bis R⁵ und m die Bedeutung gemäß einem oder mehreren der Ansprüche 1 bis 14 haben und Y für eine Abgangsgruppe, vorzugsweise für einen Halogen-Rest, besonders bevorzugt für ein Chlor oder Bromatom steht, zu wenigstens einer Verbindung der allgemeinen Formel Ib, 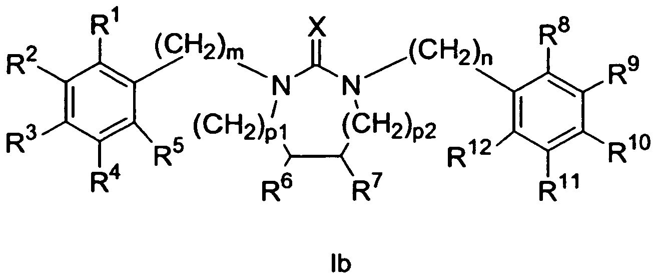 worin R¹ bis R¹², X, m, n, p1 und p2 die vorstehend genannte Bedeutung haben, mit der Maßgabe, dass wenigstens einer der Substituenten R⁸ bis R¹² für eine -NR²³-S(=O)₂-R²⁴-Gruppe steht, wobei R²³ und R²⁴ die vorstehend genannte Bedeutung haben, umgesetzt wird, und diese ggf. gereinigt und/oder isoliert wird,
und ggf. wenigstens eine Verbindung der allgemeinen Formel Ib, worin R¹ bis R¹², m, n, p1 und p2 die vorstehend genannte Bedeutung haben und X für ein Sauerstoffatom steht, mit der Maßgabe, dass wenigstens einer der Substituenten R⁸ bis R¹² für eine -NR²³-S(=O)₂-R²⁴-Gruppe steht, wobei R²³ und R²⁴ die vorstehend genannte Bedeutung haben, in einem Reaktionsmedium mit wenigstens einer Verbindung der allgemeinen Formel XVI, 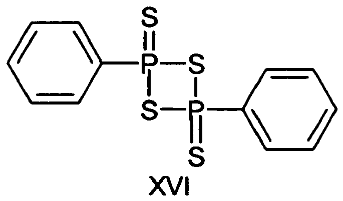 worin die Phenyl-Reste jeweils mit 1 oder 2 Substituenten unabhängig voneinander ausgewählt aus der Gruppe bestehend aus Methoxy, Phenoxy, Cl, Methyl und Br, bevorzugt jeweils mit einem Phenoxy-Rest oder Methoxy-Rest, besonders bevorzugt jeweils mit einem Methoxy-Rest in para-Position, substituiert sind, oder mit Phosphorpentasulfid, zu wenigstens einer Verbindung der allgemeinen Formel Ib, worin R¹ bis R¹², m, n und p die vorstehend genannte Bedeutung haben und X für ein Schwefelatom steht, mit der Maßgabe, dass wenigstens einer der Substituenten R⁸ bis R¹² für eine-NR²³-S(=O)₂-R²⁴-Gruppe steht, wobei R²³ und R²⁴ die vorstehend genannte Bedeutung haben, umgesetzt wird und diese ggf. gereinigt und/oder isoliert wird.

18. Arzneimittel enthaltend wenigstens eine Verbindung gemäß einem oder mehreren der Ansprüche 1 bis 14 und ggf. einen oder mehrere physiologisch verträgliche Hilfsstoffe.

19. Arzneimittel gemäß Anspruch 18 zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit;
zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

20. Verwendung wenigstens einer Verbindung gemäß einem oder mehreren der Ansprüche 1 bis 14 zur Herstellung eines Arzneimittels zur Behandlung und/oder Prophylaxe von einer oder mehreren Erkrankungen ausgewählt aus der Gruppe bestehend Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit;
zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanii und Capsavanii (DA-5018).

21. Verwendung gemäß Anspruch 20 zur Herstellung eines Arzneimittels zur Behandlung und/oder Prophylaxe von Schmerz ausgewählt aus der Gruppe bestehend aus akutem Schmerz, chronischem Schmerz, neuropathischem Schmerz und visceralem Schmerz.

## Claims

1. Substituted cyclic urea derivatives of the general formula I, 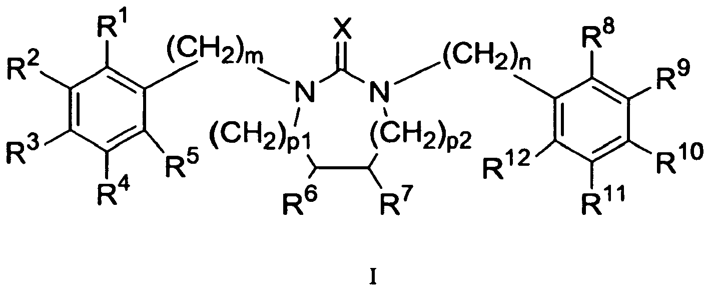 in which
X stands for O, S or N-C≡N;
m is equal to 1 or 2;
n is equal to 1 or 2;
p1 and p2, independently of one another, respectively stand for 0, 1, 2 or 3, wherein the sum of p1 and p2 amounts to 0, 1, 2 or 3;
R¹, R², R³, R⁴, R⁵, R⁸, R⁹, R¹⁰, R¹¹ and R¹², independently of one another, respectively stand for H; F; Cl; Br; I; -SF₅; -NO₂; -NH₂; -OH; -SH; -C(=O)-NH₂; -S(=O)₂-NH₂;
-NR¹³R¹⁴; -NH-R¹⁵; -OR¹⁶; -SR¹⁷; -O-(CH₂)ₐ-R¹⁸; -O-(CH₂)_{b}-OR¹⁹;
-(CH₂)_{c}-O-(CH₂)_{d}-OR²⁰;
-(CH₂)ₑ O-C(=O)-R²¹;
-(CH₂)_{f}O-C(=_{O})-OR²²;
-NR²³S(=O)₂R²⁴;
-(CH₂)_{g}-C(=O)-NR²⁵R²⁶;
-(CH₂)ₕ-C(=O)-NH-R²⁷;
-S(=O)ᵢR²⁸;
-(CH₂)ⱼ-S(=O)₂-NR²⁹R³⁰;
-(CH₂)ₖ-S(=O)₂-NHR³¹;
-(CH₂)ₗ-NR³²-C(=O)(CH₂)_{q}-OR³³;
-(CH₂)ᵣ-NH-C(=O)(CH₂)ₛ-OR³⁴;
-(CH₂)ₜ-NR³⁵-O-C(O)-OR³⁶_{;}
-(CH₂)ᵤ-NH-O-C(=O)-OR³⁷;
-(CH₂)ᵥO-S(=O)₂-R³⁸;
-(CH₂)_{w}-NR³⁹-C(=O)-SR⁴⁰;
-(CH₂)_{y}-C(=O)-NH-OR⁴¹;
-P(=O)(OR⁴²)₂;
-(CH₂)_{z}-C(=S)-NR⁴³R⁴⁴;
-(CH₂)ₐₐ-C(=S)-NH-R⁴⁵;
-(CH₂)_{bb}-NR⁴⁶-C(=O)-R⁴⁷;
-(CH₂)_{cc}-NH-C(=O)-R⁴⁸;
or -NH-C(=NH)-NH₂;
wherein
a, b, c, d, q and s, independently of one another, are respectively equal to 1, 2, 3, 4 or 5;
e, f, g, h, j, k, l, r, t, u, v, w, x, y, z, aa, bb and cc, independently of one another, are respectively equal to 0, 1, 2, 3, 4 or 5, and
i is equal to 1 or 2;
for a linear or branched, saturated or unsaturated aliphatic C₁₋₁₀ radical, which can optionally be substituted by 1, 2, 3, 4, 5, 6, 7, 8 or 9 substituents independently selected from the group consisting of F, Cl, Br, I, -CN, -NO₂, -OH, -NH₂, -SH, -O(C₁₋₅ alkyl), -S(C₁₋₅ alkyl), -NH(C₁₋₅ alkyl), -N(C₁₋₅ alkyl)(C₁₋₅ alkyl), -OCF₃ and -SCF₃;
or for an optionally substituted six-membered or ten-membered aryl radical, which can be bonded via a linear or branched, optionally substituted C₁₋₅ alkylene group;
or two adjacent radicals selected from the group consisting of R⁸, R⁹, R¹⁰, R¹¹ and R¹² together stand for a methylenedioxy(-O-CH₂-O) group;
provided that at least one of the radicals R⁸, R⁹, R¹⁰, R¹¹ and R¹² stands for -NR²³S(=O)₂R²⁴;
R⁶ and R⁷ respectively stand for a hydrogen radical
or
R⁶ and R⁷, together with the interconnecting C-C bridge, form an unsubstituted phenylene radical;
R¹³, R¹⁴, R¹⁵, R¹⁶, R¹⁷, R¹⁹, R²⁰, R²¹, R²², R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, R⁴², R⁴³, R⁴⁴, R⁴⁵, R⁴⁶, R⁴⁷ and R⁴⁸, independently of one another, respectively
stand for a linear or branched, saturated or unsaturated aliphatic C₁₋₁₀ radical, which can be optionally substituted by 1, 2, 3, 4, 5, 6, 7, 8 or 9 substituents independently selected from the group consisting of F, Cl, Br, I, -CN, -NO₂, -OH, -NH₂, -SH, -O(C₁₋₅ alkyl), -S(C₁₋₅ alkyl), -NH(C₁₋₅ alkyl), -N(C₁₋₅ alkyl)(C₁₋₅ alkyl), -OCF₃ and -SCF₃;
for an unsaturated or saturated, optionally substituted three-membered, four-membered, five-membered, six-membered, seven-membered, eight-membered or nine-membered cycloaliphatic radical
or for an optionally substituted five-membered to fourteen-membered aryl radical or an optionally substituted five-membered to fourteen-membered heteroaryl radical, which can be condensed with a saturated or unsaturated, optionally substituted monocyclic or polycyclic ring system;
R¹⁸ stands for an unsaturated or saturated, optionally substituted three-membered, four-membered, five-membered, six-membered, seven-membered, eight-membered or nine-membered cycloaliphatic radical
or for an optionally substituted five-membered to fourteen-membered aryl radical or heteroaryl radical, which can be condensed with a saturated or unsaturated, optionally substituted monocyclic or polycyclic ring system;
R²³ stands for a hydrogen radical
or for a linear or branched, saturated or unsaturated or aliphatic C₁₋₁₀ radical, which can optionally be substituted by 1, 2, 3, 4, 5, 6, 7, 8 or 9 substituents independently selected from the group consisting of F, Cl, Br, I, -CN, -NO₂, -OH, -NH₂, -SH, -O(C₁₋₅ alkyl), -S(C₁₋₅ alkyl), -NH(C₁₋₅ alkyl), -N(C₁₋₅ alkyl)(C₁₋₅ alkyl), -OCF₃ and -SCF₃;
and
R²⁴ stands for a linear or branched, saturated or unsaturated or aliphatic C₁₋₁₀ radical, which can optionally be substituted by 1, 2, 3, 4 or 5 substituents independently selected from the group consisting of -CN, -NO₂, -OH, -NH₂, -SH, -O(C₁₋₅ alkyl), -S(C₁₋₅ alkyl), -NH(C₁₋₅ alkyl) and -N(C₁₋₅ alkyl)(C₁₋₅ alkyl),
or for an optionally substituted 5-membered to 14-membered aryl or heteroaryl radical, which may be condensed with a saturated or unsaturated, optionally substituted mono- or poly-cyclic ring system;
wherein
the aforementioned cycloaliphatic radicals can optionally in each case be substituted by 1, 2, 3, 4 or 5 substituents independently selected from the group consisting of oxo (=O), thioxo (=S), F, Cl, Br, I, -CN, -CF₃, -SF₃, -OH, -O-C₁₋₅ alkyl, -NH₂, -NO₂, -O-CF₃, -S-CF₃, -SH, -S-C₁₋₅ alkyl, -C₁₋₅ alkyl, -C(=O)-OH, -C(=O)-O-C₁₋₅ alkyl, -NH-C₁₋₅ alkyl, -N(C₁₋₅ alkyl)₂, -O-phenyl, -O-benzyl, phenyl and benzyl, and in each case the cyclic moiety of the radicals -O-phenyl, -O-benzyl, phenyl and benzyl can be substituted by 1, 2, 3, 4 or 5 substituents independently selected from the group consisting of F, Cl, Br, -OH, -CF₃, -SF₅, -CN, -NO₂, -C₁₋₅ alkyl, -O-C₁₋₅ alkyl, -O-CF₃, -S-CF₃, phenyl and -O-benzyl,
and the aforementioned cycloaliphatic radicals in each case optionally exhibit 1, 2, 3, 4 or 5 heteroatom(s) independently selected from the group consisting of oxygen, nitrogen and sulphur;
the aforementioned C₁₋₅ alkylene group can optionally be substituted by 1, 2, 3, 4 or 5 substituents independently selected from the group consisting of F, Cl, Br, -OH, -SH, -NH₂, -CN and NO₂;
the rings of the aforementioned monocyclic or polycyclic ring systems can optionally in each case be substituted by 1, 2, 3, 4 or 5 substituents independently selected from the group consisting of oxo (=O), thioxo (=S), F, Cl, Br, I, -CN, -CF₃, -SF₅, -OH, -O-C₁₋₅ alkyl, -NH₂, -NO₂, -O-CF₃, -S-CF₃, -SH, -S-C₁₋₅ alkyl, -C₁₋₅ alkyl, -C(=O)-OH, -C(=O)-O-C₁₋₅ alkyl, -NH-C₁₋₅ alkyl, -N(C₁₋₅ alkyl)₂, -O-phenyl, -O-benzyl, phenyl and benzyl, and in each case the cyclic moiety of the radicals -O-phenyl, -O-benzyl, phenyl and benzyl can be substituted by 1, 2, 3, 4 or 5 substituents independently selected from the group consisting of F, Cl, Br, -OH, -CF₃, -SF₅, -CN, -NO₂, -C₁₋₅ alkyl, -O-C₁₋₅ alkyl, -O-CF₃, -S-CF₃, phenyl and -O-benzyl,
and the rings of the aforementioned monocyclic or polycyclic ring systems are each five-membered, six-membered or seven-membered and can in each case optionally exhibit 1, 2, 3, 4 or 5 heteroatom(s) as ring member(s), which are independently selected from the group consisting of oxygen, nitrogen and sulphur;
and the aforementioned aryl radicals or heteroaryl radicals can optionally in each case be substituted by 1, 2, 3, 4 or 5 substituents independently selected from the group consisting of F, Cl, Br, I, -CN, -CF₃, -SF₅, -OH, -O-C₁₋₅ alkyl, -NH₂, -NO₂, -O-CF₃, -S-CF₃, -SH, -S-C₁₋₅ alkyl, -C₁₋₅ alkyl, -C(=O)-OH, -C(=O)-O-C₁₋₅ alkyl, -NH-C₁₋₅ alkyl, -N(C₁₋₅ alkyl)₂, -NH-C(=O)-O-C₁₋₅ alkyl, -C(=O)-H, -C(=O)-C₁₋₅ alkyl, -C(=O)-NH₂, -C(=O)-NH-C₁₋₅ alkyl, -C(=O)-N-(C₁₋₅ alkyl)₂, -O-phenyl, -O-benzyl, phenyl and benzyl, and in each case the cyclic moiety of the radicals -O-phenyl, -O-benzyl, phenyl and benzyl can be substituted by 1, 2, 3, 4 or 5 substituents independently selected from the group consisting of F, Cl, Br, -OH, -CF₃, -SF₅, -CN, -NO₂, -C₁₋₅ alkyl, -O-C₁₋₅ alkyl, -O-CF₃, -S-CF₃, phenyl and -O-benzyl,
and
the aforementioned heteroaryl radicals in each case comprise 1, 2, 3, 4 or 5 heteroatom(s) independently selected from the group consisting of oxygen, nitrogen, and sulphur as ring member(s);
in each case optionally in the form of one of the pure stereoisomers thereof, particularly enantiomers or diastereoisomers or the racemates thereof, or in the form of a mixture of stereoisomers, particularly the enantiomers and/or diastereoisomers, in an arbitrary mixing ratio, or in each case in the form of corresponding salts, or respectively in the form of corresponding solvates.

2. Compounds according to claim 1, **characterised in that**
R¹, R², R⁴ and R⁵, independently of one another, respectively
stand for H; F; Cl; Br; I; -SF₅; -NO₂; -NH₂; -OH; -SH; -C(=O)-NH₂; -S(=O)₂-NH₂;
-NR¹³R¹⁴; -NH-R¹⁵; -OR¹⁶; -SR¹⁷; -O-(CH₂)ₐ-R¹⁸; -O-(CH₂)_{b}-OR¹⁹;
-(CH₂)_{c}-O-(CH₂)_{d}-OR²⁰;
-(CH₂)ₑ-O-C(=O)-R²¹;
-(CH₂)_{f}-O-C(=O)-OR²²;
-NR²³S(=O)₂R²⁴;
-(CH₂)_{g}-C(=O)-NR²⁵R²⁶;
-(CH₂)ₕ-C(=O)-NH-R²⁷;
-S(=O)ᵢR²⁸;
-(CH₂)ⱼ-S(=O)₂-NR²⁹R³⁰;
-(CH₂)ₖ-S(=O)₂-NHR³¹;
-(CH₂)ₗ-NR³²-C(=O)(CH₂)_{q}-OR³³;
-(CH₂)ᵣ-NH-C(=O)(CH₂)ₛ-OR³⁴;
-(CH₂)ₜ-NR³⁵-O-C(=O)-OR³⁶;
-(CH₂)ᵤ-NH-O-C(=O)-OR³⁷;
-(CH₂)ᵥ-O-S(=O)₂-R³⁸;
-(CH₂)_{w}-NR³⁹-C(=O)-SR⁴⁰_{;}
-(CH₂)_{y}-C(=O)-NH-OR⁴¹;
-P(=O)(OR⁴²)₂;
-(CH₂)_{z}-C(=S)-NR⁴³R⁴⁴;
-(CH₂)ₐₐ-C(=S)-NH-R⁴⁵;
-(CH₂)_{bb}-NR⁴⁶-C(=O)-R⁴⁷;
-(CH₂)_{cc}-NH-C(=O)-R⁴⁸;
or -NH-C(=NH)-NH₂;
wherein
a, b and c, d, q and s, independently of one another, are respectively equal to 1, 2, 3, 4 or 5,
e, f and g, h, j, k, l, r, t, u, v, w, x, y and z, aa, bb and cc, independently of one another, are respectively equal to 1, 1, 2, 3, 4 or 5 and
i is equal to 1 or 2;
for a radical selected from the group consisting of methyl, -CF₃, -CCl₃, -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃, -CH₂-N(CH₃)₂, -CH₂-N(C₂H₅)₂, -CH₂-N(CH₃)(C₂H₅), ethyl, -CH₂-CH₂-NH₂, -CH₂-CH₂-OH, -CH₂-CH₂-SH, -CH₂-CH₂-NH-CH₃, -CH₂-CH₂-N(CH₃)₂, -CH₂-CH₂-N(C₂H₅)₂, -CH₂-CH₂-N(CH₃)(C₂H₅), -CH₂-CF₃, -C₂F₅, -CH₂-CCl₃, -CH₂-CBr₃, -CH₂-CH₂-CN, n-propyl, -CH₂-CH₂-CH₂-OH, -CH₂-CH₂-CH₂-SH, -CH₂-CH₂-CH₂-NH₂, -CH₂-CH₂-CH₂-NH-CH₃, -CH₂-CH₂-CH₂-N(CH₃)₂, -CH₂-CH₂-CH₂-N(C₂H₅)₂, -CH₂-CH₂-CH₂-N(CH₃)(C₂H₅), -CH₂-CH₂-O-CH₃, -CF₂-CF₂-CF₃, -CF(CF₃)₂, isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), -CH(CH₃)(S-CH₃), n-butyl, -CF₂-CF₂-CF₂-CF₃, -CH₂-CH₂-CH₂-CH₂-CN, sec-butyl, isobutyl, tert-butyl, n-pentyl, sec-pentyl, neopentyl, n-hexyl, vinyl, 1-propenyl, 2-propenyl, 1-butenyl, 2-butenyl, 3-butenyl, 2-methylbuten-2-yl, (1,1,2)-trifluoro-1-butenyl, 1-pentenyl, 2-pentenyl, 3-pentenyl and 4-pentenyl;
or for an aryl radical selected from the group consisting of phenyl and naphthyl, wherein the aryl radical can be substituted by 1, 2, 3, 4 or 5 substituents independently selected from the group consisting of F, Cl, Br, I, -CN, -NO₂, -OH, -SH, methyl, ethyl, -CF₃, -O-CF₃, -S-CF₃, -SF₅, -O-CH3, -O-C₂H₅, -NH₂, -N(CH₃)₂, -N(C₂H₅)₂, -N(H)(CH₃) and -N(H)(C₂H₅);
and
R³ stands for F; Cl; Br; I; -SF₅; -NO₂; -NH₂; -OH; -SH; -C(=O)-NH₂; -S(=O)₂-NH₂; -NR¹³R¹⁴; -NH-R¹⁵; -OR¹⁶; -SR¹⁷; -O-(CH₃)ₐ-R¹⁸, -O-(CH₂)_{b}-OR¹⁹ or -NR²³S(=O)₂R²⁴;
wherein
a and b, independently of one another, are respectively equal to 1, 2, 3, 4 or 5;
or for a radical selected from the group consisting of methyl, -CF₃, -CCl₃, -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃, -CH₂-N(CH₃)₂, -CH₂-N(C₂H₅)₂, -CH₂-N(CH₃)(C₂H₅), ethyl, -CH₂-CH₂-NH₂, -CH₂-CH₂-OH, -CH₂-CH₂-SH, -CH₂-CH₂-NH-CH₃, -CH₂-CH₂-N(CH₃)₂, -CH₂-CH₂-N(C₂H₅)₂, -CH₂-CH₂-N(CH₃)(C₂H₅), -CH₂-CF₃, -C₂F₅, -CH₂-CCl₃, -CH₂-CBr₃, -CH₂-CH₂-CN, n-propyl, -CH₂-CH₂-CH₂-OH, -CH₂-CH₂-CH₂-SH, -CH₂-CH₂-CH₂-NH₂, -CH₂-CH₂-CH₂-NH-CH₃, -CH₂-CH₂-CH₂-N(CH₃)₂, -CH₂-CH₂-CH₂-N(C₂H₅)₂, -CH₂-CH₂-CH₂-N(CH₃)(C₂H₅), -CH₂-CH₂-O-CH₃, -CF₂-CF₂-CF₃, -CF(CF₃)₂, isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), -CH(CH₃)(S-CH₃), n-butyl, -CF₂-CF₂-CF₂-CF₃, -CH₂-CH₂-CH₂-CH₂-CN, sec-butyl, isobutyl, tert-butyl, n-pentyl, sec-pentyl, neopentyl, n-hexyl, vinyl, 1-propenyl, 2-propenyl, 1-butenyl, 2-butenyl, 3-butenyl, 2-methylbuten-2-yl, (1,1,2)-trifluoro-1-butenyl, 1-pentenyl, 2-pentenyl, 3-pentenyl and 4-pentenyl.

3. The compounds according to claim 1 or 2, **characterised in that**
R¹, R², R⁴ and R⁵, independently of one another, respectively stand for H; F; Cl; I; Br; -NO₂; -NH₂; -OH; and -SH; -NR¹³R¹⁴; -NH-R¹⁵; -OR¹⁶; -SR¹⁷; -NR²¹S(=O)₂R²⁴;
or for a radical selected from the group consisting of methyl, -CF₃, ethyl, -CH₂-CF₃, -C₂F₅, n-propyl, isopropyl, n-butyl, sec-butyl, isobutyl, tert-butyl or n-pentyl
and
R³ stands for F; Cl; Br; I; -OR²; -NR²³S(=O)₂R²⁴;
or for a radical selected from the group consisting of -SF₅, -CF₃, -C₂F₅, -CH₂-CF₃, -CF(CF₃)₂, isopropyl, -CH(CH₃)(O-CH₃), -CH(CH₃)(S-CH₃), sec-butyl, isobutyl and tert-butyl;

4. The compounds according to one or more of claims 1 to 3, **characterised in that**
R⁸, R⁹, R¹¹ and R¹², independently of one another, respectively stand for
H; F; Cl; Br; I; -SF₅; -NO₂; -NH₂; -OH; -SH; -C(=O)-NH₂; -S(=O)₂-NH₂; -NR¹³R¹⁴;
-NH-R¹⁵; -OR¹⁶; -SR¹⁷; -O-(CH₂)ₐ-R¹⁸; -O-(CH₂)_{b}-OR¹⁹;
-(CH₂)_{c}-O-(CH₂)_{d}-OR²⁰;
-(CH₂)ₑ-O-C(=O)-R²¹;
-(CH₂)_{f}-O-C(=O)-OR²²;
-NR²³S(=O)₂R²⁴;
-(CH₂)_{g}-C(=O)-NR²⁵R²⁶;
-(CH₂)ₕ-C(=O)-NH-R²⁷;
-S(=O)ᵢR²⁸;
-(CH₂)ⱼ-S(=O)₂-NR²⁹R³⁰;
-(CH₂)ₖ-S(=O)₂-NHR³¹;
-(CH₂)ₗ-NR³²-C(=O)(CH₂)_{q}-OR³³;
-(CH₂)ᵣ-NH-C(=O)(CH₂)ₛ-OR³⁴;
-(CH₂)ₜ-NR³⁵-O-C(=O)-OR³⁶;
-(CH₂)ᵤ-NH-O-C(=O)-OR³⁷;
-(CH₂)ᵥ-O-S(=O)₂-R³⁸;
-(CH₂)_{w}-NR³⁹-C(=O)-SR⁴⁰;
-(CH₂)_{y}-C(=O)-NH-OR⁴¹;
-P(=O)(OR⁴²)₂;
-(CH₂)_{z}-C(=S)-NR⁴³R⁴⁴;
-(CH₂)ₐₐ-C(=S)-NH-R⁴⁵;
-(CH₂)_{bb}-NR⁴⁶-C(=O)-R⁴⁷;
-(CH₂)_{cc}-NH-C(=O)-R⁴⁸;
or -NH-C(=NH)-NH₂;
wherein
a, b and c, d, q and s, independently of one another, are respectively equal to 1, 2, 3, 4 or 5,
e, f and g, h, j, k, l, r, t, u, v, w, x, y and z, aa, bb and cc each, independently of one another are respectively equal to 0, 1, 2, 3, 4 or 5, and
i is equal to 1 or 2;
for a radical selected from the group consisting of methyl, -CF₃, -CCl₃, -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃, -CH₂-N(CH₃)₂, -CH₂-N(C₂H₅)₂, -CH₂-N(CH₃)(C₂H₅), ethyl, -CH₂-CH₂-NH₂, -CH₂-CH₂-OH, -CH₂-CH₂-SH, -CH₂-CH₂-NH-CH₃, -CH₂-CH₂-N(CH₃)₂, -CH₂-CH₂-N(C₂H₅)₂, -CH₂-CH₂-N(CH₃)(C₂H₅), -CH₂-CF₃, -C₂F₅, -CH₂-CCl₃, -CH₂-CBr₃, -CH₂-CH₂-CN, n-propyl, -CH₂-CH₂-CH₂-OH, -CH₂-CH₂-CH₂-SH, -CH₂-CH₂-CH₂-NH₂, -CH₂-CH₂-CH₂-NH-CH₃, -CH₂-CH₂-CH₂-N(CH₃)₂, -CH₂-CH₂-CH₂-N(C₂H₅)₂, -CH₂-CH₂-CH₂-N(CH₃)(C₂H₅), -CH₂-CH₂-O-CH₃, -CF₂-CF₂-CF₃, -CF(CF₃)₂, isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), -CH(CH₃)(S-CH₃), n-butyl, -CF₂-CF₂-CF₂-CF₃, -CH₂-CH₂-CH₂-CH₂-CN,, sec-butyl, isobutyl, tert-butyl, n-pentyl, sec-pentyl, neopentyl, n-hexyl, vinyl, 1-propenyl, 2-propenyl, 1-butenyl, 2-butenyl, 3-butenyl, 2-methylbuten-2-yl, (1,1,2)-trifluoro-1-butenyl, 1-pentenyl, 2-pentenyl, 3-pentenyl and 4-pentenyl;
or for an aryl radical selected from the group consisting of phenyl and naphthyl, wherein the aryl radical can be substituted by 1, 2, 3, 4 or 5 substituents independently selected from the group consisting of F, Cl, Br, I, -CN, -NO₂, -OH, -SH, methyl, ethyl, -CF₃, -O-CF₅, -S-CF₃, -SF₅, -O-CH₃, -O-C₂H₅, -NH₂, -N(CH₃)₂, -N(C₂H₅)₂, -N(H)(CH₃) and -N(H)(C₂H₅);
and
R¹⁰ stands for -NR²³S(=O)₂R²⁴.

5. The compounds according to one or more of claims 1 to 4, **characterised in that** R⁸, R⁹, R¹¹ and R¹², independently of one another, respectively stand for H; F; Cl; Br; I; -NO₂; -NH₂; -OH; -SH; -NR¹³R¹⁴; -NH-R¹⁵; -OR¹⁶; -SR¹⁷; -NR²³S(=O)₂R²⁴;
or for a radical selected from the group consisting of methyl, -CF₃, ethyl, -CH₂-CF₃, -C₂F₅, n-propyl, isopropyl, n-butyl, sec-butyl, isobutyl, tert-butyl or n-pentyl and
R¹⁰ stands for -NR²³S(=O)₂R²⁴.

6. The compounds according to one or more of claims 1 to 5, **characterised in that** the radical R¹⁰ stands for -NR²³S(=O)₂R²⁴ and the radicals R⁸, R⁹, R¹¹ and R¹² respectively stand for H.

7. The compounds according to one or more of claims 1 to 6, **characterised in that** R¹³, R¹⁴, R¹⁵, R¹⁶, R¹⁷, R¹⁹, R²⁰, R²¹, R²², R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, R⁴², R⁴³, R⁴⁴, R⁴⁵, R⁴⁶, R⁴⁷ and R⁴⁸, independently of one another, respectively stand for
a radical selected from the group consisting of methyl, -CF₃, -CCl₃, -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃, -CH₂-N(CH₃)₂, -CH₂-N(C₂H₅)₂, -CH₂-N(CH₃)(C₂H₅), ethyl, -CH₂-CH₂-NH₂, -CH₂-CH₂-OH, -CH₂-CH₂-SH, -CH₂-CH₂-NH-CH₃, -CH₂-CH₂-N(CH₃)₂, -CH₂-CH₂-N(C₂H₅)₂, -CH₂-CH₂-N(CH₃)(C₂H₅), -CH₂-CF₃, -C₂F₅, -CH₂-CCl₃, -CH₂-CBr₃, -CH₂-CH₂-CN, n-propyl, -CH₂-CH₂-CH₂-OH, -CH₂-CH₂-CH₂-SH, -CH₂-CH₂-CH₂-NH₂, -CH₂-CH₂-CH₂-NH-CH₃, -CH₂-CH₂-CH₂-N(CH₃)₂, -CH₂-CH₂-CH₂-N(C₂H₅)₂, -CH₂-CH₂-CH₂-N(CH₃)(C₂H₅), -CH₂-CH₂-O-CH₃, -CF₂-CF₂-CF₃, -CF(CF₃)₂, isopropyl, -CH₂-CH₂-CH₂-CN, -CH₂-O-CH₂-CH₃, -CH₂-CH₂-SF₃, -CH₂-CH₂-OCF₃, -CH(CH₃)(O-CH₃), -CH(CH₃)(S-CH₃), n-butyl, -CF₂-CF₂-CF₂-CF₃, -CH₂-CH₂-CH₂-CH₂-CN, sec-butyl, isobutyl, tert-butyl, n-pentyl, sec-pentyl, neopentyl, n-hexyl, vinyl, 1-propenyl, 2-propenyl, 1-butenyl, 2-butenyl, 3-butenyl, 2-methylbuten-2-yl, (1,1,2)-trifluoro-1-butenyl, 1-pentenyl, 2-pentenyl, 3-pentenyl and 4-pentenyl;
for a (hetero)cycloaliphatic radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl and dithiolanyl, wherein the (hetero)cycloaliphatic radical can in each case be optionally substituted by 1, 2, 3, 4 or 5 substituents independently selected from the group consisting of thioxo (=S), F, Cl, Br, I, -CN, -CF₃, -SF₅, -OH, -NH₂, -O-CF₃, -SH, -O-CH₃, -O-C₂H₅, methyl, ethyl, n-propyl, isopropyl, n-butyl, sec-butyl, isobutyl, tert-butyl, -S-CH₃, -S-C₂H₅, -C(=O)-O-CH₃, -C(=O)-O-C₂H₅, Oxo (=O), -N(CH₃)₂, -N(C₂H₅)₂, -N(H)(CH₃), -N(H)(C₂H₅), -NO₂, -SCF₃, -C(=O)-OH, -O-phenyl, -O-benzyl, phenyl and benzyl,
or for a radical selected from the group consisting of phenyl, naphthyl, (1.3)-benzodioxolyl, (1.4)-benzodioxanyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinoxalinyl, quinolinyl and isoquinolinyl, wherein the radical can in each case be optionally substituted by 1, 2, 3, 4 or 5 substituents independently selected from the group consisting of F, Cl, Br, I, -CN, -NO₂, -OH, -SH, methyl, ethyl, n-propyl, isopropyl, n-butyl, sec-butyl, isobutyl, tert-butyl, -CF₃, -O-CF₃, -S-CF₃, -SF₅, -O-CH₃, -O-C₂H₅, -O-phenyl, -O-benzyl, -NH₂, -N(CH₃)₂, -N(C₂H₅)₂, -N(H)(CH₃) und -N(H)(C₂H₅).

8. The compounds according to one or more of claims 1 to 7, **characterised in that**
R¹⁸ stands for a (hetero)cycloaliphatic radical selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl and dithiolanyl, wherein the (hetero)cycloaliphatic radical can in each case be optionally substituted by 1, 2, 3, 4 or 5 substituents independently selected from the group consisting of thioxo (=S), F, Cl, Br, I, -CN, -CF₃, -SF₅, -OH, -NH₂, -O-CF₃, -SH, -O-CH₂, -O-C₂H₅, methyl, ethyl, n-propyl, isopropyl, n-butyl, sec-butyl, isobutyl, tert-butyl, -S-CH₃, -S-C₂H₅, -C(=O)-O-CH₃, -C(=O)-O-C₂H₅, Oxo (=O), -N(CH₃)₂, -N(C₂H₅)₂, -N(H)(CH₃), -N(H)(C₂H₅), -NO₂, -SCF₃, -C(=O)-OH, -O-phenyl, -O-benzyl, phenyl and benzyl,
or for a radical selected from the group consisting of phenyl, naphthyl, (1,3)-benzodioxolyl, (1,4)-benzodioxanyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinoxalinyl, quinolinyl and isoquinolinyl, wherein the radical can in each case be optionally substituted by 1, 2, 3, 4 or 5 substituents independently selected from the group consisting of F, Cl, Br, I, -CN, -NO₂, -OH, -SH, methyl, ethyl, n-propyl, isopropyl, n-butyl, sec-butyl, isobutyl, tert-butyl, -CF₃, -O-CF₃, -S-CF₃, -SF₅, -O-CH₃, -O-C₂H₅, -O-phenyl, -O-benzyl, -NH₂, -N(CH₃)₂, -N(C₂H₅)₂, -N(H)(CH₃) and -N(H)(C₂H₅) and -NH-C(=O)-O-CH₃.

9. The compounds according to one or more of claims 1 to 8, **characterised in that**
R²³ stands for a hydrogen radical
or for a radical selected from the group consisting of methyl, ethyl, n-propyl, isopropyl, n-butyl, sec-butyl, isobutyl, tert-butyl, n-pentyl, sec-pentyl, neopentyl and n-hexyl.

10. The compounds according to one or more of claims 1 to 9, **characterised in that**
R²⁴ stands for a radical selected from the group consisting of methyl, -CH₂-CN, ethyl, -CH₂-CH₂-CN, n-propyl, isopropyl, n-butyl, sec-butyl, isobutyl, tert-butyl, n-pentyl, sec-pentyl, neopentyl and n-hexyl.

11. The compounds according to one or more of claims 1 to 10, **characterised in that**
p1 and p2, independently of one another, respectively stand for 0 or 1, wherein the sum of p1 and p2 is 0 or 1.

12. The compounds according to one or more of claims 1 to 11, **characterised in that**
X stands for O, S or N-C≡N;
m is equal to 1;
n is equal to 1;
p1 and p2, independently of one another, respectively stand for 0 or 1, wherein the sum of p1 and p2 is 0 or 1;
R¹, R² and R⁴, R⁵, R⁸, R⁹, R¹¹ and R¹², independently of one another, respectively stand for
H; F; Cl; I; Br; -NO₂; -NH₂; -OH; -SH; -NR¹³R¹⁴; -NH-R¹⁵; -OR¹⁶; -SR¹⁷; -NR²³S(=O)₂R²⁴;
or for a radical selected from the group consisting of methyl, -CF₃, ethyl, -CH₂-CF₃, -C₂F₅, n-propyl, isopropyl, n-butyl, sec-butyl, isobutyl, tert-butyl or n-pentyl;
R³ stands for F; Cl; Br; I; -O R¹⁶; -NR²³S(=O)₂R²⁴;
or for a radical selected from the group consisting of -SF₅, -CF₃, -C₂F₅, -CH₂-CF₃, -CF(CF₃)₂, isopropyl, -CH(CH₃)(O-CH₃), -CH(CH₃)(S-CH₃), sec-butyl, isobutyl and tert-butyl;
R⁶ and R⁷ respectively stand for a hydrogen radical
or
R⁶ and R⁷, together with the interconnecting C-C bridge, form an unsubstituted phenylene radical;
R¹⁰ stands for -NR²³S(=O)₂R²⁴;
R¹³, R¹⁴, R¹⁵, R¹⁶ and R¹⁷, independently of one another, respectively stand for a radical selected from the group consisting of -CF₃, -C₂F₅, -CH₂-CF₃, methyl, ethyl, n-propyl, isopropyl, n-butyl, sec-butyl, isobutyl, tert-butyl, n-pentyl, sec-pentyl, neopentyl and n-hexyl;
or for a radical selected from the group consisting of phenyl, thiophenyl, furanyl and pyridinyl, wherein the radical can in each case be optionally substituted by 1, 2, 3, 4 or 5 substituents independently selected from the group consisting of F, Cl, Br, I, -CN, -NO₂, -OH, -SH, methyl, ethyl, n-propyl, isopropyl, n-butyl, sec-butyl, isobutyl, tert-butyl, -CF₃, -O-CF₃, -S-CF₃, -SF₅, -O-CH₃ and -O-C₂H₅;
R²³ stands for a hydrogen radical
and
R²⁴ stands for a radical selected from the group consisting of methyl, -CH₂-CN, ethyl, -CH₂-CH₂-CN, n-propyl, isopropyl, n-butyl, sec-butyl, isobutyl, tert-butyl, n-pentyl, sec-pentyl, neopentyl and n-hexyl;
in each case optionally in the form of one of the pure stereoisomers thereof, particularly enantiomers or diastereoisomers or the racemates thereof or in the form of a mixture of stereoisomers, particularly the enantiomers and/or diastereoisomers, in an arbitrary mixing ratio, or in each case in the form of corresponding salts, or in each case in the form of corresponding solvates.

13. The compounds according to one or more of claims 1 to 12, **characterised in that**
X stands for O, S or N-C≡N;
m is equal to 1;
n is equal to 1;
p1 and p2, independently of one another, respectively stand for 0 or 1, wherein the sum of p1 and p2 is 0 or 1;
R¹, R² and R⁴, R⁵, R⁸, R⁹, R¹¹ and R¹², independently of one another, respectively stand for H;
R³ stands for -OR¹⁶; -NR²³S(=O)₂R²⁴;
or for a radical selected from the group consisting of -CF₃, isopropyl, sec-butyl, isobutyl and tert-butyl;
R⁶ and R⁷ respectively stand for a hydrogen radical
or
R⁶ and R⁷, together with the interconnecting C-C bridge, form an unsubstituted phenylene radical;
R¹⁰ stands for -NR²³S(=O)₂R²⁴;
R¹⁶ stands for a radical selected from the group consisting of -CF_{3,} -C₂F₅ and -CH₂-CF₃;
R²³ stands for a hydrogen radical
and
R²⁴ stands for a radical selected from the group consisting of methyl, ethyl, n-propyl, isopropyl, n-butyl, sec-butyl, isobutyl, tert-butyl, n-pentyl, sec-pentyl, neopentyl and n-hexyl;
in each case optionally in the form of one of the pure stereoisomers thereof, particularly enantiomers or diastereoisomers or the racemates thereof or in the form of a mixture of stereoisomers, particularly the enantiomers and/or diastereoisomers, in an arbitrary mixing ratio, or in each case in the form of corresponding salts, or in each case in the form of corresponding solvates.

14. Compounds according to one or more of claims 1 to 13, selected from the group consisting of
[1] N-[4-[3-(4-tert-butylbenzyl)-2-thioxo-imidazolidinylmethyl]phenyl] methanesulphonamide,
[2] N-[4-[3-(4-tert-butylbenzyl)-2-thioxo-2,3-dihydrobenzoimidazol-1-ylmethyl]phenyl]methanesulphonamide,
[3] N-[4-[3-(4-tert-butylbenzyl)-2-thioxotetrahydropyrimidin-1-ylmethyl] phenyl]methanesulphonamide,
[4] N-[4-[3-(4-tert-butylbenzyl)-2-oxo-imidazolidin-1-ylmethyl]phenyl] methanesulphonamide,
[5] N-[4-[3-(4-tert-butylbenzyl)-2-oxotetrahydropyrimidin-1-ylmethyl] phenyl]methanesulphonamide,
[6] N-[4-[3-(4-tert-butylbenzyl)-2-oxo-2,3-dihydrobenzoimidazol-1-ylmethyl]phenyl]methanesulphonamide,
[7] N-[4-[3-(4-trifluoromethoxybenzyl)-2-oxo-2,3-dihydrobenzimidazol-1-ylmethyl] phenyl]methanesulphonamide,
[8] N-[4-[3-(4-trifluoromethoxybenzyl)-2-thioxo-2,3-dihydrobenzimidazol-1-yl-methyl]phenyl]methanesulphonamide,
[9] N-[4-[3-(4-methanesulfonylaminobenzyl)-2-oxo-2,3-dihydrobenzimidazol-1-yl-methyl]phenyl]methanesulphonamide, and
[10] N-{4-[2-oxo3-(4-trifluoromethylbenzyl)-2,3-dihydrobenzimidazol-1-yl-methyl]phenyl}methanesulphonamide,
optionally in the form of corresponding salts or in the form of corresponding solvates.

15. A process for the preparation of substituted cyclic urea derivatives according to one or more of claims 1 to 14, **characterised in that**
at least one compound of the general formula II, 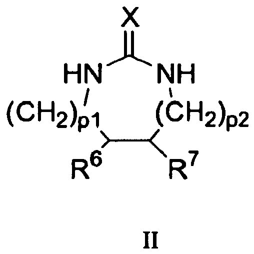 in which R⁶, R⁷, X, p1 and p2 have the meanings stated in one or more of claims 1 to 14, in a reaction medium, optionally in the presence of at least one base, preferably in the presence of at least one metal hydride salt, more preferred in the presence of potassium and/or sodium hydride, or in the presence of at least one alkali metal carbonate salt, preferably in the presence of potassium and/or sodium carbonate and at least one alkali metal iodide, preferably potassium and/or sodium iodide, is caused to react with at least one compound of the general formula III, 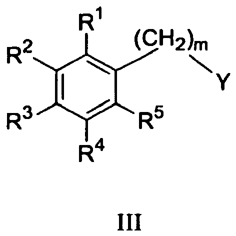 in which R¹ to R⁵ and m have the meanings stated in one or more of claims 1 to 14 and Y stands for a leaving group, preferably for a halogen radical, more preferred for a bromine atom or chlorine atom, to produce at least one compound of the general formula IV, 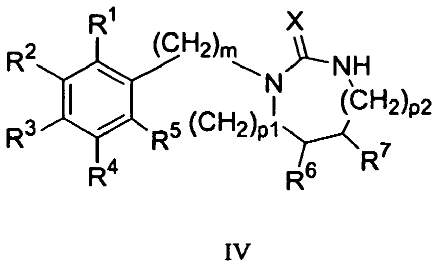 in which R¹ to R⁷, X, p1, p2 and m have the meanings stated above, and this is optionally purified and/or isolated,
or at least one compound of the general formula V, 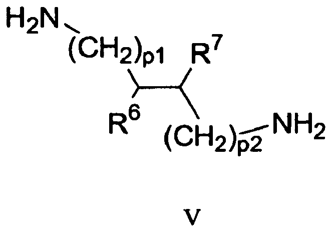 in which R⁶, R⁷, p1 and p2 have the meanings stated in one or more of claims 1 to 14, in a reaction medium, optionally in the presence of at least one base, preferably in the presence of at least one base selected from the group consisting of pyridine, triethylamine and diisopropylethylamine, is caused to react with at least one compound of the general formula III to form at least one compound of the general formula VI, 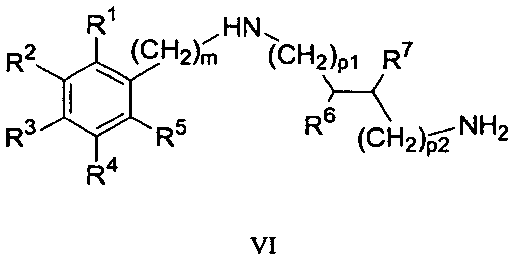 in which R¹ to R⁷, m, p1 and p2 have the meanings stated above, and this is optionally purified and/or isolated, and at least one compound of the general formula VI is caused to react, in a reaction medium, optionally in the presence of at least one base, preferably in the presence of at least one base selected from the group consisting of pyridine, triethylamine and diisopropylethylamine, with at least one compound of the general formula Z-C(=X)-Z in which X stands for an oxygen atom or sulphur atom and Z in each case for a leaving group, preferably in each case for a halogen radical, more preferred in each case for a chlorine atom, to form at least one compound of the general formula IV and this is optionally purified and/or isolated,
or
at least one compound of the general formula VII, 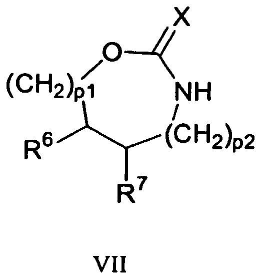 in which R⁶, R⁷, X, p1 and p2 have the meanings stated above, is caused to react, in a reaction medium, with at least one compound of the general formula VIII, 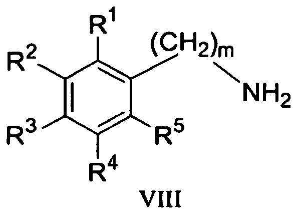 in which R¹ to R⁵ and m have the meanings stated above, to produce at least one compound of the general formula IV, and this is optionally purified and/or isolated,
and at least one compound of the general formula IV is caused to react, in a reaction medium, in the presence of at least one base, preferably in the presence of at least one metal hydride salt, more preferred in the presence of potassium and/or sodium hydride, or in the presence of at least one alkali metal carbonate salt, preferably in the presence of potassium and/or sodium carbonate and at least one alkali metal iodide, preferably potassium and/or sodium iodide, with at least one compound of the general formula IX, 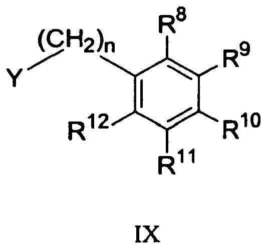 in which R⁸ to R¹² and n have the meanings stated above, provided that at least one of the substituents R⁸ to R¹² stands for an -N(PG)₂ group, wherein PG stands in each case for a protective group, preferably for a tert-butoxycarbonyl group or benzyloxycarbonyl group, or two PG groups, together with the interconnecting nitrogen atom, form a cyclic protective group, preferably a phthalimide group, and Y stands for a leaving group, preferably for a halogen atom, more preferred for a chlorine or bromine atom, to produce at least one compound of the general formula XI, 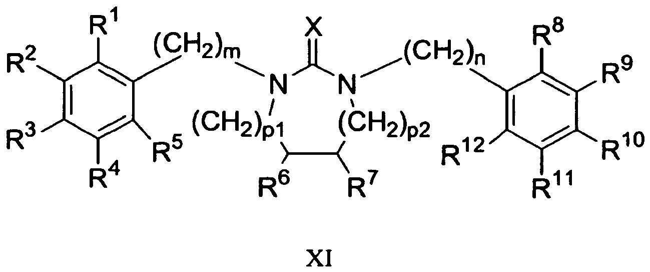 in which R¹ to R¹², X, m, n, p1 and p2 have the meanings stated in one or more of claims 1 to 14, provided that at least one of the substituents R⁸ to R¹² stands for an -N(PG)₂ group, in which PG has the meanings stated above, and this is optionally purified and/or isolated, and at least one compound of the general formula XI is converted, in a reaction medium, in the presence of at least one base, preferably in the presence of dimethylamine, or in the presence of at least one acid, preferably in the presence of at least one acid or in the presence of hydrazine and/or phenylhydrazine or in the presence of an alkali metal boron hydride, preferably in the presence of sodium
tetrahydridoborate, to form at least one compound of the general formula XII, 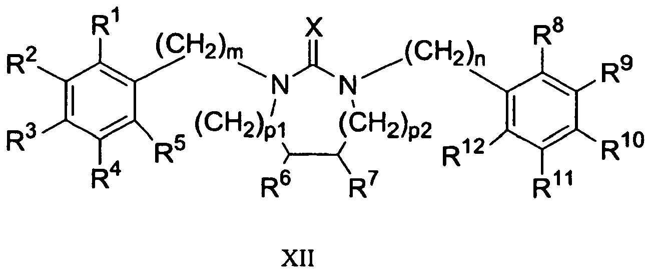 in which R¹ to R¹², X, m, n, p1 and p2 have the meanings stated above, provided that at least one of the substituents R⁸ to R¹² stands for an -NH₂ group, and this is optionally purified and/or isolated,
or
at least one compound of the general formula IV is caused to react, in a reaction medium, in the presence of at least one base, preferably in the presence of at least one metal hydride salt, more preferred in the presence of potassium and/or sodium hydride, or in the presence of at least one alkali metal carbonate salt, preferably in the presence of potassium and/or sodium carbonate, and at least one alkali metal iodide, preferably potassium and/or sodium iodide, with at least one compound of the general formula XIII, 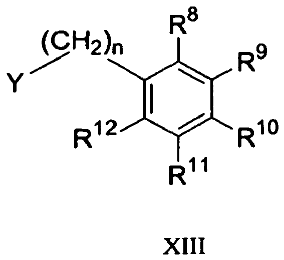 in which R⁸ to R¹² and n have the meanings stated in one or more of claims 1 to 14, provided that at least one of the substituents R⁸ to R¹² stands for an -NO₂ group, and
Y stands for a leaving group, preferably for a halogen radical, more preferred for a chlorine radical or bromine atom, to form at least one compound of the general formula XIV, 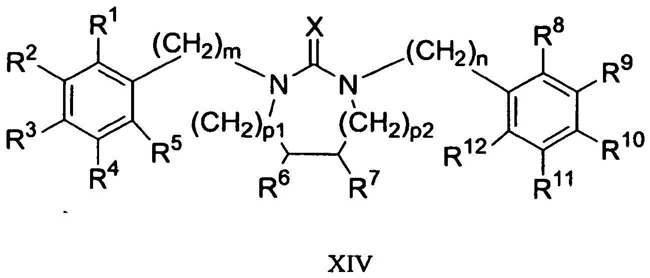 in which R¹ to R¹², X, m, n, p1 and p2 have the meanings stated above, provided that at least one of the substituents R⁸ to R¹² stands for an -NO₂ group, and this is optionally purified and/or isolated, and at least one compound of the general formula XIV is converted, in a reaction medium, in the presence of hydrogen and in the presence of a catalyst, to produce at least one compound of the general formula XII, in which R¹ to R¹², X, m, n, p1 and p2 have the meanings stated above, provided that at least one of the substituents R⁸ to R¹² stands for an -NH₂ group, and this is optionally purified and/or isolated,
and at least one compound of the general formula XII is caused to react, in a reaction medium, optionally in the presence of at least one base, preferably in the presence of at least one base selected from the group consisting of pyridine, triethylamine and diisopropylethylamine, with at least one compound of the general formula R²⁴-S(=O)₂-Z, in which R²⁴ has the meanings stated in one or more of claims 1 to 14 and Z stands for a leaving group, preferably for a halogen atom, more preferred for a chlorine atom, to form at least one compound of the general formula Ia, 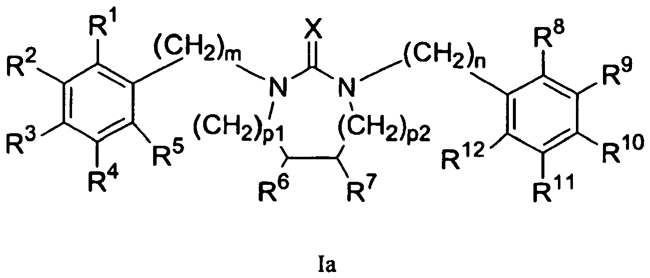 in which R¹ to R¹², X, m, n, p1 and p2 have the meanings stated above, provided that at least one of the substituents R⁸ to R¹² stands for an -NH-S(=O)₂-R²⁴ group, in which R²⁴ has the meanings stated above, and this is optionally purified and/or isolated,
or at least one compound of the general formula IV is caused to react, in a reaction medium, in the presence of at least one base, preferably in the presence of at least one metal hydride salt, more preferred in the presence of potassium and/or sodium hydride, or in the presence of at least one alkali metal carbonate salt, preferably in the presence of potassium and/or sodium carbonate, and at least one alkali metal iodide, preferably potassium and/or sodium iodide, with at least one compound of the general formula XV, 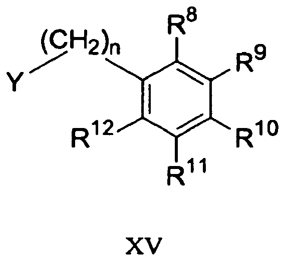 in which R⁸ to R¹² and n have the meanings stated in one or more of claims 1 to 14, provided that at least one of the substituents R⁸ to R¹² stands for an -NH-S(=O)₂-R²⁴ group, in which R²⁴ has the meanings stated above, to produce at least one compound of the general formula Ia, and this is optionally purified and/or isolated and optionally at least one compound of the general formula Ia is caused to react, in a reaction medium, optionally in the presence of at least one base, with at least one compound of the general formula R²³-Z, in which R²³ has the meanings stated above with the exception of hydrogen and Z stands for a leaving group, preferably for a halogen atom, more preferred for a chlorine atom, to produce at least one compound of the general formula Ib, 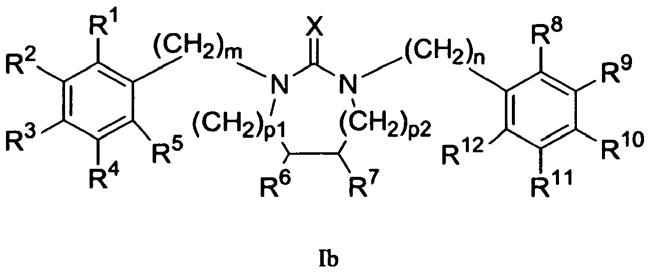 in which R¹ to R¹², X, m, n, p1 and p2 have the meanings stated above, provided that at least one of the substituents R⁸ to R¹² stands for an -NR²³-S(=O)₂-R²⁴ group, in which R²³ and R²⁴ have the meanings stated above, and this is optionally purified and/or isolated,
and optionally at least one compound of the general formula Ib, in which R¹ to R¹², m, n, p1 and p2 have the meanings stated in one or more of claims 1 to 14 and X stands for an oxygen atom, provided that at least one of the substituents R⁸ to R¹² stands for an -NR²³-S(=O)₂-R²⁴ group, in which R²³ and R²⁴ have the meanings stated above, is caused to react, in a reaction medium, with at least one compound of the general formula XVI 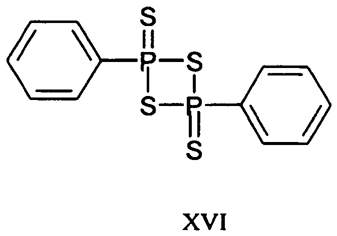 in which the phenyl radicals are each para-substituted by 1 or 2 substituents independently selected from the group consisting of methoxy, phenoxy, Cl, methyl and Br, preferably by a phenoxy radical or methoxy radical, and more preferred by a methoxy radical, or with phosphorus pentasulphide, to produce at least one compound of the general formula Ib in which R¹ to R¹², m, n and p have the meanings stated in one or more of claims 1 to 14 and X stands for or a sulphur atom, provided that at least one of the substituents R⁸ to R¹² stands for an -NR²³-S(=O)₂-R²⁴ group, in which R²³ and R²⁴ have the meanings stated above, and this is optionally purified and/or isolated.

16. The process for the preparation of substituted cyclic urea derivatives according to one or more of claims 1 to 14, **characterised in that**
at least one compound of the general formula II, 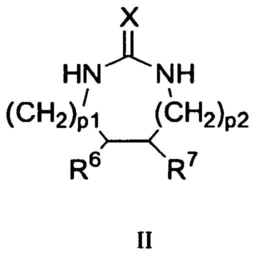 in which R⁶, R⁷, X, p1 and p2 have the meanings stated in one or more of claims 1 to 14, is caused to react, in a reaction medium, in the presence of at least one base, preferably in the presence of at least one metal hydride salt, more preferred in the presence of potassium and/or sodium hydride, or in the presence of at least one alkali metal carbonate salt, preferably in the presence of potassium and/or sodium carbonate, and at least one alkali metal iodide, preferably potassium and/or sodium iodide, with at least one compound of the general formula XVII, 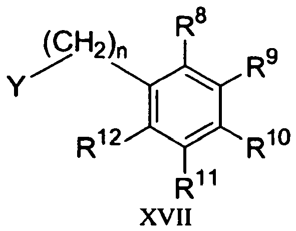 in which R⁸ to R¹² and n have the meanings stated in one or more of claims 1 to 14, provided that at least one of the substituents R⁸ to R¹² stands for an -N(PG)₂ group, wherein PG in each case stands for a protective group, or two PG groups, together with the interconnecting nitrogen atom, form a phthalimide group, or at least one of the substituents R⁸ to R¹² stands for an -NO₂ group, and Y stands for a leaving group, preferably for a halogen atom, more preferred for a chlorine or bromine atom, to produce at least one compound of the general formula XVIII, 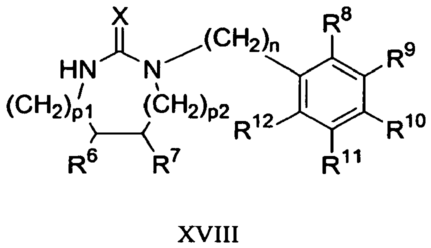 in which R⁶ to R¹², p1, p2 and n have the meanings stated above, provided that at least one of the substituents R⁸ to R¹² stands for an -N(PG)₂ group or an -NO₂ group, and this is optionally purified and/or isolated,
or
at least one compound of the general formula XIX, 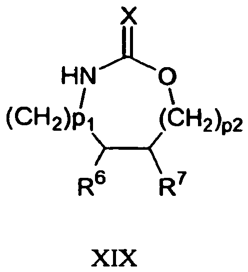 in which R⁶, R⁷, X, p1 and p2 have the meanings stated in one or more of claims 1 to 14, is caused to react, in a reaction medium, with at least one compound of the general formula XX, 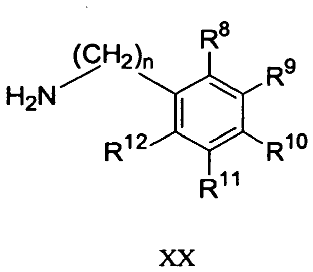 in which R⁸ to R¹² and n have the meanings stated in one or more of claims 1 to 14, provided that at least one of the substituents R⁸ to R¹² stands for an -N(PG)₂ group, in which PG in each case stands for a protective group, or two PG groups, together with the interconnecting nitrogen atom, form a cyclic protective group, preferably together with the interconnecting nitrogen atom a phthalimide group, or at least one of the substituents R⁸ to R¹² stands for an -NO₂ group, to produce at least one compound of the general formula XVIII and this is optionally purified and/or isolated,
or at least one compound of the general formula V, 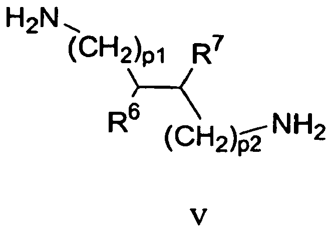 in which R⁶, R⁷, p1 and p2 have the meanings stated above, is caused to react, in a reaction medium, optionally in the presence of at least one base, with at least one compound of the general formula XVII to produce at least one compound of the general formula XXI, 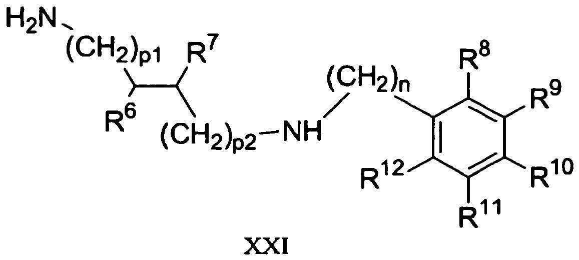 in which R⁶ to R¹², p1, p2 and n have the meanings stated in one or more of claims 1 to 14, provided that at least one of the substituents R⁸ to R¹² stands for an -N(PG)₂ group or an -NO₂ group, and this is optionally purified and/or isolated, and at least one compound of the general formula XXI is caused to react, in a reaction medium, optionally in the presence of at least one base, preferably in the presence of at least one base, with at least one compound of the general formula Z-C(=X)-Z, in which X stands for an oxygen atom or sulphur atom and Z stands for a leaving group, preferably for a halogen radical, more preferred for a chlorine atom, to produce at least one compound of the general formula XVIII, and this is optionally purified and/or isolated,
and at least one compound of the general formula XVIII is caused to react, in a reaction medium, in the presence of at least one base, preferably in the presence of at least one metal hydride salt, more preferred in the presence of potassium and/or sodium hydride, or in the presence of at least one alkali metal carbonate salt, preferably in the presence of potassium and/or sodium carbonate, and at least one alkali metal iodide, preferably potassium and/or sodium iodide, with at least one compound of the general formula III, 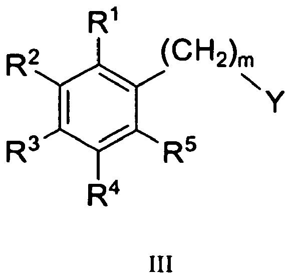 in which R¹ to R⁵ and m have the meanings stated in one or more of claims 1 to 14 and Y stands for a leaving group, preferably for a halogen radical, more preferred for a chlorine or bromine atom, to produce at least one compound of the general formula XXII, 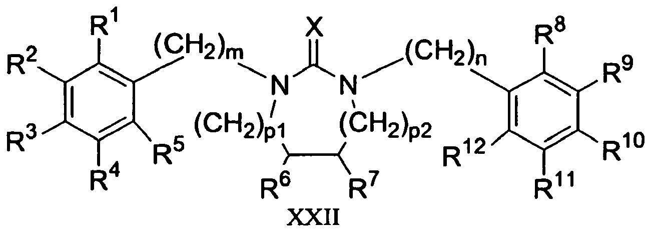 in which R¹ to R¹², X, m, n, p1 and p2 have the meanings stated in one or more of claims 1 to 14, provided that at least one of the substituents R⁸ to R¹² stands for an -N(PG)₂ group or -NO₂ group, and this is optionally purified and/or isolated
and at least one compound of the general formula XXII is caused to react, in a reaction medium, in the presence of at least one base, or in the presence of at least one acid, or in the presence of hydrazine and/or phenylhydrazine or in the presence of at least one alkali metal boron hydride, preferably in the presence of sodium tetrahydridoborate, or in the presence of hydrogen and a catalyst, to produce at least one compound of the general formula XII, 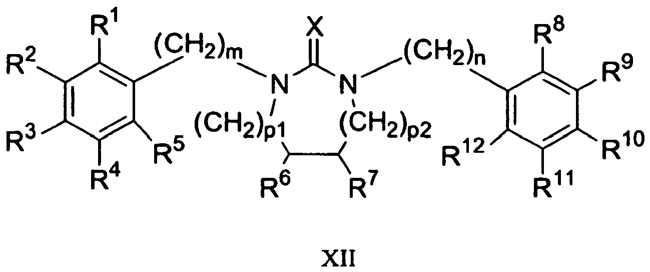 in which R¹ to R¹², X, m, n, p1 and p2 have the meanings stated above, provided that at least one of the substituents R⁸ to R¹² stands for an -NH₂ group, and this is optionally purified and/or isolated,
and at least one compound of the general formula XII is caused to react, in a reaction medium, optionally in the presence of at least one base, with at least one compound of the general formula R²⁴-S(=O)₂-Z, in which R²⁴ has the meanings stated in one or more of claims 1 to 14 and Z stands for a leaving group, preferably for a halogen atom, more preferred for a chlorine atom, to produce at least one compound of the general formula Ia, 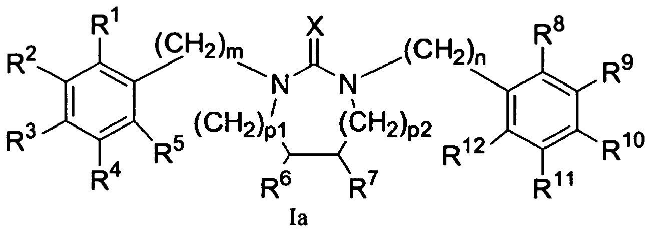 in which R¹ to R¹², X, m, n, p1 and p2 have the meanings stated above, provided that at least one of the substituents R⁸ to R¹² stands for an -NH-S(=O)₂-R²⁴ group, in which R²⁴ has the meanings stated above, and this is optionally purified and/or isolated,
and optionally at least one compound of the general formula Ia is caused to react, in a reaction medium, optionally in the presence of at least one base, with at least one compound of the general formula R²³-Z in which R²³ has the meanings stated in one or more of claims 1 to 14 with the exception of hydrogen and Z stands for a leaving group, preferably for a halogen atom, more preferred for a chlorine atom, to produce at least one compound of the general formula Ib, 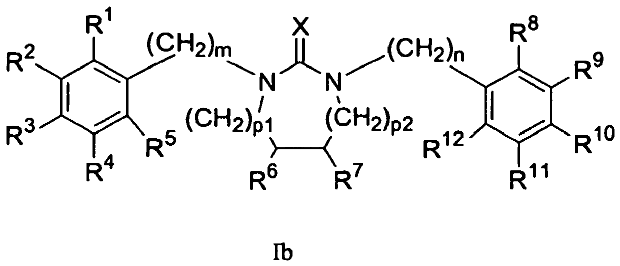 in which R¹ to R¹², X, m, n, p1 and p2 have the meanings stated above, provided that at least one of the substituents R⁸ to R¹² stands for an -NR²³-S(=O)₂-R²⁴ group, in which R²³ and R²⁴ have the meanings stated above, and this is optionally purified and/or isolated,
and optionally at least one compound of the general formula Ib in which R¹ to R¹², m, n, p1 and p2 have the meanings stated in one or more of claims 1 to 14 and X stands for an oxygen atom, provided that at least one of the substituents R⁸ to R¹² stands for an -NR²³-S(=O)₂-R²⁴ group, in which R²³ and R²⁴ have the meanings stated above, is caused to react, in a reaction medium, with at least one compound of the general formula XVI, 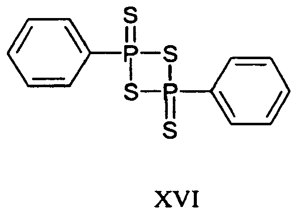 in which the phenyl radicals are each para-substituted by 1 or 2 substituents independently selected from the group consisting of methoxy, phenoxy, Cl, methyl and Br, preferably by a phenoxy radical or methoxy radical, and more preferred by a methoxy radical, or with phosphorus pentasulphide, to produce at least one compound of the general formula Ib in which R¹ to R¹², m, n and p have the meanings stated in one or more of claims 1 to 14 and X stands for a sulphur atom, provided that at least one of the substituents R⁸ to R¹² stands for an -NR²³-S(=O)₂-R²⁴ group, in which R²³ and R²⁴ have the meanings stated above, and this is optionally purified and/or isolated.

17. A process for the preparation of substituted cyclic urea derivatives according to one or more of claims 1 to 14, **characterised in that**
at least one compound of the general formula II, 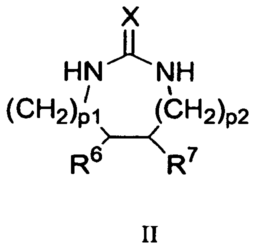 in which R⁶, R⁷, X, p1 and p2 have the meanings stated above, is caused to react, in a reaction medium, in the presence of at least one base, preferably in the presence of at least one metal hydride salt, more preferred in the presence of potassium and/or sodium hydride, or in the presence of at least one alkali metal carbonate salt, preferably in the presence of potassium and/or sodium carbonate, with at least one compound of the general formula XXIII, 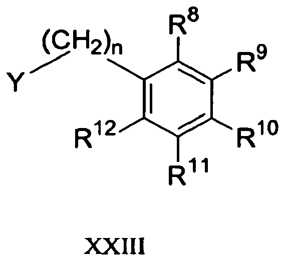 in which R⁸ to R¹² and n have the meanings stated in one or more of claims 1 to 14, provided that at least one of the substituents R⁸ to R¹² stands for an -NR²³-S(=O)₂-R²⁴ group and Y stands for a leaving group, preferably for a halogen atom, more preferred for a chlorine or bromine atom, to produce at least one compound of the general formula XXIV, 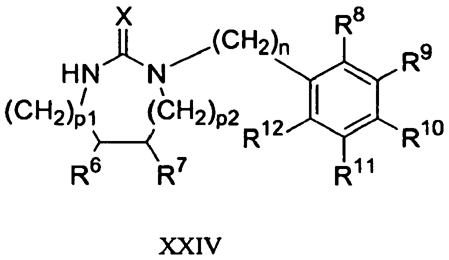 in which R⁶ to R¹², p1, p2 and n have the meanings stated above, provided that at least one of the substituents R⁸ to R¹² stands for an -NR²³-S(=O)₂-R²⁴ group, and this optionally purified and/or isolated,
and at least one compound of the general formula XXIV is caused to react, in a reaction medium, in the presence of at least one base, preferably in the presence of at least one metal hydride salt, more preferred in the presence of potassium and/or sodium hydride, or in the presence of at least one alkali metal carbonate salt, preferably in the presence of potassium and/or sodium carbonate, and at least one alkali metal iodide, preferably potassium and/or sodium iodide, with at least one compound of the general formula III, 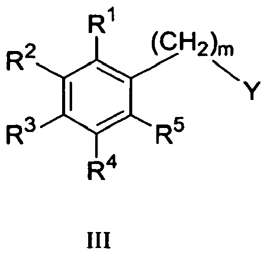 in which R¹ to R⁵ and m have the meanings stated in one or more of claims 1 to 14 and Y stands for a leaving group, preferably for a halogen radical, more preferred for a chlorine or bromine atom, to produce at least one compound of the general formula Ib, 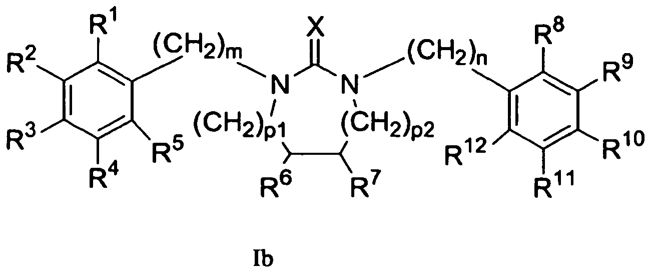 in which R¹ to R¹², X, m, n, p1 and p2 have the meanings stated above, provided that at least one of the substituents R⁸ to R¹² stands for an -NR²³-S(=O)₂-R²⁴ group, in which R²³ and R²⁴ have the meanings stated above, and this is optionally purified and/or isolated,
and optionally at least one compound of the general formula Ib in which R¹ to R¹², m, n, p1 and p2 have the meanings stated above and X stands for an oxygen atom, provided that at least one of the substituents R⁸ to R¹² stands for an -NR²³-S(=_{O})₂-R²⁴ group, in which R²³ and R²⁴ have the meanings stated above, is caused to react, in a reaction medium, with at least one compound of the general formula XVI, 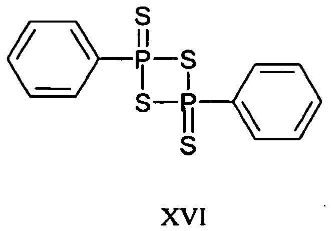 in which the phenyl radicals are each para-substituted by 1 or 2 substituents independently selected from the group consisting of methoxy, phenoxy, Cl, methyl and Br, preferably by a phenoxy radical or methoxy radical, and more preferred by a methoxy radical, or with phosphorus pentasulphide, to produce at least one compound of the general formula Ib in which R¹ to R¹² m, n and p have the meanings stated in one or more of claims 1 to 14 and X stands for a sulphur atom, provided that at least one of the substituents R⁸ to R¹² stands for an -NR²³-S(=O)₂-R²⁴ group, in which R²³ and R²⁴ have the meanings stated above, and this is optionally purified and/or isolated.

18. A medicinal drug containing at least one compound according to one or more of claims 1 to 14 and optionally one or more physiologically acceptable adjuvants.

19. A medicinal drug according to claim 18 for treatment and/or prophylaxis of one or more disorders selected from the group consisting of pain, preferably pain selected from the group consisting of acute pain, chronic pain, neuropathic pain and visceral pain; arthralgia; migraine; depression disorders; nervous disorders; neurotraumas; neurodegenerative disorders, preferably selected from the group consisting of multiple sclerosis, Alzheimer's disease, Parkinson's disease and Huntington's disease; cognitive dysfunctions, preferably cognitive deficiency states, particularly preferred memory defects; epilepsy; respiratory tract diseases, preferably selected from the group consisting of asthma and pneumonia; coughing; urinary incontinence; an overactive bladder (OAB); gastric ulcers; irritable bowel syndrome; strokes; eye irritations; skin irritations; neurotic skin conditions; inflammatory diseases, preferably inflammation of the intestine; diarrhoea; pruritus; eating disorders, preferably selected from the group consisting of bulimia, cachexia, anorexia and obesity; medication addiction; medication abuse; withdrawal symptoms following medication addiction; medication tolerance, preferably to natural or synthetic opioids; drug addiction; drug abuse; withdrawal symptoms following drug addiction; alcohol addiction; alcohol abuse and withdrawal symptoms following alcohol addiction; diuresis; antinatriuresis; for influencing the cardiovascular system; to improve vigilance; for libido enhancement; for modulation of mobility; for anxiolysis; for local anaesthesia and/or for inhibition of undesirable side effects, preferably selected from the group consisting of hyperthermia, hypertension and bronchial constriction, caused by the administration of vanilloid receptor 1 (VR1/TRPV1 receptor) agonists, preferably selected from the group consisting of capsaicin, resiniferatoxin, olvanil, arvanil, SDZ-249665, SDZ-249482, nuvanil and capsavanil.

20. The use of at least one compound according to one or more of claims 1 to 14 for the preparation of a medicinal drug for the treatment and/or prophylaxis of one or more disorders selected from the group consisting of pain, preferably pain selected from the group consisting of acute pain, chronic pain, neuropathic pain and visceral pain; arthralgia; migraine; depression disorders; nervous disorders; neurotraumas;
neurodegenerative disorders, preferably selected from the group consisting of multiple sclerosis, Alzheimer's disease, Parkinson's disease and Huntington's disease; cognitive dysfunctions, preferably cognitive deficiency states, more preferred memory defects; epilepsy; respiratory tract diseases, preferably selected from the group consisting of asthma and pneumonia; coughing; urinary incontinence; overactive bladder (OAB); gastric ulcers; irritable bowel syndrome; strokes; eye irritations; skin irritations; neurotic skin conditions; inflammatory diseases, preferably inflammation of the intestine; diarrhoea; pruritus; eating disorders, preferably selected from the group consisting of bulimia, cachexia, anorexia and obesity; medication addiction; medication abuse; withdrawal symptoms following medication addiction; medication tolerance, preferably to natural or synthetic opioids; drug addiction; drug abuse; withdrawal symptoms following drug addiction; alcohol addiction; alcohol abuse and withdrawal symptoms following alcohol addiction; for diuresis; for antinatriuresis; for influencing the cardiovascular system; to improve vigilance; for libido enhancement; for modulation of mobility; for anxiolysis; for local anaesthesia and/or for inhibition of undesirable side effects, preferably selected from the group consisting of hyperthermia, hypertension and bronchial constriction, caused by administration of vanilloid receptor 1 (VR1/TRPV1 receptor) agonists, preferably selected from the group consisting of capsaicin, resiniferatoxin, olvanil, arvanil, SDZ-249665, SDZ-249482, nuvanil and capsavanil (DA-5018).

21. The use according to claim 20 for the preparation of a medicinal drug for the treatment and/or prophylaxis of pain, selected from the group consisting of acute pain, chronic pain, neuropathic pain and visceral pain.

## Revendications

1. Dérivés d'urée cycliques substitués de formule générale 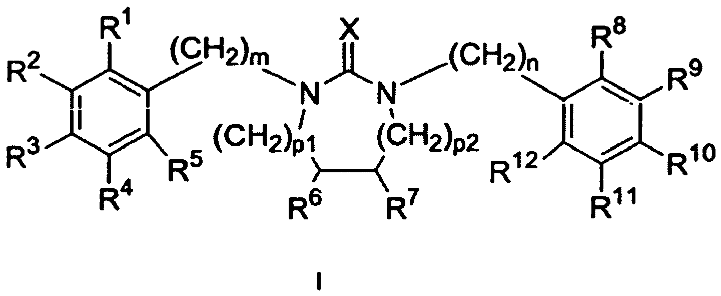 dans laquelle
X représente O, S ou N-C≡N ;
m vaut 1 ou 2 ;
n vaut 1 ou 2 ;
p1 et p2 représentent chacun indépendamment l'un de l'autre 0, 1, 2 ou 3, la somme de p1 et p2 étant de 0, 1, 2 ou 3 ;
R¹, R², R³, R⁴, R⁵, R⁸, R⁹, R¹⁰, R¹¹ et R¹² représentent chacun indépendamment les uns des autres
H ; F ; Cl ; Br ; I ; -SF₅ ; -NO₂ ; -NH₂ ; -OH ; -SH ; -C(=O)-NH₂ ; -S(=O)₂-NH₂ ; -NR¹³R¹⁴ ; -NH-R¹⁵ ; -OR¹⁶ ;-SR¹⁷ ; -O-(CH₂)ₐ-R¹⁸ ; -O-(CH₂)_{b}-OR¹⁹; -(CH₂)_{c}-O-(CH₂)_{d}-OR²⁰ ;
-(CH₂)ₑ-O-C(=O)-R²¹ ;
-(CH₂)_{f}-O-C(=O)-OR²² ;
-NR²³S(=O)₂R²⁹ ;
-(CH₂)_{g}-C(=O)-NR²⁵R²⁶ ;
-(CH₂)ₕ-C(=O)-NH-R²⁷ ;
-S(=O)ᵢR²⁸ ;
-(CH₂)ⱼ-S(=O)₂-NR²⁹R³⁰ ;
-(CH₂)ₖ-S(=O)₂-NHR³¹ ;
-(CH₂)ₗ-NR³²-C(=O)-(CH₂)_{q}-OR³³ ;
-(CH₂)ᵣ-NH-C(=O)-(CH₂)ₛ-OR³⁹ ;
-(CH₂)ₜ-NR³⁵-O-C(=O)-OR³⁶ ;
-(CH₂)ᵤ-NH-O-C(=O)-OR³⁷ ;
-(CH₂)ᵥ-O-S (=O)₂-R³⁸ ;
-(CH₂)_{w}-NR³⁹-C(=O)-SR⁴⁰ ;
-(CH₂)_{y}-C(=O)-NH-OR⁴¹ ;
-P(=O)-(OR⁴²)₂ ;
-(CH₂)_{z}-C(=S)-NR⁴³R⁴⁴ ;
-(CH₂)ₐₐ-C(=S)-NH-R⁴⁵ ;
-(CH₂)_{bb}-NR⁴⁶-C(=O)-R⁴⁷ ;
-(CH₂)_{cc}-NH-C(=O)-R⁴⁸ ;
ou -NH-C(=NH)-NH₂ ;
a, b, c, d, q et s valant chacun indépendamment les uns des autres 1, 2, 3, 4 ou 5 ;
e, f, g, h, j, k, l, r, t, u, v, w, x, y, z, aa, bb et cc valant chacun indépendamment les uns des autres 0, 1, 2, 3, 4 ou 5, et
i valant 1 ou 2 ;
un radical en C₁₋₁₀ aliphatique, saturé ou insaturé, linéaire ou ramifié, qui peut éventuellement être substitué avec 1, 2, 3, 4, 5, 6, 7, 8 ou 9 substituants choisis indépendamment les uns des autres dans le groupe constitué par F, Cl, Br, I, -CN, -NO₂, -OH, -NH₂, -SH, -O(alkyle en C₁₋₅), -S(alkyle en C₁₋₅), -NH (alkyle en C₁₋₅), -N (alkyle en C₁₋₅)(alkyle en C₁₋₅), -OCF3 et -SCF₃ ;
ou un radical aryle à 6 ou 10 éléments éventuellement substitué, qui peut être relié par un groupe alkylène en C₁₋₅ linéaire ou ramifié, éventuellement substitué ;
ou deux radicaux voisins choisis dans le groupe constitué par R⁸, R⁹, R¹⁰, R¹¹ et R¹² représentent ensemble un groupe méthylènedioxy(-O-CH₂-O) ;
à condition qu'au moins un des radicaux R⁸, R⁹, R¹⁰, R¹¹ et R¹² représente -NR²³S(=O)₂R²⁴ ;
R⁶ et R⁷ représentent chacun un radical hydrogène
ou
R⁶ et R⁷ forment ensemble avec le pont -C-C- qui les relie un radical phénylène non substitué ;
R¹³ R¹⁴, R¹⁵, R¹⁶, R¹⁷, R¹⁹, R²⁰, R²¹, R²², R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, R⁴², R⁴³, R⁴⁴, R⁴⁵, R⁴⁶, R⁴⁷ et R⁴⁸ représentent chacun indépendamment les uns des autres un radical en C₁₋₁₀ aliphatique, saturé ou insaturé, linéaire ou ramifié, qui peut éventuellement être substitué avec 1, 2, 3, 4, 5, 6, 7, 8 ou 9 substituants choisis indépendamment les uns des autres dans le groupe constitué par F, Cl, Br, I, -CN, -NO₂, -OH, -NH₂, -SH, -O(alkyle en C₁₋₅), -S(alkyle en C₁₋₅), -NH(alkyle en C₁₋₅), -N(alkyle en C₁₋₅)(alkyle en C₁₋₅), -OCF₃ et -SCF₃ ;
un radical cycloaliphatique à 3, 4, 5, 6, 7, 8 ou 9 éléments insaturé ou saturé, éventuellement substitué
ou un radical aryle ou hétéroaryle de 5 à 14 éléments éventuellement substitué, qui peut être condensé avec un système cyclique mono- ou polycyclique saturé ou insaturé, éventuellement substitué ;
R¹⁸ représente un radical cycloaliphatique à 3, 4, 5, 6, 7, 8 ou 9 éléments insaturé ou saturé, éventuellement substitué
ou un radical aryle ou hétéroaryle de 5 à 14 éléments éventuellement substitué, qui peut être condensé avec un système cyclique mono- ou polycyclique saturé ou insaturé, éventuellement substitué ;
R²³ représente un radical hydrogène
ou un radical en C₁₋₁₀ aliphatique, saturé ou insaturé, linéaire ou ramifié, qui peut éventuellement être substitué avec 1, 2, 3, 4, 5, 6, 7, 8 ou 9 substituants choisis indépendamment les uns des autres dans le groupe constitué par F, Cl, Br, I, -CN, -NO₂, -OH, -NH₂, -SH, -O(alkyle en C₁₋₅), -S(alkyle en C₁₋₅), -NH(alkyle en C₁₋₅), -N(alkyle en C₁₋₅) (alkyle en C₁₋₅), -OCF₃ et -SCF₃ ;
et
R²⁴ représente un radical en C₁₋₁₀ aliphatique, saturé ou insaturé, linéaire ou ramifié, qui peut éventuellement être substitué avec 1, 2, 3, 4 ou 5 substituants choisis indépendamment les uns des autres dans le groupe constitué par -CN, -NO₂, -OH, -NH₂, -SH, -O(alkyle en C₁₋₅), -S(alkyle en C₁₋₅), -NH(alkyle en C₁₋₅) et -N(alkyle en C₁₋₅) (alkyle en C₁₋₅), ou un radical aryle ou hétéroaryle de 5 à 14 éléments éventuellement substitué, qui peut être condensé avec un système cyclique mono- ou polycyclique saturé ou insaturé, éventuellement substitué ;
les radicaux cycloaliphatiques mentionnés précédemment pouvant chacun éventuellement être substitués avec 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -O-alkyle en C₁₋₅, -NH₂, -NO₂, -O-CF₃, -S-CF₃, -SH, -S-alkyle en C₁₋₅, -alkyle en C₁₋₅, -C(=O)-OH, -C(=O)-O-alkyle en C₁₋₅, -NH-alkyle en C₁₋₅, -N (alkyle en C₁₋₅)₂, -O-phényle, -O-benzyle, phényle et benzyle, la partie cyclique des radicaux -O-phényle, -O-benzyle, phényle et benzyle pouvant à chaque fois être substituée avec 1, 2, 3, 4, ou 5 substituants choisis indépendamment les uns des autres dans le groupe constitué par F, Cl, Br, -OH, -CF₃, -SF₅, -CN, -NO₂, -alkyle en C₁₋₅, -O-alkyle en C₁₋₅, -O-CF₃, -S-CF₃, phényle et -O-benzyle, et les radicaux cycloaliphatiques mentionnés précédemment pouvant chacun éventuellement comprendre 1, 2, 3, 4 ou 5 hétéroatomes choisis indépendamment les uns des autres dans le groupe constitué par oxygène, azote et soufre ;
le groupe alkylène en C₁₋₅ mentionné précédemment pouvant éventuellement être substitué par 1, 2, 3, 4, ou 5 substituants choisis indépendamment les uns des autres dans le groupe constitué par F, Cl, Br, -OH,-SH-, -NH₂, -CN et NO₂ ;
les cycles des systèmes cycliques mono- ou polycycliques mentionnés précédemment pouvant chacun éventuellement être substitués avec 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -O-alkyle en C₁₋₅, -NH₂, -NO₂, -O-CF₃, -S-CF₃, -SH, -S-alkyle en C₁₋₅, -alkyle en C₁₋₅, -C(=O)-OH, -C(=O)-O-alkyle en C₁₋₅,-NH-alkyle en C₁₋₅, -N (alkyle en C₁₋₅)₂, -O-phényle, -O-benzyle, phényle et benzyle, la partie cyclique des radicaux -O-phényle, -O-benzyle, phényle et benzyle pouvant à chaque fois être substituée avec 1, 2, 3, 4, ou 5 substituants choisis indépendamment les uns des autres dans le groupe constitué par F, Cl, Br, -OH,-CF₃, -SF₅, -CN, -NO₂, -alkyle en C₁₋₅, -O-alkyle en C₁₋₅, -O-CF₃, -S-CF₃, phényle et -O-benzyle,
et les cycles des systèmes cycliques mono- ou polycycliques mentionnés précédemment pouvant chacun être à 5, 6 ou 7 éléments et pouvant chacun éventuellement comprendre 1, 2, 3, 4 ou 5 hétéroatomes en tant qu'éléments de cycle, qui sont choisis indépendamment les uns des autres dans le groupe constitué par oxygène, azote et soufre ;
et les radicaux aryle ou hétéroaryle mentionnés précédemment pouvant chacun éventuellement être substitués avec 1, 2, 3, 4 ou 5 substituants choisis indépendamment les uns des autres dans le groupe constitué par F, Cl, Br, I, -CN, -CF₃, -SF₅, -OH, -O-alkyle en C₁₋₅, -NH₂, -NO₂, -O-CF₃, -S-CF₃, -SH, -S-alkyle en C₁₋₅, -alkyle en C₁₋₅, -C(=O)-OH, -C(=O)-O-alkyle en C₁₋₅, -NH-alkyle en C₁₋₅, -N (alkyle en C₁₋₅)₂,-NH-C(=O)-O-alkyle en C₁₋₅, -C(=O)-H, -C(=O)-alkyle en C₁₋₅, -C(=O)-NH₂, -C(=O)-NH-alkyle en C₁₋₅, -C(=O)-N-(alkyle en C₁₋₅)₂, -O-phényle, -O-benzyle, phényle et benzyle, la partie cyclique des radicaux -O-phényle,-O-benzyle, phényle et benzyle pouvant à chaque fois être substituée avec 1, 2, 3, 4, ou 5 substituants choisis indépendamment les uns des autres dans le groupe constitué par F, Cl, Br, -OH, -CF₃, -SF₅, -CN,-NO₂, -alkyle en C₁₋₅, -O-alkyle en C₁₋₅, -O-CF₃, -S-CF₃, phényle et -O-benzyle,
et
les radicaux hétéroaryle mentionnés précédemment comprenant chacun 1, 2, 3, 4 ou 5 hétéroatomes choisis indépendamment les uns des autres dans le groupe constitué par oxygène, azote et soufre en tant qu'éléments de cycle ;
chacun éventuellement sous la forme d'un de leurs stéréoisomères purs, notamment énantiomères ou diastéréomères, de leurs racémats ou sous la forme d'un mélange de stéréoisomères, notamment des énantiomères et/ou diastéréomères, en un rapport de mélange quelconque, ou chacun sous la forme des sels correspondants, ou chacun sous la forme des solvates correspondants.

2. Composés selon la revendication 1, **caractérisés en ce que**
R¹, R², R⁴ et R⁵ représentent chacun indépendamment les uns des autres
H ; F ; Cl ; Br ; I ; -SF₅ ; -NO₂ ; -NH₂ ; -OH ; -SH ; -C(=O)-NH₂ ; -S (=O)₂-NH₂ ; -NR¹³R¹⁴ ; -NH-R¹⁵ ; -OR¹⁶ ;-SR¹⁷ ; -O-(CH₂)ₐ-R¹⁸ ; -O-(CH₂)_{b}-OR¹⁹ ; -(CH₂)_{c}-O-(CH₂)_{d}-OR²⁰ _{;}
-(CH₂)ₑ-O-C(=O)-R²¹ ;
-(CH₂)_{f}-O-C(=O)-OR²² ;
-NR²³S(=O)₂R²⁴ ;
-(CH₂)_{g}-C(=O)-NR²⁵R²⁶ ;
-(CH₂)ₕ-C(=O)-NH-R²⁷ ;
-S(=O)ᵢR²⁸ ;
-(CH₂)ⱼ-S(=O)₂-NR²⁹R³⁰ ;
-(CH₂)ₖ-S(=O)₂-NHR³¹ ;
-(CH₂)ₗ-NR³²-C(=O)-(CH₂)_{q}-OR³³ ;
-(CH₂)ᵣ-NH-C(=O)-(CH₂)ₛ-OR³⁴ ;
-(CH₂)ₜ-NR³⁵-O-C(=O)-OR³⁶ ;
-(CH₂)ᵤ-NH-O-C(=O)-OR³⁷ ;
-(CH₂)ᵥ-O-S(=O)₂-R³⁸ ;
-(CH₂)_{w}-NR³⁹-C(=O)-SR⁴⁰ ;
-(CH2)_{y}-C(=O)-NH-OR⁴¹ ;
-P(=O)-(OR⁴²)₂ ;
-(CH₂)-C(=S)-NR⁴³R⁴⁴ ;
-(CH₂)ₐₐ-C(=S)-NH-R⁴⁵ ;
-(CH₂)_{bb}-NR⁴⁶-C(=O)-R⁴⁷ ;
-(CH₂)_{cc}-NH-C(=O)-R⁴⁸ ;
ou -NH-C(=NH)-NH₂ ;
a, b, c, d, q et s valant chacun indépendamment les uns des autres 1, 2, 3, 4 ou 5 ;
e, f, g, h, j, k, l, r, t, u, v, w, x, y, z, aa, bb et cc valant chacun indépendamment les uns des autres 0, 1, 2, 3, 4 ou 5, et
i valant 1 ou 2 ;
un radical choisi dans le groupe constitué par méthyle, CF₃, -CCl₃, -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃,-CH₂-N(CH₃)₂, -CH₂-N (C₂H₅)₂, -CH₂-N (CH₃) (C₂H₅), éthyle,-CH₂-CH₂-NH₂, -CH₂-CH₂-OH, -CH₂-CH₂-SH, -CH₂-CH₂-NH-CH₃,-CH₂-CH₂-N (CH3)2, -CH₂-CH₂-N (C₂H₅)2, -CH₂-CH₂-N (CH₃) (C₂H₅), -CH₂-CF₃, -C₂F₅, -CH₂-CCl₃, -CH₂-CBr3, -CH₂-CH₂-CN, n-propyle, -CH₂-CH₂-CH₂-OH, -CH₂-CH₂-CH₂-SH, -CH₂-CH₂-CH₂-NH₂, -CH₂-CH₂-CH₂-NH-CH₃, -CH₂-CH₂-CH₂-N(CH₃)₂, -CH₂-CH₂-CH₂-N(C₂H₅)₂, -CH₂-CH₂-CH₂-N(CH₃) (C₂H₅), -CH₂-CH₂-O-CH₃, - CF₂-CF₂-CF₃, -CF(CF₃)₂, isopropyle, -CH₂-CH₂-CH₂-CN, - CH₂-O-CH₂-CH₃, -CH₂-CH₂-SF₃, -CH₂-CH₂-OCF₃, -CH(CH₃)(O-CH₃), -CH(CH₃) (S-CH₃), n-butyle, -CF₂-CF₂-CF₂-CF₃, -CH₂-CH₂-CH₂-CH₂-CN, sec-butyle, isobutyle, tert-butyle, n-pentyle, sec-pentyle, néo-pentyle, n-hexyle, vinyle, 1-propényle, 2-propényle, 1-butényle, 2-butényle, 3-butényle, 2-méthyl-butén-2-yle, (1,1,2)-trifluoro-1-butényle, 1-pentényle, 2-pentényle, 3-pentényle et 4-pentényle ;
ou un radical aryle choisi dans le groupe constitué par phényle et naphtyle, le radical aryle pouvant à chaque fois être substitué avec 1, 2, 3, 4 ou 5 substituants choisis indépendamment les uns des autres dans le groupe constitué par F, Cl, Br, I, -CN, -NO₂, -OH, -SH, méthyle, éthyle, -CF₃, -O-CF₃, -S-CF₃, -SF₅, -O-CH₃, -O-C₂H₅, -NH₂, -N(CH₃)₂, -N(C₂H₅)₂,-N(H)(CH₃) et -N(H)(C₂H₅) ;
et
R³ représente F ; Cl ; Br ; I ; -SF₅ ; -NO₂ ; -NH₂ ; -OH ; -SH ; -C(=O)-NH₂ ; -S(=O)₂-NH₂ ; -NR¹³R¹⁴ ; -NH-R¹⁵ ; -OR¹⁶ ; -SR¹⁷ ; -O-(CH₂)ₐ-R¹⁸, -O-(OH₂)_{b}-OR¹⁹ ou-NR²³S(=O)₂R²⁴ ;
a et b représentant chacun indépendamment l'un de l'autre 1, 2, 3, 4 ou 5 ;
ou un radical choisi dans le groupe constitué par méthyle, CF₃, -CCl₃, -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃,-CH₂-N(CH₃)₂, -CH₂-N(C₂H₅)₂, -CH₂-N(CH₃) (C₂H₅), éthyle,-CH₂-CH₂-NH₂, -CH₂-CH₂-OH, -CH₂-CH₂-SH, -CH₂-CH₂-NH-CH₃,-CH₂-CH₂-N (CH₃)₂, -CH₂-CH₂-N (C2H₅) ₂, -CH₂-CH₂-N (CH₃) (C₂H₅), -CH₂-CF₃, -C₂F₅, -CH₂-CCl₃, -CH₂-CBr₃, -CH₂-CH₂-CN, n-propyle, -CH₂-CH₂-CH₂-OH, -CH₂-CH₂-CH₂-SH, -CH₂-CH₂-CH₂-NH₂, -CH₂-CH₂-CH₂-NH-CH₃, -CH₂-CH₂-CH₂-N(CH₃)₂, -CH₂-CH₂-CH₂-N (C₂H₅)₂, -CH₂-CH₂-CH₂N (CH₃) (C₂H₅), -CH₂-CH₂-O-CH₃,-CF₂-CF₂-CF₃, -CF(CF₃)₂, isopropyle, -CH₂-CH₂-CH₂-CN,-CH₂-O-CH₂-CH₃, -CH₂-CH₂-SF₃, -CH₂-CH₂-OCF₃, -CH(CH₃)(O-CH₃), -CH(CH₃) (S-CH₃), n-butyle, -CF₂-CF₂-CF₂-CF₃, -CH₂-CH₂-CH₂-CH₂-CN, sec-butyle, isobutyle, tert-butyle, n-pentyle, sec-pentyle, néo-pentyle, n-hexyle, vinyle, 1-propényle, 2-propényle, 1-butényle, 2-butényle, 3-butényle, 2-méthyl-butén-2-yle, (1,1,2)-trifluoro-1-butényle, 1-pentényle, 2-pentényle, 3-pentényle et 4-pentényle.

3. Composés selon la revendication 1 ou 2, **caractérisés en ce que**
R¹, R², R⁹ et R⁵ représentent chacun indépendamment les uns des autres
H ; F ; Cl; I ; Br ; -NO₂ ; -NH₂ ; -OH ; -SH ; - NR¹³R¹⁴ ; -NH-R¹⁵ ; -OR¹⁶ ; -SR¹⁷ ; NR²³S(=O)₂R²⁴ ;
ou un radical choisi dans le groupe constitué par méthyle, -CF₃, éthyle, -CH₂-CF₃, -C₂F₅, n-propyle, isopropyle, n-butyle, sec-butyle, isobutyle, tert-butyle ou n-pentyle
et
R³ représente F ; Cl; Br ; I ; -OR¹⁶ ; NR²³S(=O)₂R²⁴ ;
ou un radical choisi dans le groupe constitué par-SF₅, -CF₃, -C₂F₅, -CH₂-CF₃, -CF(CF₃)₂, isopropyle,-CH (CH₃) (O-CH₃), -CH (CH₃) (S-CH₃), sec-butyle, isobutyle et tert-butyle.

4. Composés selon une ou plusieurs des revendications 1 à 3, **caractérisés en ce que**
R⁸, R⁹, R¹¹ et R¹² représentent chacun indépendamment les uns des autres
H ; F ; Cl ; Br ; I ; -SF₅ ; -NO₂ ; -NH₂ ; -OH ; -SH ; -C(=O)-NH₂ ; -S(=O)₂-NH₂ ; -NR¹³R¹⁴ ; -NH-R¹⁵ ; -OR¹⁶ ; SR¹⁷ ; -O-(CH₂)ₐ-R¹⁸ ; -O-(CH₂)_{b}-OR¹⁹ ; -(CH₂)_{c}-O-(CH₂)_{d}-OR²⁰ ;
-(CH₂)ₑ-O-C(=O)-R²¹ ;
-(CH₂)_{f}-O-C(=O)-OR²² ;
-NR²³S(=O)₂R²⁴ ;
-(CH₂)_{g}-C(=O)-NR²⁵R²⁶ ;
-(CH₂)ₕ-C(=O)-NH-R²⁷ ;
-S(=O)ᵢR²⁸ ;
-(CH₂)ⱼ-S(=O)₂-NR²⁹R³⁰ ;
-(CH₂)ₖ-S(=O)₂-NHR³¹ ;
-(CH₂)ₗ-NR³²-C(=O)-(CH₂)_{q}-OR³³ ;
-(CH₂)ᵣ-NH-C(=O)-(CH₂)ₛ-OR³⁴ ;
-(CH₂)ₜ-NR³⁵-O-C(=O)-OR³⁶ ;
-(CH₂)ᵤ-NH-O-C(=O)-OR³⁷ ;
-(CH₂)ᵥ-O-S(=O)₂-R³⁸ ;
-(CH₂)_{w}-NR³⁹-C(=O)-SR⁴⁰ ;
-(CH₂)_{y}-C(=O)-NH-OR⁴¹ ;
-P(=O)-(OR⁴²)₂ ;
-(CH₂)_{z}-C(=S)-NR⁴³R⁴⁴ ;
-(CH₂)ₐₐ-C(=S)-NH-R⁴⁵ ;
-(CH₂)_{bb}-NR-C(=O)-R⁴⁷ ;
-(CH₂)_{cc}-NH-C(=O)-R⁴⁸ ;
ou -NH-C(=NH)-NH₂ ;
a, b, c, d, q et s valant chacun indépendamment les uns des autres 1, 2, 3, 4 ou 5 ;
e, f, g, h, j, k, l, r, t, u, v, w, x, y, z, aa, bb et cc valant chacun indépendamment les uns des autres 0, 1, 2, 3, 4 ou 5, et
i valant 1 ou 2 ;
un radical choisi dans le groupe constitué par méthyle, CF₃, -CCl₃, -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃,-CH₂-N(CH₃)₂, -CH₂-N(C₂H₅)₂, -CH₂-N(CH₃) (C₂H₅), éthyle,-CH₂-CH₂-NH₂, -CH₂-CH₂-OH, -CH₂-CH₂-SH, -CH₂-CH₂-NH-CH₃,-CH₂-CH₂-N (CH₃)₂, -CH₂-CH₂-N (C₂H₅)₂, -CH₂-CH₂-N (CH₃) (C₂H₅), -CH₂-CF3, -C₂F5, -CH₂-CCl₃, -CH₂-CBr3, -CH₂-CH₂-CN, n-propyle, -CH₂-CH₂-CH₂-OH, -CH₂-CH₂-CH₂-SH, -CH₂-CH₂-CH₂-NH₂, -CH₂-CH₂-CH₂-NH-CH₃, -CH₂-CH₂-CH₂-N(CH₃)₂, -CH₂-CH₂-CH₂-N(C2H₅)₂, -CH₂-CH₂-CH₂-N (CH₃)(C₂H₅), -CH₂-CH₂-O-CH₃,-CF₂-CF₂-CF₃, -CF(CF₃)₂, isopropyle, -CH₂-CH₂-CH₂-CN,-CH₂-O-CH₂-CH₃, -CH₂-CH₂-SF₃, -CH₂-CH₂-OCF₃, -CH(CH₃)(O-CH₃), -CH(CH₃)(S-CH₃), n-butyle, -CF₂-CF₂-CF₂-CF₃, -CH₂-CH₂-CH₂-CH₂-CN, sec-butyle, isobutyle, tert-butyle, n-pentyle, sec-pentyle, néo-pentyle, n-hexyle, vinyle, 1-propényle, 2-propényle, 1-butényle, 2-butényle, 3-butényle, 2-méthyl-butén-2-yle, (1,1,2)-trifluoro-1-butényle, 1-pentényle, 2-pentényle, 3-pentényle et 4-pentényle ;
ou un radical aryle choisi dans le groupe constitué par phényle et naphtyle, le radical aryle pouvant à chaque fois être substitué avec 1, 2, 3, 4 ou 5 substituants choisis indépendamment les uns des autres dans le groupe constitué par F, Cl, Br, I, -CN, -NO₂, -OH, -SH, méthyle, éthyle, -CF₃, -O-CF₃, -S-CF₃, -SF₅, -O-CH₃, -O-C₂H₅, -NH₂, -N(CH₃)₂, -N(C₂H₅)₂,-N(H)(CH₃) et -N(H)(C₂H₅) ;
et
R¹⁰ représente -NR²³S (=O) ₂R²⁴.

5. Composés selon une ou plusieurs des revendications 1 à 4, **caractérisés en ce que**
R⁸, R⁹, R¹¹ et R¹² représentent chacun indépendamment les uns des autres
H ; F ; Cl ; Br ; I ; -NO₂ ; -NH₂ ; -OH ; -SH ;-NR¹³R¹⁴ ; -NH-R¹⁵ ; -OR¹⁶ ; -SR¹⁷ ; NR²³S(=O)₂R²⁴ ;
ou un radical choisi dans le groupe constitué par méthyle, -CF₃, éthyle, -CH₂-CF₃, -C₂F₅, n-propyle, isopropyle, n-butyle, sec-butyle, isobutyle, tert-butyle ou n-pentyle
et
R¹⁰ représente -NR²³S (=O) ₂R²⁴.

6. Composés selon une ou plusieurs des revendications 1 à 5, **caractérisés en ce que**
le radical R¹⁰ représente -NR²³S(=O)₂R²⁴ et les radicaux R⁸, R⁹, R¹¹ et R¹² représentent chacun H.

7. Composés selon une ou plusieurs des revendications 1 à 6, **caractérisés en ce que**
R¹³, R¹⁴, R¹⁵, R¹⁶, R¹⁷, R¹⁹, R²⁰, R²¹, R²², R²⁵, R²⁶, R²⁷, R²⁸, R²⁹, R³⁰, R³¹, R³², R³³, R³⁴, R³⁵, R³⁶, R³⁷, R³⁸, R³⁹,
R⁴⁰, R⁴¹, R⁴², R⁹³, R⁴⁹, R⁴⁵, R⁴⁶, R⁴⁷ et R⁴⁸ représentent chacun indépendamment les uns des autres
un radical choisi dans le groupe constitué par méthyle, -CF₃, -CCl₃, -CBr₃, -CH₂-CN, -CH₂-O-CH₃, -CH₂-O-CF₃, -CH₂-SF₃, -CH₂-NH₂, -CH₂-OH, -CH₂-SH, -CH₂-NH-CH₃, -CH₂-N(CH₃)₂, -CH₂-N(C₂H₅)₂, -CH₂-N(CH₃) (C₂H₅), éthyle,-CH₂-CH₂-NH₂, -CH₂-CH₂-OH, -CH₂-CH₂-SH, -CH₂-CHz-NH-CH₃,-CH₂-CH₂-N (CH₃)₂, -CH₂-CH₂-N (C₂H₅)₂, -CH₂-CH₂-N(CH₃)(C₂H₅) , -CH₂-CF₃, -C₂F₅, -CH₂-CCl₃, -CH₂-CBr₃, -CH₂-CH₂-CN, n-propyle, -CH₂-CH₂-CH₂-OH, -CH₂-CH₂-CH₂-SH, -CH₂-CH₂-CH₂-NH₂, -CH₂-CH₂-CH₂-NH-CH₃, -CH₂-CHrCHrN (CH₃)₂, -CH₂-CH₂-CH₂-N(C₂H₅)₂, -CH₂-CH₂-CH₂-N(CH₃) (C₂H₅), -CH₂-CH₂-O-CH₃,-CF₂-CF₂-CF₃, -CF(CF₃)₂, isopropyle, -CH₂-CH₂-CH₂-CN,-CH₂-O-CH₂-CH₃, -CH₂-CH₂-SF₃, -CH₂-CH₂-OCF₃, -CH(CH₃)(O-CH₃), -CH(CH₃)(S-CH₃), n-butyle, -CF₂-CF₂-CF₂-CF₃, -CH₂-CH₂-CH₂-CH₂-CN, sec-butyle, isobutyle, tert-butyle, n-pentyle, sec-pentyle, néo-pentyle, n-hexyle, vinyle, 1-propényle, 2-propényle, 1-butényle, 2-butényle, 3-butényle, 2-méthyl-butén-2-yle, (1,1,2)-trifluoro-1-butényle, 1-pentényle, 2-pentényle, 3-pentényle et 4-pentényle ;
un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle et dithiolanyle, le radical (hétéro)cycloaliphatique pouvant à chaque fois éventuellement être substitué avec 1, 2, 3, 4 ou 5 substituants choisis indépendamment les uns des autres dans le groupe constitué par thioxo (=S), F, Cl, Br, I, -CN, -CF₃,-SF₅, -OH, -NH₂, -O-CF₃, -SH, -O-CH₃, -O-C₂H₅, méthyle, éthyle, n-propyle, isopropyle, n-butyle, sec-butyle, isobutyle, tert-butyle, S-CH₃, -S-C₂H₅, -C(=O)-O-CH₃, - C(=O)-O-C₂H₅, oxo (=O), -N(CH₃)₂, -N(C₂H₅)₂, -N (H) (CH₃), -N (H) (C₂H₅), -NO₂, -SCF₃, -C (=O) -OH, -O-phényle, -O-benzyle, phényle et benzyle
ou un radical choisi dans le groupe constitué par phényle, naphtyle, (1,3)-benzodioxolyle, (1,4)-benzodioxanyle, thiophényle, furanyle, pyrrolyle, pyrazolyle, pyrazinyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinoxalinyle, quinolinyle et isoquinolinyle, le radical pouvant à chaque fois éventuellement être substitué avec 1, 2, 3, 4 ou 5 substituants choisis indépendamment les uns des autres dans le groupe constitué par F, Cl, Br, I, -CN, -NO₂, -OH, -SH, méthyle, éthyle, n-propyle, isopropyle, n-butyle, sec-butyle, isobutyle, tert-butyle, -CF₃, -O-CF₃, -S-CF₃, -SF₅, -O-CH₃, -O-C₂H₅, O-phényle, -O-benzyle, -NH₂, -N(CH₃)₂, -N(C₂H₅)₂,-N(H)(CH₃) et -N(H)(C₂H₅).

8. Composés selon une ou plusieurs des revendications 1 à 7, **caractérisés en ce que**
R¹⁸ représente un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofuranyle, tétrahydrothiophényle, pyrrolidinyle, pipéridinyle, morpholinyle, pipérazinyle, thiomorpholinyle, tétrahydropyranyle, azépanyle, diazépanyle et dithiolanyle, le radical (hétéro)cycloaliphatique pouvant à chaque fois éventuellement être substitué avec 1, 2, 3, 4 ou 5 substituants choisis indépendamment les uns des autres dans le groupe constitué par thioxo (=S), F, Cl, Br, I, -CN, -CF₃,-SF₅, -OH, -NH₂, -O-CF₃, -SH, -O-CH₃, -O-C₂H₅, méthyle, éthyle, n-propyle, isopropyle, n-butyle, sec-butyle, isobutyle, tert-butyle, S-CH₃, -S-C₂H₅, -C(=O)-O-_{C}H3,-C(=O)-O-C₂H₅, oxo (=O), -N(CH₃)₂, -N(C₂H₅)₂, -N(H)(CH₃), -N (H) (C₂H₅) -NO₂, -SCF₃, -C(=O)-OH, -O-phényle, -O-benzyle, phényle et benzyle,
ou un radical choisi dans le groupe constitué par phényle, naphtyle, (1,3)-benzodioxolyle, (1,4)-benzodioxanyle, thiophényle, furanyle, pyrrolyle, pyrazolyle, pyrazinyle, pyranyle, triazolyle, pyridinyle, imidazolyle, indolyle, isoindolyle, benzo[b]furanyle, benzo[b]thiophényle, thiazolyle, oxazolyle, isoxazolyle, pyridazinyle, pyrazinyle, pyrimidinyle, indazolyle, quinoxalinyle, quinolinyle et isoquinolinyle, le radical pouvant à chaque fois éventuellement être substitué avec 1, 2, 3, 4 ou 5 substituants choisis indépendamment les uns des autres dans le groupe constitué par F, Cl, Br, I, -CN, -NO₂, -OH, -SH, méthyle, éthyle, n-propyle, isopropyle, n-butyle, sec-butyle, isobutyle, tert-butyle, -CF₃, -O-CF₃, -S-CF₃, -SF₅, -O-CH₃, -O-C₂H₅, O-phényle, -O-benzyle, -NH₂, -N(CH₃)₂, -N (C₂H₅)₂,-N(H)(CH₃), -N(H)(C₂H₅) et -NH-C(=O)-O-CH₃.

9. Composés selon une ou plusieurs des revendications 1 à 8, **caractérisés en ce que**
R²³ représente un radical hydrogène
ou un radical choisi dans le groupe constitué par méthyle, éthyle, n-propyle, isopropyle, n-butyle, sec-butyle, isobutyle, tert-butyle, n-pentyle, sec-pentyle, néo-pentyle et n-hexyle.

10. Composés selon une ou plusieurs des revendications 1 à 9, **caractérisés en ce que**
R²⁴ représente un radical choisi dans le groupe constitué par méthyle, -CH₂-CN, éthyle, -CH₂-CH₂-CN, n-propyle, isopropyle, n-butyle, sec-butyle, isobutyle, tert-butyle, n-pentyle, sec-pentyle, néo-pentyle et n-hexyle.

11. Composés selon une ou plusieurs des revendications 1 à 10, **caractérisés en ce que**
p1 et p2 représentent chacun indépendamment l'un de l'autre 0 ou 1, la somme de p1 et p2 étant de 0 ou 1.

12. Composés selon une ou plusieurs des revendications 1 à 11, **caractérisés en ce que**
X représente O, S ou N-C≡N ;
m vaut 1 ;
n vaut 1 ;
p1 et p2 représentent chacun indépendamment l'un de l'autre 0 ou 1, la somme de p1 et p2 étant de 0 ou 1 ; R¹, R² , R⁴, R⁵, R⁸, R⁹, R¹¹ et R¹² représentent chacun indépendamment les uns des autres
H ; F ; Cl ; I ; Br ; -NO₂ ; -NH₂ ; -OH ; -SH ;-NR¹³R¹⁴ ; -NH-R¹⁵ ; -OR¹⁶ ; -SR¹⁷ ; NR²³S (=O)₂R²⁴;
ou un radical choisi dans le groupe constitué par méthyle, -CF₃, éthyle, -CH₂-CF₃, -C₂F₅, n-propyle, isopropyle, n-butyle, sec-butyle, isobutyle, tert-butyle ou n-pentyle ;
R³ représente F ; Cl ; Br ; I ; -OR¹⁶ ; NR²³S (=O)₂R²⁴; ou un radical choisi dans le groupe constitué par-SF₅, -CF₃, -C₂F₅, -CH₂-CF₃, -CF(CF₃)₂, isopropyle,-CH(CH₃)(O-CH₃), -CH(CH₃)(S-CH₃), sec-butyle, isobutyle et tert-butyle ;
R⁶ et R⁷ représentent chacun un radical hydrogène
ou
R⁶ et R⁷ forment ensemble avec le pont -C-C- qui les relie un radical phénylène non substitué ;
R¹⁰ représente NR²³S(=O)₂R²⁴;
R¹³, R¹⁴, R¹⁵, R¹⁶ et R¹⁷ représentent chacun indépendamment les uns des autres
un radical choisi dans le groupe constitué par -CF₃,-C₂F₅, -CH₂-CF₃, méthyle, éthyle, n-propyle, isopropyle, n-butyle, sec-butyle, isobutyle, tert-butyle, n-pentyle, sec-pentyle, néo-pentyle et n-hexyle ;
ou un radical choisi dans le groupe constitué par phényle, thiophényle, furanyle et pyridinyle, le radical pouvant à chaque fois éventuellement être substitué avec 1, 2, 3, 4 ou 5 substituants choisis indépendamment les uns des autres dans le groupe constitué par F, Cl, Br, I, -CN, -NO₂, -OH, -SH, méthyle, éthyle, n-propyle, isopropyle, n-butyle, sec-butyle, isobutyle, tert-butyle, -CF₃, -O-CF₃, -S-CF₃,-SF₅, -O-CH₃ et -O-C₂H₅ ;
R²³ représente un radical hydrogène
et
R²⁴ représente un radical choisi dans le groupe constitué par méthyle, -CH₂-CN, éthyle, -CH₂-CH₂-CN, n-propyle, isopropyle, n-butyle, sec-butyle, isobutyle, tert-butyle, n-pentyle, sec-pentyle, néo-pentyle et n-hexyle ;
chacun éventuellement sous la forme d'un de leurs stéréoisomères purs, notamment énantiomères ou diastéréomères, de leurs racémats ou sous la forme d'un mélange de stéréoisomères, notamment des énantiomères et/ou diastéréomères, en un rapport de mélange quelconque, ou chacun sous la forme des sels correspondants, ou chacun sous la forme des solvates correspondants.

13. Composés selon une ou plusieurs des revendications 1 à 12, **caractérisés en ce que**
X représente O, S ou N-C≡N ;
m vaut 1 ;
n vaut 1 ;
p1 et p2 représentent chacun indépendamment l'un de l'autre 0 ou 1, la somme de p1 et p2 étant de 0 ou 1 ;
R¹ R², R⁴, R⁵, R⁸, R⁹, R¹¹ et R¹² représentent chacun H ;
R³ représente -OR¹⁶ ; NR²³S(=O)₂R²⁴;
ou un radical choisi dans le groupe constitué par -CF₃, isopropyle, sec-butyle, isobutyle et tert-butyle ;
R⁶ et R⁷ représentent chacun un radical hydrogène
ou
R⁶ et R⁷ forment ensemble avec le pont -C-C- qui les relie un radical phénylène non substitué ;
R¹⁰ représente NR²³S(=O)₂R²⁴;
R¹⁶ représente un radical choisi dans le groupe constitué par -CF₃, -C₂F₅ et -CH₂-CF₃ ;
R²³ représente un radical hydrogène
et
R²⁴ représente un radical choisi dans le groupe constitué par méthyle, éthyle, n-propyle, isopropyle, n-butyle, sec-butyle, isobutyle, tert-butyle, n-pentyle, sec-pentyle, néo-pentyle et n-hexyle ;
chacun éventuellement sous la forme d'un de leurs stéréoisomères purs, notamment énantiomères ou diastéréomères, de leurs racémats ou sous la forme d'un mélange de stéréoisomères, notamment des énantiomères et/ou diastéréomères, en un rapport de mélange quelconque, ou chacun sous la forme des sels correspondants, ou chacun sous la forme des solvates correspondants.

14. Composés selon une ou plusieurs des revendications 1 à 13, choisis dans le groupe constitué par
[1] N-[4-[3-(4-tert-butyl-benzyl)-2-thioxo-imidazolidin-ylméthyl]-phényl]-méthanesulfonamide,
[2] N-[4-[3-(4-tert-butyl-benzyl)-2-thioxo-2,3-dihydro-benzoimidazol-1-ylméthyl]-phényl]-méthanesulfonamide,
[3] N-[4-[3-(4-tert-butyl-benzyl)-2-thioxo-tétrahydro-pyrimidin-1-ylméthyl]-phényl]-méthanesulfonamide,
[4] N-[4-[3-(4-tert-butyl-benzyl)-2-oxo-imidazolidin-1-ylméthyl]phényl]-méthanesulfonamide,
[5] N-[4-[3-(4-tert-butyl-benzyl)-2-oxo-tétrahydro-pyrimidin-1-ylméthyl]-phényl]-méthanesulfonamide,
[6] N-[4-[3-(4-tert-butyl-benzyl)-2-oxo-2,3-dihydro-benzoimidazol-1-ylméthyl]-phényl]-méthanesulfonamide,
[7] N-[4-[3-(4-trifluorométhoxybenzyl)-2-oxo-2,3-dihydrobenzimidazol-1-ylméthyl]-phényl]-méthanesulfonamide,
[8] N-[4-[3-(4-trifluorométhoxybenzyl)-2-thioxo-2,3-dihydrobenzimidazol-1-ylméthyl]-phényl]-méthanesulfonamide,
[9] N-[4-[3-(4-méthanesulfonylaminobenzyl)-2-oxo-2,3-dihydrobenzimidazol-1-ylméthyl]-phényl]-méthanesulfonamide et
[10] N-{4-[2-oxo-3-(4-trifluorométhylbenzyl)-2,3-dihydrobenzimidazol-1-ylméthyl]phényl}méthanesulfonamide ;
éventuellement à chaque fois sous la forme des sels correspondants, ou à chaque fois sous la forme des solvates correspondants.

15. Procédé de fabrication de dérivés d'urée cycliques substitués selon une ou plusieurs des revendications 1 à 14, **caractérisé en ce qu'**au moins un composé de formule générale II 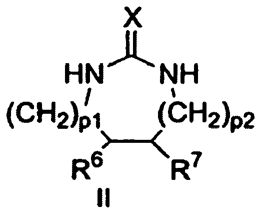 dans laquelle R⁶, R⁷, X, p1 et p2 ont la signification selon une ou plusieurs des revendications 1 à 14, est mis en réaction dans un milieu réactionnel, éventuellement en présence d'au moins une base, de préférence en présence d'au moins un sel d'hydrure de métal, de manière particulièrement préférée en présence d'hydrure de potassium et/ou de sodium,
ou en présence d'au moins un sel de carbonate de métal alcalin, de préférence en présence de carbonate de potassium et/ou de sodium, et d'au moins un iodure de métal alcalin, de préférence d'iodure de potassium et/ou de sodium, avec au moins un composé de formule générale III 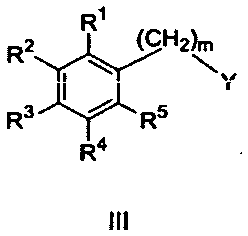 dans laquelle R¹ à R⁵ et m ont la signification selon une ou plusieurs des revendications 1 à 14 et Y représente un groupe partant, de préférence un radical halogène, de manière particulièrement préférée un atome de brome ou de chlore, pour former au moins un composé de formule générale IV 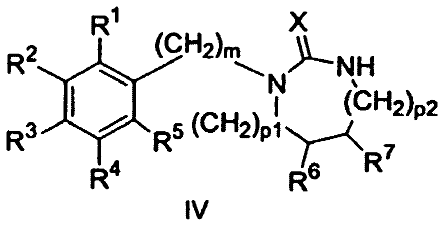 dans laquelle R¹ à R⁷, X, p1, p2 et m ont la signification mentionnée précédemment, et celui-ci est éventuellement purifié et/ou isolé,
ou au moins un composé de formule générale V 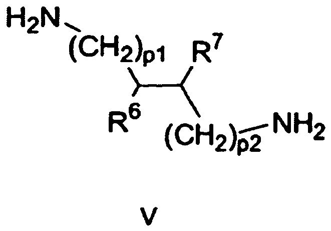 dans laquelle R⁶, R⁷, p1 et p2 ont la signification selon une ou plusieurs des revendications 1 à 14, est mis en réaction dans un milieu réactionnel, éventuellement en présence d'au moins une base, de préférence d'au moins une base choisie dans le groupe constitué par la pyridine, la triéthylamine et la diisopropyléthylamine, avec au moins un composé de formule générale III pour former au moins un composé de formule générale VI 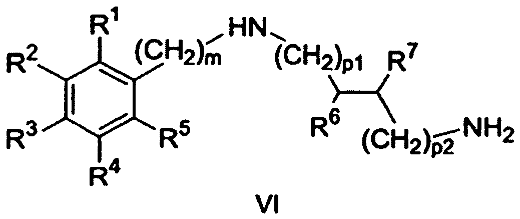 dans laquelle R¹ à R⁷, m, p1 et p2 ont la signification mentionnée précédemment, et celui-ci est éventuellement purifié et/ou isolé, et au moins un composé de formule générale VI est mis en réaction dans un milieu réactionnel, éventuellement en présence d'au moins une base, de préférence d'au moins une base choisie dans le groupe constitué par la pyridine, la triéthylamine et la diisopropyléthylamine, avec au moins un composé de formule générale Z-C(=X)-Z, dans laquelle X représente un atome d'oxygène ou de soufre et Z représente à chaque fois un groupe partant, de préférence à chaque fois un radical halogène, de manière particulièrement préférée à chaque fois un atome de chlore, pour former au moins un composé de formule générale IV, et celui-ci est éventuellement purifié et/ou isolé
ou
au moins un composé de formule générale VII 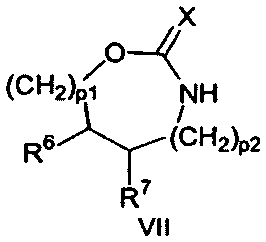 dans laquelle R⁶, R⁷, X, p1 et p2 ont la signification mentionnée précédemment, est mis en réaction dans un milieu réactionnel avec au moins un composé de formule générale VIII 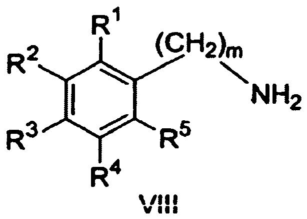 dans laquelle R¹ à R⁵ et m ont la signification mentionnée précédemment, pour former au moins un composé de formule générale IV, et celui-ci est éventuellement purifié et/ou isolé,
et au moins un composé de formule générale IV est mis en réaction dans un milieu réactionnel, en présence d'au moins une base, de préférence en présence d'au moins un sel d'hydrure de métal, de manière particulièrement préférée en présence d'hydrure de potassium et/ou de sodium,
ou en présence d'au moins un sel de carbonate de métal alcalin, de préférence en présence de carbonate de potassium et/ou de sodium, et d'au moins un iodure de métal alcalin, de préférence d'iodure de potassium et/ou de sodium, avec au moins un composé de formule générale IX 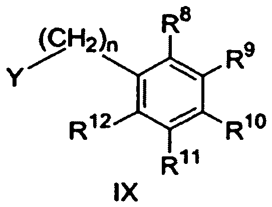 dans laquelle R⁸ à R¹² et n ont la signification selon une ou plusieurs des revendications 1 à 14, à condition qu'au moins un des substituants R⁸ à R¹² représente un groupe -N-(PG)₂, PG représentant à chaque fois un groupe protecteur ou deux groupes PG formant avec l'atome d'azote qui les relie un groupe protecteur cyclique, de préférence formant avec l'atome d'azote qui les relie un groupe phtalimide, et Y représente un groupe partant, de préférence un atome d'halogène, de manière particulièrement préférée un atome de chlore ou de brome, pour former au moins un composé de formule générale XI 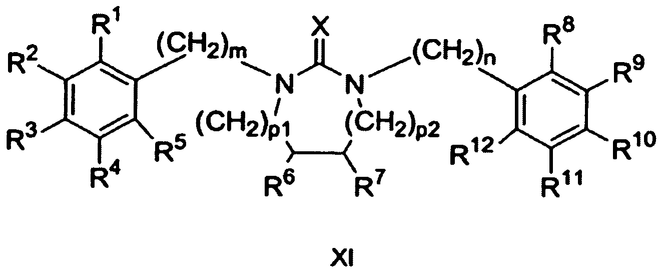 dans laquelle R¹ à R¹², X, m, n, p1 et p2 ont la signification mentionnée précédemment, à condition qu'au moins un des substituants R⁸ à R¹² représente un groupe -N-(PG)₂, PG ayant la signification mentionnée précédemment, et celui-ci est éventuellement purifié et/ou isolé, et au moins un composé de formule générale XI est mis en réaction dans un milieu réactionnel en présence d'au moins une base, de préférence en présence de diméthylamine, ou en présence d'au moins un acide ou en présence d'hydrazine et/ou de phénylhydrazine ou en présence d'au moins un borohydrure de métal alcalin, de préférence en présence de borohydrure de sodium, pour former au moins un composé de formule générale XII 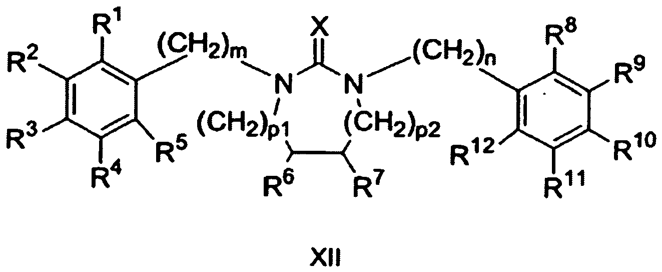 dans laquelle R¹ à R¹², X, m, n, p1 et p2 ont la signification mentionnée précédemment, à condition qu'au moins un des substituants R⁸ à R¹² représente un groupe -NH₂, et celui-ci est éventuellement purifié et/ou isolé,
ou
au moins un composé de formule générale IV est mis en réaction dans un milieu réactionnel, en présence d'au moins une base, de préférence en présence d'au moins un sel d'hydrure de métal, de manière particulièrement préférée en présence d'hydrure de potassium et/ou de sodium,
ou en présence d'au moins un sel de carbonate de métal alcalin, de préférence en présence de carbonate de potassium et/ou de sodium, et d'au moins un iodure de métal alcalin, de préférence d'iodure de potassium et/ou de sodium, avec au moins un composé de formule générale XIII 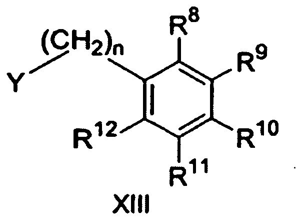 dans laquelle R⁸ à R¹² et n ont la signification selon une ou plusieurs des revendications 1 à 14, à condition qu'au moins un des substituants R⁸ à R¹² représente un groupe -NO₂, et Y représente un groupe partant, de préférence un radical halogène, de manière particulièrement préférée un atome de chlore ou de brome, pour former au moins un composé de formule générale XIV 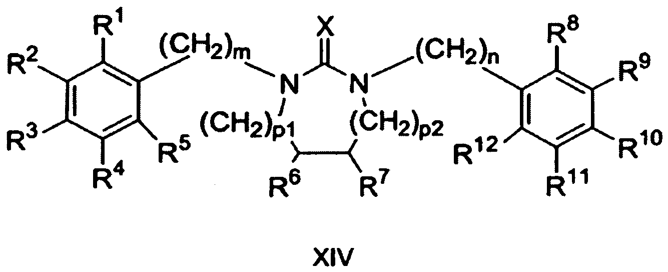 dans laquelle R¹ à R¹², X, m, n, p1 et p2 ont la signification mentionnée précédemment, à condition qu'au moins un des substituants R⁸ à R¹² représente un groupe -NO₂, et celui-ci est éventuellement purifié et/ou isolé, et au moins un composé de formule générale XIV est mis en réaction dans un milieu réactionnel en présence d'hydrogène et en présence d'un catalyseur pour former au moins un composé de formule générale XII, dans laquelle R¹ à R¹², X, m, n, p1 et p2 ont la signification mentionnée précédemment, à condition qu'au moins un des substituants R⁸ à R¹² représente un groupe -NH₂, et celui-ci est éventuellement purifié et/ou isolé,
et au moins un composé de formule générale XII est mis en réaction dans un milieu réactionnel, éventuellement en présence d'au moins une base, avec au moins un composé de formule générale R²⁴-S(=O)₂-Z, dans laquelle R²⁴ a la signification selon une ou plusieurs des revendications 1 à 14, et Z représente un groupe partant, de préférence un atome d'halogène, de manière particulièrement préférée un atome de chlore, pour former au moins un composé de formule générale Ia 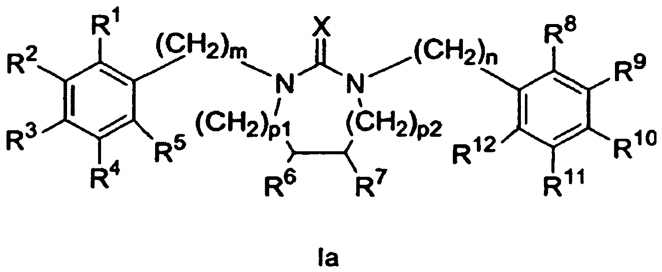 dans laquelle R¹ à R¹², X, m, n, p1 et p2 ont la signification mentionnée précédemment, à condition qu'au moins un des substituants R⁸ à R¹² représente un groupe -NH-S(=O)₂-R²⁴, R²⁴ ayant la signification mentionnée précédemment, et celui-ci est éventuellement purifié et/ou isolé,
ou au moins un composé de formule générale IV est mis en réaction dans un milieu réactionnel, en présence d'au moins une base, de préférence en présence d'au moins un sel d'hydrure de métal, de manière particulièrement préférée en présence d'hydrure de potassium et/ou de sodium,
ou en présence d'au moins un sel de carbonate de métal alcalin, de préférence en présence de carbonate de potassium et/ou de sodium, et d'au moins un iodure de métal alcalin, de préférence d'iodure de potassium et/ou de sodium, avec au moins un composé de formule générale XV 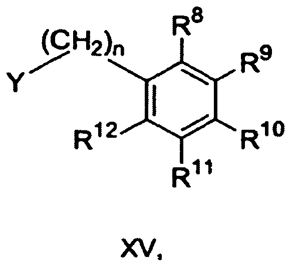 dans laquelle R⁸ à R¹² et n ont la signification selon une ou plusieurs des revendications 1 à 14, à condition qu'au moins un des substituants R⁸ à R¹² représente un groupe -NH-S(=O)₂-R²⁴ R²⁴ ayant la signification mentionnée précédemment, pour former au moins un composé de formule générale Ia, et celui-ci est éventuellement purifié et/ou isolé,
et éventuellement au moins un composé de formule générale Ia est mis en réaction dans un milieu réactionnel, éventuellement en présence d'au moins une base, avec au moins un composé de formule générale R²³-Z, dans laquelle R²³ a la signification selon une ou plusieurs des revendications 1 à 14 à l'exception de l'hydrogène et Z représente un groupe partant, de préférence un atome d'halogène, de manière particulièrement préférée un atome de chlore, pour former au moins un composé de formule générale Ib 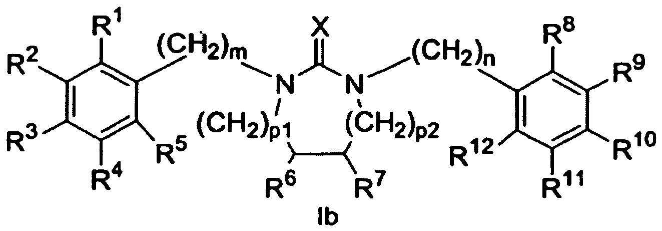 dans laquelle R¹ à R¹², X, m, n, p1 et p2 ont la signification mentionnée précédemment, à condition qu'au moins un des substituants R⁸ à R¹² représente un groupe -NR²³-S(=O)₂-R²⁹, R²³ et R²⁴ ayant la signification mentionnée précédemment, et celui-ci est éventuellement purifié et/ou isolé,
et éventuellement au moins un composé de formule générale Ib, dans laquelle R¹ à R¹², m, n, p1 et p2 ont la signification mentionnée précédemment et X représente un atome d'oxygène, à condition qu'au moins un des substituants R⁸ à R¹² représente un groupe -NR²³-*S*(=O)₂-R²⁴, R²³ et R²⁴ ayant la signification mentionnée précédemment, est mis en réaction dans un milieu réactionnel avec au moins un composé de formule générale XVI 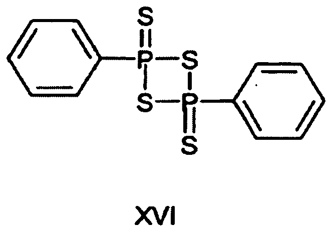 dans laquelle les radicaux phényle sont chacun substitués avec 1 ou 2 substituants choisis indépendamment l'un de l'autre dans le groupe constitué par méthoxy, phénoxy, Cl, méthyle et Br, de préférence chacun avec un radical phénoxy ou un radical méthoxy, de manière particulièrement préférée chacun avec un radical méthoxy en position para, ou avec du pentasulfure de phosphore, pour former au moins un composé de formule générale Ib, dans laquelle R¹ à R¹², m, n et p ont la signification mentionnée précédemment et X représente un atome de soufre, à condition qu'au moins un des substituants R⁸ à R¹² représente un groupe -NR²³-S(=O)₂-R²⁴, R²³ et R²⁴ ayant la signification mentionnée précédemment, et celui-ci est éventuellement purifié et/ou isolé.

16. Procédé de fabrication de dérivés d'urée cycliques substitués selon une ou plusieurs des revendications 1 à 14, **caractérisé en ce qu'**au moins un composé de formule générale II 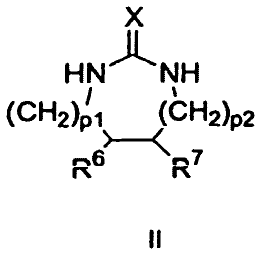 dans laquelle R⁶, R⁷, X, p1 et p2 ont la signification selon une ou plusieurs des revendications 1 à 14, est mis en réaction dans un milieu réactionnel, en présence d'au moins une base, de préférence en présence d'au moins un sel d'hydrure de métal, de manière particulièrement préférée en présence d'hydrure de potassium et/ou de sodium,
ou en présence d'au moins un sel de carbonate de métal alcalin, de préférence en présence de carbonate de potassium et/ou de sodium, et d'au moins un iodure de métal alcalin, de préférence d'iodure de potassium et/ou de sodium, avec au moins un composé de formule générale XVII 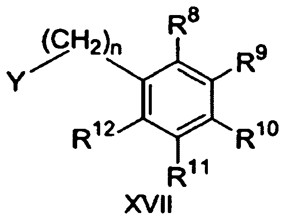 dans laquelle R⁸ à R¹² et n ont la signification selon une ou plusieurs des revendications 1 à 14, à condition qu'au moins un des substituants R⁸ à R¹² représente un groupe -N(PG)₂, PG représentant à chaque fois un groupe protecteur ou deux groupes PG formant avec l'atome d'azote qui les relie un groupe protecteur cyclique, de préférence formant avec l'atome d'azote qui les relie un groupe phtalimide, ou au moins un des substituants R⁸ à R¹² représente un groupe -NO₂, et Y représente un groupe partant, de préférence un atome d'halogène, de manière particulièrement préférée un atome de chlore ou de brome, pour former au moins un composé de formule générale XVIII 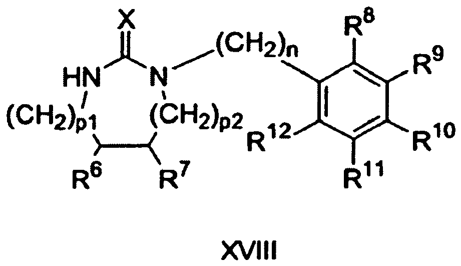 dans laquelle R⁶ à R¹², p1, p2 et n ont la signification mentionnée précédemment, à condition qu'au moins un des substituants R⁸ à R¹² représente un groupe -N(PG)₂ ou un groupe -NO₂, et celui-ci est éventuellement purifié et/ou isolé,
ou
au moins un composé de formule générale XIX 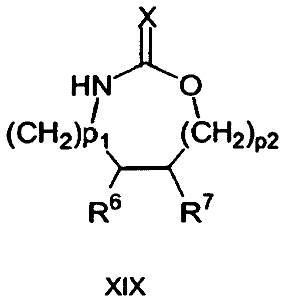 dans laquelle R⁶, R⁷, X, p1 et p2 ont la signification selon une ou plusieurs des revendications 1 à 14, est mis en réaction dans un milieu réactionnel avec au moins un composé de formule générale XX 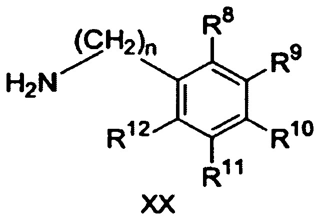 dans laquelle R⁸ à R¹² et n ont la signification selon une ou plusieurs des revendications 1 à 14, à condition qu'au moins un des substituants R⁸ à R¹² représente un groupe -N(PG)₂, PG représentant à chaque fois un groupe protecteur ou deux groupes PG formant avec l'atome d'azote qui les relie un groupe protecteur cyclique, de préférence formant avec l'atome d'azote qui les relie un groupe phtalimide, ou au moins un des substituants R⁸ à R¹² représente un groupe -NO₂, pour former au moins un composé de formule générale XVIII, et celui-ci est éventuellement purifié et/ou isolé,
ou au moins un composé de formule générale V 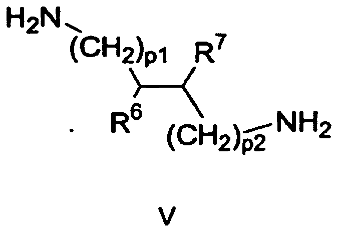 dans laquelle R⁶, R⁷, p1 et p2 ont la signification selon une ou plusieurs des revendications 1 à 14, est mis en réaction dans un milieu réactionnel, éventuellement en présence d'au moins une base, avec au moins un composé de formule générale XVII pour former au moins un composé de formule générale XXI 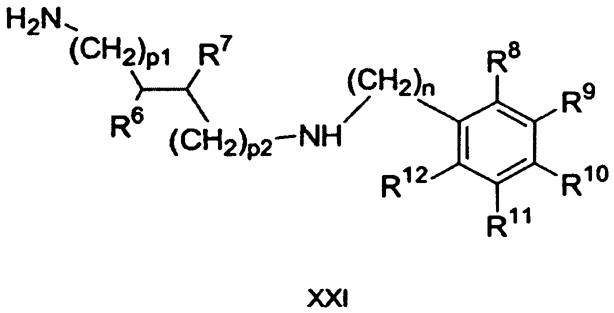 dans laquelle R⁶ à R¹², p1, p2 et n ont la signification selon une ou plusieurs des revendications 1 à 14, à condition qu'au moins un des substituants R⁸ à R¹² représente un groupe -N(PG)₂ ou un groupe -NO₂, et celui-ci est éventuellement purifié et/ou isolé, et au moins un composé de formule générale XXI est mis en réaction dans un milieu réactionnel, éventuellement en présence d'au moins une base, avec au moins un composé de formule générale Z-C(=X)-Z, dans laquelle X représente un atome d'oxygène ou de soufre et Z représente à chaque fois un groupe partant, de préférence à chaque fois un radical halogène, de manière particulièrement préférée à chaque fois un atome de chlore, pour former au moins un composé de formule générale XVIII, et celui-ci est éventuellement purifié et/ou isolé,
et au moins un composé de formule générale XVIII est mis en réaction dans un milieu réactionnel, en présence d'au moins une base, de préférence en présence d'au moins un sel d'hydrure de métal, de manière particulièrement préférée en présence d'hydrure de potassium et/ou de sodium,
ou en présence d'au moins un sel de carbonate de métal alcalin, de préférence en présence de carbonate de potassium et/ou de sodium, et d'au moins un iodure de métal alcalin, de préférence d'iodure de potassium et/ou de sodium, avec au moins un composé de formule générale III 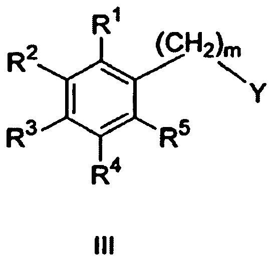 dans laquelle R¹ à R⁵ et m ont la signification selon une ou plusieurs des revendications 1 à 14, et Y représente un groupe partant, de préférence un radical halogène, de manière particulièrement préférée un atome de chlore ou de brome, pour former au moins un composé de formule générale XXII 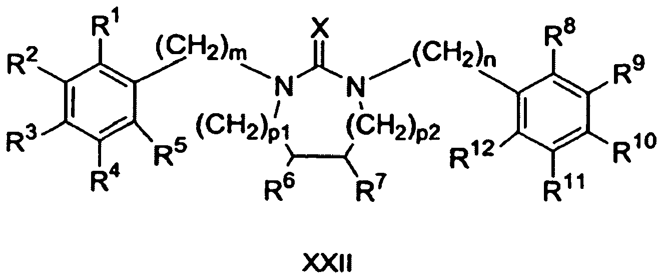 dans laquelle R¹ à R¹², X, m, n, p1 et p2 ont la signification selon une ou plusieurs des revendications 1 à 14, à condition qu'au moins un des substituants R⁸ à R¹² représente un groupe -N(PG)₂ ou un groupe -NO₂, et celui-ci est éventuellement purifié et/ou isolé,
et au moins un composé de formule générale XXII est mis en réaction dans un milieu réactionnel en présence d'au moins une base, de préférence en présence de diméthylamine, ou en présence d'au moins un acide ou en présence d'hydrazine et/ou de phénylhydrazine ou en présence d'au moins un borohydrure de métal alcalin, de préférence en présence de borohydrure de sodium, ou en présence d'hydrogène et d'un catalyseur, pour former au moins un composé de formule générale XII 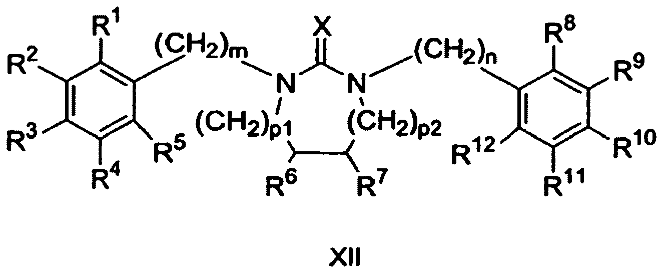 dans laquelle R¹ à R¹², X, m, n, p1 et p2 ont la signification mentionnée précédemment, à condition qu'au moins un des substituants R⁸ à R¹² représente un groupe -NH₂, et celui-ci est éventuellement purifié et/ou isolé,
et au moins un composé de formule générale XII est mis en réaction dans un milieu réactionnel, éventuellement en présence d'au moins une base, avec au moins un composé de formule générale R²⁴-S(=O)₂-Z, dans laquelle R²⁴ a la signification selon une ou plusieurs des revendications 1 à 14 et Z représente un groupe partant, de préférence un atome d'halogène, de manière particulièrement préférée un atome de chlore, pour former un composé de formule générale Ia 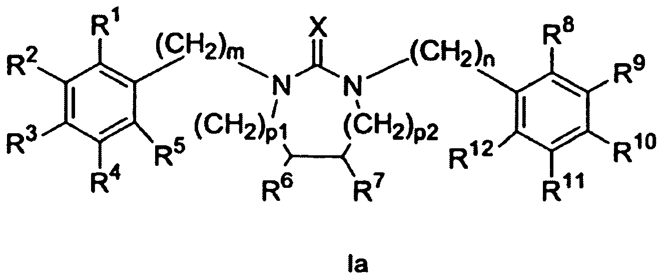 dans laquelle R¹ à R¹², X, m, n, p1 et p2 ont la signification mentionnée précédemment, à condition qu'au moins un des substituants R⁸ à R¹² représente un groupe -NH-S (=O)₂-R²⁴ R²⁴ ayant la signification mentionnée précédemment, et celui-ci est éventuellement purifié et/ou isolé,
et éventuellement au moins un composé de formule générale Ia est mis en réaction dans un milieu réactionnel, éventuellement en présence d'au moins une base, avec au moins un composé de formule générale R²³-Z, dans laquelle R²³ a la signification selon une ou plusieurs des revendications 1 à 14 à l'exception de l'hydrogène et Z représente un groupe partant, de préférence un atome d'halogène, de manière particulièrement préférée un atome de chlore, pour former au moins un composé de formule générale Ib 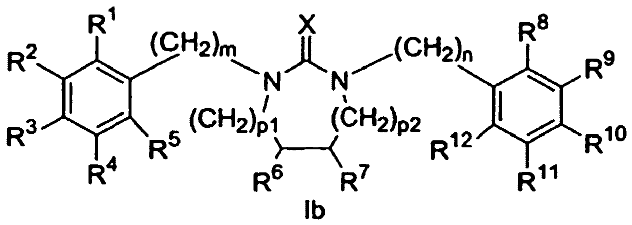 dans laquelle R¹ à R¹², X, m, n, p1 et p2 ont la signification mentionnée précédemment, à condition qu'au moins un des substituants R⁸ à R¹² représente un groupe -NR²³-S(=O)₂-R²⁴ R²³ et R²⁴ ayant la signification mentionnée précédemment, et celui-ci est éventuellement purifié et/ou isolé,
et éventuellement au moins un composé de formule générale Ib, dans laquelle R¹ à R¹², m, n, p1 et p2 ont la signification mentionnée précédemment et X représente un atome d'oxygène, à condition qu'au moins un des substituants R⁸ à R¹² représente un groupe -NR²³-S(=O)₂-R²⁴, R²³ et R²⁹ ayant la signification mentionnée précédemment, est mis en réaction dans un milieu réactionnel avec au moins un composé de formule générale XVI 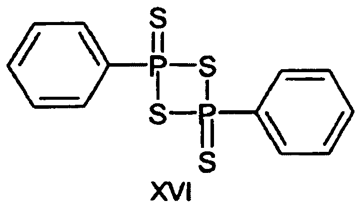 dans laquelle les radicaux phényle sont chacun substitués avec 1 ou 2 substituants choisis indépendamment l'un de l'autre dans le groupe constitué par méthoxy, phénoxy, Cl, méthyle et Br, de préférence chacun avec un radical phénoxy ou un radical méthoxy, de manière particulièrement préférée chacun avec un radical méthoxy en position para, ou avec du pentasulfure de phosphore, pour former au moins un composé de formule générale Ib, dans laquelle R¹ à R¹², m, n et p ont la signification mentionnée précédemment et X représente un atome de soufre, à condition qu'au moins un des substituants R⁸ à R¹² représente un groupe -NR²³-S(=O)₂-R²⁴, R²³ et R²⁴ ayant la signification mentionnée précédemment, et celui-ci est éventuellement purifié et/ou isolé.

17. Procédé de fabrication de dérivés d'urée cycliques substitués selon une ou plusieurs des revendications 1 à 14, **caractérisé en ce qu'**au moins un composé de formule générale II 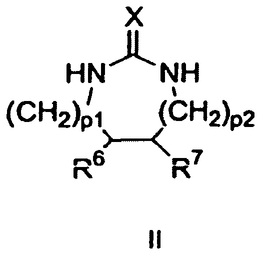 dans laquelle R⁶, R⁷, X, p1 et p2 ont la signification selon une ou plusieurs des revendications 1 à 14, est mis en réaction dans un milieu réactionnel, en présence d'au moins une base, de préférence en présence d'au moins un sel d'hydrure de métal, de manière particulièrement préférée en présence d'hydrure de potassium et/ou de sodium,
ou en présence d'au moins un sel de carbonate de métal alcalin, de préférence en présence de carbonate de potassium et/ou de sodium, et d'au moins un iodure de métal alcalin, de préférence d'iodure de potassium et/ou de sodium, avec au moins un composé de formule générale XXIII 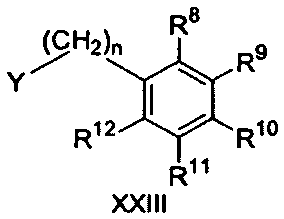 dans laquelle R⁸ à R¹² et n ont la signification selon une ou plusieurs des revendications 1 à 14, à condition qu'au moins un des substituants R⁸ à R¹² représente un groupe -NR²³-S(=O)₂-R²⁴ et Y représente un groupe partant, de préférence un atome d'halogène, de manière particulièrement préférée un atome de chlore ou de brome, pour former au moins un composé de formule générale XXIV 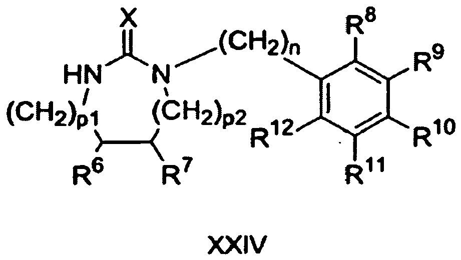 dans laquelle R⁶ à R¹², p1, p2 et n ont la signification mentionnée précédemment, à condition qu'au moins un des substituants R⁸ à R¹² représente un groupe -NR²³-S(=O)₂-R²⁴, et celui-ci est éventuellement purifié et/ou isolé,
et au moins un composé de formule générale XXIV est mis en réaction dans un milieu réactionnel en présence d'au moins une base, de préférence en présence d'au moins un sel d'hydrure de métal, de manière particulièrement préférée en présence d'hydrure de potassium et/ou de sodium,
ou en présence d'au moins un sel de carbonate de métal alcalin, de préférence en présence de carbonate de potassium et/ou de sodium, et d'au moins un iodure de métal alcalin, de préférence d'iodure de potassium et/ou de sodium, avec au moins un composé de formule générale III 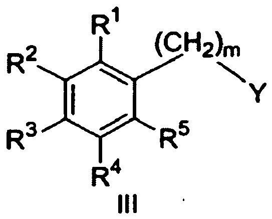 dans laquelle R¹ à R⁵ et m ont la signification selon une ou plusieurs des revendications 1 à 14 et Y représente un groupe partant, de préférence un radical halogène, de manière particulièrement préférée un atome de chlore ou de brome, pour former au moins un composé de formule générale Ib 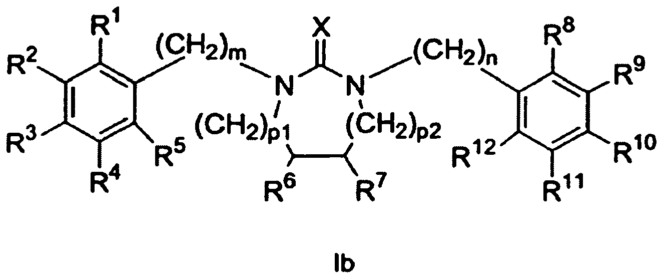 dans laquelle R¹ à R¹², X, m, n, p1 et p2 ont la signification mentionnée précédemment, à condition qu'au moins un des substituants R⁸ à R¹² représente un groupe -NR²³-S(=O)₂-R²⁴, R²³ et R²⁴ ayant la signification mentionnée précédemment, et celui-ci est éventuellement purifié et/ou isolé,
et éventuellement au moins un composé de formule générale Ib, dans laquelle R¹ à R¹², m, n, p1 et p2 ont la signification mentionnée précédemment et X représente un atome d'oxygène, à condition qu'au moins un des substituants R⁸ à R¹² représente un groupe -NR²³-S(=O)₂-R²⁴, R²³ et R²⁴ ayant la signification mentionnée précédemment, est mis en réaction dans un milieu réactionnel avec au moins un composé de formule générale XVI 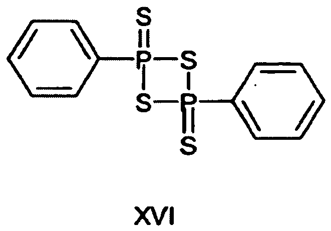 dans laquelle les radicaux phényle sont chacun substitués avec 1 ou 2 substituants choisis indépendamment l'un de l'autre dans le groupe constitué par méthoxy, phénoxy, Cl, méthyle et Br, de préférence chacun avec un radical phénoxy ou un radical méthoxy, de manière particulièrement préférée chacun avec un radical méthoxy en position para, ou avec du pentasulfure de phosphore, pour former au moins un composé de formule générale Ib, dans laquelle R¹ à R¹², m, n et p ont la signification mentionnée précédemment et X représente un atome de soufre, à condition qu'au moins un des substituants R⁸ à R¹² représente un groupe -NR²³-S(=O)₂-R²⁴, R²³ et R²⁴ ayant la signification mentionnée précédemment, et celui-ci est éventuellement purifié et/ou isolé.

18. Médicament contenant au moins un composé selon une ou plusieurs des revendications 1 à 14 et éventuellement un ou plusieurs adjuvants physiologiquement compatibles.

19. Médicament selon la revendication 18 pour le traitement et/ou la prophylaxie d'une ou de plusieurs maladies choisies dans le groupe constitué par la douleur, de préférence la douleur choisie dans le groupe constitué par la douleur aigüe, la douleur chronique, la douleur neuropathique et la douleur viscérale ; la douleur articulaire ; la migraine ; les dépressions ; les neuropathies ; les lésions nerveuses ; les maladies neurodégénératives, de préférence choisies dans le groupe constitué par la sclérose en plaques, la maladie d'Alzheimer, la maladie de Parkinson et la maladie d'Huntington ; les dysfonctionnements cognitifs, de préférence les déficiences cognitives, de manière particulièrement préférée les troubles de la mémoire ; l'épilepsie ; les maladies des voies respiratoires, de préférence choisies dans le groupe constitué par l'asthme et l'inflammation pulmonaire ; la toux ; l'incontinence urinaire ; une vessie hyperactive (overactive bladder, OAB) ; les ulcères gastriques ; le syndrome du colon irritable ; les accidents vasculaires cérébraux ; les irritations oculaires ; les irritations cutanées ; les névroses cutanées ; les maladies inflammatoires, de préférence les inflammations de l'intestin ; la diarrhée ; le prurit ; les troubles de l'alimentation, de préférence choisis dans le groupe constitué par la boulimie, la cachexie, l'anorexie et l'obésité ; la dépendance à des médicaments ; l'abus de médicaments ; les phénomènes de sevrage en cas de dépendance à des médicaments ; le développement d'une tolérance à des médicaments, de préférence aux opioïdes naturels ou synthétiques ; la dépendance à des drogues ; l'abus de drogues ; les phénomènes de sevrage en cas de dépendance à des drogues ; la dépendance à l'alcool ; l'abus d'alcool et les phénomènes de sevrage en cas de dépendance à l'alcool ; pour la diurèse ; pour l'antinatriurèse ; pour influer sur le système cardiovasculaire ; pour augmenter la vigilance ; pour augmenter la libido ; pour moduler l'activité physique ; pour l'anxiolyse ; pour l'anesthésie locale et/ou pour l'inhibition d'effets secondaires indésirables, de préférence choisis dans le groupe constitué par l'hyperthermie, l'hypertension et le rétrécissement des bronches, déclenchés par l'administration d'agonistes des récepteurs vanilloïdes 1 (récepteurs VR1/TRPV1), de préférence choisis dans le groupe constitué par la capsaïcine, la résinifératoxine, l'olvanil, l'arvanil, SDZ-249665, SDZ-249482, nuvanil et capsavanil.

20. Utilisation d'au moins un composé selon une ou plusieurs des revendications 1 à 14 pour la fabrication d'un médicament pour le traitement et/ou la prophylaxie d'une ou de plusieurs maladies choisies dans le groupe constitué par la douleur, de préférence la douleur choisie dans le groupe constitué par la douleur aigüe, la douleur chronique, la douleur neuropathique et la douleur viscérale ; la douleur articulaire ; la migraine ; les dépressions ; les neuropathies ; les lésions nerveuses ; les maladies neurodégénératives, de préférence choisies dans le groupe constitué par la sclérose en plaques, la maladie d'Alzheimer, la maladie de Parkinson et la maladie d'Huntington ; les dysfonctionnements cognitifs, de préférence les déficiences cognitives, de manière particulièrement préférée les troubles de la mémoire ; l'épilepsie ; les maladies des voies respiratoires, de préférence choisies dans le groupe constitué par l'asthme et l'inflammation pulmonaire ; la toux ; l'incontinence urinaire ; une vessie hyperactive (overactive bladder, OAB) ; les ulcères gastriques ; le syndrome du colon irritable ; les accidents vasculaires cérébraux ; les irritations oculaires ; les irritations cutanées ; les névroses cutanées ; les maladies inflammatoires, de préférence les inflammations de l'intestin ; la diarrhée ; le prurit ; les troubles de l'alimentation, de préférence choisis dans le groupe constitué par la boulimie, la cachexie, l'anorexie et l'obésité ; la dépendance à des médicaments ; l'abus de médicaments ; les phénomènes de sevrage en cas de dépendance à des médicaments ; le développement d'une tolérance à des médicaments, de préférence aux opioïdes naturels ou synthétiques ; la dépendance à des drogues ; l'abus de drogues ; les phénomènes de sevrage en cas de dépendance à des drogues ; la dépendance à l'alcool ; l'abus d'alcool et les phénomènes de sevrage en cas de dépendance à l'alcool ; pour la diurèse ; pour l'antinatriurèse ; pour influer sur le système cardiovasculaire ; pour augmenter la vigilance ; pour augmenter la libido ; pour moduler l'activité physique ; pour l'anxiolyse ; pour l'anesthésie locale et/ou pour l'inhibition d'effets secondaires indésirables, de préférence choisis dans le groupe constitué par l'hyperthermie, l'hypertension et le rétrécissement des bronches, déclenchés par l'administration d'agonistes des récepteurs vanilloïdes 1 (récepteurs VR1/TRPV1), de préférence choisis dans le groupe constitué par la capsaïcine, la résinifératoxine, l'olvanil, l'arvanil, SDZ-249665, SDZ-249482, nuvanil et capsavanil (DA-5018).

21. Utilisation selon la revendication 20, pour la fabrication d'un médicament pour le traitement et/ou la prophylaxie de la douleur choisie dans le groupe constitué par la douleur aigüe, la douleur chronique, la douleur neuropathique et la douleur viscérale.
